# EUROPEAN PATENT APPLICATION

(11) **EP 4 643 948 A2**
(43) Date of publication of application: **05.11.2025**
(21) Application number: 25198868.9
(22) Date of filing: 14.06.2022
(51) Int. Cl.: A61P 11/14

(54) **TREATMENT OF MST1 RELATED DISEASES AND DISORDERS**

(30) Priority: 16.06.2021 US 202163211364 P
(62) Divisional of application: 22825624.4
(71) Applicant: EMPIRICO INC., San Diego, CA 92122 (US)
(72) Inventor: GOTTESMAN, Omri, San Diego, CA 92122 (US); BRUSE, Shannon, San Diego, CA 92122 (US); BUSKE, Paul, San Diego, CA 92122 (US); CAJES, Brian, San Diego, CA 92122 (US); JAKUBOSKY, David, San Diego, CA 92122 (US); KLEINSTEIN, Sarah, San Diego, CA 92122 (US); LEWIS, David, San Diego, CA 92122 (US); ROZEMA, David, San Diego, CA 92122 (US); VEKICH, John, San Diego, CA 92122 (US)
(74) Representative: HGF

(57) **Abstract**

Disclosed herein are compositions comprising an oligonucleotide that inhibits the expression of MST1. The oligonucleotide includes a small interfering RNA (siRNA), as well as these compositions for use in methods of treating lung disorders

## Description

### CROSS-REFERENCE

This application claims the benefit of U.S. Provisional Application No. 63/211,364, filed June 16, 2021. which application is incorporated herein by reference

### BACKGROUND

Lung disorders are a common problem, and may affect a wide variety of persons. Improved therapeutics are needed for treating these disorders.

### SUMMARY

Disclosed herein, in some embodiments, are compositions comprising an oligonucleotide that targets *MST1.* Disclosed herein, in some embodiments, are compositions comprising an oligonucleotide that targets *MST1* and when administered to a subject in an effective amount increases a lung function measurement In some embodiments, the lung function measurement comprises a forced expiratory volume in 1 second (FEV1) measurement, a forced expiratory volume in 1 second percent predicted (FEVlpp) measurement, a forced vital capacity (FVC) measurement, a FEV 1/FVC ratio measurement, a forced expiratory volume, or a peak expiratory flow measurement In some embodiments, the lung function measurement is increased by about 10% or more, as compared to prior to administration. Disclosed herein, in some embodiments, are compositions comprising an oligonucleotide that targets *MST1* and when administered to a subject in an ef fective amount decreases a leukocyte measurement In some embodiments, the leukocyte measurement comprises a lung leukocyte measurement. In some embodiments, the leukocyte measurement comprises a circulating leukocyte measurement. In some embodiments, the leukocyte measurement comprises a neutrophil measurement, eosinophil measurement, basophil measurement, monocyte measurement, or lymphocyte measurement, or a combination thereof. In some embodiments, the leukocyte measurement is decreased by about 10% or more, as compared to prior to administration. Disclosed herein, in some embodiments, are compositions comprising an oligonucleotide that targets *MST1* and when administered to a subject in an effective amount decreases a chronic obstructive pulmonary disease (COP D) or asthma exacerbation measurement. In some embodiments, the COPD or asthma exacerbation measurement is decreased by about 10% or more, as compared to prior to administration. In some embodiments, the oligonucleotide comprises a modified internucleoside linkage. In some embodiments, the modified internucleoside linkage comprises alk ylphosphonate, phosphorothioate, methy lphosphonate, phosphorodithioate, alkylphosphotiothioate, phosphoramidate, carbamate, carbonate, phosphate triester, acetamidate, or carboxymethyl ester, or a combination thereof. In some embodiments, the modified internucleoside linkage comprises one or more phosphorothioate linkages. In some embodiments, the oligonucleotide comprises 1, 2, 3, 4,5,6.7,8, 9, 10, 11, 12, 13, 14, 15, 16. 17, 18,19, or 20 modified internucleoside linkages. In some embodiments, the oligonucleotide comprises a modified nucleoside. In some embodiments, the modified nucleoside comprises a locked nucleic acid (LNA), hexitol nucleic acid (HLA), cyclohexene nucleic acid (CeNA), 2'-methoxyethyl, 2'-O-alkyl, 2'-O-allyl, 2'-O-allyl, 2'-fluoro. or 2'-deoxy, or a combination thereof. In some embodiments, the modified nucleoside comprises a LNA. In some embodiments, the modified nucleoside comprises a 2',4' constrained ethyl nucleic acid. In some embodiments, the modified nucleoside comprises a 2'-O-methyl nucleoside, 2'-deoxyfluoro nucleoside, 2'-O-N-methylacetamido (2'-O-NMA) nucleoside, a 2'-O-dimethylaminoethoxyethyl (2'-O-DMAEOE) nucleoside, 2'-O-aminopropyl(2'-O-AP) nucleoside, or 2'-ara-F, or a combination thereof. In some embodiments, the modified nucleoside comprises one or more 2'fluoro modified nucleosides. In some embodiments, the modified nucleoside comprises a 2' O-alkyl modified nucleoside. In some embodiments, the oligonucleotide comprises 1, 2, 3, 4, 5, 6, 7, 8, 9, 10, 11, 12, 13, 14, 15, 16, 17, 18, 19,20, or 21 modified nucleosides. In some embodiments, the chgonucleotide comprises a lipid attached at a 3' or 5' terminus of the oligonucleotide. In some embodiments, the lipid comprises cholesterol, myristoyl, palmitoyl, stearoyl, lithocholoyl, docosanoyl, docosahexaenoyl, myristyl, palmityl stearyl, or α-tocopherol, or a combination thereof In some embodiments, the oligonucleotide comprises a sugar moiety attached at a 3' or 5' terminus of the oligonucleotide. In some embodiments, the sugar comprises N-acetylgalactosamine (GalNAc), N-acetylglucost unine (GlcNAc), or mannose. In some embodiments, the oligonucleotide comprises an integrin targeting ligand attached at a 3' or 5' terminus of the oligonucleotide. In some embodiments, the integrin comprises integrin alpha-v-beta-6. In some embodiments, the integrin targeting ligand comprises an arginine-glycine-aspartic acid (RGD) peptide. In some embodiments, the oligonucleotide comprises a small interfering RNA (siRNA) comprising a sense strand and an antisense strand. In some embodiments, the sense strand is 12-30 nucleosides in length. In some embodiments, the antisense strand is 12-30 nucleosides in length. Disclosed herein, in some embodiments, are compositions comprising an oligonucleotide that inhibits the expression of *MST1,* wherein the oligonucleotide comprises an siRNA comprising a sense strand and an antisense strand, each strand is independently about 12-30 nucleosides in length, and at least one of the sense strand and the anti sense strand comprises a nucleoside sequence comprising about 12-30 contiguous nucleosides of SEQ ID NO: 6185. In some embodiments, any one of the following is true with regard to the sense strand: (i) all purines comprise 2' fluoro modified purines, and all pyrimidines comprise a mixture of 2' fluoro and 2' methyl modified pyrimidines; (ii) all purines comprise 2' methyl modified purines, and all pyrimidines comprise a mixture of 2' fluoro and 2' methyl modified pyrimidines; (iii) all purines comprise 2' fluoro modified purines, and all pyrimidines comprise 2' methyl modified pyrimidines; (iv) all pyrimidines comprise 2' fluoro modified pyrimidines, and all purines comprise a mixture of 2' fluoro and 2' methyl modified purines; (v) all pyrimidines comprise 2' methyl modified pyrimidines, and all purines comprise a mixture of 2' fluoro and 2' methyl modified purines; or (vi) all pyrimidines comprise 2' fluoro modified pyrimidines, and all purines comprise 2' methyl modified purines. In some embodiments, the sense strand comprises any one of modification patterns 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 24S, or 35S. In some embodiments, any one of the following is true with regard to the antisense strand: (i) all purines comprise 2' fluoro modified purines, and all pyrimidines comprise a mixture of 2' fluoro and 2' methyl modified pyrimidines: (ii) all purines comprise 2' methyl modified purines, and all pyrimidines comprise a mixture of 2' fluoro and2' methyl modified pyrimidines: (iii) all purines comprise 2' methyl modified purines, and all pyrimidines comprise 2' fluoro modified pyrimidines; (iv) all pyrimidines comprise 2' fluoro modified pyrimidines, and all purines comprise a mixture of 2' fluoro and 2' methyl modified purines: (v) all pyrimidines comprise 2' methyl modified pyrimidines, and all purines comprise a mixture of 2' fluoro and 2' methyl modified purines: or (vi) all pyrimidines comprise 2' methyl modified pyrimidines, and all purines comprise 2' fluoro modified purines. In some embodiments. the antisense strand comprises any one of modification patterns 1AS, 2AS, 3AS, 4AS, 5AS, 6AS, 7AS, 8AS, 9AS, 10AS, 11AS, 12AS, 13AS, 14AS, 15AS, 16AS, 17AS, 19AS., 20AS, or 21 AS. In some embodiments, the sense strand comprises the nucleic acid sequence of any one of SEQ ID NOs: 1-3024 or 6358-6397. and the antisense strand comprises the nucleic acid sequence of any one of SEQ ID NOs: 3025-6048 or 6398-6417. In some embodiments, the sense strand comprises the nucleic acid sequence of any one of SEQ ID NOs 6373. 6375, 6385, 6386, or 6387, and the antisense strand comprises the nucleic acid sequence of any one of SEQ ID NOs: 6403, 6405, 6415, 6416, or 6417. In some embodiments, the oligonucleotide comprises an antisense oligonucleotide (ASO). In some embodiments, the ASO is 12-30 nucleosides in length. Disclosed herein, in some embodiments, are compositions comprising an oligonucleotide that inhibits the expression of *MST1,* wherein the oligonucleotide comprises an ASO about 12-30 nucleosides in length and a nucleoside sequence complementary to about 12-30 contiguous nucleosides of SEQ ID NO: 6185. Some embodiments include a pharmaceutically acceptable carrier. . Some embodiments include a method of treating a subject having a lung disorder, comprising administering an effective amount of the composition to the subject. In some embodiments, the lung disorder comprises COPD, acute exacerbation of COPD, emphysema, chronic bronchitis, asthma, status asthmaticus, asthma-COPD overlap syndrome (ACOS), cough, lung cancer, interstitial lung disease, or pulmonary fibrosis.

### BRIEF DESCRIPTION OF THE DRAWINGS

The novel features of the invention are set forth with particularity in the appended claims. A better understanding of the features and advantages of the present invention will be obtained by reference to the f allowing detailed description that sets forth illustrative embodiments, in which the principles of the invention are utilized, and the accompanying drawings of which:

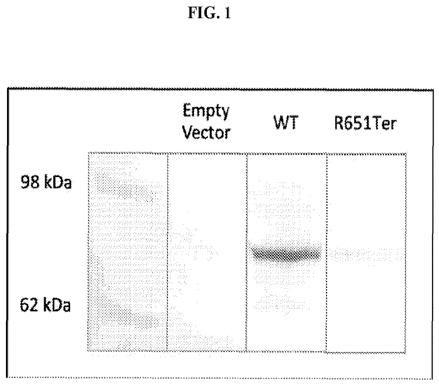

FIG. 1 shows a western blot for MST1 protein.

### DETAILED DESCRIPTION

Large-scale human genetic data can improve the success rate of pharmaceutical discovery and development. A Genome Wide Association Study (GWAS) may detect associations between genetic variants and traits in a population sample. A GWAS may enable better understanding of the biology of disease, and provide applicable treatments. A GWAS can utilize genotyping and/or sequencing data, and often involves an evaluation of millions of genetic variants that are relatively evenly distributed across the genome. The most common GWAS design is the case-control study, which involves comparing variant frequencies in cases versus controls. If a variant has a significantly different frequency in cases versus controls, that variant is said to be associated with disease. Association statistics that may be used in a GWAS are p-values, as a measure of statistical significance; odds ratios (OR), as a measure of effect size; or beta coefficients (beta), as a measure of effect size. Researchers often assume an additive genetic model and calculate an allelic odds ratio, which is the increased (or decreased) risk of disease conferred by each additional copy of an allele (compared to carrying no copies of that allele). An additional concept in design and interpretation of GWAS is that of linkage disequilibrium, which is the non-random association of alleles. The presence of linkage disequilibrium can obfuscate which variant is "causal."

Functional annotation of variants and/or wet lab experimentation can identify the causal genetic variant identified via GWAS, and in many cases may lead to the identification of disease-caus ing genes. In particular, understanding the functional effect of a causal genetic variant (for example, loss or protein function, gain of protein function, increase in gene expression, or decrease in gene expression) may allow that variant to be used as a proxy for therapeutic modulation of the target gene, or to gain insight into potential therapeutic efficacy and safety of a therapeutic that modulates that target.

Identification of such gene-disease associations has provided insights into disease biology and may be used to identify novel therapeutic targets for the pharmaceutical industry. In order to translate the therapeutic insights derived from human genetics, disease biology in patients may be exogenously 'programmed' into replicating the observation from human genetics. There are several potential options for therapeutic modalities that may be brought to bear in translating therapeutic targets identified via human genetics into novel medicines. These may include well established therapeutic modalities such as small molecules and monoclonal antibodies, maturing modalities such as oligonucleotides, and emerging modalities such as gene therapy and gene editing. The choice of therapeutic modality can depend on several factors including the location of a target (for example, intracellular, extracellular, or secreted), a relevant tissue (for example, lung or liver) and a relevant indication.

The *MST1* (*macrophage-stimulating 1*) gene is located on chromosome 3, and encodes macrophage-stimulating protein (MSP), also known as hepatocyte growth factor-like protein (HLP, HGFL, or HGFLP). MSP may also be referred to as an MST1 protein. The *MST1* gene may encode various transcripts or splice variants. MSP may include 711 amino acids and have a mass of about 80.3 kDa. MSP may be cleaved into an alpha and beta chain. MSP may be cytoplasmic. MSP may be secreted. MSP may interact with the macrophage-stimulating protein receptor, encoded by *MST1R (macrophage-stimulating 1 receptor*). *MST1* may be expressed in liver cells such as hepatocytes. Secreted MSP may bind or interact with macrophage-stimulating protein receptor in the lungs. MS P may stimulate lung ciliary motility. *MST1* may be expressed in lung cells. An example of an MSP amino acid sequence, and further description of MSP is included at uniprot.org under accession no. P26927 (last modified May 15, 2007).

Here, it is shown that genetic variants that may result in loss of function of the *MST1* gene in humans are associated with decreased risk of chronic obstructive pulmonary disease (COPD), family history of COPD, asthma, and use of inhaled beta agonist medication. Therefore, inhibition of *MST1* or MSP may serve as a therapeutic strategy for treatment of a lung disorder such as COPD, acute exacerbation of COPD, emphysema, chronic bronchitis, asthma, status asthmaticus, asthma-COPD overlap syndrome (ACOS), cough, lung cancer, interstitial lung disease, or pulmonary fibrosis.

Disclosed herein, are methods or compositions that inhibit or target *MST1* or MSP. Where inhibition or targeting of *MST1* is disclosed, it is contemplated that some embodiments may include inhibiting or targeting MSP, or vice versa. For example, by inhibiting or targeting an RNA (e.g. mRNA) encoded by the *MST1* gene using an oligonucleotide described herein, MSP may be inhibited or targeted as a result of there being less production of MSP by translation of the *MST1* RNA; or MSP may be targeted or inhibited by an oligonucleotide that binds or interacts with an *MST1* RNA and reduces production of MSP from the *MST1* RNA. Thus, targeting *MST1* may refer to binding an *MST1* RNA and reducing *MST1* RNA levels or MSP levels. The oligonucleotide may include a small interfering RNA (siRNA) or an anti sense oligonucleotide (ASO). Also provided herein are methods of treating a lung disorder by providing an oligonucleotide that targets *MST1* to a subject in need thereof.

### I. COMPOSITIONS

Disclosed herein, in some embodiments, are compositions comprising an oligonucleotide. In some embodiments, the composition comprises an oligonucleotide that targets *MST1.* In some embodiments, the composition consists of an oligonucleotide that targets *MST1.* In some embodiments, the oligonucleotide reduces*MST1* mRNA expression in the subject. In some embodiments, the oligonucleotide reduces MSP expression in the subject. The oligonucleotide may include a small interfering RNA (siRNA) described herein The oligonucleotide may include an antisense oligonucleotide (ASO) described herein. In some embodiments, a composition described herein is used in a method of treating a disorder in a subject in need thereof. Some embodiments relate to a composition comprising an oligonucleotide for use in a method of treating a disorder as described herein. Some embodiments relate to use of a composition comprising an oligonucleotide, in a method of treating a disorder as described herein.

Some embodiments include a composition comprising an oligonucleotide that targets *MST1* and when administered to a subject in an eff ective amount decreases *MST1* mRNA or MSP levels in a cell, fluid or tissue. In some embodiments, the composition comprises an oligonucleotide that targets *MST1* and when administered to a subject in an effective amount decreases *MST1* mRNA levels in a cell or tissue. In some embodiments, the cell is a liver cell or hepatocyte. In some embodiments, the cell is a lung cell, lung epithelial cell, type I or II alveolar cell, macrophage, alveolar macrophage, goblet cell, club cell, or fibroblast. In some embodiments, the tissue is liver tissue. In some embodiments, the tissue is lung tissue. In some embodiments, the *MST1* mRNA levels are decreased by about 2.5% or more, about 5% or more, or about 7.5% or more, as compared to prior to administration. In some embodiments, the *MST1* mRNA levels are decreased by about 10% or more, as compared to prior to administration. In some embodiments, the *MST1* mRNA levels are decreased by about 20% or more, about 30% or more, about 40% or more, about 50% or more, about 60% or more, about 70% or more, about 80% or more, about 90% or more, or about 100%, as compared to prior to administration. In some embodiments, the *MST1* mRNA levels are decreased by no more than about 2.5%, no more than about 5%, or no more than about 7.5%, as compared to prior to administration. In some embodiments, the *MST1* mRNA levels are decreased by no more than about 10%, as compared to prior to administration. In some embodiments, the *MST1* mRNA levels are decreased by no more than about 20%, no more than about 30%, no more than about 40%, no more than about 50%, no more than about 60%, no more than about 70%, no more than about 80%, or no more than about 90%, as compared to prior to administration In some embodiments, the *MST1* mRNA levels are decreased by 2.5%, 5%, 7.5%, 10%, 15%, 20%, 25%, 30%, 35%, 40%, 45%, 50%, 55%, 60% 65%, 70%, 75%, 80%, 85%, 90%,95%, or 100%, or by a range defined by any of the two aforementioned percentages.

In some embodiments, the composition comprises an oligonucleotide that targets *MST1* and when administered to a subject in an effective amount decreases MSP levels in a cell, fluid or tissue. In some embodiments, the cell is a liver cell or hepatocyte. In some embodiments, the cell is a lung cell, lung epithelial cell, type I or 11 alveolar cell, macrophage, alveolar macrophage, goblet cell, club cell, or fibroblast. In some embodiments, the tissue is liver tissue. In some embodiments, the tissue is lung tissue. In some embodiments, the fluid is a blood, serum, or plasma sample. In some embodiments, the fluid is a lung fluid such as a bronchoalveolar fluid. In some embodiments, the MSP levels are decreased by about 2.5% or more, about 5% or more, or about 7.5%or more, as compared to prior to administration. In some embodiments, the MSP levels are decreased by about 10% or more, as compared to prior to administration. In some embodiments, the MSP levels are decreased by about 20% or more. about 30% or more, about 40% or more, about 50% or more, about 60% or more, about 70% or more, about 80% or more, about 90% or more, or about 100%, as compared to prior to administration. In some embodiments, the MSP levels are decreased by no more than about 2.5%. no more than about 5%. or no more than about 7.5%, as compared to prior to administration. In some embodiments, the MSP levels are decreased by no more than about 10%, as compared to prior to administration. In some embodiments, the MSP levels are decreased by no more than about 20%, no more than about 30%, no more than about 40%, no more than about 50%, no more than about 60%, no more than about 70%, no more than about 80%, or no more than about 90%, as compared to prior to administration. In some embodiments, the MSP levels are decreased by 2.5%, 5%,75%, 10%, 15%, 20%, 25%, 30%, 35%,40%, 45%, 50%, 55%, 60% 65%, 70%, 75%, 80%, 85%, 90%, 95%, or 100%, or by a range defined by any of the two aforementioned percentages.

In some embodiments, the composition comprises an oligonucleotide that targets *MST1* and when administered to a subject in an effective amount diminishes an adverse phenotype of lung disorder in the subject. The lung disorder may include chronic obstructive pulmonary disease(COPD), acute exacerbation of COPD, emphysema, chronic bronchitis, asthma, status asthmaticus, asthma-COPD overlap syndrome (ACOS), cough, lung cancer, interstitial lung disease, or pulmonary fibrosis. In some embodiments, the adverse phenotype is decreased by about2.5% or more, about 5% or more, or about 7.5% or more, as compared to prior to administration. In some embodiments, the adverse phenotype is decreased by about 10% or more, as compared to prior to administration. In some embodiments, the adverse phenotype is decreased by about 20% or more, about 30% or more, about 40% or more, about 50% or more, about 60% or more, about 70% or more, about 80% or more, about 90% or more, or about 100%, as compared to prior to administration. In some embodiments, the adverse phenotype is decreased by no more than about2.5%, no more than about 5%, or no more than about 75%, as compared to prior to administration. In some embodiments, the adverse phenotype is decreased by no more than about 10%, as compared to prior to administration. In some embodiments, the adverse phenotype is decreased by no more than about 20%, no more than about 30%, no more than about 40%, no more than about 50%, no more than about 60%, no more than about 70%, no more than about 80%, or no more than about 90%, as compared to prior to administration. In some embodiments, the adverse phenotype is decreased by 2.5%, 5%, 7.5%, 10%, 15%, 20%, 25%, 30%, 35%, 40%, 45%, 50%, 55%, 60%, 65%, 70%, 75%, 80%, 85%, 90%, 95%. or 100%, or by a range defined by any of the two aforementioned percentages.

In some embodiments, the composition comprises an oligonucleotide that targets*MST1* and when administered to a subject in an effective amount enhances a protective phenotype of a lung disorder. The lung disorder may include chronic obstructive pulmonary disease (COPD), acute exacerbation of COPD, emphysema, chronic bronchitis, asthma, status asthmaticus, asthma-COPD overlap syndrome (ACOS), cough, lung cancer, interstitial lung disease, or pulmonary fibrosis. In some embodiments, the protective phenotype is increased by about 2.5% or more, about 5% or more, or about 7.5% or more, as compared to prior to administration. In some embodiments, the protective phenotype is increased by about 10% or more, as compared to prior to administration. In some embodiments, the protective phenotype is increased by about 20% or more. about 30% or more, about 40% or more, about 50% or more, about 60% or more, about 70% or more, about 80% or more, about 90% or more, or about 100% or more, as compared to prior to administration. In some embodiments, the protective phenotype is increased by about 200% or more, about 300% or more, about 400% or more, about 500% or more, about 600% or more, about 700% or more, about 800% or more, about 900% or more, or about 1000% or more, as compared to prior to administration. In some embodiments, the protective phenotype is increased by no more than about 2.5%, no more than about 5%, or no more than about 7.5%, as compared to prior to administration. In some embodiments, the protective phenotype is increased by no more than about 10%, as compared to prior to administration. In some embodiments, the protective phenotype is increased by no more than about 20%, no more than about 30%, no more than about 40%, no more than about 50%, no more than about 60%. no more than about 70%, no more than about 80%, no more than about 90%, or no more than about 100%, as compared to prior to administration. In some embodiments, the protective phenotype is increased by no more than about 200%, no more than about 300%, no more than about 400%, no more than about 500%, no more than about 600%, no more than about 700%, no more than about 800%, no more than about 900%, or no more than about 1000%, as compared to prior to administration. In some embodiments, the protective phenotype is increased by 2.5%, 5%, 7.5%, 10%, 15%, 20%, 25%, 30%, 35%, 40%, 45%, 50%, 55%, 60%, 65%, 70%, 75%, 80%, 85%, 90%, 95%, 100%, 150%, 200%, 250% 300%, 400%, 500%, 600%, 700%, 800%, 900%, or 1000%, or by a range defined by any of the two aforementioned percentages.

In some embodiments, the composition comprises an oligonucleotide that targets *MST1* and when administered to a subject in an effective amount improves (e.g. increases) a lung function measurement. The lung function measurement may include a measurement of forced expiratory volume in 1 second (FEV1), forced expiratory volume in 1 second percent predicted (FEV 1pp), forced vital capacity (FVC), FEVI/FVC ratio, forced expiratory volume, or peak expiratory flow. In some embodiments, the lung function measurement is improved by about 2.5% or more, about 5% or more, or about 7.5% or more, as compared to prior to administration. In some embodiments, the lung function measurement is improved by about 10% or more. as compared to prior to administration. In some embodiments, the lung function measurement is improved by about 20% or more, about 30% or more, about 40% or more, about 50% or more, about 60% or more, about 70% or more, about80% or more, about 90% or more, or about 100% or more, as compared to prior to administration. In some embodiments, the lung function measurement is improved by about 200% or more, about 300% or more, about 400% or more, about 500% or more, about 600% or more, about 700% or more, about 800% or more. about 900% or more, or about 1000% or more, as compared to prior to administration. In some embodiments, the lung function measurement is improved by no more than about 2.5%, no more than about 5%, or no more than about 7.5%, as compared to prior to administration. In some embodiments, the lung function measurement is improved by no more than about 10%, as compared to prior to administration. In some embodiments, the lung function measurement is improved by no more than about 20%, no more than about 30%, no more than about 40%, no more than about 50%, no more than about 60%, no more than about 70%, no more than about 80%, no more than about 90%, or no more than about 100%, as compared to prior to administration. In some embodiments, the lung function measurement is improved by no more than about 200%, no more than about 300%, no more than about 400%, no more than about 500%, no more than about 600%, no more than about 700%, no more than about 800%, no more than about 900%, or no more than about 1000%, as compared to prior to administration. In some embodiments, the lung function measurement is improved by 2.5%, 5%. 7.5%, 10%, 15%, 20%, 25%, 30%, 35%, 40%,45%, 50%, 55%, 60%, 65%, 70%, 75%, 80%, 85%, 90%, 95%, 100%, 150%, 200%, 250%, 300%, 400%, 500%, 600%, 700%, 800%, 900%, or 1000%, or by a range defined by any of the two af orementioned percentages.

A leukocyte measurement may be affected by a lung disorder. For example, some inflammatory lung disorders that may include chronic obstructive pulmonary disease (COPD) or asthma may lead to increased inflammation and circulating white blood cell counts that may be treated using a composition comprising an oligonucleotide; or lung inflammation concomitant with a lung disorder may include an increase in leukocytes in a lung tissue or lung fluid (e.g. bronchoalveolar fluid). In some embodiments, the composition comprises an oligonucleotide that targets *MST1* and when administered to a subject in an effective amount changes a leukocyte measurement in a cell, fluid or tissue of the subject. In some embodiments, the cell is a liver cell or hepatocyte. In some embodiments, the cell is a lung cell, lung epithelial cell, type 1 or II alveolar cell, macrophage, alveolar macrophage, goblet cell, club cell, or fibroblast. In some embodiments, the tissue is liver tissue. In some embodiments, the tissue is lung tissue. In some embodiments, the fluid is a blood, serum, or plasma sample. In some embodiments, the fluid is a lung fluid such as a bronchoalveolar fluid. The change may be a decrease (for example, when circulating levels of leukocytes, or levels of leukocytes in lungs are increased due to an inflammatory lung disorder). The change may be an increase in some embodiments. In some embodiments, the leuk ocyte measurement is changed by about 2.5% or more, about 5% or more, or about 7.5%or more, as compared to prior to administration. In some embodiments, the leukocyte measurement is changed by about 10% or more, as compared to prior to administration. In some embodiments, the leukocyte measurement is changed by about 20% or more, about 30% or more, about 40% or more, about 50% or more, about 60% or more, about 70% or more. or about 80% or more, as compared to prior to administration. In some embodiments, the leukocyte measurement is changed by no more than about 2.5%, no more than about 5%, or no more than about 7.5%, as compared to prior to administration. In some embodiments, the leukocyte measurement is changed by no more than about 10%. as compared to prior to administration. In some embodiments, the leukocyte measurement is changed by no more than about 20%, no more than about 30%, no more than about 40%, no more than about 50%, no more than about 60%, no more than about 70%, no more than about 80%, or no more than about 90%, as compared to prior to administration. In some embodiments, the leukocyte measurement is changed by 2.5%, 5%, 7.5%, 10%, 15%, 20%, 25%, 30%, 35%, 40%, 45%, 50%, 55%, 60%, 65%. 70%, 75%,80%, 85%, or90%, or by a range defined by any of the two aforementioned percentages.

In some embodiments, the composition comprises an oligonucleotide that targets *MST1* and when administered to a subject in an effective amount decreases chronic obstructive pulmonary disease (COPD) exacerbations in the subject. In some embodiments, the COPD exacerbations are decreased by about 2.5% or more, about 5% or more, or about 7.5% or more, as compared to prior to administration. In some embodiments, the COPD exacerbations are decreased by about 10% or more, as compared to prior to administration. In some embodiments, the COPD exacerbations are decreased by about 20% or more, about 30% or more, about 40% or more, about 50% or more, about 60% or more, about 70% or more, about 80% or more, about 90% or more, or about 100%. as compared to prior to administration. In some embodiments, the COPD exacerbations are decreased by no more than about 2.5%, no more than about 5%, or no more than about 7.5%, as compared to prior to administration. In some embodiments, the COPD exacerbations are decreased by no more than about 10%, as compared to prior to administration. In some embodiments, the COPD exacerbations are decreased by no more than about 20%, no more than about 30%. no more than about 40%, no more than about 50%, no more than about 60% no more than about 70%, no more than about 80%, or no more than about 90%, as compared to prior to administration. In some embodiments, the COPD exacerbations are decreased by 2.5%, 5%, 7.5%, 10%, 15%, 20%, 25%, 30%, 35%, 40%, 45%, 50%, 55%, 60%, 65%, 70%, 75%, 80%, 85%, 90%, 95%, or 100%, or by a range defined by any of the two aforementioned percentages.

In some embodiments, the composition comprises an oligonucleotide that targets *MST1* and when administered to a subject in an effective amount decreases asthma exacerbations in the subject. In some embodiments, the asthma exacerbations are decreased by about 2.5%or more, about 5% or more, or about 7.5% or more, as compared to prior to administration. In some embodiments, the asthma exacerbations are decreased by about 10% or more, as compared to prior to administration. In some embodiments, the asthma exacerbations are decreased by about 20% or more, about 30% or more, about 40% or more, about 50% or more, about 60% or more, about 70% or more, about 80% or more, about 90% or more, or about 100%, as compared to prior to administration. In some embodiments, the asthma exacerbations are decreased by no more than about2.5%, no more than about 5%, or no more than about 7.5%, as compared to prior to administration. In some embodiments. the asthma exacerbations are decreased by no more than about 10%, as compared to prior to administration. In some embodiments, the asthma exacerbations are decreased by no more than about 20%, no more than about 30%, no more than about 40%, no more than about 50%, no more than about 60%, no more than about 70%, no more than about 80%, or no more than about 90% as compared to prior to administration. In some embodiments, the asthma exacerbations are decreased by 2.5%, 5%. 7.5%. 10%, 15%, 20%, 25%, 30%, 35%, 40%. 45%, 50%, 55%, 60% 65%, 70%. 75%, 80%, 85%, 90%, 95%, or 100%,or by a range defined by any of the two aforementioned percentages.

### A. siRNAs

In some embodiments, the composition comprises an oligonucleotide that targets *MST1,* wherein the oligonucleotide comprises a small interfering RNA (siRNA). In some embodiments, the composition comprises an oligonucleotide that targets *MST1,* wherein the oligonucleotide comprises a small interfering RNA (siRNA) comprising a sense strand and an anti sense strand.

In some embodiments, the composition comprises an oligonucleotide that inhibits the expression of *MST1,* wherein theoligonucleotide comprises an siRNA comprising a sense strand and an antisense strand, wherein the sense strand is 12-30 nucleosides in length. In some embodiments, the composition comprises a sense strange that is 10, 11, 12, 13, 14, 15, 16, 17, 18,19, 20,21, 22, 23, 24, 25, 26, 27, 28, 29, or 30 nucleosides in length, or a range defined by any of the two aforementioned numbers. The sense strand may be 14-30 nucleosides in length. In some embodiments, the composition comprises an anti sense strand is 12-30 nucleosides in length. In some embodiments, the composition comprises an anti sense strand that is 10, 11, 12, 13, 14, 15, 16, 17, 18,19, 20, 21, 22, 23, 24, 25, 26, 27, 28, 29, or 30 nucleosides in length, or a range defined by any of the two aforementioned numbers. The anti sense strand may be 14-30 nucleosides in length.

In some embodiments, the composition comprises an oligonucleotide that inhibits the expression of *MST1,* wherein theoligonucleotide comprises an siRNA comprising a sense strand and an antisense strand, each strand is independently about 12-30 nucleosides in length, and at least one of the sense strand and the antisense strand comprises a nucleoside sequence comprising about 12-30 contiguous nucleosides of a full-length human *MST1* mRNA sequence such as SEQ ID NO: 6163. In some embodiments, at least one of the sense strand and the antisense strand comprise a nucleoside sequence comprising at least about 10, 11, 12, 13, 14, 15, 16, 17, 18,19, 20, 21, 22, 23, 24, 25, 26, 27, 28, 29, 30, or more contiguous nucleosides of one of SEQ ID NO: 6163.

In some embodiments, the composition comprises an oligonucleotide that inhibits the expression of *MST1,* wherein the oligonucleotide comprises an si RNA comprising a sense strand and an antisense strand each strand is independently about 12-30 nucleosides in length, and at least one of the sense strand and the antisense strand comprises a nucleoside sequence comprising about 12-30 contiguous nucleosides of a full-length human *MST1* mRNA sequence such as SEQ ID NO: 6185. In some embodiments, at least one of the sense strand and the antisense strand comprise a nucleoside sequence comprisingatleastabout 10, 11, 12, 13, 14, 15, 16, 17, 18,19,20,21,22,23,24,25,26,27,28,29,30,or more contiguous nucleosides of one of SEQ ID NO: 6185.

In some embodiments, the composition comprises an oligonucleotide that inhibits the expression of *MST1,* wherein the oligonucleotide comprises an siRNA comprising a sense strand and an antisense strand, wherein the sense strand and the antisense strand form a double-stranded RNA duplex. In some embodiments, the first base pair of the double-stranded RNA duplex is an AU base pair.

In some embodiments. the sense strand further comprises a 3' overhang. In some embodiments, the 3' overhang comprises 1, 2, 3, 4, 5,6,7, 8,9, or 10 nucleosides, or a range of nucleotides defined by any two of the aforementioned numbers. In some embodiments, the 3' overhang comprises 1, 2. or more nucleosides. In some embodiments, the 3' overhang comprises 2 nucleosides. In some embodiments, the sense strand further comprises a5' overhang. In some embodiments, the 5' overhang comprises 1,2. 3, 4, 5, 6, 7, 8,9, or 10 nucleosides, or a range of nucleotides defined by any two of the aforementioned numbers. In some embodiments, the 5' overhang comprises 1, 2, or more nucleosides. In some embodiments, the 5' overhang comprises 2 nucleosides.

In some embodiments, the antisense strand further comprises a 3' overhang. In some embodiments, the 3' overhang comprises 1, 2, 3, 4, 5, 6, 7, 8, 9, or 10 nucleosides, or a range of nucleotides defined by any two of the aforementioned numbers. In some embodiments, the 3' overhang comprises 1, 2, or more nucleosides. In some embodiments, the 3' overhang comprises 2 nucleosides. In some embodiments, the antisense strand further comprises a5'overhang. In some embodiments, the 5' overhang comprises 1, 2, 3, 4, 5, 6, 7, 8, 9, or 10 nucleosides, or a range of nucleotides defined by any two of the aforementioned numbers. In some embodiments, the 5' overhang comprises 1, 2, or more nucleosid es. In some embodiments, the5' overhang comprises 2 nucleosides.

In some embodiments, the composition comprises an oligonucleotide that inhibits the expression of *MST1*, wherein the oligonucleotide comprises an siRNA comprising a sense strand and an antisense strand, wherein the sRNA binds with a 19mer in a human *MST1* mRNA In some embodiments, the siRNA binds with a 12mer, a 13mer, a 14mer, a 15mer, a 16mer, a 17mer, a 18mer, a 19mer, a 20mer. a 21mer, a 22mer, a 23mer, a 24mer. or a 25mer in a human *MST1* mRNA.

In some embodiments, the composition comprises an oligonucleotide that inhibits the expression of *MST1,* wherein the oligonucleotide comprises an siRNA comprising a sense strand and an antisense strand, wherein the siRNA binds with a 17mer in a non-human primate *MST1* mRNA. In some embodiments, the siRNA binds with a 12mer, a 13mer, a 14mer, a 15mer, a 16mer, a 17mer, a 18mer, a 19mer, a 20mer, a 21 mer, a 22mer, a 23mer, a 24mer, or a 25mer in a non-human primate *MST1* mRNA.

In some embodiments, the composition comprises an oligonucleotide that inhibits the expression of *MST1,* wherein the oligonucleotide comprises an siRNA comprising a sense strand and an antisense strand, wherein the siRNA binds with a human *MST1* mRNA and less than or equal to 20 human off-targets, with no more than 2 mismatches in the antisense strand. In some embodiments, the siRNA binds with a human *MST1* mRNA and less than or equal to 10 human off-targets, with no more than 2 mismatches in the antisense strand. In some embodiments, the siRNA binds with a human *MST1* mRNA and less than or equal to 30 human off -targets, with no more than 2 mismatches in the anti sense strand. In some embodiments, the siRNA binds with a human *MST1* mRNA and less than or equal to 40 human off-targets, with no more than 2 mismatches in the antisense strand. In some embodiments, the siRNA binds with a human *MST1* mRNA and less than or equal to 50 human of f-targets, with no more than 2 mismatches in the antisense strand. In some embodiments, the siRNA binds with a human *MST1* mRNA and less than or equal to 10 human off-targets, with no more than 3 mismatches in the antisense strand. In some embodiments, the siRNA binds with a human *MST1* mRNA and less than or equal to 20 human off-targets, with no more than 3 mismatches in the antisense strand. In some embodiments, the siRNA binds with a human *MST1* mRNA and less than or equal to 30 human off-targets, with no more than 3 mismatches in the antisense strand. In some embodiments, the siRNA binds with a human *MST1* mRNA and less than or equal to 40 human off-targets, with no more than 3 mismatches in the antisense strand. In some embodiments, the siRNA binds with a human *MST1* mRNA and less than or equal to 50 human off-targets, with no more than 3 mismatches in the anti sense strand.

In some embodiments, the composition comprises an oligonucleotide that mhibits the expression of *MST1,* wherein the oligonucleotide comprises an siRNA comprising a sense strand and an antisense strand, siRNA binds with a human *MST1* mRNA target site that does not harbor an SNP, with a minor allele frequency (MAF) greater or equal to 1% (pos. 2-18). In some embodiments, the MAF is greater or equal to about 2%. about 3%, about 4%, about 5%, about 6% about 7%, about 8%, about 9%, about 10%, about 11%, about 12%, about 13%, about 14%, about 15%, about 16%, about 17%, about 18%, about 19%, or about 20%.

In some embodiments, the composition comprises an oligonucleotide that inhibits the expression of *MST1,* wherein the oligonucleotide comprises an siRNA comprising a sense strand and an antisense strand, wherein the sense strand comprises a nucleoside sequence comprising or consisting of the sequence of any one of SEQ 1D NOs: 1-3024, or a nucleic acid sequence thereof having 1 or 2 nucleoside substitutions, additions, or deletions. In some embodiments, the sense strand comprises a nucleoside sequence comprising or consisting of the sequence of any one of SEQ ID NOs: 1-3024, or a nucleic acid sequence thereof having 3 or 4 nucleoside substitutions, additions, or deletions. In some embodiments, the sense strand further comprises a 3' overhang. In some embodiments, the 3' overhang comprises 1, 2, 3, 4, 5,6, 7, 8, 9, or 10 nucleosides, or a range of nucleotides defined by any two of the aforementioned numbers. In some embodiments, the 3' overhang comprises 1, 2, or more nucleosides. In some embodiments, the 3' overhang comprises 2 nucleosides. In some embodiments, the sense strand further comprises a 5' overhang. In some embodiments. the 5' overhang comprises 1, 2, 3, 4, 5, 6, 7.8,9, or 10 nucleosides, or a range of nucleotides defined by any two of the aforementioned numbers. In some embodiments, the 5' overhang comprises 1, 2, or more nucleosides. In some embodiments, the 5' overhang comprises 2nucleosides. In some embodiments, the sense strand comprises a nucleoside sequence comprising or consisting of the sequence of any one of SEQ ID NOs: 1-3024, or a nucleic acid sequence there of having 1 or 2 nucleoside additions at the 3'end. In some embodiments, the composition comprises an oligonucleotide that inhibits the expression of *MST1,* wherein the oligonucleotide comprises an siRNA comprising a sense strand and an anti sense strand, wherein the sense strand comprises a nucleoside sequence comprising or consisting of the sequence of any one of SEQ ID NOs: 1-3024. The sense strand or antisense strand may comprise any modifications described herein. The sense strand or antisense strand may comprise a lipid moiety or a GalNAc moiety.

In some embodiments, the composition comprises an oligonucleotide that inhibits the expression of *MST1,* wherein the oligonucleotide comprises an siRNA comprising a sense strand and an antisense strand, wherein the antisense strand comprises a nucleoside sequence comprising or consisting of the sequence of any one of SEQ ID NOs: 3025-6048, or a nucleic acid sequence thereof having 1 or 2 nudeoside substitutions, additions, or deletions. In some embodiments, the anti sense strand sequence comprises a nucleoside sequence comprising or consisting of the sequence of any one of SEQ ID NOs: 3025-6048, or a nucleic acid sequence thereof having 3 or 4 nucleoside substitutions, additions, or deletions. In some embodiments, the anti sense strand further comprises a3' overhang. In some embodiments, the 3' overhang comprises 1, 2, 3, 4, 5.6, 7,8,9, or 10 nucleosides, or a range of nucleotides defined by any two of the aforementioned numbers. In some embodiments, the 3' overhang comprises 1, 2, or more nucleosides. In some embodiments, the 3' overhang comprises 2nucleosides. In some embodiments, the antisense strand further comprises a 5' overhang. In some embodiments, the 5' overhang comprises 1, 2, 3, 4, 5, 6, 7, 8, 9, or 10 nucleosides, or a range of nucleotides defined by any two of the aforementioned numbers. In some embodiments, the 5' overhang comprises 1. 2, or more nucleosides. In some embodiments, the 5' overhang comprises 2 nucleosides. In some embodiments, the antisense strand comprises a nucleoside sequence comprising or consisting of the sequence of any one of SEQ ID NOs: 3025-6048, or a nucleic acid sequence thereof having 1 or 2 nucleoside additions at the 3⁻end In some embodiments, the composition comprises an oligonucleotide that inhibits the expression of *MST1,* wherein the oligonucleotide comprises an siRNA comprising a sense strand and an antisense strand, wherein the antisense strand comprises a nucleoside sequence comprising or consisting of the sequence of any one of SEQ ID NOs: 3025-6048. The sense strand or anti sense strand may comprise any modifications described herein. The sense strand or anti sense strand may comprise a lipid moiety or a GalNAc moiety.

In some embodiments, the composition comprises an oligonucleotide that inhibits the expression of *MST1* wherein the oligonucleotide comprises an siRNA comprising a sense strand and an antisense strand, wherein the sense strand comprises a nucleoside sequence comprising or consisting of the sequence of any one of SEQ ID NOs: 6358-6397, or a nucleic acid sequence thereof having 1 or 2 nucleoside substitutions, additions, or deletions. In some embodiments, the sense strand comprises a nucleoside sequence comprising or consisting of the sequence of any one of SEQ ID NOs: 6358-6397, or a nucleic acid sequence thereof having 3 or 4 nucleoside substitutions, additions, or deletions. In some embodiments, the sense strand further comprises a 3' overhang. In some embodiments, the 3' overhang comprises 1, 2, 3, 4, 5,6, 7, 8, 9, or 10 nucleosides, or a range of nucleotides defined by any two of the aforementioned numbers. In some embodiments, the 3' overhang comprises 1, 2, or more nucleosides. In some embodiments, the 3 overhang comprises 2 nucleosides. In some embodiments, the sense strand further comprises a5' overhang. In some embodiments, the 5' overhang comprises 1, 2, 3, 4,5,6,7,8,9, or 10 nucleosides, or a range of nucleotides defined by any two of the aforementioned numbers. In some embodiments, the 5' overhang comprises 1, 2, or more nucleosides. In some embodiments, the 5' overhang comprises 2 nucleosides. In some embodiments, the sense strand comprises a nucleoside sequence comprising or consisting of the sequence of any one of SEQ ID NOs: 6358-6397, or a nucleic acid sequence thereof having 1 or 2nucleoside additions at the 3' end. In some embodiments, the composition comprises an oligonucleotide that inhibits the expression of *MST1,* wherein the oligonucleotide comprises an siRNA comprising a sense strand and an antisense strand, wherein the sense strand comprises a nucleoside sequence comprising or consisting of the sequence of any one of SEQ ID NOs: 6358-63()7. The sense strand or antisense strand may comprise any modifications described herein. The sense strand or antisense strand may comprise a lipid moiety or a GalNAc moiety.

In some embodiments, the composition comprises an oligonucleotide that inhibits the expression of *MST1*, wherein the oligonucleotide comprises an siRNA comprising a sense strand and an antisense strand, wherein the antisense strand comprises a nucleoside sequence comprising or consisting of the sequence of any one of SEQ ID NOs: 6398-6417, or a nucleic acid sequence thereof having 1 or 2 nucleoside substitutions, additions, or deletions. In some embodiments, the antisense strand sequence comprises a nucleoside sequence comprising or consisting of the sequence of any one of SEQ ID NOs: 6398-6 417, or a nucleic acid sequence thereof having 3 or 4 nucleoside substitutions, additions, or deletions. In some embodiments, the antisense strand further comprises a3' overhang. In some embodiments, the 3' overhang comprises 1,2,3, 4, 5, 6, 7, 8, 9, or 10 nucleosides. or a range of nucleotides defined by any two of the aforementioned numbers. In some embodiments, the 3'overhang comprises 1, 2, or more nucleosides. In some embodiments, the 3' overhang comprises 2 nucleosides. In some embodiments, the antisense strand further comprises a5' overhang. In some embodiments, the 5' overhang comprises 1,2, 3, 4, 5, 6, 7, 8, 9, or 10 nucleosides, or a range of nucleotides defined by any two of the aforementioned numbers. In some embodiments, the 5 overhang comprises 1,2, or more nucleosides. In some embodiments, the 5' overhang comprises 2 nucleosides. In some embodiments, the antisense strand comprises a nucleoside sequence comprising or consisting of the sequence of any one of SEQ ID NOs: 6398-6417, or a nucleic acid sequence thereof having 1 or 2 nucleoside additions at the 3' end. In some embodiments, the composition compnses an oligonucleotide that inhibits the expression of *MST1,* wherein the oligonucleotide comprises an siRNA comprising a sense strand and an antisense strand, wherein the antisense strand comprises a nucleoside sequence comprising or consisting of the sequence of any one of SEQ 1D NOs: 6398-6417, The sense strand or antisense strand may comprise any modifications described herein. The sense strand or antisense strand may comprise a lipid moiety or a GalNAc moiety.

In some embodiments, the siRNA comprises the sense strand and/or the antisense strand sequence of an siRNA in any one of **Tables 3-8,** or a nucleic acid sequence thereof having 3 or 4 nucleoside substitutions, additions, or deletions. In some embodiments, the siRNA comprises the sense strand and/or the antisense strand sequence of an siRNA in any one of **Tables 3-8,** or a nucleic acid sequence thereof having 1 or 2 nucleoside substitutions, additions, or deletions. In some embodiments, the siRNA comprises the sense strand and/or the antisense strand sequence of an siRNA in any one of **Tables 3-8.** In some embodiments, the siRNA is cross-reactive with anon-human primate (NHP) *MST1* mRNA. The sense strand or antisense strand may comprise any modifications described herein. The sense strand or anti sense strand may comprise a lipid moiety or a GalNAc moiety.

In some embodiments, the siRNA comprises the sequence of a sense strand in **Table 24B,** or a nucleic acid sequence thereof having 3 or 4 nucleoside substitutions, additions, or deletions. In some embodiments, the siRNA comprises the sequence of a sense strand in **Table 24B,** or a nucleic acid sequence thereof having 1 or 2 nucleoside substitutions, additions, or deletions. In some embodiments, the siRNA comprises the sequence of a sense strand in **Table 24B.** The sense strand may include any of these sequences may include an overhang such as a3' UU overhang. The sense strand may include any modifications described herein. The sense strand may include a lipid moiety or a GalNAc moiety. In some embodiments, the siRNA comprises the sequence of an antisense strand in **Table 24B,** or a nucleic acid sequence thereof having 3 or 4 nucleoside substitutions, additions, or deletions. In some embodiments, the siRNA comprises the sequence of an antisense strand in **Table 24B.** or a nucleic acid sequence thereof having 1 or 2 nucleoside substitutions, additions, or deletions. In some embodiments, the siRNA comprises the sequence of an antisense strand in **Table 24B.** The antisense strand may include any of these sequences may include an overhang such as a 3' UU overhang. The anti sense strand may include any modifications described herein. The antisense strand may include a lipid moiety or a GalNAc moiety.

In some embodiments, the siRNA comprises the sequence of a sense strand in **Table 24D,** or a nucleic acid sequence thereof having 3 or 4 nucleoside substitutions, additions, or deletions. In some embodiments, the siRNA comprises the sequence of a sense strand in **Table 24D,** or a nucleic acid sequence thereof having 1 or 2 nucleoside substitutions, additions, or deletions. In some embodiments, the siRNA comprises the sequence of a sense strand in **Table 24D.** The sense strand may include any of these sequences may include an overhang such as a3' UU overhang. The sense strand may include any modifications described herein. The sense strand may include a lipid moiety or a GalNAc moiety. In some embodiments, the siRNA comprises the sequence of an anti sense strand in **Table 24D,** or a nucleic acid sequence thereof having 3 or 4 nucleoside substitutions, additions, or deletions. In some embodiments, the siRN A comprises the sequence of an anti sense strand in **Table 24D,** or a nucleic acid sequence thereof having 1 or 2 nucleoside substitutions, additions, or deletions. In some embodiments, the siRNA comprises the sequence of an antisense strand in **Table 24D.** The antisense strand may include any of these sequences may include an overhang such as a3' UU overhang. The antisense strand may include any modifications described herein. The antisense strand may include a lipid moiety or a GalNAc moiety.

In some embodiments, the siRNA comprises the sense strand and/or the antisense strand sequence of an siRNA in **Table 33B** or **Table 33C,** or a nucleic acid sequence thereof having 3 or 4 nucleoside substitutions, additions, or deletions. In some embodiments, the siRNA comprises the sense strand and/or the antisense strand sequence of an siRNA in **Table 33B** or **Table 33C,** or a nucleic acid sequence thereof having 1 or 2 nucleoside substitutions, additions, or deletions. In some embodiments, the siRNA comprises the sense strand and/or the antisense strand sequence of an siRNA in **Table 33B** or **Table 33C.** In some embodiments, the siRNA is cross-reactive with a non-human primate (NHP) *MST1* mRNA. The sense strand or anti sense strand may comprise any modifications described herein. The sense strand or antisense strand may comprise a lipid moiety or a GalNAc moiety.

The siRNA may comprise the sense strand and/or the antisense strand base sequence (e.g. unmodified sequence, or base sequence with other modifications) of an siRNA in any table included herein, or may include a nucleic acid sequence thereof having 1 or 2 nucleoside substitutions, additions, or deletions; or may include a nucleic acid sequence thereof having 3 or 4 nucleoside substitutions, additions, or deletions. In some cases, the sequence does not include an overhang (e.g. UU) that is included in a table.

In some embodiments, the siRNA comprises the sense strand and/or the antisense strand sequence of an siRNA of subset A, or a nucleic acid sequence thereof having 3 or 4 nucleoside substitutions, additions, or deletions. In some embodiments, the siRNA comprises the sense strand and/or the antisense strand sequence of an siRNA of subset A, or a nucleic acid sequence thereof having 1 or 2 nucleoside substitutions, additions. or deletions. In some embodiments, the siRNA comprises the sense strand and/or the anti sense strand sequence of an siRNA of subset A. In some embodiments, the siRNA is cross-reactive with a non-human primate (NHP) *MST1* mRNA. The sense strand or antisense strand may comprise any modifications described herein. The sense strand or antisense strand may comprise a lipid moiety or a GalNAc moiety.

In some embodiments, the siRNA comprises the sense strand and/or the antisense strand sequence of an siRNA of subset B or a nucleic acid sequence thereof having 3 or 4 nucleoside substitutions. additions, or deletions. In some embodiments, thesiRNA comprises the sense strand and/or the antisense strand sequence of an siRNA of subset B, or a nucleic acid sequence thereof having 1 or 2 nucleoside substitutions, additions, or deletions. In some embodiments, the siRNA comprises the sense strand and/or the anti sense strand sequence of an siRNA of subset B. In some embodiments, the siRNA is cross-reactive with a non-human primate (NHP) *MET1* mRNA. The sense strand or antisense strand may comprise any modifications described herein. The sense strand or antisense strand may comprise a lipid motely or a GalNAc moiety.

In some embodiments, the siRNA comprises the sense strand and/or the antisense strand sequence of an siRNA of subset C, or a nucleic acid sequence thereof having 3 or 4 nucleoside substitutions, additions, or deletions. In some embodiments, the siRNA comprises the sense strand and/or the antisense strand sequence of an siRNA of subset C, or a nucleic acid sequence thereof having 1 or 2 nucleoside substitutions, additions, or deletions. In some embodiments, the siRNA comprises the sense strand and/or the antisense strand sequence of an siRNA of subset C. In some embodiments, the siRNA is cross-reactive with a non-human primate (NHP) *MST1* mRNA. The sense strand or antisense strand may comprise any modifications described herein. The sense strand or antisense strand may comprise a lipid moiety or a GalNAc moiety.

In some embodiments, the siRNA comprises the sense strand and/or the antisense strand sequence of an siRNA of subset D, or a nucleic acid sequence thereof having 3 or 4 nucleoside substitutions, additions, or deletions. In some embodiments, the siRNA comprises the sense strand and/or the anti sense strand sequence of an siRNA of subset D, or a nucleic acid sequence thereof having 1 or 2 nucleoside substitutions, additions, or deletions. In some embodiments, the siRNA comprises the sense strand and/or the anti sense strand sequence of an siRNA of subset D. In some embodiments, the siRNA is cross-reactive with a non-human primate (NHP) *MST1* mRNA. The sense strand or antisense strand may comprise any modifications described herein. The sense strand or antisense strand may comprise a lipid moiety or a GalNAc moiety.

In some embodiments, the siRNA comprises the sense strand and/or the antisense strand sequence of an siRNA of subset E, or a nucleic acid sequence thereof having 3 or 4 nucleoside substitutions, additions, or deletions. In some embodiments, the siRNA comprises the sense strand and/or the anti sense strand sequence of an siRNA of subset E, or a nucleic acid sequence thereof having 1 er 2 nucleoside substitutions, additions, or deletions. In some embodiments, the siRNA comprises the sense strand and/or the anti sense strand sequence of an siRNA of subset E. In some embodiments, the siRNA is cross-reaclive with a non-human primate (NHP) *MST1* mRNA. The sense strand or antisense strand may comprise any modifications described herein. The sense strand or antisense strand may comprise a lipid moiety or a GaINAc moiety.

In some embodiments, the siRNA comprises the sense strand and/or the antisense strand sequence of an siRNA of subset F, or a nucleic acid sequence thereof having 3 or 4 nucleoside substitutions, additions, or deletions. In some embodiments, the siRNA comprises the sense strand and/or the antisense strand sequence of an siRNA of subset F, or a nucleic acid sequence thereof having 1 or 2 nucleoside substitutions. additions, or deletions. In some embodiments, the siRNA comprises the sense strand and/or the antisense strand sequence of an siRNA of subset F. In some embodiments, the siRNA is cross-reactive with a non-human primate (NHP) *MST1* mRNA The sense strand or antisense strand may comprise any modifications described herein. The sense strand or antisense strand may comprise a lipid moiety or a GalNAc moiety.

In some embodiments, the siRNA comprises a sense strand having a sequence in accordance with any of SEQ ID NOs: 6373, 6375, 6385, 6386, or 6387. In some embodiments, the sense strand sequence comprises or consists of sequence at least 75% identical to any one of SEQ ID NOs: 6373, 6375, 6385, 6386, or 6387, at least 80% identical to any one of SEQ ID NOs: 6373, 6375, 6385, 6386, or 6387, at least 85% identical to of any one of SEQ ID NOs: 6373, 6375, 6385, 6386, or 6387, at least 90% identical to any one of SEQ ID NOs: 6373, 6375, 6385, 6386, or 6387, or at least 95% identical to any one of SEQ ID NOs: 6373, 6375, 6385, 6386, or 6387. In some embodiments, the sense strand sequence comprises or consists of the sequence of any one of SEQ ID NOs6373, 6375, 6385, 6386, or 6387, or a sense strand sequence thereof having 1, 2, 3, or 4 nucleoside substitutions, additions, or deletions. In some embodiments, the sense strand sequence comprises or consists of the sequence of any one of SEQ ID NOs: 6373, 6375, 6385, 6386, or 6387, or a sense strand sequence thereof having 1 or 2 nucleoside substitutions, additions, or deletions. In some embodiments, the sense strand sequence comprises or consists of a sequence 100% identical to SEQ ID NOs: 6373, 6375, 6385, 6386, or 6387. Thesense strand sequence may include the first 5, 6,7. 8, 9, 10, 11, 12, 13, 14, 15, 16, 17, 18, or 19 nucleotides (in the5' to 3⁻ direction) of any of the aforementioned sequences. The sense strand sequence may include the last 5, 6, 7, 8, 9,10, 11, 12, 13, 14, 15, 16, 17, 18, or 19 nucleotides (in the 5' to 3' direction) of any of the aforementioned sequences. The sense strand may comprise a modification pattern described herein. The sense strand may comprise an overhang. The sense strand may comprise a lipid moiety The sense grand may comprise a GalNAc moiety.

In some embodiments, the siRNA comprises an antisense strand having a sequence in accordance with any of SEQ ID NOs: 6403, 6405, 6415, 6416, or 6417. In some embodiments, the anti sense strand sequence comprises or consists of sequence at least 75% identical to any one of SEQ ID NOs: 6403, 6405, 6415, 6416, or 6417, at least 80% identical to any one of SEQ ID NOs: 6403, 6405, 6415, 6416, or 6417, at least 85% identical to of any one of SEQ ID NOs: 6403, 6405, 6415,6416, or 6417, at least 90% identical to any one of SEQ ID NOs: 6403, 6.405, 6415, 6416 or 6417, or at least 95% identical to any one of SEQ ID NOs: 6403, 6405, 6415, 6416, or 6417. In some embodiments, the anti sense strand sequence comprises or consists of the sequence of any one of SEQ ID NOs: 6403, 6405, 6415, 6416, or 6417, or an anti sense strand sequence thereof having 1, 2, 3, or 4 nucleoside substitutions, additions, or deletions. In some embodiments, the antisense strand sequence comprises or consists of the sequence of any one of SEQ ID NOs: 6403, 6405, 6415, 6416, or 6417, or an antisense strand sequence thereof having 1 or 2 nucleoside substitutions, additions, or deletions. In some embodiments, the antisense strand sequence comprises or consists of a sequence 100% identical to SEQ ID NOs: 6403, 6405, 6415, 6416, or 6417. The antisense strand sequence may include the first 5, 6, 7, 8, 9, 10, 11, 12, 13, 14, 15, 16, 17, 18, or 19 nucleotides (in the 5' to 3' direction) of any of the aforementioned sequences. The antisense strandsequencemayincludethelast5,6, 7,8,9, 10, 11, 12, 13, 14, 15, 16, 17, 18, or 19 nucleotides (in the 5' to 3' direction) of any of the aforementioned sequences. The antisense strand may comprise an overhang. The antisense strand may comprise a modification pattern described herein The antisense strand any comprise a lipid moiety or a GalNAc moiety.

In some embodiments, the siRNA comprises a sense strand having a sequence in accordance with SEQ ID NO: 6373. In some embodiments, the sense strand sequence comprises or consists of sequence at least 75% identical to SEQ ID NO: 6373, at least 80% identical to SEQ ID NO: 6373, at least 85% identical to SEQ ID NO: 6373, at least 90% identical to SEQ ID NO: 6373, or at least 95% identical to SEQ ID NO 6373. In some embodiments, the sense strand sequence comprises or consists of the sequence of SEQ ID NO 6373, or a sense strand sequence thereof having 1,2,3, or 4 nucleoside substitutions, additions, or deletions. In some embodiments, the sense strand sequence comprises or consists of the sequence of SEQ ID NO: 6373, or a sense strand sequence thereof having 1 or 2 nucleoside substitutions, additions, or deletions. In some embodiments, the sense strand sequence comprises or consists of a se quence 100% identical to SEQ ID NO: 6373. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6374. In some embodiments, the sense strand sequence comprises or consists of sequence at least 75% identical to SEQ ID NO: 6374, at least 80% identical to SEQ ID NO: 6374, at least 85% identical to SEQ ID NO: 6374, at least 90% identical to SEQ ID NO: 6374, or at least 95% identical to SEQ ID NO: 6374. In some embodiments, the sense strand sequence comprises or consists of the sequence of SEQ ID NO 6374, or a sense strand sequence thereof having 1, 2, 3, or 4 nucleoside substitutions, additions, or deletions. In some embodiments, the sense strand sequence comprises or consists of the sequence of SEQ ID NO: 6374, or a sense strand sequence thereof having 1 or 2 nucleoside substitutions, additions, or deletions. In some embodiments, the sense strand sequence comprises or consists of a sequence 100% identical to SEQ ID NO: 6374. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6385. In some embodiments, the sense strand sequence comprises or consists of sequence at least 75% identical to SEQ ID NO: 6385, at least 80% identical to SEQ ID NO: 6385, at least 85% identical to SEQ ID NO: 6385, at least 90% identical to SEQ ID NO: 6385, or at least 95% identical to SEQ IDNO: 6385, In some embodiments, the sense strand sequence comprises or consists of the sequence of SEQ ID NO 6385, or a sense strand sequence thereof having 1, 2, 3, or 4 nucleoside substitutions, additions, or deletions. In some embodiments, the sense strand sequence comprises or consists of the sequence of SEQ ID NO: 6385, or a sense strand sequence thereof having 1 or 2 nucleoside substitutions. additions, or deletions. In some embodiments, the sense strand sequence comprises or consists of a sequence 100% identical to SEQ ID NO: 6385. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6386. In some embodiments, the sense strand sequence comprises or consists of sequence at least 75% identical to SEQ ID NO: 6386, at least 80% identical to SEQ ID NO: 6386, at least 85% identical to SEQ ID NO: 6386, a least 90% identical to SEQ ID NO: 6386, or at least 95% identical to SEQ ID NO: 6386. In some embodiments, the sense strand sequence comprises or consists of the sequence of SEQ ID NO 6386, or a sense strand sequence thereof having 1, 2, 3, or 4 nucleoside substitutions, additions, or deletions. In some embodiments, the sense strand sequence comprises or consists of the sequence of SEQ ID NO: 6386, or a sense strand sequence thereof having 1 or 2 nucleoside substitutions, additions, or deletions. In some embodiments, the sense strand sequence comprises or consists of a sequence 100% identical to SEQ ID NO: 6386. The sense strand may comprise a modification pattern described herein. The sense strand may comprise an overhang. The sense strand may comprise a lipid moiety. The sense strand maycomprise a GalNAc moiety.

In some embodiments, the siRNA comprises a sense strand having a sequence in accordance with SEQ ID NO: 6387. In some embodiments, the sense strand sequence comprises or consists of sequence at least 75% identical to SEQ ID NO: 6387, at least 80% identical to SEQ ID NO: 6387, at least 85% identical to SEQ ID NO: 6387, at least 90% identical to SEQ ID NO: 6387, or at least 95% identical to SEQ ID NO: 6387. In some embodiments, the sense strand sequence comprises or consists of the sequence of SEQ ID NO 6387, or a sense strand sequence thereof having 1, 2, 3, or 4 nucleoside substitutions, additions. or deletions. In some embodiments, the sense strand sequence comprises or consists of the sequence of SEQ ID NO: 6387, or a sense strand sequence thereof having 1 or 2 nucleoside substitutions. additions, or deletions. In some embodiments, the sense strand sequence comprises or consists of a sequence 100% identical to SEQ ID NO: 6387. The sense strand may comprise a modification pattern described herein. The sense strand may comprise an overhang. The sense strand may comprise a lipid moiety. The sense strand may comprise a GalNAc moiety.

In some embodiments, the siRNA comprises an antisense strand having a sequence in accordance with SEQ ID NO: 6403. In some embodiments, the antisense strand sequence comprises or consists of sequence at least 75% identical to SEQ ID NO: 6403, at least 80% identical to SEQ ID NO: 6403, at least 85% identical to SEQ ID NO: 6403, at least 90% identical to SEQ ID NO: 6403, or at least 95% identical to SEQ ID NO: 6403. In some embodiments, the anti sense strand sequence comprises or consists of the sequence of SEQ IDNO 6403, or an antisense strand sequence thereof having 1, 2, 3, or4 nucleoside substitutions, additions, or deletions. In some embodiments, the antisense strand sequence comprises or consists of the sequence of SEQ ID NO: 6403, or an antisense strand sequence thereof having 1 or 2 nucleoside substitutions, additions, or deletions. In some embodiments, the antisense strand sequence comprises or consists of a sequence 100% identical to SEQ ID NO: 6403. The antisense strand may comprise a modification pattern described herein. The antisense strand may comprise an overhang. The anti sense strand may comprise a lipid moiety. The antisense strand may comprise a GalNAc moiety.

In some embodiments, the siRNA comprises an antisense strand having a sequence in accordance with SEQ ID NO: 6405. In some embodiments, the antisense strand sequence comprises or consists of sequence at least 75% identical to SEQ ID NO: 6405, at least 80% identical to SEQ ID NO 6405, at least 85% identical to SEQ ID NO: 6405, at least 90% identical to SEQ ID NO: 6405, or at least 95% identical to SEQ ID NO: 6405. In some embodiments, the antisense strand sequence comprises or consists of the sequence of SEQ ID NO 6405, or an antisense strand sequence thereof having 1, 2, 3, or 4 nucleoside substitutions, additions, or deletions. In some embodiments, the antisense strand sequence comprises or consists of the sequence of SEQ ID NO: 6405, or an anti sense strand sequence thereof having 1 or 2 nucleoside substitutions, additions, or deletions. In some embodiments, the antisense strand sequence comprises or consists of a sequence 100% identical to SEQ ID NO: 6405. The anti sense strand may comprise a modification pattern described herein. The antisense strand may comprise an overhang. The antisense strand may comprise a lipid moiety. The antisense strand may comprise a GalNAc moiety.

In some embodiments, the siRNA comprises an antisense strand having a sequence in accordance with SEQ ID NO: 6415. In some embodiments, the anti sense strand sequence comprises or consists of sequence at least 75% identical to SEQ ID NO: 6415, at least 80% identical to SEQ ID NO: 6415, at least 85% identical to SEQ ID NO: 6415, at least 90% identical to SEQ ID NO: 6415, or at least 95% identical to SEQ ID NO: 6415. In some embodiments, the antisense strand sequence comprises or consists of the sequence of SEQ ID NO 6415, or an antisense strand sequence thereof having 1, 2, 3, or 4 nucleoside substitutions, additions, or deletions. In some embodiments, the anti sense strand sequence comprises or consists of the sequence of SEQ ID NO: 6415, or an antisense strand sequence thereof having 1 or 2 nucleoside substitutions, additions, or deletions. In some embodiments, the antisense strand sequence comprises or consists of a sequence 100% identical to SEQ ID NO: 6415. The antisense strand may comprise a modification pattern described herein. The antisense strand may comprise an overhang. The antisense strand may comprise a lipid moiety. The antisense strand may comprise a GalNAc moiety.

In some embodiments, the siRNA comprises an antisense strand having a sequence in accordance with SEQ ID NO: 6416. In some embodiments, the antisense strand sequence comprises or consists of sequence at least 75% identical to SEQ ID NO: 6416, at least 80% identical to SEQ ID NO: 6416, at least 85% identical to SEQ ID NO: 6416, at least 90% identical to SEQ ID NO: 6 416, or at least 95% identical to SEQ ID NO: 6416. In some embodiments, the antisense strand sequence comprises or consists of the sequence of SEQ ID NO 6416, or an antisense strand sequence thereof having 1, 2, 3, or 4 nucleoside substitutions, additions, or deletions. In some embodiments, the antisense strand sequence comprises or consists of the sequence of SEQ ID NO: 6416, or an antisense strand sequence thereof having 1 or 2 nucleoside substitutions, additions, or deletions. In some embodiments, the anti sense strand sequence comprises or consists of a sequence 100% identical to SEQ ID NO: 6416. The antisense strand may comprise a modification pattern described herein. The antisense strand may comprise an overhang. The antisense strand may comprise a lipid moiety. The antisense strand may comprise a GalNAc moiety.

In some embodiments, the siRNA comprises an antisense strand having a sequence in accordance with SEQ ID NO: 6417. In some embodiments, the antisense strand sequence comprises or consists of sequence at least 75% identical to SEQ ID NO: 6417, at least 80% identical to SEQ ID NO: 6417, at least 85% identical to SEQ ID NO 6417, at least 90% identical to SEQ ID NO: 6417, or at least 95% identical to SEQ ID NO: 6417. In some embodiments, the antisense strand sequence comprises or consists of the sequence of SEQ ID NO 6417, or an antisensestrand sequence thereof having 1, 2, 3, or 4 nucleoside substitutions, additions, or deletions. in some embodiments, the antisense strand sequence comprises or consists of the sequence of SEQ ID NO: 6417, or an antisense strand sequence thereof having 1 or 2 nucleoside substitutions, additions, or deletions. In some embodiments, the antisense strand sequence comprises or consists of a sequence 100% identical to SEQ ID NO: 6417. The antisense strand may comprise a modification pattern described herein. The antisense strand may comprise an overhang, The antisense strand may comprise a lipid moiety. The antisense strand may comprise a GalNAc moiety.

In some embodiments, the siRNA comprises a sense strand having a sequence in accordance with SEQ ID NO: 6440. In some embodiments, the sense strand sequence comprises or consists of sequence at least 75% identical to SEQ ID NO: 6440, at least 80% identical to SEQ ID NO: 6440, at least 85% identical to SEQ ID NO: 6440, at least 90% identical to SEQ ID NO: 6440, or at least 95% identical to SEQ ID NO: 6440, In some embodiments, the sense strand sequence comprises or consists of the sequence of SEQ ID NO 6440, or a sense strand sequence thereof having 1, 2, 3, or 4 nucleoside substitutions, additions, or deletions. In some embodiments, the sense strand sequence comprises or consists of the sequence of SEQ ID NO: 6440, or a sense strand sequence thereof having 1 or 2 nucleoside substitutions, additions, or deletions. In some embodiments, the sense strand sequence comprises or consists of a sequence 100% identical to SEQ ID NO: 6440. The sense strand may comprise a modification pattern described herein. The sense strand may comprise an overhang. The sense strand may comprise a moiety such as a GalNAc moiety or a lipid moiety. In some embodiments, the siRNA comprises an antisense strand having a sequence in accordance with SEQ ID NO: 6499. In some embodiments, the antisense strand sequence comprises or consists of sequence at least 75% identical to SEQ ID NO: 6499, at least 80% identical to SEQ ID NO: 6499, at least 85% identical to SEQ ID NO: 6499, at least 90% identical to SEQ ID NO: 6499, or at least 95% identical to SEQ ID NO: 6499. In some embodiments, the antisense strand sequence comprises or consists of the sequence of SEQ ID NO 6499, or an anti sense strand sequence thereof having 1, 2, 3, or 4 nucleoside substitutions, additions, or deletions. In some embodiments, the antisense strand sequence comprises or consists of the sequence of SEQ ID NO: 6499, or an antisense strand sequence thereof having 1 or 2 nucleoside substitutions, additions, or deletions. In some embodiments, the anti sense strand sequence comprises or consists of a sequence 100% identical to SEQ ID NO: 6499. The antisense strand may comprise a modification pattern described herein. The antisense strand may comprise an overhang. The antisense strand may comprise a moiety such as a GalNAc moiety or a lipid moiety.

In some embodiments, the siRNA comprises a sense strand having a sequence in accordance with SEQ ID NO: 6446. In some embodiments, the sense strand sequence comprises or consists of sequence at least 75% identical to SEQ ID NO: 6446, at least 80% identical to SEQ ID NO: 6446, at least 85% identical to SEQ ID NO: 6446, at least 90% identical to SEQ ID NO: 6446, or at least 95% identical to SEQ ID NO: 6446. In some embodiments, the sense strand sequence comprises or consists of the sequence of SEQ ID NO 6446, or a sense strand sequence thereof having 1, 2, 3, or 4 nucleoside substitutions, additions, or deletions. In some embodiments, the sense strand sequence comprises or consists of the sequence of SEQ ID NO: 6446, or a sense strand sequence thereof having 1 or 2 nucleoside substitutions, additions, or deletions. In some embodiments, the sense strand sequence comprises or consists of a sequence 100% identical to SEQ ID NO: 6446. The sense strand may comprise a modification pattern described herein. The sense strand may comprise an overhang. The sense strand may comprise a moiety such as a GalNAc moiety or a lipid moiety. In some embodiments, the siRNA comprises an anti sense strand having a sequence in accordance with SEQ ID NO: 6505. In some embodiments, the anti sense strand sequence comprises or consists of sequence at least 75% identical to SEQ ID NO: 6505, at least 80% identical to SEQ ID NO: 6505, at least 85% identical to SEQ ID NO 6505, at least 90% identical to SEQ ID NO: 6505, or at least 95% identical to SEQ ID NO: 6505. In some embodiments, the antisense strand sequence comprises or consists of the sequence of SEQ ID NO 6505, or an antisense strand sequence thereof having 1, 2, 3, or 4 nucleoside substitutions, additions, or deletions. In some embodiments, the anti sense strand sequence comprises or consists of the sequence of SEQ ID NO: 6505, or an antisense strand sequence thereofhaving 1 or 2 nucleoside substitutions, additions, or deletions. In some embodiments, the antisense strand sequence comprises or consists of a sequence 100% identical to SEQ ID NO: 6505. The antisense strand may comprise a modification pattern described herein. The anti sense strand may comprise an overhang. The anti sense strand may comprise a moiety such as a GalNAc moiety or a lipid moiety.

In some embodiments, the siRNA comprises a sense strand having a sequence in accordance with SEQ ID NO: 6447. In some embodiments, the sense strand sequence comprises or consists of sequence at least 75% identical to SEQ ID NO: 6447, at least 80% identical to SEQ ID NO: 6447, at least 85% identical to SEQ ID NO: 6447, at least 90% identical to SEQ ID NO: 6447, or at least 95% identical to SEQ ID NO: 6447. In some embodiments, the sense strand sequence comprises or consists of the sequence of SEQ ID NO 6447, or a sense strand sequence thereofhaving 1, 2, 3, or 4 nucleoside substitutions, additions, or deletions. In some embodiments, the sense strand sequence comprises or consists of the sequence of SEQ ID NO: 6447, or a sense strand sequence thereof having 1 or 2 nucleoside substitutions. additions, or deletions. In some embodiments, the sense strand sequence comprises or consists of a sequence 100% identical to SEQ ID NO: 6447. The sense strand may comprise a modification pattern described herein. The sense strand may comprise an overhang. The sense strand may comprise a moiety such as a GalNAc moiety or a lipid moiety. In some embodiments, the siRNA comprises an antisense strand having a sequence in accordance with SEQ ID NO: 6506. In some embodiments, the anti sense strand sequence comprises or consists of sequence at least 75% identical to SEQ ID NO:6506, at least 80% identical to SEQ ID NO: 6506, at least 85% identical to SEQ ID NO: 6506, at least 90% identical to SEQ ID NO: 6506, or at least 95% identical to SEQ ID NO: 6506. In some embodiments, the antisense strand sequence comprises or consists of the sequence of SEQ ID NO 6506, or an antisense strand sequence thereof having 1, 2,3, or 4 nucleoside substitutions, additions, or deletions. In some embodiments, the anti sense strand sequence comprises or consists of the sequence of SEQ ID NO: 6506, or an antisense strand sequence thereof having 1 or 2 nucleoside substitutions, additions, or deletions. In some embodiments, the antisense strand sequence comprises or consists of a sequence 100% identical to SEQ ID NO: 6506. The antisense strand may comprise a modification pattern described herein. The antisense strand may comprise an overhang. The antisense strand may comprise a moiety such as a GalNAc moiety or a lipid moiety.

In some embodiments, the siRNA comprises a sense strand having a sequence in accordance with SEQ ID NO: 6448. In some embodiments, the sense strand sequence comprises or consists of sequence a least 75% identical to SEQ ID NO: 6448, at least 80% identical to SEQ ID NO: 6448, at least 85% identical to SEQ ID NO: 6448, at least 90% identical to SEQ ID NO: 6448, or at least 95% identical to SEQ ID NO: 6448. In some embodiments, the sense strand sequence comprises or consists of the sequence of SEQ ID NO 6448, or a sense strand sequence thereof having 1, 2, 3, or 4 nucleoside substitutions, additions, or deletions. In some embodiments, the sense strand sequence comprises or consists of the sequence of SEQ ID NO: 6448, or a sense strand sequence thereof having 1 or 2 nucleoside substitutions, additions, or deletions. In some embodiments, the sense strand sequence comprises or consists of a sequence 100% identical to SEQ ID NO: 6448. The sense strand may comprise a modification pattern described herein. The sense strand may comprise an overhang. The sense strand may comprise a moiety such as a GalNAc moiety or a lipid moiety. In some embodiments, the siRNA comprises an antisense strand having a sequence in accordance with SEQ ID NO: 6507. In some embodiments, the antisense strand sequence comprises or consists of sequence at least 75% identical to SEQ ID NO: 6507, at least 80% identical to SEQ ID NO: 6507, at least 85% identical to SEQ ID NO: 6507, at least 90% identical to SEQ ID NO: 6507, or a least 95% identical to SEQ ID NO: 6507. In some embodiments, the antisense strand sequence comprises or consists of the sequence of SEQ ID NO 6507, or an antisense strand sequence thereof having 1, 2,3, or 4 nucleoside substitutions, additions, or deletions. In some embodiments, the antisense strand sequence comprises or consists of the sequence of SEQ ID NO: 6507, or an antisense strand sequence thereof having 1 or 2 nucleoside substitutions, additions, or deletions. In some embodiments, the antisense strand sequence comprises or consists of a sequence 100% identical to SEQ ID NO: 6507. The antisense strand may comprise a modification pattern described herein. The antisense strand may comprise an overhang. The antisense strand may comprise a moiety such as a GalNAc moiety or a lipid moiety.

In some embodiments, the siRNA comprises a sense strand having a sequence in accordance with SEQ ID NO: 6461. In some embodiments, the sense strand sequence comprises or consists of sequence at least 75% identical to SEQ ID NO: 6461, at least 80% identical to SEQ ID NO: 6461, at least 85% identical to SEQ ID NO: 6461, at least 90% identical to SEQ ID NO: 6 461, or at least 95% identical to SEQ ID NO: 6461. In some embodiments, the sense strand sequence comprises or consists of the sequence of SEQ ID NO 6461, or a sense strand sequence thereof having 1, 2, 3, or 4 nucleoside substitutions, additions, or deletions. In some embodiments, the sense strand sequence comprises or consists of the sequence of SEQ ID NO: 6461, or a sense strand sequence thereof having 1 or 2 nucleoside substitutions, additions, or deletions. In some embodiments, the sense strand sequence comprises or consists of a sequence 100% identical to SEQ ID NO: 6461. The sense strand may comprise a modification pattern described herein. The sense strand may comprise an overhang. The sense strand may comprise a moiety such as a GalNAc moiety or a lipid moiety. In some embodiments, the siRNA comprises an antisense strand having a sequence in accordance with SEQ ID NO: 6520. In some embodiments, the anti sense strand sequence comprises or consists of sequence at least 75% identical to SEQ ID NO: 6520, at least 80% identical to SEQ ID NO: 6520, at least 85% identical to SEQ ID NO: 6520, a least 90% identical to SEQ ID NO: 6520, or at least 95% identical to SEQ ID NO: 6520. In some embodiments, the anti sense strand sequence comprises or consists of the sequence of SEQ ID NO 6520, or an antisense strand sequence thereof having 1, 2, 3, or 4 nucleoside substitutions, additions, or deletions. In some embodiments, the anti sense strand sequence comprises or consists of the sequence of SEQ ID NO: 6520, or an antisense strand sequence thereof having 1 or 2 nucleoside substitutions, additions, or deletions. In some embodiments, the antisense strand sequence comprises or consists of a sequence 100% identical to SEQ ID NO: 6520. The antisense strand may comprise a modification pattern described herein. The antisense strand may comprise an overhang. The antisense strand may comprise a moiety such as a GalNAc moiety or a lipid moiety.

In some embodiments, the siRNA comprises a sense strand having a sequence in accordance with SEQ ID NO: 6466. In some embodiments, the sense strand sequence comprises or consists of sequence at least 75% identical to SEQ ID NO: 6466, at least 80% identical to SEQ ID NO: 6466, at least 85% identical to SEQ ID NO: 6466, at least 90% identical to SEQ ID NO: 6466, or at least 95% identical to SEQ ID NO: 6466. In some embodiments, the sense strand sequence comprises or consists of the sequence of SEQ ID NO 6466, or a sense strand sequence thereof having 1, 2,3, or 4 nucleoside substitutions. additions, or deletions. In some embodiments, the sense strand sequence comprises or consists of the sequence of SEQ ID NO: 6466, or a sense strand sequence thereof having 1 or 2 nucleoside substitutions, additions, or deletions. In some embodiments, the sense strand sequence comprises or consists of a sequence 100% identical to SEQ ID NO: 6466. The sense strand may comprise a modification pattern described herein. The sense strand may comprise an overhang. The sense strand may comprise a moiety such as a GalNAc moiety or a lipid moiety. In some embodiments, the siRNA comprises an antisense strand having a sequence in accordance with SEQ ID NO: 6525. In some embodiments, the anti sense strand sequence comprises or consists of sequence at least 75% identical to SEQ ID NO: 6525, at least 80% identical to SEQ ID NO: 6525, at least 85% identical to SEQ ID NO: 6525, at least 90% identical to SEQ ID NO: 6525, or at least 95% identical to SEQ ID NO: 6525. In some embodiments, the antisense strand sequence comprises or consists of the sequence of SEQ ID NO 6525, or an antisense strand sequence thereof having 1, 2, 3, or 4 nucleoside substitutions, additions, or deletions. In some embodiments, the anti sense strand sequence comprises or consists of the sequence of SEQ ID NO: 6525, or an antisense strand sequence thereof having I or 2 nucleoside substitutions, additions, or deletions. In some embodiments, the antisense strand sequence comprises or consists of a sequence 100% identical to SEQ ID NO: 6525. The antisense strand may comprise a modification pattern described herein. The antisense strand may comprise an overhang. The antisense strand may comprise a moiety such as a GalNAc moiety or a lipid moiety.

In some embodiments, the siRNA comprises a sense strand having a sequence in accordance with SEQ ID NO: 6470. In some embodiments, the sense strand sequence comprises or consists of sequence at least 75% identical to SEQ ID NO: 6470, at least 80% identical to SEQ ID NO: 6470, at least 85% identical to SEQ ID NO: 6470, at least 90% identical to SEQ ID NO: 6470, or a least 95% identical to SEQ ID NO: 6470. In some embodiments, the sense strand sequence comprises or consists of the sequence of SEQ ID NO 6470, or a sense strand sequence thereof having 1,2,3, or 4 nucleoside substitutions, additions, or deletions. In some embodiments, the sense strand sequence comprises or consists of the sequence of SEQ ID NO: 6470, or a sense strand sequence thereof having 1 or 2 nucleoside substitutions, additions, or deletions. In some embodiments, the sense strand sequence comprises or consists of a sequence 100% identical to SEQ ID NO: 6470. The sense strand may comprise a modification pattern described herein. The serse strand may comprise an overhang. The sense strand may comprise a moiety such as a GalNAc moiety or a lipid moiety. In some embodiments, the siRNA comprises an antisense strand having a sequence in accordance with SEQ ID NO: 6529. In some embodiments, the antisense strand sequence comprises or consists of sequence at least 75% identical to SEQ ID NO: 6529, at least 80% identical to SEQ ID NO: 6529, at least 85% identical to SEQ IDNO: 6529, at least 90% identical to SEQ ID NO: 6529, or at least 95% identical to SEQ ID NO: 6529. In some embodiments, the antisense strand sequence comprises or consists of the sequence of SEQ ID NO 6529, or an antisense strand sequence thereof having 1, 2, 3, or 4 nucleoside substitutions, additions, or deletions. In some embodiments, the antisense strand sequence comprises or consists of the sequence of SEQ ID NO: 6529, or an antisense strand sequence thereof having 1 or 2 nucleoside substitutions, additions, or deletions. In some embodiments, the antisense strand sequence comprises or consists of a sequence 100% identical to SEQ ID NO: 6529. The anti sense strand may comprise a modification pattern described herein. The antisense strand may comprise an overhang. The antisense strand may comprise a moiety such as a GalNAc moiety or a lipid moiety.

In some embodiments, the siRNA comprises a sense strand having a sequence in accordance with SEQ ID NO: 6476. In some embodiments, the sense strand sequence comprises or consists of sequence at least 75% identical to SEQ ID NO: 6476. at least 80% identical to SEQ ID NO: 6476, at least 85% identical to SEQ ID NO: 6476, at least 90% identical to SEQ ID NO: 6476, or at least 95% identical to SEQ ID NO: 6476. In some embodiments, the sense strand sequence comprises or consists of the sequence of SEQ ID NO 6476, or a sense strand sequence thereof having 1, 2, 3, or 4 nucleoside substitutions, additions, or deletions. In some embodiments, the sense strand sequence comprises or consists of the sequence of SEQ ID NO: 6476, or a sense strand sequence thereof having 1 or 2 nucleoside substitutions. additions, or deletions. In some embodiments, the sense strand sequence comprises or consists of a sequence 100% identical to SEQ ID NO: 6476. The sense strand may comprise a modification pattern described herein. The sense strand may comprise an overhang. The sense strand may comprise a moiety such as a GalNAc moiety or a lipid moiety. In some embodiments, the siRNA comprises an antisense strand having a sequence in accordance with SEQ ID NO: 6535. In some embodiments, the antisense strand sequence comprises or consists of sequence at least 75% identical to SEQ ID NO: 6535, at least 80% identical to SEQ ID NO: 6535, at least 85% identical to SEQ ID NO: 6535, at least 90% identical to SEQ ID NO: 6535, or at least 95% identical to SEQ ID NO: 6535. In some embodiments, the antisense strand sequence comprises or consists of the sequence of SEQ ID NO 6535, or an antisense strand sequence thereof having 1, 2, 3, or 4 nucleoside substitutions, additions, or deletions. In some embodiments, the antisense strand sequence comprises or consists of the sequence of SEQ ID NO: 6535, or an anti sense strand sequence thereof having 1 or 2 nucleoside substitutions, additions, or deletions. In some embodiments, the antisense strand sequence comprises or consists of a sequence 100% identical to SEQ ID NO: 6535. The anti sense strand may comprise a modification pattern described herein. The antisense strand may comprise an overhang. The antisense strand may comprise a moiety such as a GalNAc moiety or a lipid moiety.

### B. ASOs

In some embodiments, the composition comprises an oligonucleotide that inhibits the expression of *MST1,* wherein the oligonucleotide comprises an antisense oligonucleotide (ASO). In some embodiments, the ASO is 12-30 nucleosides in length. In some embodiments, the ASO is 14-30 nucleosides in length. In some embodiments, the ASO is at least about 10, 11, 12, 13, 14, 15, 16, 17, 18,19, 20, 21, 22, 23, 24, 25, 26, 27, 28, 29, or 30 nucleosides in length, or a range defined by any of the two aforementioned numbers. In some embodiments, the ASO is 15-25 nucleosides in length. In some embodiments, the ASO is 20 nucleosides in length.

In some embodiments, the composition comprises an oligonucleotide that inhibits the expression of *MST1,* wherein theoligonucleotide comprises an ASO about 12-30 nucleosides in length and comprising a nucleoside sequence complementary to about 12-30 contiguous nucleosides of a full-length human *MST1* mRNA sequence such as SEQ ID NO: 6163; wherein (i) the oligonucleotide comprises a modification comprising a modified nucleoside and/or a modified internucleoside linkage, and/or (ii) the composition comprises a pharmaceutically acceptable carrier. In some embodiments, the ASO comprise a nucleoside sequence complementary to at least about 10, 11, 12, 13, 14, 15, 16, 17, 18,19, 20, 21, 22, 23, 24, 25,26, 27, 28, 29, 30, or more contiguous nucleosides of one of SEQ ID NO: 6163.

In some embodiments, the composition comprises an oligonucleotide that inhibits the expression of *MST1,* wherein the oligonucleotide comprises an ASO about 12-30 nucleosides in length and comprising a nucleoside sequence complementary to about 12-30 contiguous nucleosides of a fulllength human *MST1* mRNA sequence such as SEQ ID NO: 6185; wherein (i) the oligonucleotide comprises a modification comprising a modified nucleoside and/or a modified internucleoside linkage, and/or (ii) the composition comprises a pharmaceutically acceptable carrier. In some embodiments, the ASO comprise a nucleoside sequence complementary to at least about 10, 11, 12, 13, 14, 15, 16, 17, 18,19, 20, 21, 22, 23, 24, 25,26, 27, 28, 29, 30, or more contiguous nucleosides of one of SEQ ID NO: 6185.

### C. Modification patterns

In some embodiments, the composition comprises an oligonucleotide that inhibits the expression of *MST1,* wherein the oligonucleotide comprises a modification comprising a modified nucleoside and/or a modified internucleoside linkage. and/or (ii) the composition comprises a pharmaceutically acceptable carrier. In some embodiments, the oligonucleotide comprises a modification comprising a modified nucleoside and/or a modified internucleoside linkage. In some embodiments, the oligonucleotide comprises a modified internucleoside linkage. In some embodiments, the modified internucleoside linkage comprise s alkylphosphonate, phosphorothioate, methylphosphonate, phosphorodithioate, alkylphosphonothioate, phosphoramidate, carbamate, carbonate, phosphate triester, acetamidate, or carboxymethyl ester, or a combination thereof. In some embodiments, the modified internucleoside linkage comprises one or more phosphorothioate linkages. A phosphorothioate may include a nonbridging oxygen atom in a phosphate backbone of the oligonucleotide that is replaced by sulfur. Modified internucleoside linkages may be included in siRNAs or ASOs. Benefits of the modified internucleoside linkage may include decreased toxicity or improved pharmacokinetics.

In some embodiments, the composition comprises an oligonucleotide that inhibits the expression of *MST1,* wherein the oligonucleotide comprises a modified internucleoside link age, wherein the oligonucleotide comprises 1, 2, 3, 4, 5, 6, 7, 8, 9, 10, 11, 12, 13, 14, 15,16, 17, 18,19, or 20 modified internucleoside linkages, or a range of modified internucleoside linkag es defined bv any two of the aforementioned numbers. In some embodiments, the oligonucleotide comprises no more than 18 modified internucleoside linkages. In some embodiments, the oligonucleotide comprises no more than 20 modified internucleoside linkages. In some embodiments, the oligonucleotide comprises 2 or more modified internucleoside linkages, 3 or more modified internucleoside linkages, 4 or more modified intemucleoside linkages, 5 or more modified internucleoside linkages, 6 or more modified internucleoside linkages, 7 or more modified internucleoside linkages, 8 or more modified internucleoside linkages, 9 or more modified internucleoside linkages, 10 or more modified internucleoside linkages, 11 or more modified internucleoside linkages, 12 or more modified internucleoside linkages, 13 or more modified internucleoside linkages, 14 or more modified internucleoside linkages, 15 or more modified internucleoside linkages, 16 or more modified internucleoside linkages, 17 or more modified internucleoside linkages. 18 or more modified internucleoside linkages, 19 or more modified internucleoside link ages, or 20 or more modified intemucleoside linkages.

In some embodiments, the composition comprises an oligonucleotide that inhibits the expression of *MST1,* wherein theoligonucleotide comprises the modified nucleoside. In some embodiments, the modified nucleoside comprises a locked nucleic acid (LNA), hexitol nucleic acid (HLA), cyclohexene nucleic acid (CeNA), 2'-methoxyethyl, 2'-O-alkyl, 2'-O-allyl, 2'-fluoro, or 2'-deoxy, or a combination thereof. In some embodiments, the modified nucleoside comprises a LNA. In some embodiments, the modified nucleoside comprises a 2',4' constrained ethyl nucleic acid. In some embodiments, the modified nucleoside comprises HLA. In some embodiments, the modified nucleoside comprises CeNA. In some embodiments, the modified nucleoside comprises a 2'-methoxyethyl group. In some embodiments, the modified nucleoside comprises a2'-O-alkyl group. In some embodiments, the modified nucleosidecomprises a 2'-O-allyl group. In some embodiments, the modified nucleoside comprises a 2'-fluoro group. In some embodiments, the modified nucleoside comprises a 2'-deoxy group. In some embodiments, the modified nucleoside comprises a 2'-O-methyl nucleoside. 2'-deoxyfluoro nucleoside, 2'-O-N-methylacetamido (2'-O-NMA) nucleoside, a 2'-O-dimethylaminoethoxyethyl(2'-O-DMAEOE) nucleoside, 2'-O-aminopropyl (2'-O-AP) nucleoside, or 2'-ara-F, or a combination thereof. In some embodiments, the modified nucleoside comprises a 2'-O-methyl nucleoside. In some embodiments, the modified nucleoside comprises a 2'-deoxyfluoro nucleoside. In some embodiments, the modified nucleoside comprises a 2'-O-NMA nucleoside. In some embodiments, the modified nucleoside comprises a 2'-O-DMAEOE nucleoside. In some embodiments, the modified nucleoside comprises a 2'-O-aminopropyl (2'-O-AP) nucleoside. In some embodiments, the modified nucleoside comprises 2'-ara-F. In some embodiments, the modified nucleoside comprises one or more 2'fluoro modified nucleosides. In some embodiments, the modified nucleoside comprises a 2' O-alkyl modified nucleoside. Benefits of the modified nucleoside may include decreased toxicity or improved pharmacokinetics.

In some embodiments, the oligonucleotide comprises 1, 2, 3, 4, 5, 6, 7, 8, 9, 10, 11, 12, 13, 14, 15, 16, 17, 18, 19, 20, or 21 modified nucleosides, or a range of nucleosides defined by any two of the aforementioned numbers. In some embodiments, the oligonucleotide comprises no more than 19 modified nucleosides. In some embodiments, the oligonucleotide comprises no more than 21 modified nucleosides. In some embodiments, the oligonucleotide comprises 2 or more modified nucleosides, 3 or more modified nucleosides, 4 or more modified nucleosides, 5 or more modified nucleosides, 6 or more modified nucleosides, 7 or more modified nucleosides, 8 or more modified nucleosides, 9 or more modified nucleosides, 10 or more modified nucleosides, 11 or more modified nucleosides, 12 or more modified nucleosides, 13 or more modified nucleosides, 14 or more modified nucleosides, 15 or more modified nucleosides, 16 or more modified nucleosides, 17 or more modified nucleosides, 18 or more modified nucleosides, 19 or more modified nucleosides, 20 or more modified nucleosides, or 21 or more modified nucleosides.

In some embodiments, the composition comprises an oligonucleotide that inhibits the expression of *MSTI*, wherein the oligonucleotide comprises a moiety attached at a 3' or 5' terminus of the oligonucleotide. Examples of moieties include an integrin targeting ligand, a hydrophobic moiety, a sugar moiety, or a combination thereof. In some embodiments, the oligonucleotide is an siRNA having a sense strand, and the moiety is attached to a 5' end of the sense strand. In some embodiments, the oligonucleotide is an siRNA having a sense strand, and the moiety is attached to a 3' end of the sense strand. In some embodiments, the oligonucleotide is an siRNA having an anti sense strand, and the moiety is attached to a 5' end of the antisense strand. In some embodiments, the oligonucleotide is an siRNA having an antisense strand, and the moiety is attached to a 3'end of the antisense strand. In some embodiments, the oligonucleotide is an ASO, and the moiety is attached to a5'end of the ASO. In some embodiments, the oligonucleotide is an ASO, and the moiety is attached to a3'end of the ASO.

In some embodiments, the oligonucleotide is delivered to a cell or tissue by linking the oligonucleotide to a targeting group. In some embodiments, the targeting group includes a cell receptor ligand, such as an integrin targeting ligand. Integrins may include a family of transmembrane receptors that facilitate cell-extracellular matrix (ECM) adhesion. In some embodiments, the moiety includes an epithelial-specific integrin. Integrin alpha-v-beta-6 (αvβ6) bay be an example of an epithelial-specific integrin αvβ6 may be a receptor for an ECM protein or TGF-beta latency-associated peptide (LAP). Integrin αvβ6 may be expressed in a cell or tissue. Integrin αvβ36 may be expressed or upregulated in injured pulmonary epithelium.

In some embodiments, the oligonucleotide is linked to an integrin targeting ligand that has affinity for integrin αvβ6. An integrin targeting ligand may include a compound that has affinity for integrin αvβ6 or integrin alpha-v-beta-3 (αvβ3), may be useful as a ligand to facilitate targeting or delivery of the oligonucleotide to which it is attached to a particular cell type or tissue (e.g., to cells expressing integrin αvβ3 or αvβ6). In some embodiments, multiple integrin targeting ligands are linked to the oligonucleotide. In some embodiments, the oligonucleotide-inleg rin targeting ligand conjugates are selectively internalized by lung epithelial cells, either through receptor-mediated endocytosis or by other means.

Examples of targeting groups useful for delivering the oligonucleotide that include integrin targeting ligands may be based upon peptides or peptide mimics containing an arginine-glycine-aspartic acid (RGD) peptide. In some embodiments, the composition comprises an oligonucleotide that inhibits the expression of *MSTI,* wherein the oligonucleotide comprises an RGD peptide. In some embodiments, the composition comprises an RGD peptide. In some embodiments, the composition comprises an RGD peptide derivative. In some embodiments, the RGD peptide is attached a a3'terminus of the oligonucleotide. In some embodiments, the RGD peptide is attached at a 5' terminus of the oligonucleotide. In some embodiments, the composition comprises a sense strand, and the RGD peptide is attached to the sense strand (e.g. attached to a5' end of the sense strand, or attached to a 3' end of the sense strand). In some embodiments, the composition comprises an antisense strand, and the RGD peptide is attached to the antisense strand (e.g. attached to a5' end of the antisense strand, or attached to a 3' end of the antisense strand). In some embodiments, the composition comprises an RGD peptide attached a a 3' or 5' terminus of the oligonucleotide. In some embodiments, the oligonucleotide comprises an RGD peptide and a lipid attached at a 3' or 5' terminus of the oligonucleotide. The RGD peptide may be linear. The RGD peptide may be cyclic. An RGD peptide may include a D-amino acid. In some embodiments, the RGD peptide comprises Cyclo(-Arg-Gly-Asp-D-Phe-Cys) (SEQ ID NO: 6182). In some embodiments, the RGD peptide comprises Cyclo(-Arg-Gly-Asp-D-Phe-Lys) (SEQ ID NO: 6183). In some embodiments, the RGD peptide comprises Cyclo(-Arg-Gly-Asp-D-Phe-azido)(SEQ ID NO: 6184). In some embodiments, the RGD peptide comprises an aminobenzoic acid derived RGD. In some embodiments, the RGD peptide comprises Cyclo(-Arg-Gly-Asp-D-Phe-Cys) (SEQ ID NO: 6182),Cyclo(-Arg-Gly-Asp-D-Phe-Lys) (SEQ ID NO: 6183), Cyclo(-Arg-Gly-Asp-D-Phe-azido)(SEQ ID NO: 6184), an aminobenzoic acid derived RGD, or a combination thereof In some embodiments, the RGD peptide comprises multiple of such RGD peptides. For example, the RGD peptide may include 2, 3, or 4RGD peptides. Some embodiments include an arginine-glycine-glutamic acid peptide.

The oligonucleotide may include purines. Examples of purines include adenine (A) or guanine (G), or modified versions thereof. The oligonucleotide may include pyrimidines. Examples of pyrimidines include cytosine (C), thymine (T), or uracil (U), or modified versions thereof.

In some embodiments, purines of the oligonucleotide comprise 2' fluoro modified purines. In some embodiments, purines of the oligonucleotide comprise 2' methyl modified purines. In some embodiments, purines of the oligonucleotide comprise a mixture of 2' fluoro and 2' methyl modified purines. In some embodiments, all purines of the oligonucleotide comprise 2' fluoro modified purines. In some embodiments, all purines of the oligonucleotide comprise 2' methyl modified purines. In some embodiments, all purines of the oligonucleotide comprise a mixture of 2' fluoro and 2' methyl modified purines.

In some embodiments, pyrimidines of the oligonucleotide comprise 2' fluoro modified pyrimidines. In some embodiments, pyrimidines of the oligonucleotide comprise 2' methyl modified pyrimidines. In some embodiments, pyrimidines of the oligonucleotide comprise a mixture of 2' fluoro and 2' methyl modified pyrimidines. In some embodiments, all pyrimidines of the oligonucleotide comprise 2' fluoro modified pyrimidines. In some embodiments, all pyrimidines of the oligonucleotide comprise 2' methyl modified pyrimidines. In some embodiments, all pyrimidines of the oligonucleotide comprise a mixture of 2' fluoro and 2' methyl modified pyrimidines.

In some embodiments, purines of the oligonucleotide comprise 2' fluoro modified purines, and pyrimidines of the oligonucleotide comprise a mixture of 2' fluoro and 2' methyl modified pyrimidines. In some embodiments, purines of the oligonucleotide comprise 2' methyl modified purines, and pyrimidines of the oligonucleotide comprise a mixture of 2' fluoro and 2' methyl modified pyrimidines. In some embodiments, purines of the oligonucleotide comprise 2' fluoro modified purines, and pyrimidines of the oligonucleotide comprise 2' methyl modified pyrimidines. In some embodiments, purines of the oligonucleotide comprise 2' methyl modified purines, and pyrimidines of the oligonucleotide comprise 2' fluoro modified pyrimidines. In some embodiments, pyrimidines of the oligonucleotide comprise 2' fluoro modified pyrimidines, and purines of the oligonucleotide comprise a mixture of 2' fluoro and 2' methyl modified purines. In some embodiments, pyrimidines of the oligonucleotide comprise 2' methyl modified pyrimidines, and purines of the oligonucleotide comprise a mixture of 2' fluoro and 2' methyl modified purines. In some embodiments, pyrimidines of the oligonucleotide comprise 2' fluoro modified pyrimidines, and purines of the oligonucleotide comprise 2' methyl modified purines. In some embodiments, pyrimidines of the oligonucleotide comprise 2' methyl modified pyrimidines, and purines of the oligonucleotide comprise 2' fluoro modified purines.

In some embodiments, all purines of the oligonucleotide comprise 2' fluoro modified purines, and all pyrimidines of the oligonucleotide comprise a mixture of 2' fluoro and 2' methyl modi fied pyrimidines. In some embodiments, all purines of the oligonucleotide comprise 2' methyl modified purines, and all pyrimidines of the oligonucleotide comprise a mixture of 2' fluoro and 2' methyl modified pyrimidines. In some embodiments, all purines of the oligonucleotide comprise 2' fluoro modified purines, and all pyrimidines of the oligonucleotide comprise 2' methyl modified pyrimidines. In some embodiments, and all purines of the oligonucleotide comprise 2' methyl modified purines, and all pyrimidines of the oligonucleotide comprise 2' fluoro modified pyrimidines. In some embodiments, all pyrimidines of the oligonucleotide comprise 2' fluoro modified pyrimidines, and all purines of the oligonucleotide comprise a mixture of 2' fluoro and 2' methyl modified purines. In some embodiments, all pyrimidines of the oligonucleotide comprise 2' methyl modified pyrimidines, and all purines of the oligonucleotide comprise a mixture of 2' fluoro and 2' methyl modified purines. In some embodiments, all pyrimidines of the oligonucleotide comprise 2' fluoro modified pyrimidines, and all purines of the oligonucleotide comprise 2' methyl modified purines. In some embodiments, all pyrimidines of the oligonucleotide comprise 2' methyl modified pyrimidines, and all purines of the oligonucleotide comprise 2' fluoro modified purines.

In some cases, the oligonucleotide comprises a particular modification pattern. In some embodiments, position 9 counting from the 5' end of the of a strand of the oligonucleotide may have a 2'F modification. In some embodiments, when position 9 of a strand of the oligonucleotide is a pyrimidine, then all purines in a strand of the oligonucleotide have a 2'OMe modification. In some embodiments, when position 9 is the only pyrimidine between positions 5 and 11 of the sense stand, then position 9 is the only position with a 2'F modification in a strand of the oligonucleotide. In some embodiments, when position 9 and only one other base between positions 5 and 11 of a strand of the oligonucleotideare pyrimidines, then both of these pyrimidines are the only two positions with a2'F modification in a strand of the oligonucleotide. In some embodiments, when position 9 and only two other bases between positions 5 and 11 of a strand of the oligonucleotide are pyrimidines, and those two other pyrimidines are in adjacent positions so that there would be not three 2'F modifications in a row, then any combination of 2'F modifications can be made that give three 2'F modifications in total In some embodiments, when there are more than 2 pyrimidines between positions 5 and 11 of a strand of the oligonucleotide, then all combinations of pyrimidines having the 2'F modification are allowed that have three to five 2'F modifications in total, provided that a strand of the oligonucleotide does not have three 2'F modifications in a row. In some cases, a strand of the oligonucleotide of any of the siRNAs comprises a modification pattern which conforms to any or all of these a strand of the oligonucleotide rules.

In some embodiments, when position 9 of a strand of the oligonucleotide is a purine, then all purines in a strand of the oligonucleotide have a 2'OMe modification. In some embodiments, when position 9 is the only purine between positions 5 and 11 of the sense stand, then position 9 is the only position with a2'F modification in a strand of the oligonucleotide. In some embodiments, when position 9 and only one other base between positions 5 and 11 of a strand of the oligonucleotide are purines, then both of these purines are the only two positions with a 2'F modification in a strand of the oligonucleotide. In some embodiments, when position 9 and only two other bases between positions 5 and 11 of a strand of the oligonucleotide are purines, and those two other purines are in adjacent positions so that there would be not three 2'F modificaions in a row, then any combination of 2'F modifications can be made that give three 2'F modifications in total. In some embodiments, when there are more than 2 purines between positions 5 and 11 of a strand of the oligonucleotide, then all combinations of purines having the 2'F modification are allowed that have three to the 2'F modifications in total, provided that a strand of the oligonucleotide does not have three 2'F modifications in a row. In some cases, a strand of the oligonucleotide of any of the siRNAs comprises a modification pattern which conforms to any or all of these a strand of the oligonucleotide rules.

In some cases, position 9 of a strand of the oligonucleotide can be a2'deoxy. In these cases, 2'F and 2' OMe modifications may occur at the other positions of a strand of the oligonucleotide. In some cases, a strand of the oligonucleotide of any of the siRNAs comprises a modification pattern which conforms to these a strand of the oligonucleotide rules.

In some embodiments, position nine of the sense strand comprises a 2' fluoro-modified pyrimidine. In some embodiments, all purines of the sense strand comprise 2'-O-methyl modified purines. In some embodiments, 1, 2, 3, 4, or 5 pyrimidines between positions 5 and 11 comprise a 2'flouro-modified pyrimidine, provided there are not three 2' fluoro-modified pyrimidines in a row. In some embodiments, the odd-numbered positions of the antisense strand comprise 2' -O-methyl modified nucleotides. In some embodiments, the even-numbered positions of the antisense strand comprise 2'flouro-modified nucleotides and unmodified deoxyribonucleotide. In some embodiments, the even-numbered positions of the antisense strand comprise 2'flouro-modified nucleotides, 2'-O-methyl modified nucleotides and unmodified deoxyribonucleotide. In some embodiments, position nine of the sense strand comprises a 2' fluoro-modified pyrimidine; all purines of the sense strand comprises 2'-O-methyl modified purines; 1, 2, 3, 4, or 5 pyrimidines between positions 5 and 11 comprise a 2'flouro-modified pyrimidine, provided there are not three 2' fluoro-modified pyrimidines in a row; the odd-numbered positions of the antisense strand comprise 2'-O-methyl modified nucleotides; and the even-numbered positions of the antisense strand comprise 2'flouro-modified nucleotides and unmodified deoxyribonucleotides.

In some embodiments, position nine of the sense strand comprises a2' fluoro-modified purine. In some embodiments, all pyrimidines of the sense strand comprise 2'-O-methyl modified purines. In some embodiments, 1, 2, 3, 4, or 5 purines between positions 5 and 11 comprise a 2'flouro-modified purine, provided there are not three 2' fluoro-modified purine in a row. In some embodiments, the odd-numbered positions of the antisense strand comprise 2'-O-methyl modified nucleotides. In some embodiments, the even-numbered positions of the antisense strand comprise 2 flouro-modified nucleotides and unmodified deoxyribonucleotide. In some embodiments, the even-numbered positions of the antisense strand comprise 2'flouro-modified nucleotides, 2'-O-methyl modified nucleotides and unmodified deoxyribonucleotide. In some embodiments, position nine of the sense strand comprises a2' fluoro-modified purine; all pyrimidine of the sense strand comprises 2'-O-methyl modified pyrimidines; 1,2, 3, 4, or 5 purines between positions 5 and 11 comprise a 2' flouro-modified purines, provided there are not three 2' fluoro-modified purines in a row; the odd-numbered positions of the antisense strand comprise 2'-O-methyl modified nucleotides; and the even-numbered positions of the anti sense strand comprise 2'flouro-modified nucleotides and unmodified deoxyribcnucleotides. In some embodiments, there are not three 2' fluoro-modified purines in a row. In some embodiments, there are not three2' fluoro-modified pyrimidines in a row.

In some embodiments, position nine of the sense strand comprises an unmodified deoxyribonucleotide. In some embodiments, positions 5, 7, and 8 of the sense strand comprise 2'fluoro-modifed nucleotides. In some embodiments, all pyrimidines in positions 10 to 21 of the sense strand comprise 2'-O-methyl modified pyrimidines and all purines in positions 10 to 21 of the comprise2'-O-methyl modified purines or 2' fluoro-modified purines. In some embodiments, the odd-numbered positions of the antisense strand comprise 2'-O-methyl modified nucleotides. In some embodiments, the even-numbered positions of the antisense strand comprise 2'flouro-modified nucleotides and unmodified deoxyribonucleotides. In some embodiments, the even-numbered positions of the antisense strand comprise 2'flouro-modified nucleotides, 2'-O-methyl modified nucleotides and unmodified deoxyribonucleotides. In some embodiments, position nine of the sense strand comprises an unmodified deoxyribonucleotide; positions 5, 7, and 8 of the sense strand comprise 2'fluoro-modifed nucleotides; all pyrimidines in positions 10 to 21 of the sense strand comprise 2' -O-methyl modified pyrimidines and all purines in positions 10 to 21 of the comprise 2'-O-methyl modified purines or 2 fluoro-modified purines: the odd-numbered positions of the antisense strand comprise 2'-O-methyl modified nucleotides; and the even-numbered positions of the antisense strand comprise 2 flouro-modified nucleotides and unmodified deoxyribonucleotides.

In some embodiments, position nine of the sense strand comprises an unmodified deoxyribonucleotide. In some embodiments, positions 5, 7, and 8 of the sense strand comprise 2'fluoro-modifed nucleotides. In some embodiments, all purines in positions 10 to 21 of the sense strand comprise 2'-O-methyl modified purines and all pyrimidines in positions 10 to 21 of the comprise 2'-O-methyl modified pyrimidines or 2'fluoro-modified pyrimidines. In some embodiments, the odd-numbered positions of the antisense strand comprise 2'-O-methyl modified nucleotides. In some embodiments, the even-numbered positions of the antisense strand comprise 2'flouro-modified nucleotides and unmodified deoxyribonucleotides. In some embodiments, the even-numbered positions of the antisense strand comprise 2'flouro-modified nucleotides, 2'-O-methyl modified nucleotides and unmodified deoxyribonucleotides. In some embodiments, position nine of the sense strand comprises an unmodified deoxyribonucleotide; positions 5, 7, and 8 of the sense strand comprise 2'fluoro-modifed nucleotides; and all purines in positions 10 to 21 of the sense strand comprise 2'-O-methyl modified purines and and all pyrimidines in positions 10 to 21 of the comprise 2'-O-methyl modified pyrimidines or 2'fluoro-modified pyrimidines; the odd-numbered positions of the anti sense strand comprise 2'-O-methyl modified nucleotides; and the even-numbered positions of the antisense strand comprise 2' flouro-modified nucleotides and unmodified deoxyribonucleotide.

In some embodiments, the moiety includes a negatively charged group attached at a 5' end of the oligonucleotide. This may be referred to as a 5'-end group. In some embodiments, the negatively charged group is attached at a5' end of an antisense strand of an siRNA disclosed herein. The 5'-end group may be or include a 5'-end phosphorothioate, 5'-end phosphorodithioate, 5'-end vinylphosphonate (5'-VP), 5' -end methylphosphonate, 5'-end cyclopropyl phosphonate, or a 5'-deoxy-5'-C-malonyl. The 5'-end group may comprise 5'-VP. In some embodiments, the 5'-VP comprises a trans-vinyl phosphate or cis-vinyl phosphate. The 5'-end group may include an extra 5' phosphate. A combination of 5'-end groups may be used.

In some embodiments, the oligonucleotide includes a negatively charged group. The negatively charged group may aid in cell or tissue penetration. The negatively charged group may be attached at a5'or3'end(e.g. a5'end) of the oligonucleotide. This may be referred to as an end group. The end group may be or include a phosphorothioate, phosphorodithioate, vinylphosphonate, methylphosphonate, cyclopropyl phosphonate, or a deoxy-C-malonyl. The end group may include an extra 5' phosphate such as an extra 5' phosphate. A combination of end groups may be used

In some embodiments, the oligonucleotide includes a phosphate mimic. In some embodiments, the phosphate mimic comprises vinyl phosphonate. In some embodiments, the vinyl phosphonate comprises a trans-vinyl phosphate. In some embodiments, the vinyl phosphonate comprises a cis-vinyl phosphate. An example of a nucleotide that includes a vinyl phosphonate is shown below.

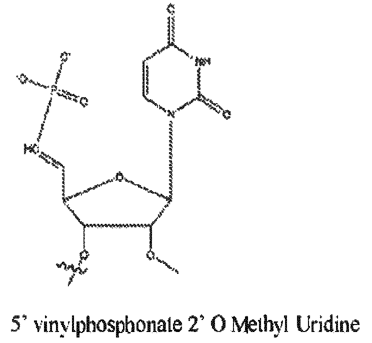

In some embodiments, the vinyl phosphonate increases the stability of the oligonucleotide. In some embodiments, the vinyl phosphonate increases the accumulation of the oligonucleotide in tissues. In some embodiments, the vinyl phosphonate protects the oligonucleotide from an exonuclease or a phosphatase. In some embodiments, the vinyl phosphonate improves the binding affinity of the oligonucleotide with the siRNA processing machinery.

In some embodiments, the oligonucleotide includes 1 vinyl phosphonate. In some embodiments, the oligonucleotide includes 2 vinyl phosphonates. In some embodiments, the oligonucleotide includes 3 vinyl phosphonates. In some embodiments, the oligonucleotide includes 4 vinyl phosphonates. In some embodiments, the antisense strand of the oligonucleotide comprises a vinyl phosphonate at the 5' end. In some embodiments, the antisense strand of: the oligonucleotide comprises a vinyl phosphonate at the 3'end. In some embodiments, the sense strand of the oligonucleotide comprises a vinyl phosphonate at the 5' end. In some embodiments, the sense strand of the oligonucleotide comprises a vinyl phosphonate at the 3' end.

In some embodiments, the composition comprises an oligonucleotide that inhibits the expression of *MSTI*, wherein the oligonucleotide comprises an siRNA comprising a sense strand and an antisense strand, wherein the sense strand comprises a nucleoside sequence comprising or consisting of the sequence of any one of SEQ ID NOs: 6049-6086, 6125-6162, or 6186-6242, or a nucleic acid sequence thereof having 1 or 2 nucleoside substitutions, additions, or deletions. In some embodiments, the sense strand comprises a nucleoside sequence comprising or consisting of the sequence of any one of SEQ ID NOs: 6049-6086, 6125-6162, or 6186-6242, or a nucleic acid sequence thereof having 3 or 4 nucleoside substitutions, additions, or deletions. In some embodiments, the sense strand further comprises a 3' overhang. In some embodiments, the 3' overhang comprises 1, 2, 3, 4, 5, 6, 7, 8,9, or 10 nucleosides, or a range of nucleotides defined by any two of the aforementioned numbers. In some embodiments, the 3' overhang comprises 1, 2, or more nucleosides. In some embodiments, the3' overhang comprises 2 nucleosides. In some embodiments, the sense strand further comprises a5' overhang. In some embodiments, the 5' overhang comprises 1, 2, 3, 4, 5.6, 7, 8,9, or 10 nucleosides, or a range of nucleotides defined by any two of the aforementioned numbers. In some embodiments, the 5' overhang comprises 1, 2, or more nucleosides. In some embodiments, the 5' overhang comprises 2nucleosides. In some embodiments, the sense strand comprises a nucleoside sequence comprising or consisting of the sequence of any one of SEQ ID NOs: 6049-6086, 6125-6162, or 6186-6242, or a nucleic acid sequence thereof having 1 or 2 nucleoside additions at the 3' end. In some embodiments, the composition comprises an oligonucleotide that inhibits the expression of *MSTI,* wherein the oligonucleotide comprises an siRNA comprising a sense strand and an antisense strand, wherein the sense strand comprises a nucleoside sequence comprising or consisting of the sequence of any one of SEQ ID NOs: 6049-6086, 6125-6162, or 6186-6242. The sense strand or antisense strand may comprise any modifications described herein. The sense strand or antisense strand may comprise a lipid moiety or a GalNAc moiety.

In some embodiments, the composition comprises an oligonucleotide that inhibits the expression of *MSTI,* wherein theoligonucleotide comprises an siRNA comprising a sense strand and an anti sense strand, wherein the antisense strand comprises a nucleoside sequence comprising or consisting of the sequence of any one of SEQ ID NOs: 6087-6124 or 6253-6309, or a nucleic acid sequence thereof having 1 or 2 nucleoside substitutions, additions, or deletions. In some embodiments, the antisense strand sequence comprises a nucleoside sequence comprising or consisting of the sequence of any one of SEQ ID NOs: 6087-6124 or 6253-6309, or a nucleic acid sequence thereof having 3 or 4 nucleoside substitutions, additions, or deletions. In some embodiments, the antisense strand further comprises a 3' overhang. In some embodiments, the 3' overhang comprises 1, 2, 3,4,5,6,7, 8,9, or 10 nucleosides, or a range of nucleotides defined by any two of the aforementioned numbers. In some embodiments, the 3' overhang comprises 1, 2, or more nucleosides. In some embodiments, the 3' overhang comprises 2 nucleosides. In some embodiments, the anti sense strand further comprises a5'overhang. In some embodiments, the 5' overhang comprises 1,2, 3, 4, 5, 6, 7, 8,9, or 10 nucleosides, or a range of nucleotides defined by any two of the aforementioned numbers. In some embodiments, the 5' overhang comprises 1, 2, or more nucleosides. In some embodiments, the 5' overhang comprises 2 nucleosides. In some embodiments, the antisense strand comprises a nucleoside sequence comprising or consisting of the sequence of any one of SEQ ID NOs: 6087-6124 or 6253-6309, or a nucleic acid sequence thereof having I or 2 nucleoside additions at the 3'end. In some embodiments, the composition comprises an oligonucleotide that inhibits the expression of *MSTI,* wherein the oligonucleotide comprises an siRNA comprising a sense strand and an antisense strand, wherein the antisense strand comprises a nucleoside sequence comprising or consisting of the sequence of any one of SEQ ID NOs: 6087-6124 or 6253-6309. The sense strand or antisense strand may comprise any modifications described herein. The sense strand or antisense strand may comprise a lipid moiety or a GalNAc moiety.

In some embodiments, the siRNA comprises a sense strand having a sequence in accordance with any of SEQ ID NOs: 6206, 6212, 6213, 6214, 6227, 6232, 6236. or 6242. In some embodiments, the sense strand sequence comprises or consists of sequence at least 75% identical to any one of SEQ ID NOs: 6206, 6212, 6213, 6214, 6227, 6232, 6236, or 6242, at least 80% identical to any one of SEQ ID NOs: 6206, 6212, 6213, 6214, 6227, 6232, 6236, or 6242, at least 85% identical to of any one of SEQ ID NOs: 6206, 6212, 6213, 6214, 6227, 6232, 6236, or 6242, at least 90% identical to any one of SEQ ID NOs: 6206, 6212, 6213, 6214, 6227, 6232, 6236, or 6242, or at least 95% identical to any one of SEQ ID NOs: 6206, 6212, 6213. 6214, 6227, 6232, 6236, or 6242. In some embodiments, the sense strand sequence comprises or consists of the sequence of any one of SEQ ID NOs 6206, 6212, 6213, 6214, 6227, 6232, 6236, or 6242, or a sense strand sequence thereof having 1, 2, 3, or 4 nucleoside substitutions. additions, or deletions. In some embodiments, the sense strand sequence comprises or consists of the sequence of any one of SEQ ID NOs: 6206, 6212, 6213, 6214, 6227, 6232, 6236, or 6242, or a sense strand sequence thereof having 1 or 2 nucleoside substitutions, additions, or deletions. In some embodiments, the sense strand sequence comprises or consists of a sequence 100% identical to SEQ ID NOs: 6206, 6212, 6213, 6214, 6227, 6232, 6236, or 6242. The sense strand sequence may include the first 5,6, 7, 8, 9,10, 11, 12, 13, 14, 15, 16, 17, 18, or 19 nucleotides (in the 5' to 3' direction) of any of the aforementioned sequences. The sense strand sequence may include the last 5, 6, 7, 8, 9, 10, 11, 12, 13, 14, 15, 16, 17, 18, or 19 nucleotides (in the 5' to 3' direction) of any of the aforementioned sequences. The sense strand may comprise a modification pattern described herein. The sense strand may comprise an overhang. The sense strand may comprise a lipid moiety. The sense strand may comprise a GalNAc moiety.

In some embodiments, the siRNA comprises an antisense strand having a sequence in accordance with any of SEQ ID NOs: 6273, 6279, 6280, 6281, 6294, 6299, 6303, or 6309. In some embodiments, the anti sense strand sequence comprises or consists of sequence at least 75% identical to any one of SEQ ID NOs: 6273, 6279, 6280, 6281, 6294, 6299, 6303, or 6309, at least 80% identical to any one of SEQ ID NOs: 6273, 6279, 6280, 6281, 6294, 6299, 6303, or 6309, at least 85% identical to of any one of SEQ ID NOs: 6273, 6279, 6280, 6281, 6294, 6299, 6303, or 6309, at least 90% identical to any one of SEQ ID NOs: 6273, 6279, 6280, 6281, 6294, 6299, 6303, or 6309, or at least 95% identical to any one of SEQ ID NOs: 6273, 6279, 6280, 6281, 6294, 6299, 6303, or 6309. In some embodiments, the antisense strand sequence comprises or consists of the sequence of any one of SEQ ID NOs: 6273, 6279. 6280, 6281, 6294, 6299, 6303, or 6309, or an antisense strand sequence thereof having 1, 2, 3, or 4 nucleoside substitutions, additions, or deletions. In some embodiments, the antisense strand sequence comprises or consists of the sequence of any one of SEQ ID NOs 6273, 6279, 6280, 6281, 6294, 6299, 6303, or 6309, or an antisense strand sequence thereof having 1 or 2 nucleoside substitutions, additions, or deletions. In some embodiments, the antisense strand sequence comprises or consists of a sequence 100% identical to SEQ ID NOs: 6273, 6279, 6280, 6281, 6294, 6299, 6303, or 6309. The anti sense strand sequence any include the first 5, 6, 7, 8, 9, 10, 11, 12, 13, 14, 15, 16, 17, 18, or 19 nucleotides (in the 5' to 3' direction) of any of the aforementioned sequences. The antisense strand sequence may include the last 5, 6,7,8,9, 10, 11, 12, 13, 14, 15, 16, 17, 18, or 19 nucleotides (in the 5' to 3' direction) of any of the aforementioned sequences. The antisense strand may comprise an overhang. The anti sense strand may comprise a modification pattern described herein. The antisense strand may comprise a lipid moiety or a GalNAc moiety.

In some embodiments, the siRNA comprises a sense strand having a sequence in accordance with SEQ ID NO: 6206. In some embodiments, the sense strand sequence comprises or consists of sequence at least 75% identical to SEQ ID NO: 6206, at least 80% identical to SEQ ID NO: 6206, at least 85% identical to SEQ ID NO: 6206, at least 90% identical to SEQ ID NO: 6206, or at least 95% identical to SEQ ID NO 6206. In some embodiments, the sense strand sequence comprises or consists of the sequence of SEQ ID NO 6206, or a sense strand sequence thereof having 1,2, 3, or 4 nucleoside substitutions, additions, or deletions. In some embodiments, the sense strand sequence comprises or consists of the sequence of SEQ IDNO: 6206, or a sense strand sequence thereof having 1 or 2 nucleoside substitutions, additions, or deletions. In some embodiments, the sense strand sequence comprises or consists of a sequence 100% identical to SEQ ID NO: 6206. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6212. In some embodiments, the sense strand sequence comprises or consists of sequence at least 75% identical to SEQ IDNO: 6212, at least 80% identical to SEQ ID NO: 6212, at least 85% identical to SEQ ID NO: 6212, a least 90% identical to SEQ ID NO: 6212, or at least 95% identical to SEQ ID NO: 6212. In some embodiments, the sense strand sequence comprises or consists of the sequence of SEQ ID NO 6212, or a sense strand sequence thereof having 1, 2, 3, or 4 nucleoside substitutions, additions, or deletions. In some embodiments, the sense strand sequence comprises or consists of the sequence of SEQ ID NO: 6212, or a sense strand sequence thereof having 1 or 2 nucleoside substitutions, additions, or deletions. In some embodiments, the sense strand sequence comprises or consists of a sequence 100% identical to SEQ ID NO: 6212. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6213. In some embodiments, the sense strand sequence comprises or consists of sequence at least 75% identical to SEQ ID NO: 6213, at least 80% identical to SEQ ID NO: 6213, at least 85% identical to SEQ ID NO: 6213, at least 90% identical to SEQ ID NO: 6213, or at least 95% identical to SEQ ID NO: 6213. In some embodiments, the sense strand sequence comprises or consists of the sequence of SEQ ID NO 6213, or a sense strand sequence thereof having 1, 2, 3, or 4 nucleoside substitutions, additions, or deletions. In some embodiments, the sense strand sequence comprises or consists of the sequence of SEQ ID NO: 6213, or a sense strand sequence thereof having 1 or 2 nucleoside substitutions, additions, or deletions. In some embodiments, the sense strand sequence comprises or consists of a sequence 100% identical to SEQ ID NO: 6213. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6214. In some embodiments, the sense strand sequence comprises or consists of sequence at least 75% identical to SEQ ID NO: 6214, at least 80% identical to SEQ ID NO: 6214, at least 85% identical to SEQ ID NO: 6214, at least 90% identical to SEQ ID NO: 6214, or at least 95% identical to SEQ ID NO: 6214. In some embodiments, the sense strand sequence comprises or consists of the sequence of SEQ ID NO 6214, or a sense strand sequence thereof having 1, 2, 3, or 4 nucleoside substitutions, additions, or deletions. In some embodiments, the sense strand sequence comprises or consists of the sequence of SEQ ID NO: 6214, or a sense strand sequence thereof having 1 or 2 nucleoside substitutions, additions, or deletions. In some embodiments, the sense strand sequence comprises or consists of a sequence 100% identical to SEQ ID NO: 6214. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6227. In some embodiments, the sense strand sequence comprises or consists of sequence at least 75% identical to SEQ ID NO: 6227, at least 80% identical to SEQ ID NO: 6227, at least 85% identical to SEQ ID NO: 6227, at least 90% identical to SEQ ID NO: 6227, or at least 95% identical to SEQ ID NO 6227. In some embodiments, the sense strand sequence comprises or consists of the sequence of SEQ ID NO 6227, or a sense strand sequence thereof having 1, 2,3, or 4 nucleoside substitutions, additions, or deletions. In some embodiments, the sense strand sequence comprises or consists of the sequence of SEQ ID NO: 6227, or a sense strand sequence thereof having 1 or 2 nucleoside substitutions, additions, or deletions. In some embodiments, the sense strand sequence comprises or consists of a sequence 100% identical to SEQ ID NO: 6227. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6232. In some embodiments, the sense strand sequence comprises or consists of sequence at least 75% identical to SEQ ID NO: 6232, at least 80% identical to SEQ ID NO: 6232, at least 85% identical to SEQ ID NO: 6232, at least 90% identical to SEQ ID NO: 6232, or at least 95% identical to SEQ ID NO: 6232. In some embodiments, the sense strand sequence comprises or consists of the sequence of SEQ ID NO 6232. or a sense strand sequence thereof having 1, 2, 3, or 4 nucleoside substitutions, additions, or deletions. In some embodiments, the sense strand sequence comprises or consists of the sequence of SEQ ID NO: 6232, or a sense strand sequence thereof having 1 or 2nucleoside substitutions, additions, or deletions. In some embodiments, the sense strand sequence comprises or consists of a sequence 100% identical to SEQ ID NO: 6232. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6236. In some embodiments, the sense strand sequence comprises or consists of sequence at least 75% identical to SEQ ID NO: 6236, at least 80% identical to SEQ ID NO: 6236, at least 85% identical to SEQ ID NO: 6236, at least 90% identical to SEQ ID NO: 6236, or at least 95% identi cal to SEQ ID NO: 6236. In some embodiments, the sense strand sequence comprises or consists of the sequence of SEQ ID NO 6236, or a sense strand sequence thereof having 1, 2, 3, or 4 nucleoside substitutions, additions, or deletions. In some embodiments, the sense strand sequence comprises or consists of the sequence of SEQ IDNO: 6236, or a sense strand sequence thereof having 1 or 2 nucleoside substitutions, additions, or deletions. In some embodiments, the sense strand sequence comprises cr consists of a sequence 100% identical to SEQ ID NO: 6236. The sense strand may comprise a modification pattern described herein. The sense strand may comprise an overhang. The sense strand may comprise a lipid moiety. The sense strand may comprise a GalNAc moiety.

In some embodiments, the siRNA comprises a sense strand having a sequence in accordance with SEQ ID NO: 6242. In some embodiments, the sense strand sequence comprises or consists of sequence at least 75% identical to SEQ ID NO: 6242, at least 80% identical to SEQ ID NO: 6242, at least 85% identical to SEQ ID NO: 6242, at least 90% identical to SEQ ID NO: 6242, or at least 95% identical to SEQ ID NO: 6242. In some embodiments, the sense strand sequence comprises or consists of the sequence of SEQ ID NO 6242, or a sense strand sequence thereof having 1, 2, 3, or 4 nucleoside substitutions, additions, or deletions. In some embodiments, the sense strand sequence comprises or consists of the sequence of SEQ ID NO: 6242, or a sense strand sequence thereof having 1 or 2 nucleoside substitutions. additions. or deletions. In some embodiments, the sense strand sequence comprises or consists of a sequence 100% identical to SEQ ID NO: 6242. The sense strand may comprise a modification pattern described herein. The sense strand may comprise an overhang. The sense strand may comprise a lipid moiety. The sense strand may comprise a GalNAc moiety.

In some embodiments, the siRNA comprises an antisense strand having a sequence in accordance with SEQ ID NO: 6273. In some embodiments, the antisense strand sequence comprises or consists of sequence at least 75% identical to SEQ ID NO: 6273. at least 80% identical to SEQ ID NO: 6273, at least 85% identical to SEQ ID NO: 6273, at least 90% identical to SEQ ID NO: 6273, or at least 95% identical to SEQ ID NO: 6273. In some embodiments, the antisense strand sequence comprises or consists of the sequence of SEQ ID NO 6273, or an antisense strand sequence thereof having 1,2,3, or 4 nucleoside substitutions, additions, or detetions. In some embodiments, the antisense strand sequence comprises or consists of the sequence of SEQ ID NO: 6273, or an antisense strand sequence thereof having 1 or 2 nucleoside substitutions, additions, or deletions. In some embodiments, the antisense strand sequence comprises or consists of a sequence 100% identical to SEQ ID NO: 6273. The antisense strand may comprise a modification pattern described herein. The antisense strand may comprise an overhang. The antisense strand may comprise a lipid moiety. The anti sense strand may comprise a GalNAc moiety.

In some embodiments, the siRNA comprises an antisense strand having a sequence in accordance with SEQ ID NO: 6279. In some embodiments, the antisense strand sequence comprises or consists of sequence at least 75% identical to SEQ ID NO: 6279, at least 80% identical to SEQ ID NO: 6279, at least 85% identical to SEQ ID NO: 6279, at least 90% identical to SEQ ID NO: 6279, or at least 95% identical to SEQ ID NO: 6279. In some embodiments, the anti sense strand sequence comprises or consists of the sequence of SEQ ID NO 6279, or an antisense strand sequence thereof having 1, 2, 3, or4 nucleoside substitutions, additions, or deletions. In some embodiments, the antisense strand se quence comprises or consists of the sequence of SEQ ID NO: 6279, or an anti sense strand sequence thereof having 1 or 2 nucleoside substitutions, additions, or deletions. In some embodiments, the antisense strand sequence comprises or consists of a sequence 100% identical to SEQ ID NO: 6279. The antisense strand may comprise a modification pattern described herein. The anti sense strand may comprise an overhang. The antisense strand may comprise a lipid moiety. The antisense strand may comprise a GalNAc moiety.

In some embodiments, the siRNA comprises an antisense strand having a sequence in accordance with SEQ ID NO: 6280. In some embodiments, the antisense strand sequence comprises or consists or sequence at least 75% identical to SEQ ID NO: 6280, at least 80% identical to SEQ ID NO: 6280, at least 85% identical to SEQ ID NO: 6280, a least 90% identical to SEQ ID NO: 6280, or at least 95% identical to SEQ ID NO: 6280. In some embodiments, the anti sense strand sequence comprises or consists of the sequence of SEQ ID NO 6280, or an antisense strand sequence thereof having 1,2, 3, or 4 nucleoside substitutions, additions, or deletions. In some embodiments, the antisense strand sequence comprises or consists of the sequence of SEQ ID NO: 6280, or an anti sense strand sequence thereof having 1 or 2 nucleoside substitutions, additions, or deletions. In some embodiments, the antisense strand sequence comprises or consists of a sequence 100% identical to SEQ ID NO: 6280. The anti sense strand may comprise a modification pattern described herein. The antisense strand may comprise an overhang. The antisense strand may comprise a lipid moiety. The anti sense strand may comprise a GalNAc moiety.

In some embodiments, the siRNA comprises an antisense strand having a sequence in accordance with SEQ ID NO: 6281. In some embodiments, the antisense strand sequence comprises or consists of sequence at least 75% identical to SEQ ID NO: 6281, at least 80% identical to SEQ ID NO: 6281, at least 85% identical to SEQ ID NO 6281, a least 90% identical to SEQ ID NO: 6281, or at least 95% identical to SEQ ID NO: 6281. In some embodiments, the antisense strand sequence comprises or consists of the sequence of SEQ ID NO 6281, or an antisense strand sequence thereof having 1, 2, 3, or 4 nucleoside substitutions, additions, or deletions. In some embodiments, the antisense strand sequence comprises or consists of the sequence of SEQ ID NO: 6281, or an antisense strand sequence thereof having 1 or 2nucleoside substitutions, additions, or deletions. In some embodiments, the antisense strand sequence comprises or consists of a sequence 100% identical to SEQ ID NO: 6281. The antisense strand may comprise a modification pattern described herein. The antisense strand may comprise an overhang. The anti sense strand may comprise a lipid moiety. The antisense strand may comprise a GalNAc moiety.

In some embodiments, the siRNA comprises an antisense strand having a sequence in accordance with SEQ ID NO: 6294. In some embodiments, the antisense strand sequence comprises or consists of sequence a least 75% identi cal to SEQ ID NO: 6294, at least 80% identical to SEQ ID NO: 6294, at least 85% identical to SEQ ID NO: 6294, at least 90% identical to SEQ ID NO: 6294, or at least 95% identical to SEQ ID NO: 6294. In some embodiments, the anti sense strand sequence comprises or consists of the sequence of SEQ ID NO 6294, or an anti sense strand sequence thereof having 1, 2, 3, or 4 nucleoside substitutions, additions, or deletions. In some embodiments, the anti sense strand sequence comprises or consists of the sequence of SEQ ID NO: 6294, or an antisense strand sequence thereof having 1 or 2 nucleoside substitutions, additions, or deletions. In some embodiments, the antisense strand sequence comprises or consists of a sequence 100% identical to SEQ ID NO: 6294. The antisense strand may comprise a modification pattern described herein. The antisense strand may comprise an overhang. The antisense strand may comprise a lipid moiety. The antisense strand may comprise a GalNAc moiety.

In some embodiments, the siRNA comprises an antisense strand having a sequence in accordance with SEQ ID NO: 6299. In some embodiments, the antisense strand sequence comprises or consists of sequence at least 75% identical to SEQ ID NO: 6299, at least 80% identical to SEQ ID NO: 6299, at least 85% identical to SEQ ID NO: 6299, at least 90% identical to SEQ ID NO: 6299, or at least 95% identical to SEQ ID NO: 6299. In some embodiments, the antisense strand sequence comprises or consists of the sequence of SEQ ID NO 6299, or an anti sense strand sequence thereof having 1, 2, 3, or 4 nucleosid e substitutions, additions, or deletions. In some embodiments, the antisense strand sequence comprises or consists of the sequence of SEQ ID NO: 6299, or an antisense strand sequence thereof having 1 or 2 nucleoside substitutions, additions, or deletions. In some embodiments, the anti sense strand sequence comprises or consists of a sequence 100% identical to SEQ ID NO: 6299. The anti sense strand may comprise a modification pattern described herein. The anti sense strand may comprise an overhang. The anti sense strand may comprise a lipid moiety. The anti sense strand may comprise a GalNAc moiety.

In some embodiments, the siRNA comprises an antisense strand having a sequence in accordance with SEQ ID NO: 6303. In some embodiments, the antisense strand sequence comprises or consists of sequence at least 75% identical to SEQ ID NO: 6303, at least 80% identical to SEQ ID NO: 6303, at least 85% identical to SEQ ID NO: 6303, at least 90% identical to SEQ ID NO: 6303, or at least 95% identical to SEQ ID NO: 6303. In some embodiments, the antisense strand sequence comprises or consists of the sequence of SEQ ID NO 6303, or an antisense strand sequence thereof having 1, 2, 3, or 4 nucleoside substitutions. additions, or deletions. In some embodiments, the antisense strand sequence comprises or consists of the sequence of SEQ ID NO: 6303, or an antisense strand sequence thereof having 1 or 2 nucleoside substitutions, additions, or deletions. In some embodiments, the antisense strand sequence comprises or consists of a sequence 100% identical to SEQ ID NO: 6303. The antisense strand may comprise a modification pattern described herein. The antisense strand may comprise an overhang. The antisense strand may comprise a lipid moiety. The antisense strand may comprise a GalNAc moiety.

In some embodiments, the siRNA comprises an antisense strand having a sequence in accordance with SEQ ID NO: 6309. In some embodiments, the antisense strand sequence comprises or consists of sequence a least 75% identical to SEQ ID NO: 6309, at least 80% identical to SEQ ID NO: 6309, at least 85% identical to SEQ ID NO: 6309, at least 90% identical to SEQ ID NO: 6309, or at least 95% identical to SEQ ID NO: 6309. In some embodiments, the anti sense strand sequence comprises or consists of the sequence of SEQ ID NO 6309, or an anti sense strand sequence thereof having 1, 2, 3, or 4 nucleoside substitutions, additions, or deletions. In some embodiments, the anti sense strand sequence comprises or consists of the sequence of SEQ ID NO: 6309, or an antisense strand sequence thereof having 1 or 2 nucleoside substitutions, additions, or deletions. In some embodiments, the anti sense strand sequence comprises or consists of a sequence 100% identical to SEQ ID NO: 6309. Theantisense strand may comprise a modification pattern described herein. The antisense strand may comprise an overhang. The antisense strand may comprise a lipid moiety. The antisense strand may comprise a GalNAc moiety.

### 1. HydropHobic moieties

In some embodiments, the composition comprises an oligonucleotide that inhibits the expression of MST1, wherein the oligonucleotide comprises a hydrophobic moiety. The hydrophobic moiety nny be attached at a 3' or 5' terminus of the oligonucleotide. The hydrophobic moiety may include a lipid such as a fatty acid. The hydrophobic moiety may include a hydrocarbon. The hydrocarbon may be linear. The hydrocarbon may be non-linear. The hydrophobic moiety may include a lipid moiety or a cholesterol moiety, or a combination thereof.

In some embodiments, the composition comprises an oligonucleotide that inhibits the expression of MST1, wherein the oligonucleotide comprises a lipid attached at a 3' or 5' terminus of the oligonucleotide. In some embodiments, the lipid comprises cholesterol, myristonyl, palmitoyl, stearoyl, lithocholoyl, docosanoyl, docosahexaenoyl, myristyl, palmityl, stearyl, or α-tocopherol, or a combination thereof.

In some embodiments, the composition comprises an oligonucleotide that inhibits the expression of MST1, wherein the oligonucleotide comprises a hydrophobic ligand or moiety. In some embodiments, the hydrophobic ligand or moiety comprises cholesterol. In some embodiments, the hydrophobic ligand or moiety comprises a cholesterol derivative. In some embodiments, the hydrophobic ligand or moiety is attached at a 3' terminus of the oligonucleotide. in some embodiments, the hydrophobic ligand or moiety s attached at a 5' terminus of the oligonucleotide. In some embodiments, the composition comprises a sense strand, and the hydrophobic ligand or moiety is attached to the sense strand (e.g. attached to a 5' end of the sense strand, or attached to a 3' end of the sense strand). In some embodiments, the composition comprises an antisense strand, and the hydrophobic ligand or moiety is attached to the antisense strand (e.g. attached to a5'end of the antisense strand, or attached to a 3'end of the antisense strand). In some embodiments, the composition comprises a hydrophobic ligand or moiety attached at a3'or 5' terminus of the oligonucleotide.

In some embodiments, a hydrophobic moiety is attached to the oligonucleotide (e.g. a sense strand and/or an antisense strand of a siRNA). In some embodiments, a hydrophobic moiety is attached at a 3' terminus of the oligonucleotide. In some embodiments, a hydrophobic moiety is attached at a5' terminus of the oligonucleotide. In some embodiments, the hydrophobic moiety comprises cholesterol. In some embodiments, the hydrophobic moiety includes a cyclohexanyl.

In some embodiments, the composition comprises an oligonucleotide that inhibits the expression of MST1, wherein the oligonucleotide comprises a lipid attached at a 3' or 5' terminus of the oligonucle otide. In some embodiments, a lipid is attached at a 3'terminus of the oligonucleotide. In some embodiments, a lipid is attached at a 5' terminus of the oligonucleotide. In some embodiments, the lipid comprises cholesterol, myristoyl, palmitoyl, stenroyl, lithocholoyl, docosanoyl, docosanexaneoyl, myristyl, palmityl, stearyl, or α-tocopherol, or a combination thereof. In some embodiments, the lipid comprises stearyl, lithocholyl, docosanyl, docosahexaenyl, or myristyl. In some embodiments, the lipid comprises cholesterol. In some embodiments, the lipid includes a sterol such as cholesterol. In some embodiments, the lipid comprises stearyl, t-butylphenol, n-butylphenol, octylphenol, dodecyl phenol, phenyl n-dodecyl, octadecylbenzamide, hexadecylbenzamide, or octadecylcyclohexyl. In some embodiments, the lipid comprises phenyl para C12.

In some embodiments, the oligonucleotide comprises any aspect of the following structure: 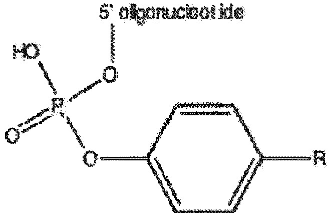 . In some embodiments, the oligonucleotide comprises any aspect of the following structure: 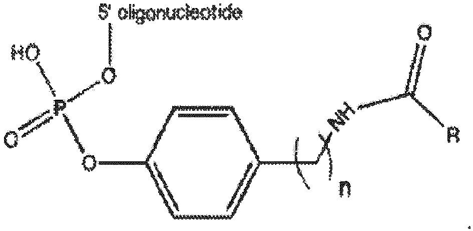 In some embodiments, the oligonucleotide comprises any aspect of the following structure: 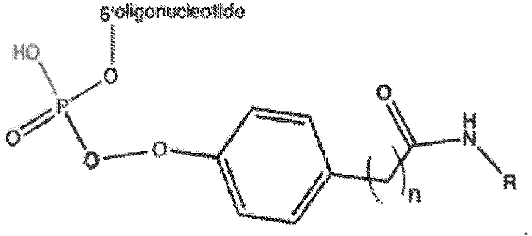 In some embodiments, the oligonucleotide comprises any aspect of the following structure: The aspect included in the oligonucleotide may include the entire structure, or may include the lipid moiety, of any of the structures shown. In some embodiments, n is 1-3. In some embodiments, n is 1. In some embodiments, n is 2. In some embodiments, n is 3. In some embodiments, R is an alkyl group. In some embodiments, the alkyl group contains 1, 2, 3, 4, 5,6,7, 8, 9, 10, 11, 12, 13, 14, 15, 16,17, 18, 19, or 20 carbons. In some embodiments, the alkyl group contains 4, 5, 6, 7, 8, 9, 10, 11, 12, 13, 14, 15, 16, 17, or 18 carbons, or a range defined by any two of the aforementioned numbers of carbons. In some embodiments, the alkyl group contains 4-18 carbons. In some embodiments, the lipid moiety comprises an alcohol or ether.

In some embodiments, the lipid includes a fatty acid.In some embodiments, the lipid comprises a lipid depicted in Table 1. The example lipid moieties in Table 1 are shown attached at a 5' end of an oligonucleotide, in which the 5' terminal phosphate of the oligonucleotide is shown with the lipid moiety. In some embodiments, a lipid moiety in Table 1 may be attached at a different point of attachment than shown For example, the point of attachment of any of the lipid moieties in the table may be at a oligonucleotide end. In some embodiments, the lipid is used for targeting the oligonucleotide to a non-hepatic cell or tissue.

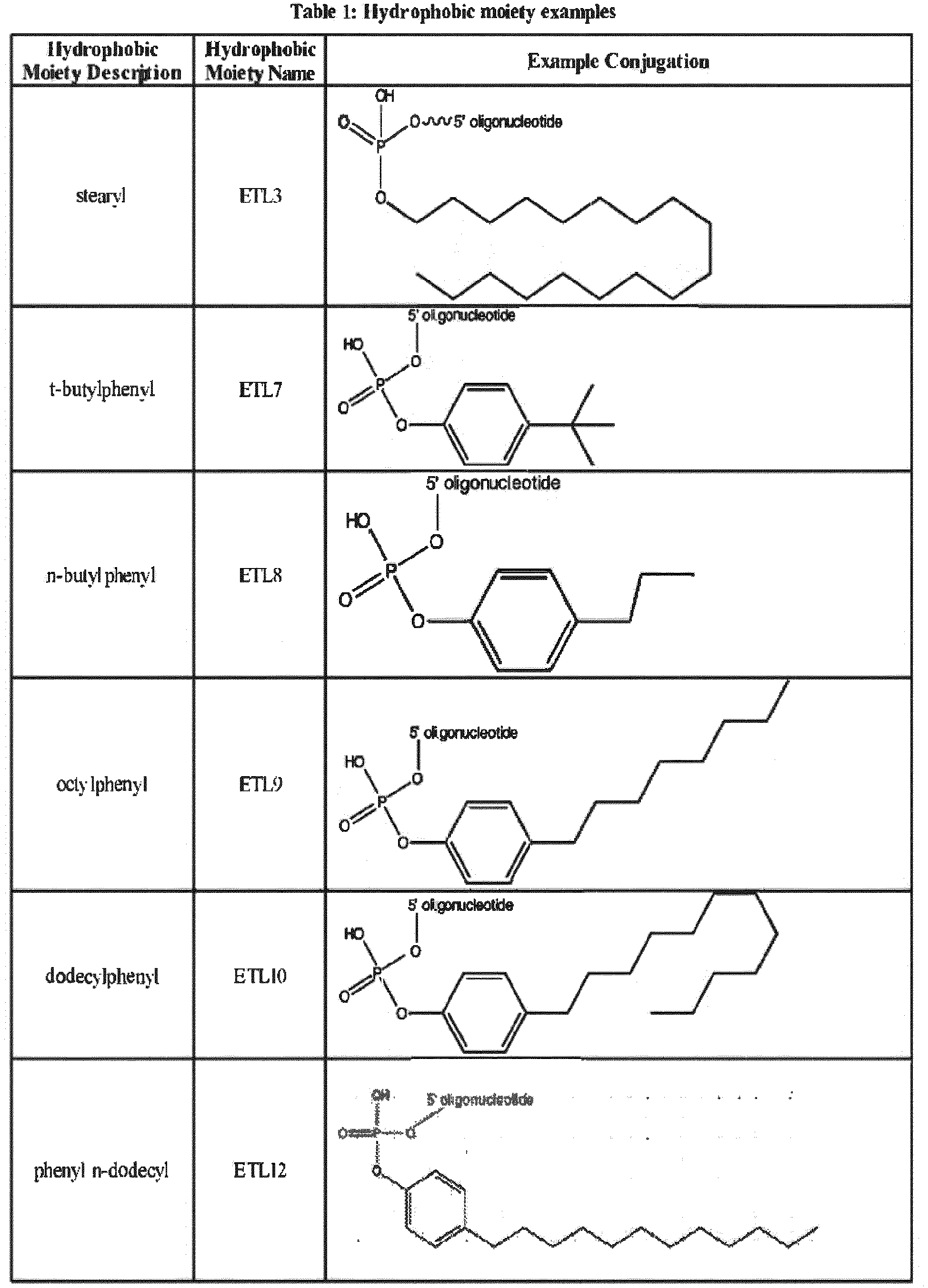

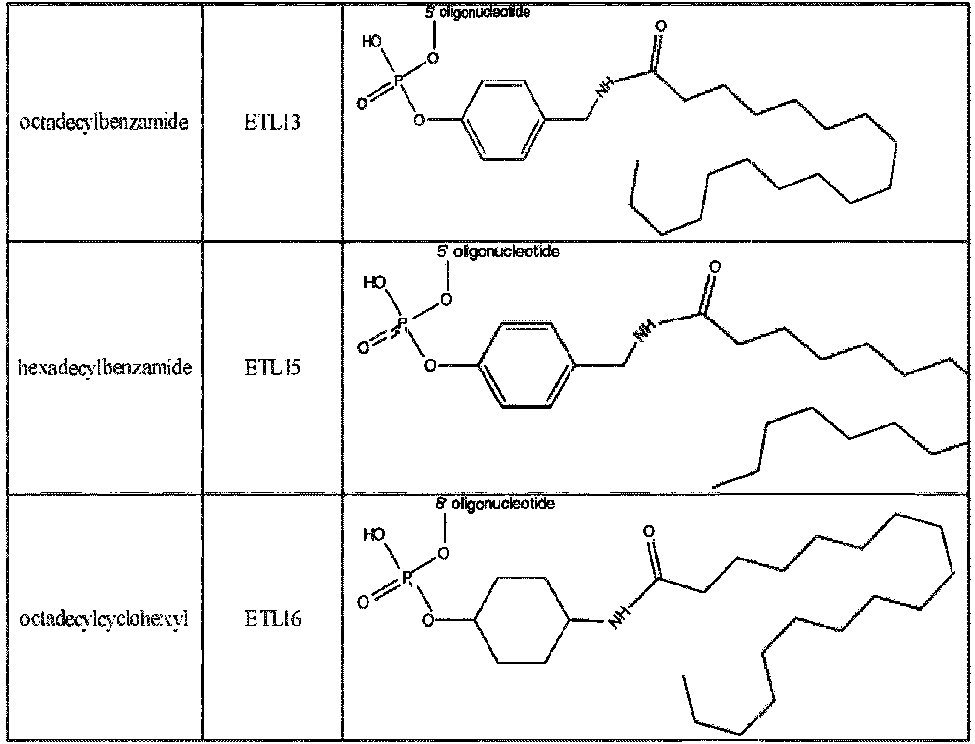

In some embodiments, the lipid or lipid moiety includes 16 to 18 carbons. In some embodiments, the lipid includes 16 carbons. In some embodiments, the lipid includes 17 carbons. In some embodiments, the lipid includes 18 carbons. In some embodiments, the lipid moiety includes 16 carbons. In some embodiments, the lipid moiety includes 17 carbons. In some embodiments, the lipid moiety includes 18 carbons.

The hydrophobic moiety may include a linker that comprises a carbocycle. The carbocycle may be six-membered. Some examples of a carbocycle include phenyl or cyclohexyl. The linker may include a phenyl. The linker may include a cyclohexyl. The lipid may be attached to the carbocycle, which may in turn be attached at a phosphate (e.g 5' or 3' phosphate) of the oligonucleotide. In some embodiments, the lipid or hydrocarbon, and the end of the sense are connected to the phenyl or cyclohexyl linker in the 1.4; 1,3; or 1,2 substitution pattern (e.g_{.} the para, meta, or ortho phenyl configuration). In some embodiments, the lipid or hydrocarbon, and the end of the sense are connected to the phenyl or cyclohexyl linker in the 1,4 substitution pattern (e.g. the para phenyl configuration). The lipid may be attached to the carbocycle in the 1,4 substitution pattern relative to the oligonucleotide. The lipid may be attached to the carbocycle in the 1,3 substitution pattern relative to the oligonucleotide. The lipid may be attached to the carbocycle in the 1,2 substitution pattern relative to the oligonucleotide. The lipid may be attached to the carbocycle in the ortho orientation relative to the oligonucleotide. The lipid any be attached to the carbocycle in the para orientation relative to the oligonucleotide. The lipid may be attached to the carbocycle in the meta orientation relative to the oligonucleotide.

The lipid moiety may comprise or consist of the following structure 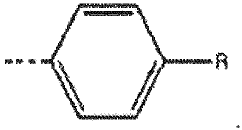 In some embodiments, the lipid moiety comprises or consists of the f allowing structure: 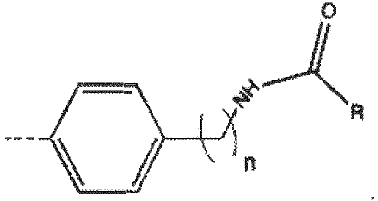 **In** some embodiments, the lipid moiety comprises the following structure: 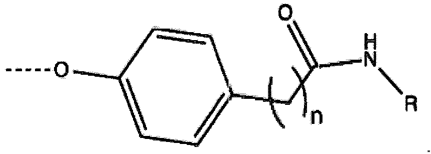 In some embodiments, the lipid moiety comprises or consist of the following structure: 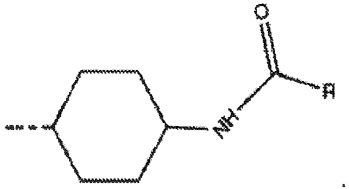 In some embodiments, the dotted line indicates a covalent connection. The covalent connection may between an end of the sense or antisense strand. For example, the connection may be to the5' end of the sense strand. In some embodiments, n is0-3. In some embodiments, n is 1-3. In some embodiments, n is 0. In some embodiments, n is 1. In some embodiments, n is 2. In some embodiments, n is 3. In some embodiments, n is 4. In some embodiments, n is 5. In some embodiments, n is 1, 2, 3, 4, 5, 6, 7, 8, 9, or 10. In some embodiments, R is an alkyl group. In some embodiments, the alkyl group contains 1, 2, 3, 4, 5, 6, 7, 8, 9, 10, 11, 12, 13, 14, 15, 16, 17, 18, 19, or 20 carbons. In some embodiments, the alkyl group contains 4, 5, 6, 7, 8, 9, 10, 11, 12, 13**,** 14, 15, 16,17, or 18 carbons, or a range defined by any two of the aforementioned numbers of carbons. In some embodiments, R comprises or consists of an alkyl group containing 4-18 carbons.

The lipid moiety may be attached at a 5'end of the oligonucleotide. The 5' erd may have one phosphate linking the lipid moiety to a 5' carbon of a sugar of the oligonucleotide. The 5' end may have two phosphates linking the lipid moiety to a5' carbon of a sugar of the oligonucleotide. The5' end may have three phosphates linking the lipid moiety to a5' carbon of a sugar of the oligonucleotide. The5' end may have one phosphate connected to the 5' carbon of a sugar of the oligonucleotide, where the one phosphate is connected to the lipid moiety. The 5' end may have two phosphates connected to the 5' carbon of a sugar of the oligonucleotide, where the one of the two phosphates is connected to the lipid moiety. The 5' end may have three phosphates connected to the 5' carbon of a sugar of the oligonucleotide, where the one of the three phosphates is connected to the lipid moiety. The sugar may include a ribose. The sugar may include a deoxyribose. The sugar may be modified a such as a 2' modified sugar (e.g. a2' O-methyl or 2' fluoro ribose). A phosphate of the 5' end may include a modification such as a sulfur in place of an oxygen. Two phosphates of the 5' end may include a modification such as a sulfur in place of an oxygen Three phosphates of the 5' end may include a modification such as a sulfur in place of an oxygen.

In some embodiments, the oligonucleotide includes 1 lipid moiety. In some embodiments, the oligonucleotide includes 2 lipid moieties. In some embodiments, the oligonucleotide includes 3 lipid moieties. In some embodiments, the oligonucleotide includes 4 lipid moieties.

Some embodiments relate to a method of making an oligonucleotide comprising a hydrophobic conjugate. A strategy for making hydrophobic conjugates may include use or a phosphoramidite reagent based upon a 6-membered ring alcohol such as a phenol or cyclohexanol. The phosphoramidite may be reacted to a nucleotide to connect the nucleotide to the hydrophobic moiety, and thereby produce the hydrophobic conjugate. Some examples of phosphoramidite reagents that may be used to produce a hydrophobic conjugate are provided as follows: 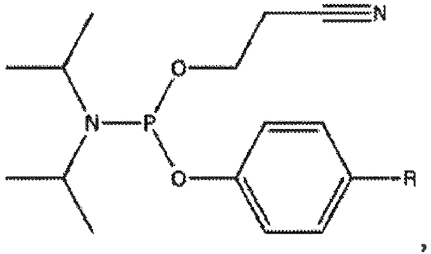 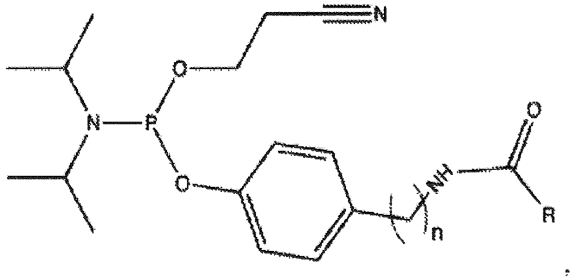 or 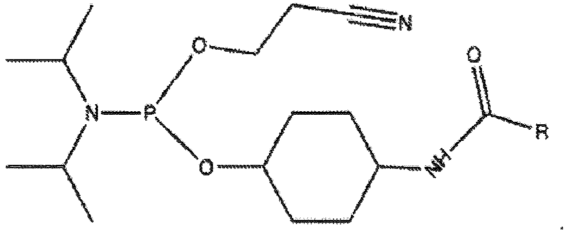 In some embodiments, n is 1-3. In some embodiments, n is 1. In some embodiments, n is2. In some embodiments, n is 3. In some embodiments, R is an alkyl group. In some embodiments, the alkyl group contains 1, 2,3, 4, 5, 6, 7, 8, 9, 10, 11, 12, 13, 14, 15, 16, 17, 18, 19, or 20 carbons. In some embodiments, the alkyl group contains 4, 5, 6, 7, 8, 9, 10, 11, 12, 13, 14, 15,16, 17, or 18 carbons, or a range defined be any two of the aforementioned numbers of carbons. In some embodiments. R comprises or consists of an alkyl group containing 4-18 carbons. Any one of the phosphoramidite reagents may be reacted to a 5'end of an oligonucleotide to produce an oligonucleotide comprising a hydrophobic moiety. In some embodiments, the phosphoramidite reagents is reacted to a 5' end of a sense strand of an siRNA. The sense strand may then be hybridized to an antisense strand to form a duplex. The hybridization may be performed by incubating the sense and antisense strands in solution at a given temperature. The temperature may be gradually reduced. The temperature may comprise or include a temperature comprising an annealing temperature for the sense and antisense strands. The temperature may be below or include a temperature below the annealing temperature for the sense and antisense strands. The temperature may be below a melting temperature of the sense and antisense strands.

The lipid may be attached to the oligonucleotide by a linker. The linker may include a polyethyleneglycol (e.g. tetraethyleneglycol).

The modifications described herein may be useful for delivery to a cell or tissue, for example, extrahepatic delivery or targeting of an oligonucleotide composition. The modifications described herein may be useful for targeting an oligonucleotide composition to a cell or tissue.

### 2. Sugar moieties

In some embodiments, the composition comprises an oligonucleotide that inhibits the expression of MST1, wherein the oligonucleotide comprises a sugar moiety. The sugar moiety may include an N-acetyl galactose moiety (e.g. an N-acetylgalactosamine (GalNAc) moiety), an N-acetyl glucose moiety (e.g. an N-acetylglucosamine (GlcNAc) moiety), a fucose moiety, or a mannose moiety. The sugar moiety may include 1, 2, 3, or more sugar molecules. The sugar moiety may be attached at a 3' or 5'terminus of the oligonucleotide. The sugar moiety may include an N-acetyl galactose moiety. The sugar moiety may include an N-acetylgalactosamine (GalNAc) moiety. The sugar moiety may include an N-acetyl glucose moiety. The sugar moiety may include N-acetylglucosamine (GlcNAc) moiety. The sugar moiety may include a fucose moiety. The sugar moiety may include a mannose moiety. N-acetyl glucose, GlcNAc, fucose, or mannose may be useful for targeting macrophages when they target or bind a mannose receptor such as CD206. The sugar moiety may be useful for binding or targeting an asialoglycoprotein receptor such as an asialoglycoprotein receptor of a hepatocyte. The GalNAc moiety may bind to an asialoglycoprotein receptor. The GalNAc moiety may target a hepatocyte.

In some embodiments, the composition comprises an oligonucleotide that inhibits the expression of MST1, wherein the oligonucleotide comprises an N-acetylgalactosamine (GalNAc) moiety. GalNAc may be useful for hepatocyte targeting. The GalNAc moiety may include a bivalent or trivalent branched linker. The oligo may be attached to 1, 2 or 3 GalNAcs through a bivalent or trivalent branched linker. The GalNAc moiety may include 1, 2, 3, or more GalNAc molecules. The GalNAc moiety may be attached at a 3' or 5' terminus of the oligonucleotide.

In some embodiments. the composition comprises an oligonucleotide that inhibits the expression of MST1, wherein the oligonucleotide comprises an N-acetylgalactosamine (GalNAc) ligand for hepatocyte targeting. In some embodiments, the composition comprises GalNAc. In some embodiments, the composition comprises a GalNAc derivative. In some embodiments, the GalN Ac ligand is attached at a 3' terminus of the oligonucleotide. In some embodiments, the Gal NAc ligand is attached at a 5' terminus of the oligonucleotide. In some embodiments, the composition comprises a sense strand, and the GalNAc ligand is attached to the sense strand (e.g. attached to a 5'end of the sense strand, or attached to a 3'end of the sense strand). In some embodiments, the composition comprises an anti sense strand, and the GalNAc ligand is attached to the antisense strand (e.g attached to a 5' end of the anti sense strand, or attached to a 3' end of the antisense strand). In some embodiments, the composition comprises a GalNAc ligand attached at a 3' or 5' terminus of the oligonucleotide.

Disclosed herein, in some embodiments. are compositions comprising an oligonucleotide that inhibits the expression of MST1, wherein the oligonucleotide comprises a GalNAc moiety. The Gal NAc moiety may be included in any formula, structure, or GalNAc moiety shown below. In some embodiments, described herein is a compound (e.g. oligonucleotide) represented by Formula (I) or (II): 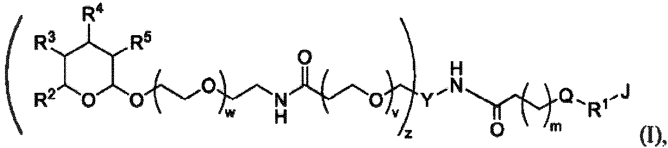 or 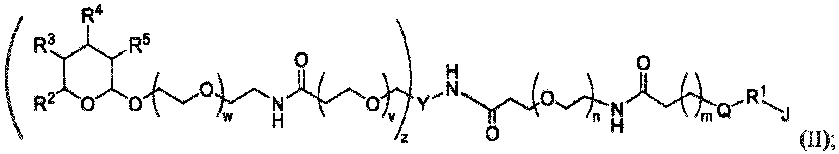 or a salt thereof, wherein
J is an oligonucleotide;
each w is independently selected from any value from 1 to 20;
each w is independently selected from any value from 1 to 20;
n is selected from any value from 1 to 20,
m is selected from any value from 1 to 20;
z is selected from any value from 1 to 3, wherein
   if zis 3, Yis C
   if zis 2, Y is CR⁶, or
   if zis 1, Y is C(R⁶)₂;
Q is selected from:
   C₃₋₁₀ carbocycle optionally substituted with one or more substituents independently selected from halogen, -CN, -NO₂, -OR⁷, -SR⁷, -N(R⁷)₂, -C(O)R⁷,-C(O)N(R⁷)₂,-N(R⁷)C(O)R⁷,-N(R⁷)C(O)N(R⁷)₂, -OC(O)N(R⁷)₂, -N(R⁷)C(O)OR⁷, -C(O)OR⁷, -OC(O)R⁷, -S(O)R⁷, and C₁₋₆ alkyl, wherein the C₁₋₆ alkyl, is optionally substituted with one or more substituents independently selected from halogen, -CN, -OH, -SH, -NO₂, and -NH₂;
R¹is a linker selected from:
   -O-,-S-,-N(R⁷)-, -C(O)N(R⁷)-, -C(O)N(R⁷)-,-N(R⁷)C(O)-,-N(R⁷)C(O)N(R⁷)-,-OC(O)N(R⁷)-,-N(R⁷)C(O)O-,-C(O)O-, -OC(O)-,-S(O)-, -S(O)₂-, -OS(O)₂, -OP(O)(OR⁷)O-,-SP(O)(OR⁷)O-,-OP(S)(OR⁷)O-,-OP(O)(SR⁷)O-,-OP(O)(OR⁷)S-, -OP(O)(O⁻)O-, -SP(O)(O⁻)O-, -OP(S)(O⁻)O-,-OP(O)(S⁻)O-,-OP(O)(O-)S-, -OP(O)(OR⁷)NR⁷-,-OP(O)(N(R⁷)₂₎NR⁷-, -OP(OR⁷)O-,-OP(N(R⁷)₂)O-, -OP(OR⁷)N(R⁷)-, and -OPN(R⁷)₂NR⁷-;
each R² is independently selected from:
   C₁₋₆ alkyl optionally substituted with one or more substituents independently selected from halogen, -OR⁷, -SR⁷, -N(R⁷)₂, -C(O)R⁷, -C(O)N(R⁷)₂,-N(R⁷)C(O)R⁷, -N(R⁷)C(O)N(R⁷)₂, - OC(O)N(R⁷)₂, -N(R⁷)C(O)OR⁷,-C(O)OR⁷,-OC(O)R⁷, and -S(O)R⁷;
R³ and R⁴ are each independently selected from:
   -OR⁷, -SR⁷, -N(R⁷)2, -C(O)R⁷, -C(O)N(R⁷)₂, -N(R⁷)C(O)R⁷-N(R⁷)C(O)N(R⁷)₂,-OC(O)N(R⁷)₂, -N(R⁷)C(O)OR⁷,-C(O)OR⁷, -OC(O)R⁷, and -S(O)R⁷;
each R⁵ is independently selected from:
   -OC(O)R⁷, -OC(O)N(R⁷)₂₋, -N(R⁷)C(O)R⁷-N(R⁷)C(O)N(R⁷)₂₋,-N(R⁷)C(O)OR⁷, -C(O)R⁷, -C(O)OR⁷, and -C(O)N(R⁷)₂;
each R⁶ is independently selected from:
   hydrogen;
   halogen, -CN, -NO₂, -OR⁷, -SR⁷, -N(R⁷)₂, -C(O)R⁷, -C(O)N(R⁷)₂,-N(R⁷)C(O)R⁷,-N(R⁷)C(O)N(R⁷)₂, -OC(O)N(R⁷)₂, -N(R⁷)C(O)OR⁷, -C(O)OR⁷, -OC(O)R⁷, and -S(O)R⁷; and
   C₁₋₆ alkyl optionally substituted with one or more substituents independently selected from halogen, -CN, -NO₂, -OR⁷, -SR⁷, -N(R⁷)₂, -C(O)R⁷, -C(O)N(R⁷)₂,-N(R⁷)C(O)R⁷-N(R⁷)C(O)N(R⁷)₂, -OC(O)N(R⁷)₂,-N(R⁷)C(O)OR⁷, -C(O)OR⁷, -OC(O)R⁷, and -S(O)R⁷;
each R⁷ is independently selected from: hydrogen:
   C₁₋₆ alkyl, C₂₋₆ alkenyl, and C₂₋₆ alkynyl, each of which is optionally substituted with one or more substituents independently selected from halogen, -CN. -OH, -SH, -NO₂, -NH₂, =O, =S,-O-C₁₋₆ alkyl, -S-C₁₋₆, alkyl, -N(C₁₋₆ alkyl)₂, -NH(C₁₋₆ alkyl), C₃₋₁₀ carbocycle, and 3- to 10-membered heterocycle; and
   C₃₋₁₀ carbocycle, and 3- to 10-membered heterocycle, each of which is optionally substituted with one or more substituents independently selected from halogen, -CN, -OH, -SH. - NO₂, -NH₂, =O, =S,-O-C₁₋₆ alkyl, -S-C₁₋₆alkyl, -N(C₁₋₆ alkyl)₂, -NH(C₁₋₆ alkyl), C₁₋₆ alkyl, C₂₋₆ alkenyl, C₂₋₆ alkynyl, C₃₋₁₀ carbocycle, 3- to 10-membered heterocycle, and C ₁₋₆haloalkyl.
In some embodiments, each w is independently selected from any value from 1 to 10. In some embodiments, each w is independently selected from any value from 1 to 5. In some embodiments, each w is 1. In some embodiments, each v is independently selected from any value from 1 to 10. In some embodiments, each vis independently selected from any value from 1 to5. In some embodiments, each v is 1. In some embodiments, n is selected from any value from 1 to 10. In some embodiments, n is selected from any value from 1 to 5. In some embodiments, n is 2. In some embodiments, mis selected from any value from 1 to 10. In some embodiments, m is selected from any value from 1 to 5. In some embodiments, m is selected from 1 and 2. In some embodiments, z is 3 and Y is C. In some embodiments, Q is selected from C₅₋₆ carbocycle optionally substituted with one or more substituents independently selected from halogen, -CN, -NO₂. -OR⁷, -SR⁷. -N(R⁷)₂, -C(O)R⁷, -C(O)N(R⁷)₂. -N(R⁷)C(O)R⁷.-N(R⁷)C(O)N(R⁷)₂, -OC(O)N(R⁷)₂, -N(R⁷)C(O)OR⁷, -C(O)OR⁷, -OC(O)R⁷, and -S(O)R⁷. In some embodiments, Q is selected from C₅₋₆ carbocycle optionally substituted with one or more substituents independently selected from halogen, -CN, -OH, -SH, -NO₂, and -NH₂. In some embodiments, Q is selected from phenyl and cyclohexyl, each of which is optionally substituted with one or more substituents independently selected from halogen. -CN, -OH, -SH, -NO₂, and -NH₂. In some embodiments, Q is selected from phenyl. In some embodiments, Q is selected from cyclohexyl. In some embodiments, R¹ is selected from -OP(O)(OR⁷)O-,-SP(O)(OR⁷)O-,-OP(S)(OR⁷)O-,-OP(O)(SR⁷)O-,-OP(O)(OR⁷)S-,-OP(O)(O-)O-, -SP(O)(O⁻)O-,-OP(S)(O)O-,-OP(O)(S⁻)O-,-OP(O)(O-)S-,-OP(O)(OR⁷)NR⁷-, -OP(O)(N(R⁷)₂)NR⁷-, -OP(OR⁷)O-,-OP(N(R⁷)₂)O-,-OP(OR⁷)N(R⁷)-, and -OPN(R⁷)₂₋NR⁷. In some embodiments, R¹ is selected from -OP(O)(OR¹)O-,-SP(O)(OR⁷)O-,-OP(S)(OR⁷)O-,-OP(O)(SR ⁷)O-,-OP(O)(OR⁷)S-,-OP(O)(O⁻)O-, -SP(O)(O⁻)O-, -OP(S)(O⁻}O-,-OP(O)(S-)O-, -OP(O)(O-)S-, and -OP(OR⁷)O-. In some embodiments, R^{I} is selected from -OP(O)(OR⁷)O-, -OP(S)(OR⁷)O-,-OP(O)(O-)O-,-OP(S)(O⁻)O-,-OP(O)(S-)O-,and -OP(OR⁷)O-. In some embodiments, R¹ is selected from-OP(O)(OR⁷)O- and -OP(OR⁷)O-. In some embodiments, R¹ is selected from C₁₋₃alkyl substituted with one or more substituents independently selected from halogen, -OR⁷, -OC(O)R¹, -SR⁷, -N(R⁷)₃. -C(·O)R⁷, and -S(O)R⁷. In some embodiments, R² is selected from C₁₋₃ alkyl substituted with one or more substituents independently selected from -OR⁷, -OC(O)R⁷, -SR⁷, and -N(R⁷)₂. In some embodiments, R² is selected from C₁₋₃ alkyl substituted with one or more substituents independently selected from -OR⁷ and-OC(O)R⁷, In some embodiments, R³ is selected from halogen, -OR⁷, -SR⁷, -N(R⁷)₂, -C(O)R⁷, -OC(O)R⁷, and -S(O)R⁷. In some embodiments, R³ is selected from -OR⁷ -SR⁷,-OC(O)R⁷, and -N(R⁷)₂. In some embodiments, R³ is selected from -OR⁷ - and -OC(O)R⁷. In some embodiments, R⁴ is selected from halogen, -OR⁷, -SR⁷, -N(R⁷)₂, -C(O)R⁷, -OC(O)R⁷, and -S(O)R⁷. In some embodiments, R⁴ is selected from -OR⁷ -SR⁷, -OC(O)R⁷, and -N(R⁷)₂ In some embodiments, R⁴ is selected from -OR⁷ - and -OC(O)R⁷. In some embodiments, R⁵ is selected from -OC(O)R⁷, -OC(O)N(R⁷)₂, -N(R⁷)C(O)R⁷,-N(R⁷)C(O)N(R⁷)₂. and -N(R⁷)C(O)OR⁷. In some embodiments, R⁵ is selected from -OC(O)R⁷ and -N(R⁷)C(O)R⁷. In some embodiments, each R⁷ is independently selected front hydrogen; and C₁₋₆ alkyl optionally substituted with one or more substituents independently selected from halogen, -CN, -OH, -SH, -NO₂, -NH₂, =O,=S,-O-C₁₋₆ alkyl, -S-C₁₋₆ alkyl -N(C₁₋₆ alkyl)₂, -NH(C₁₋₆ alkyl), C₃₋₁₀ carbocycle. or 3-to 10-membered heterocycle. In some embodiments, each R⁷ is independently selected from C₁₋₆ alkyl optionally substituted with one or more substituents independently selected from halogen, -CN, -OH, -SH, -NO₂, - NH₂, =O, =S, -O-C₁₋₆ alkyl, -S-C₁₋₆ alkyl, -N(C₁₋₆alkyl)₂, and -NH(C₁₋₆ alkyl). In some embodiments, each R⁷ is independently selected from C₁₋₆ alkyl optionally substituted with one or more substituents independently selected from halogen, -CN, -OH, and -SH. In some embodiments, w is 1; vis 1; n is 2; m is 1 or 2; zis 3 and Y is C; Qis phenyl or cyclohexyl, each of which is optionally substituted with one or more substituents independently selected from halogen, -CN, -OH, -SH, -NO₂, -NH₂, and C₁₋₃alkyl; R¹ is selected from -OP)(O)(OR⁷)O-, -OP(S)(OR⁷)O-, -OP(O)(O⁻)O-,-OP(S)(O⁻)O-, -OP(O)(S⁻)O-, and-OP(OR⁷)O-; R² is C₁ alkyl substituted with -OH or -OC(O)CH₃;
R³is -OH or -OC(O)CH₃; R⁴ is -OH or -OC(O)CH₃; and R⁵ is-NH(O)CH₃. In some embodiments, the compound comprises: 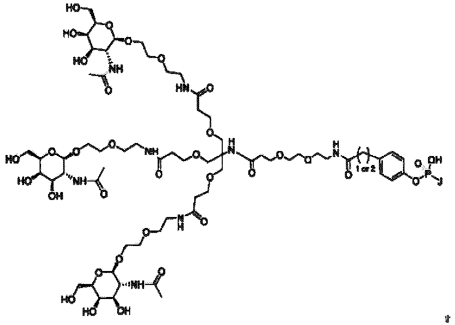 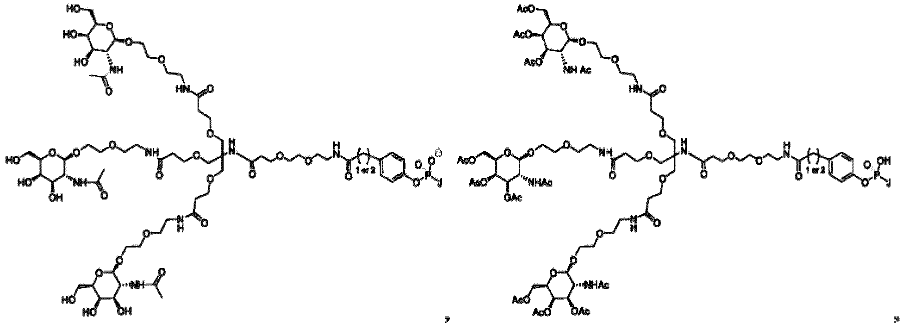 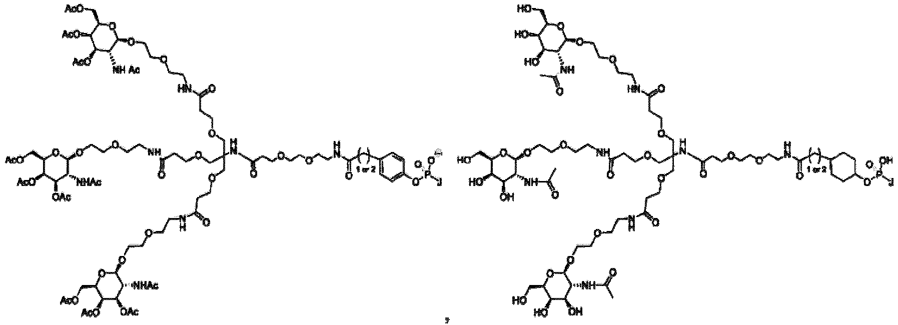 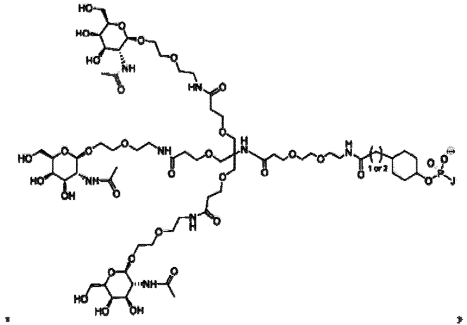 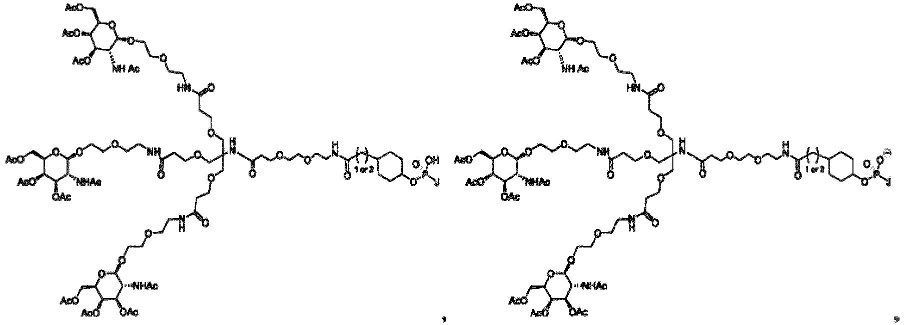 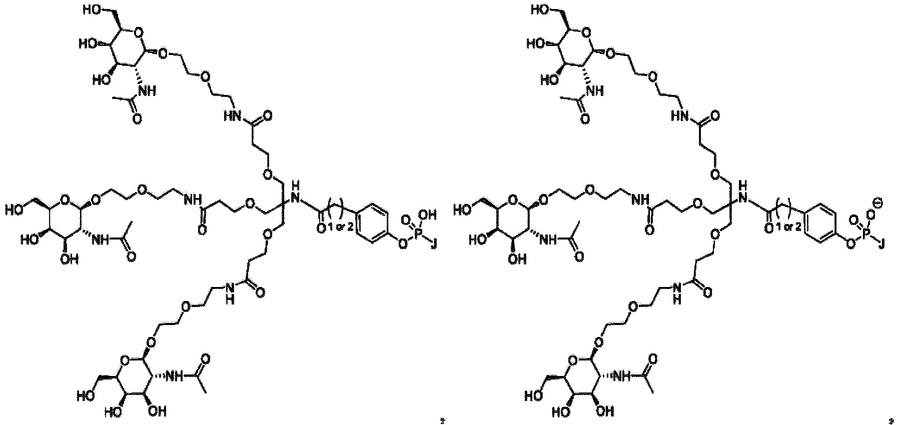 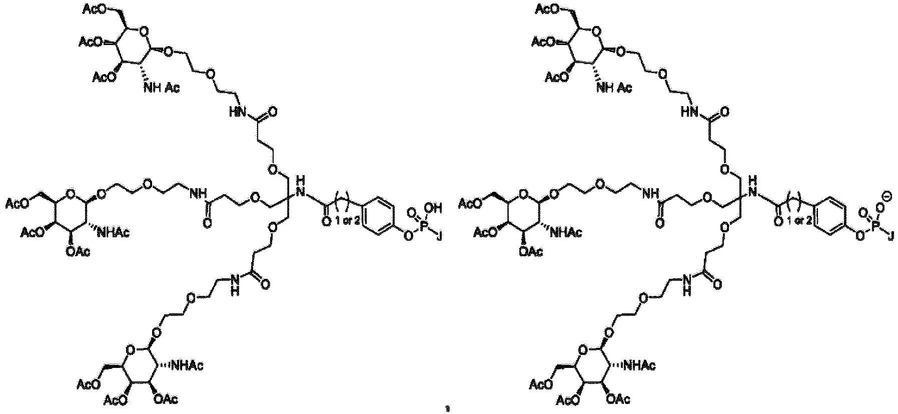 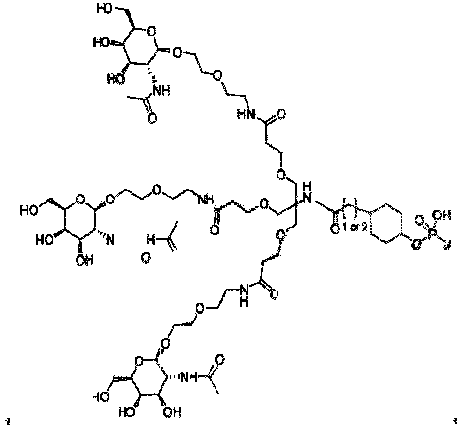 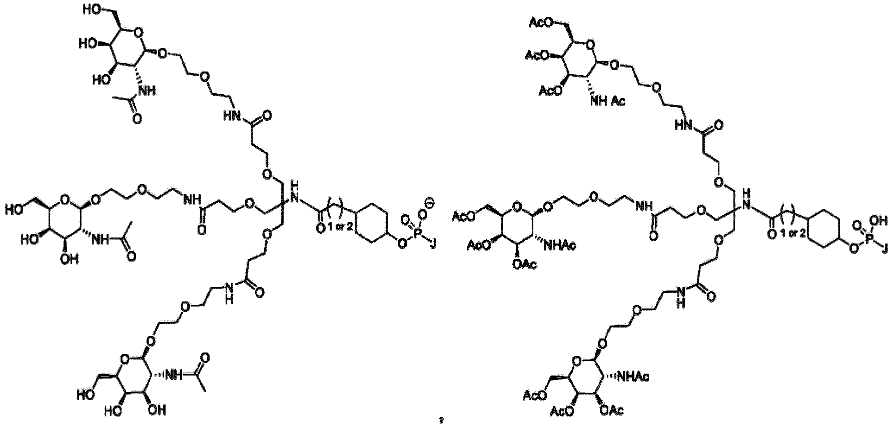 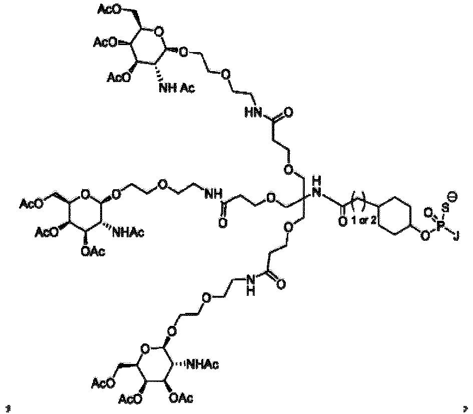 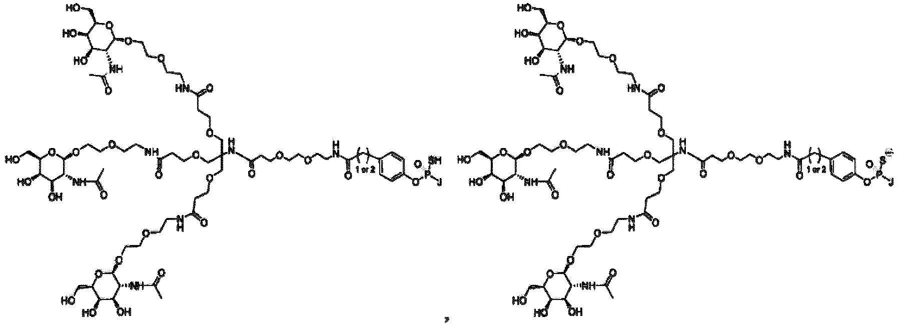 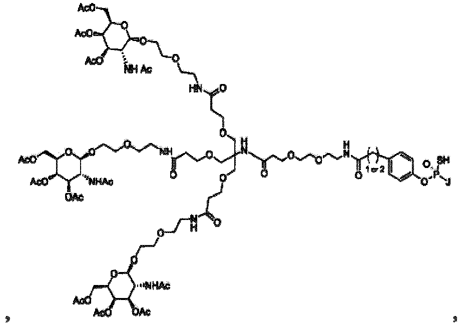 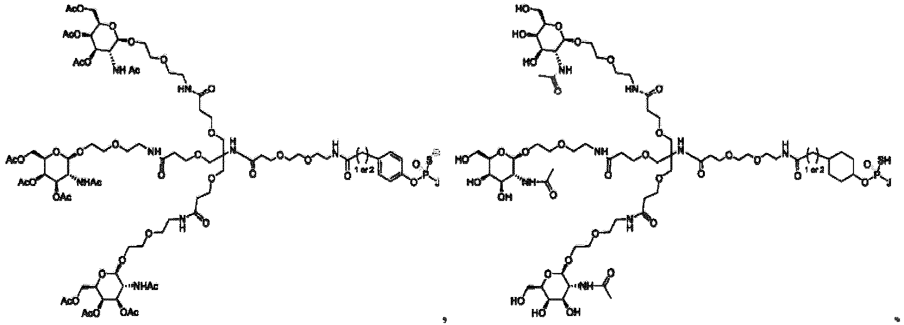 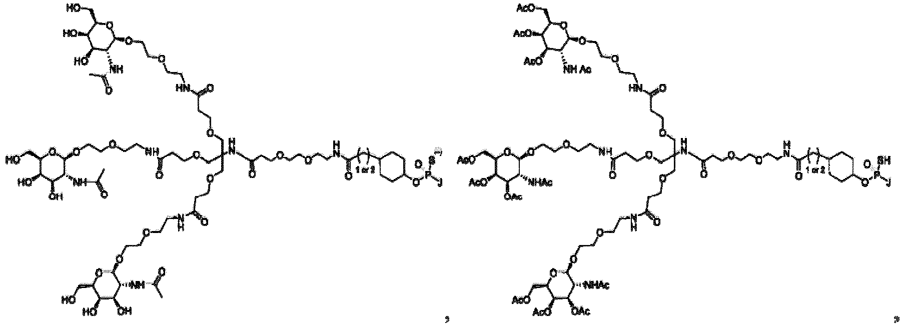 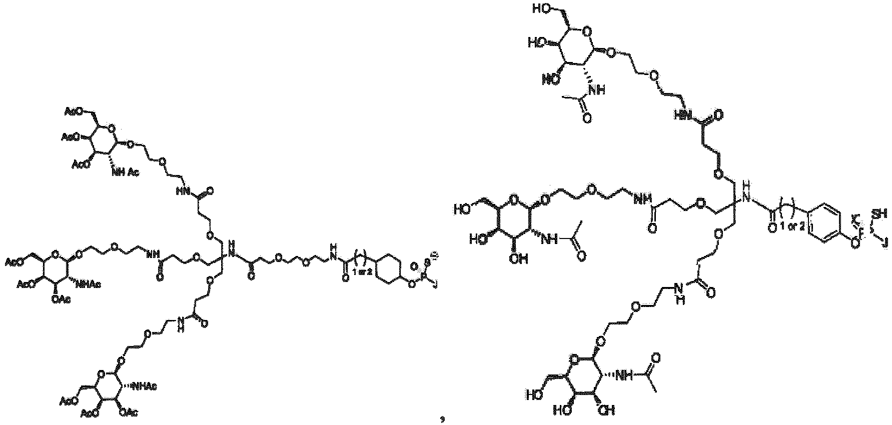 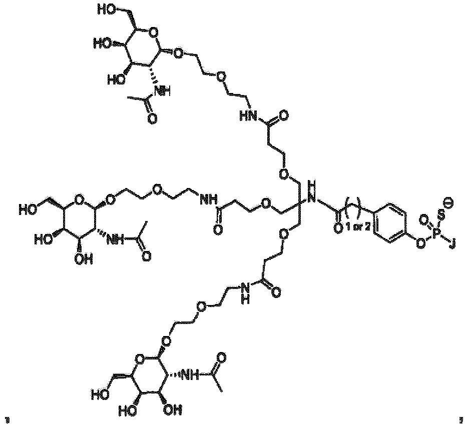 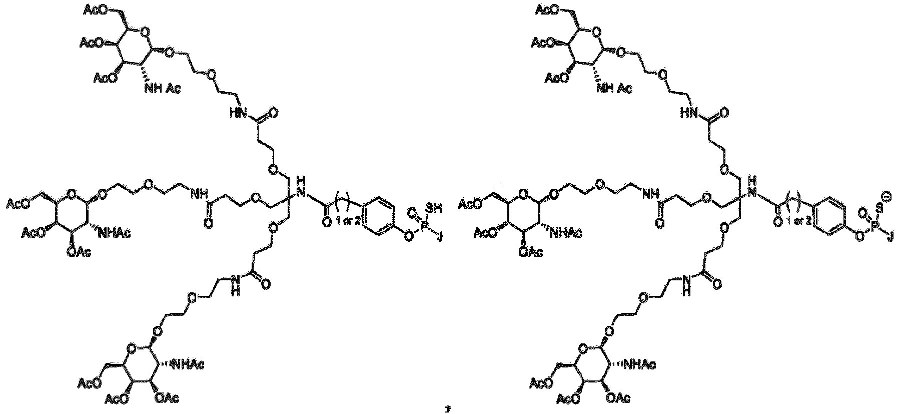 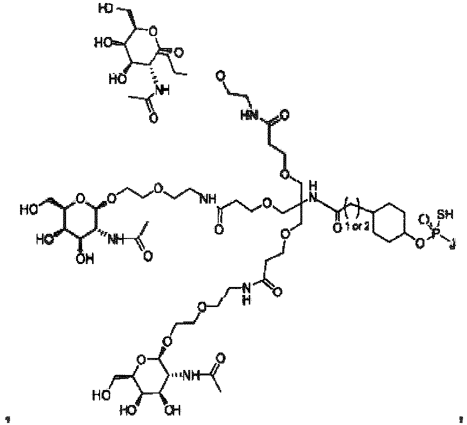 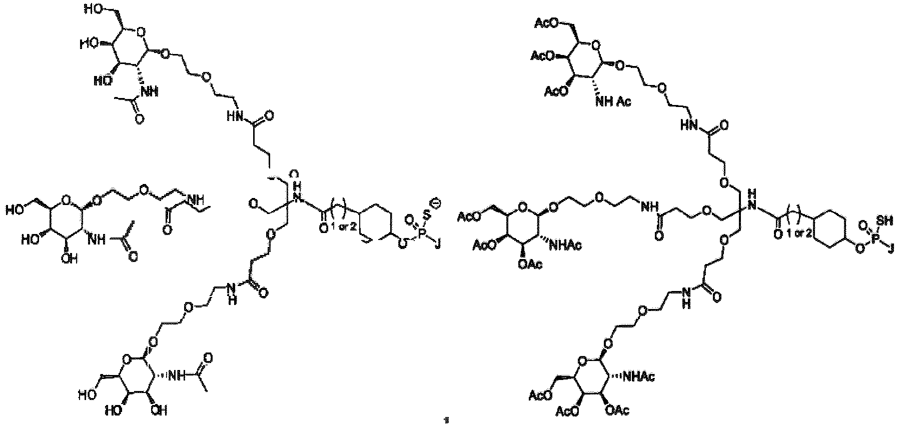 , or 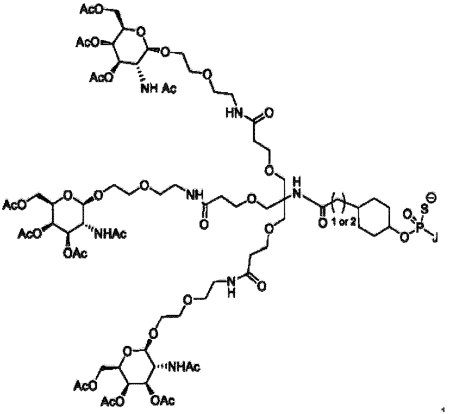
In some embodiments, the oligonucleotide (J) is attached a a5' end or a3' end of the oligonucleotide. In some embodiments, the oligonucleotide comprises DNA. In some embodiments, the oligonucleotide comprises RNA. In some embodiments, the oligonucleotide comprises one or more modified internucleoside link ages. In some embodiments, the one or more modifie d internucleoside linkages comprise alkylphosphonate, phosphorothioate, methylphosphonate, phosphorodithioate, alkyl phosphonothioale, phosphoramidate, carbamate, carbonate, phosphate triester, acetamidate, or carboxymethyl ester, or a combination thereof. In some embodiments, the oligonucleotide comprises 1, 2, 3, 4,5,6,7, 8,9, 10, 11, 12, 13, 14, 15, 16, 17, 18, 19, or 20 modified internucleosidelinkages. In some embodiments, the compound binds to an asialoglycoprotein receptor. In some embodiments, the compound targets a hepatocyte.

Some embodiments include the following, where J is the oligonucleotide: 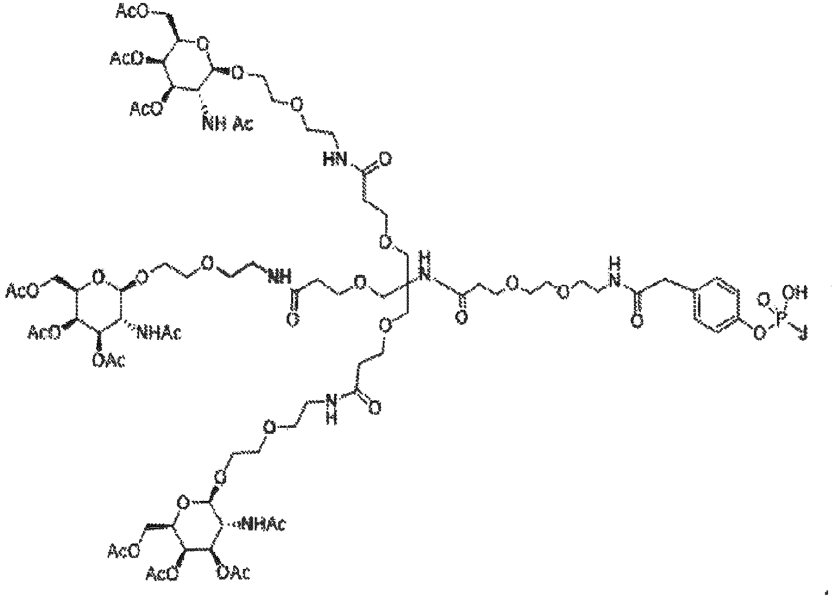 J may include one or more additional phosphates, or one or more phosphorothioates linking to the oligonucleotide. J may include one or more additional phosphates linking to the oligonucleotide. J may include one or more phosphorothioates linking to the oligonucleotide.

Some embodiments include the following, where J is the oligonucleotide: 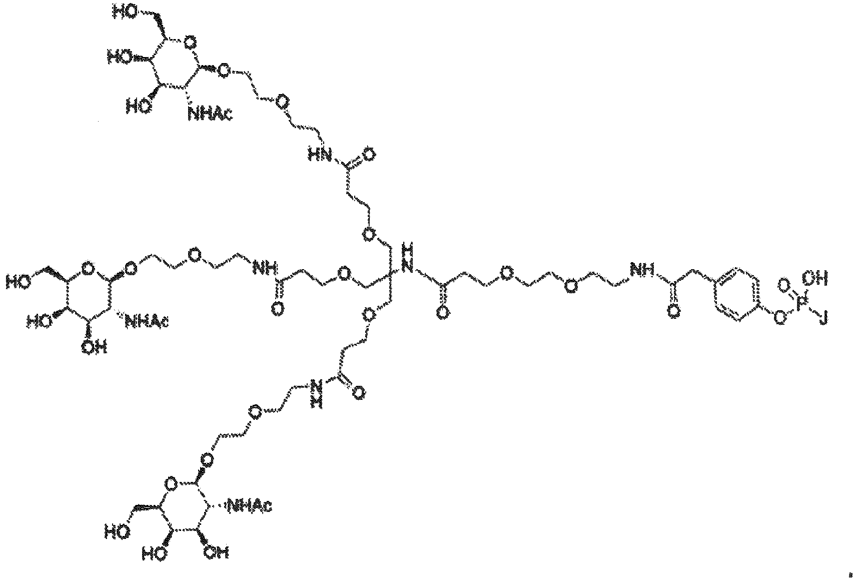 J may include one or more additional phosphates, or one or more phosphorothioates linking to the oligonucleotide. J may include one or more additional phosphates linking to the oligonucleotide. J may include one or more phosphorothioates linking to the oligonucleotide.

Some embodiments include the following, where J is the oligonucleotide: 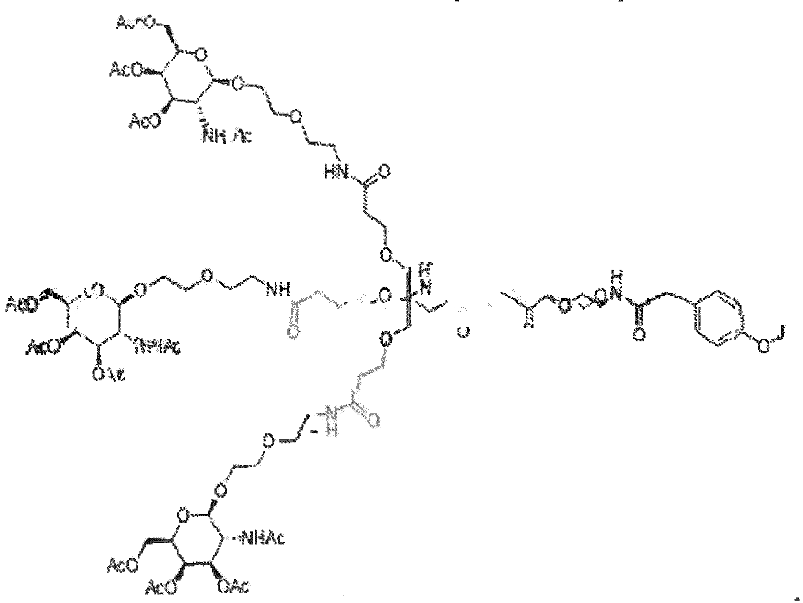 J may include one or more phosphates or phosphorothioates linking to the oligonucleotide. J may include one or more phosphates linking to the oligonucleotide. J may include a phosphate linking to the oligonucleotide. J may include one or more phosphorothioates linking to the oligonucleotide. J may include a phosphorothioate linking to the oligonucleotide.

Some embodiments include the following, where J is the oligonucleotide: 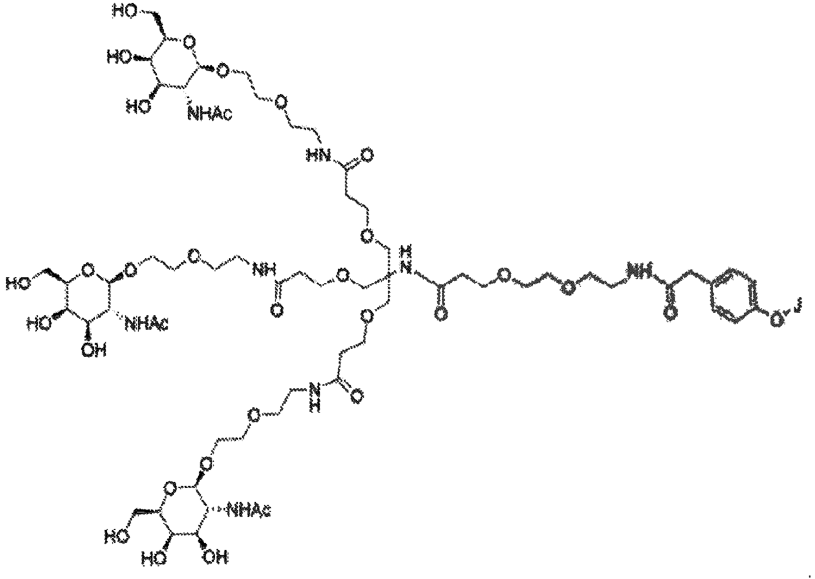 The structure in this compound attached to the oligonucleotide (J) may be referred to as "ETLI7," and is an example of a GalNAc moiety. J may include one or more phosphates or phosphorothioates linking to the oligonucleotide. J may include one or more phosphates linking to the oligonucleotide.J may include a phosphate linking to the oligonucleotide. J may include one or more phosphorothioates linking to the oligonucleotide. J may include a phosphorothioate linking to the oligonucleotide.

Some embodiments include the following, where the phosphate or "5'" indicates a connection to the oligonucleotide:

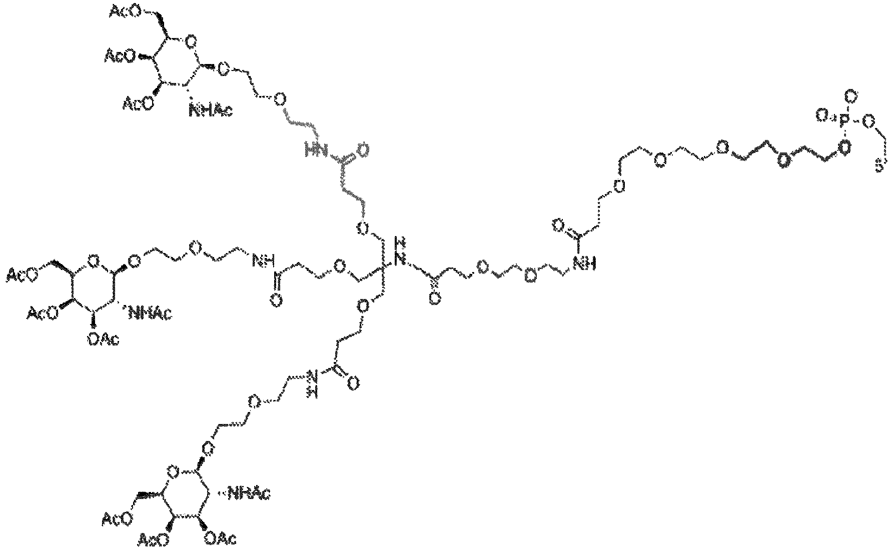

Some embodiments include the following, where the phosphate or "5'" indicates a connection to the oligonucleotide:

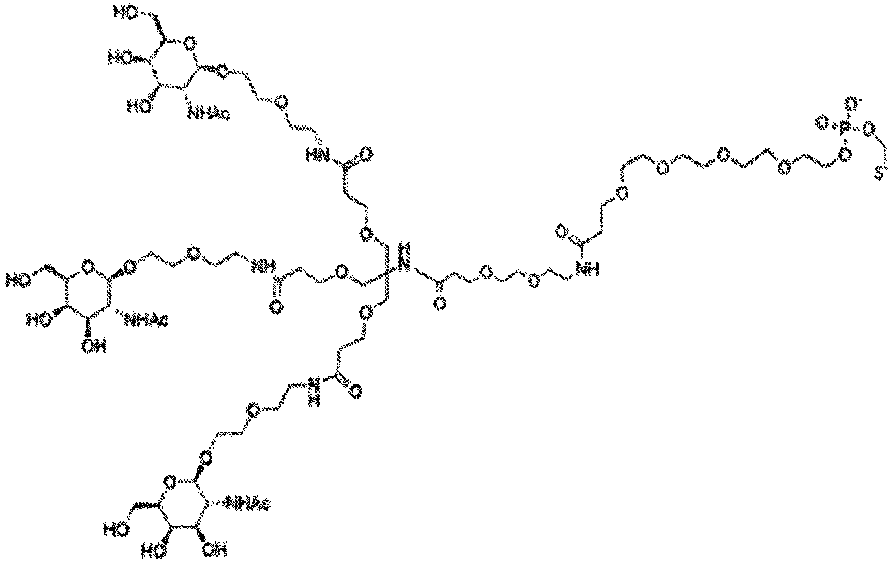

Some embodiments include the following, where J is the oligonucleotide: 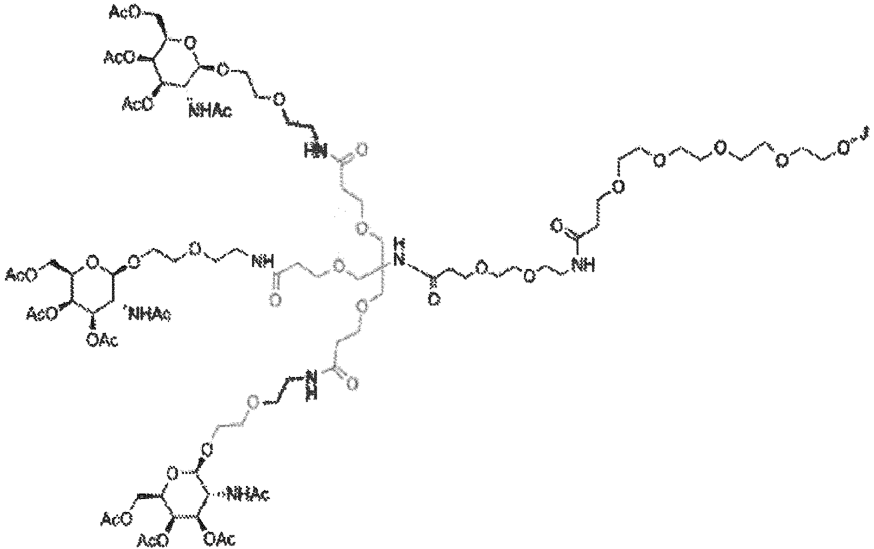 include one or more phosphates or phosphorothioates linking to the oligonucleotide.J may include one or more phosphates linking to the oligonucleotide. J may include a phosphate linking to the oligonucleotide. J may include one or more phosphorothioates linking to the oligonucleotide. J may include a phosphorothioate linking to the oligonucleotide.

Some embodiments include the following, where J is the oligonucleotide: 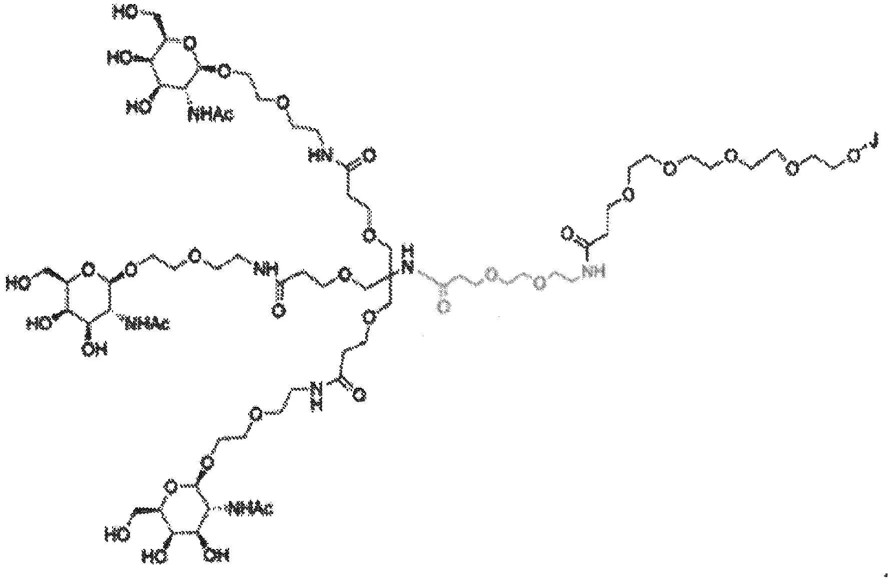 The structure in this compound attached to the oligonucleotide (J) may be referred to as "ETL1," and is an example of a GalNAc moiety. J may include one or more phosphates or phosphorothioates linking to the oligonucleotide.J may include one or more phosphates linking to the oligonucleotide.J may include a phosphate linking to the oligonucleotide. J may include one or more phosphorothioates linking to the oligonucleotide. J may include a phosphorothioate linking to the oligonucleotide.

### 3. siRNA modification patterns

In some embodiments, the composition comprises an oligonucleotide that inhibits the expression of *MST1,* wherein the oligonucleotide comprises an siRNA comprising a sense strand and an antisense strand, wherein the sense strand comprises modification pattern IS: 5'-NfsnsNfnNfnNfNfNfnNfnNfnNfnNfnNfsnsn-3' (SEQ ID NO: 6164), wherein "Nf" is a2' fluoro-modified nucleoside. '"n"is a2' O-methyl modified nucleoside, and "s"is a phosphorothioate linkage. In some embodiments, the sense strand comprises modification pattern 2S: 5'-nsnsnnNfnNfNfNfnnnnnmnnnsnsn-3' (SEQ ID NO: 6165), wherein "Nf" is a2' fluoro-modified nucleoside, "n"is a2' O-methyl modified nucleoside, and "s" is a phosphorothioate linkage. In some embodiments, the sense strand comprises modification pattern 3S: 5'-nsnsnnNfnNfnNfinnnnnmnnnsnsn-3'(SEQID NO: 6166), wherein "Nf" is a2' fluoro-modified nucleoside, "n"is a2' O-methyl modified nucleoside, and "s" is a phosphorothioate linkage. In some embodiments, the sense strand comprises modification pattern 4S: 5'-NfsnsNfnNfnNfNfNfnNFnNfnNfnNfnNfsnN-moiety-3' (SEQ ID NO: 6167), wherein "Nf" is a2' fluoro-modified nucleoside, "n"is a 2' O-methyl modified nucleoside, "s" is a phosphorothioate linkage, and N comprises one or more nucleosides. In some embodiments, the sense strand comprises modification pattern 5S: 5'-nNfNfNfnnnnnnnnnnsnsnN-moiety-3 (SEQ ID NO: 6168), wherein "Nf" is a2' fluoro-modified nucleoside. "n" is a2' O-methyl modified nucleoside, "s" is a phosphorothioate linkage, and N comprises one or more nucleosides. In some embodiments, the moiety in modification pattern 4S or 5S includes an integrin targeting ligand. In some embodiments, the moiety in modification pattern 4S or 5S is a sugar moiety. In some embodiments, the sense strand comprises modification pattern 6S: 5'-NfsnsNfnNfnNfnNfnNfnNfnNfnNfnNfsnsn-3' (SEQ ID NO: 6169), wherein "Nf" is a2' fluoro-modified nucleoside, "n" is a2' O-methyl modified nucleoside. and "s" is a phosphorothioate linkage. In some embodiments, the sense strand comprises modification pattern 7S: 5:nsnsnnNfNfNfNfNfnnnnnnnnnnsnsn-3' (SEQ ID NO: 6170), wherein "Nf'is a2' fluoro-modified nucleoside, "n"is a2' O-methyl modified nucleoside, and "s" is a phosphorothioate linkage. In some embodiments, the sense strand comprises modification pattern 8S: 5'-nsnsnnnNfNfNfNfnnnnnnnnnnsnsn-3'(SEQ ID NO: 6171), wherein "Nf" is a2" fluoro-modified nucleoside, "n"is a2' O-methyl modified nucleoside, and "s" is a phosphorothioate linkage. In some embodiments, the sense strand comprises modification pattern 9S: 5'-nsnsnnnnNNNNfnnnnnnnnnsnsn-3' (SEQ ID NO:6172), wherein "Nf"is a 2' fluoro-modified nucleoside, "n"is a2' O-methyl modified nucleoside, and "s"is a phosphorothioate linkage. In some embodiments, the sense strand comprises modification pattern 10S: 5'-snnnnNfnnnNfNfnnnnnnnnnsnsn-3' (SEQ ID NO: 6320), wherein "Nf" is a2' fluoro-modified nucleoside, "n"is a2' O-methyl modified nucleoside, and "s" is a phosphorothioate linkage. In some embodiments, the sense strand comprises modification pattern 11S: 5'-sNfnNfnNfnNfndNnNfnnnNfnNfnnsnsn-3' (SEQ ID NO: 6321), wherein "Nf" is a2' fluoro-modified nucleoside, "n"is a2' O-methyl modified nucleoside, and "s" is a phosphorothioate linkage. In some embodiments, the sense strand comprises modification pattern 12S: 5'nNfnnnnsnsn-3'(SEQ ID NO: 6322), wherein "Nf" is a 2' fluoro-modified nucleoside, "n''is a2' O-methyl modified nucleoside, ard "s" is a phosphorothioate linkage. In some embodiments, the sense strand comprises modification pattern 13S: 5'-snnnnNfNfnNfNfnNfnnnnnnnnsnsn-3' (SEQ ID NO: 6323), wherein "Nf" is a 2' fluoro-modified nucleoside, "n"is a2' O-methyl modified nucleoside, and "s"is a phosphorothioate linkage. In some embodiments, the sense strand comprises modification pattern 14S: 5*-snnnnnNfNfNfNfnnnnnnnnnsnsn-3'(SEQ ID NO: 6324), wherein "N" is a2' fluoro-modified nucleoside, "n"is a2' O-methyl modified nucleoside, and "s" is a phosphorothioate linkage. In some embodiments, the sense strand comprises modification pattern 15S: 5'snnnnNfnNfnNfnnnnnannnnsnsn-3' (SEQ ID NO: 6325), wherein "Nf" is a 2' fluoro-modified nucleoside, "n" is a 2' O-methyl modified nucleoside, and "s" is a phosphorothioate linkage. In some embodiments, the sense strand comprises modification pattern 16S: 5'-nsnsnnNfNfnNfNfnnnnnnnnnnsnsn-3'(SEQ ID NO: 6326), wherein "Nf" is a2' fluoro-modified nucleoside, "n"is a2' O-methyl modified nucleoside, and "s"is a phosphorothioate linkage. In some embodiments, the sense strand comprises modification pattern 17S: 5 -NfsnsNfnNfnNfndNnNfnnnNfnNfnnsnsn-3' (SEQ ID NO: 6327), wherein "Nf" is a 2' fluoro-modified nucleoside, "n"is a2' O-methyl modified nucleoside, and "s" is a phosphorothioate linkage. In some embodiments, the sense strand comprises modification pattern 18S: 5:nsnsnnnnNfNfNfNfNftnnnnnnnnsnsn-3' (SEQ ID NO 6328), wherein "Nf"is a2' fluoro-modified nucleoside, "n"is a2' O-methyl modified nucleoside, and "s" is a phosphorothioate linkage. In some embodiments, the sense strand comprises modification pattern 19S: 5' -nsnsnnnNtNfNfNtNfnnnnnnnnnsnsn-3' (SEQ ID NO: 6329), wherein "Nf"is a2' fluoro-modified nucleoside, "n"is a2' O-methyl modified nucleoside, and "s" is a phosphorothioate linkage. In some embodiments, the sense strand comprises modification pattern 20S: 5'-snnnnnNfnNfNfnnnnnnnnsnsn-3'(SEQ ID NO: 6330), wherein "Nf" is a2' fluoro-modified nucleoside, "n"is a2' O-methyl modified nucleoside, and "s" is a phosphorothioate linkage. In some embodiments, the sense strand comprises modification pattern 21S: 5'-snnnnnnNfNfNfNfNfnnnnnnnnsnsn-3' (SEQ ID NO: 6331), wherein "Nf"' is a 2' fluoro-modified nucleoside, "n"is a2' O-methyl modified nucleoside, and "s" is a phosphorothioate linkage. In some embodiments, the sense strand comprises modification pattern 22S: 5'-snnnnNfNfnnNfnnnnnnnnnnsnsn-3' (SEQ ID NO: 6332), wherein "Nf"is a2' fluoro-modified nucleoside, "n"is a2' O-methyl modified nucleoside, and "s'" is a phosphorothioate linkage. In some embodiments, the sense strand comprises modification pattern 23S; 5'-snnnnNfNfnNfNfnnnnnnnnnnsnsn-3'(SEQ ID NO: 6333), wherein "Nf" is a2' fluoro-modified nucleoside, "n"is a2' O-methyl modified nucleoside, and "s" is a phosphorothioate linkage. In some embodiments, the sense strand comprises modification pattern 24S: 5'-snnnnnNfNfnNfNfnnnnnnnnnsnsn-3' (SEQ ID NO: 6334), wherein "Nf" is a 2' fluoro-modified nucleoside, "n"is a2' O-methyl modified nucleoside, and "s" is a phosphorothioate linkage. In some embodiments, the sense strand comprises modification pattern 25S: 5'-snnnnnnNfnNfnNfnnnmnnnsnsn-3'(SEQ ID NO: 6335), wherein "Nf" is a2' fluoro-modified nucleoside, "n"is a2' O-methyl modified nucleoside, and "s" is a phosphorothioate linkage. In some embodiments, the sense strand comprises modification pattern 26S: 5'-snnnnnNfnnNfnNfnnnnnnnnsnsn-3'(SEQ ID NO: 6336), wherein "Nf' is a2' fluoro-modified nucleoside, "n"is a2' O-methyl modified nucleoside, and "s" is a phosphorothioate linkage. In some embodiments, the sense strand comprises modification pattern 27S: 5'-snnnnNfnnNfNfNfNfnnnnnnnnsnsn-3' (SEQ ID NO: 6337), wherein "Nf"is a 2' fluoro-modified nucleoside, "n"is a2' O-methyl modified nucleoside, and "s" is a phosphorothioate linkage. In some embodiments, the sense strand comprises modification pattern 28S: 5'-snnnnnNfNfNfNfNfnnnnnnnnnnsnsn-3 (SEQ ID NO: 6338), wherein "Nf" is a2' fluoro-modified nucleoside, "n" is a2' O-methyl modified nucleoside, and "s" is a phosphorothioate linkage. In some embodiments, the sense strand comprises modification pattern 29S: 5'-snnnnnNfnnNfNfnnnnnnnnnsns-3'(SEQ ID NO: 6339), wherein "Nf" is a2⁻ fluoro-modified nucleoside, "n" is a2' O-methyl modified nucleoside, and "s" is a phosphorothioate linkage. In some embodiments, the sense strand comprises modification pattern 30S: 5'-snnnnnnNfnNfNfnnnnnnnnnsnsn-3' (SEQ ID NO: 6340), wherein "Nf" is a 2' fluoro-modified nucleoside, "n" is a2' O-methyl modified nucleoside, and "s" is a phosphorothioate linkage. In some embodiments, the sense strand comprises modification pattern 31s: 5:snnnnNfNfnnNfNfnnnnnnnnnsnsn-3' (SEQ ID NO: 6341), wherein "Nf" is a2⁻ fluoro-modified nucleoside, "n"is a2' O-methyl modified nucleoside, and "s" is a phosphorothioate linkage. In some embodiments, the sense strand comprises modification pattern 32S: 5'-snnnnNfnnNfNfnnnnnnnnnsnsn-3' (SEQ ID NO: 6342), wherein "Nf" is a2' fluoro-modified nucleoside, "n"is a2' O-methyl modified nucleoside, and "'s" is a phosphorothioate linkage. In some embodiments, the sense strand comprises modification pattern 33S: 5'-snnnnNfndNnNfnNfnnnnnnnnsnsn-3'(SEQ ID NO: 6343), wherein "'Nf" is a 2' fluoro-modified nucleoside, "n"is a2' O-methyl modified nucleoside, and "s" is a phosphorothioate linkage. In some embodiments, the sense strand comprises modification pattern 34S: 5'-snnnnnnnnNfdNNfnnnnnnnsnsn-3' (SEQ ID NO: 6344), wherein "Nf" is a2' fluoro-modified nucleoside, "n"is a2' O-methyl modified nucleoside, and "'s" is a phosphorothioate linkage. In some embodiments, the sense strand comprises modification pattern 35S: 5'-snnnnNfnnnNfnNfnnnnnnnnsnsn-3'(SEQ ID NO: 6319), wherein "Nf" is a2' fluoro-modified nucleoside, "n" is a2' O-methyl modified nucleoside, and "s" is a phosphorothioate linkage.

In some embodiments, the composition comprises an oligonucleotide that inhibits the expression of *MST1,* wherein the oligonucleotide comprises an siRNA comprising a sense strand and an antisense strand, wherein the antisense strand comprises modification pattern 1AS: 5'-nsNfsnNfnNfnNfnNfnnnNfnNfnNfnsnsn-3' (SEQ ID NO: 6173), wherein "Nf" is a2' fluoro-modified nucleoside, "n"is a2' O-methyl modified nucleoside, and "s" is a phosphorothioate linkage. In some embodiments, the antisense strand comprises modification pattern 2AS: 5'-nsNfsnnnNfnNfNfnnnnNfnNfnnnsnsn-3' (SEQ ID NO: 6174), wherein "Nf" is a2' fluoro-modified nucleoside, "n"is a2' O-methyl modified nucleoside, and "s" is a phosphorothioate linkage. In some embodiments, the antisense strand comprises modification pattern 3AS: 5'-nsNfsnnnNfnnnnnnnNfnNfnnnsnsn-3'(SEQ ID NO: 6175), wherein "Nf" is a2' fluoro-modifi ed nucleoside, "n" is a 2' O-methyl modified nucleoside, and "s" is a phosphorothioate linkage. In some embodiments, the anti sense strand comprises modi fication pattern 4AS: 5:nsNfsnNfnNfnnnnnnnNfnNfnnnsnsn-3' (SEQ ID NO: 6176), wherein "Nf" is a2' fluoro-modified nucleoside, "n" is a2' O-methyl modified nucleoside, and "s" is a phosphorothioate linkage. In some embodiments, the antisense strand comprises modification pattern 5AS: 5'-nsNfsnnnnnnnnnnnNfnNfnmsnsn-3' (SEQ ID NO: 6177), wherein "Nf" is a 2' fluoro-modified nucleoside, "n"is a2' O-methyl modified nucleoside, and "s" is a phosphorothioate linkage. In some embodiments, the antisense strand comprises modification pattern 6AS: 5"-nsNfsnnnNfnnNfnnnnNfnNfnnnsnsn-3' (SEQ ID NO. 6178), wherein "Nf" is a2' fluoro-modified nucleoside, "n"is a2' O-methyl modified nucleoside, and "s" is a phosphorothioate linkage. In some embodiments, the antisense strand comprises modification pattern 7AS: 5'-nsNfsnNfnNfnNfnNfnNfnNfnNfnNfnsnsn-3' (SEQ ID NO: 6179), wherein "Nf"is a2' fluoro-modified nucleoside, "n" is a2' O-methyl modified nucleoside, and "s" is a phosphorothioate linkage. In some embodiments, the antisense strand comprises modification pattern 8AS: 5'-nsNfsnnnnnnnnnn nNfnnnnn snsn-3'(SEQ ID NO: 6180), wherein "Nf" is a2' fluoro-modified nucleoside, "n"is a2' O-methyl modified nucleoside, and "s" is a phosphorothioate linkage. In some embodiments, the anti sense strand comprises modification pattern 9AS: 5'-nsNfsnNfnnnNfnnnNfnNfnNfnNfnsnsn-3'(SEQ ID NO: 6345), wherein "Nf" is a2' fluoro-modified nucleoside, "n"is a2' O-methyl modified nucleoside, and "s" is a phosphorothioate linkage. In some embodiments, the antisense strand comprises modification pattern 10AS: 5'-nsNfsnNfnNfnnnNfnNfnNfnNfNfnsnsn-3' (SEQ ID NO: 6346), wherein "Nf" is a 2' fluoro-modified nucleoside, "n"is a2' O-methyl modified nucleoside, and "s" is a phosphorothioate linkage. In some embodiments, the antisensestrand comprises modification pattern 11AS: 5'-nsNfsnNfnNfnnnnnNfnNfnNfnNfnsnsn-3⁻(SEQ ID NO: 6347), wherein "Nf" is a 2' fluoro-modifi ed nucleoside, "n "is a2' O-methyl modified nucleoside, and "'s" is a phosphorothioate linkage. In some embodiments, the antisense strand comprises modification pattern I2As: 5'-nsNfsnNfnnNfNfnNfnNfnNfnNfnNfnsnsn-3' (SEQ ID NO: 6348), wherein "Nf" is a2' fluoro-modified nucleoside, "n"is a2' O-methyl modified nucleoside, and "s" is a phosphorothioate linkage. In some embodiments, the antisense strand comprises modification pattern 13AS: 5'-nsNfsnNfnnnNfnNfnNfnNfnNfnNfnsnsn-3' (SEQ ID NO: 6349). wherein "Nf" is a2' fluoro-modified nucleoside, "n"is a2' O-methyl modified nucleoside, and "s" is a phosphorothioate linkage. In some embodiments, the anti sense strand comprises modification pattern 14AS: 5'-nsNfsnnNfnNfnnNfaNfnNfnNfnNfnsnsn-3' (SEQ ID NO: 6350), wherein "Nf" is a2' fluoro-modified nucleoside, "n"is a2' O-methyl modified nucleoside, and "s" is a phosphorothioate linkage. In some embodiments, the antisense strand comprises modification pattern 15AS: 5'-nsNfsnnnnNfnnNfnNfnnNfnNfnNfnsnsn-3' (SEQ ID NO: 6351), wherein "Nf"is a 2' fluoro-modified nucleoside, "n" is a2' O-methyl modified nucleoside, and "s" is a phosphorothioate linkage. In some embodiments, the antisense strand comprises modification pattern 16AS: 5°-nsNfsnnnNfnnnNfnNfnNfnNfnNfnsnsn-3'(SEQ ID NO: 6352), wherein "Nf" is a2' fluoro-modified nucleoside, "n"is a 2' O-methyl modified nucleoside, and "s" is a phosphorothioate linkage. In some embodiments, the antisense strand comprises modification pattern 17AS: 5'-nsNfsnNfnnNfnnNfnNfNfnNfnNfrmsn-3' (SEQ ID NO: 6353), wherein "Nf" is a2' fluoro-modified nucleoside, "n"is a2' O-methyl modified nucleoside, and "s" is a phosphorothioate linkage. In some embodiments, the antisense strand comprises modification pattern 18AS: 5'-nsNfsnnnNfnNfnNfnNfNfnNfNfnsnsn-3' (SEQ ID NO: 6354), wherein "Nf" is a 2' fluoro-modified nucleoside, "n" is a2' O-methyl modified nucleoside, and "s" is a phosphorothioate link age. In some embodiments, the antisense strand comprises modification pattern 19AS: 5'-nsNfsnNfnNfnNfnnnNfnNfnNfnNfnsnsn-3' (SEQ ID NO: 6355), wherein "Nf" is a2' fluoro-modified nucleoside, "n"is a2' O-methyl modified nucleoside, and "s" is a phosphorothioate linkage. In some embodiments, the antisense strand comprises modification pattern 20AS: 5'-nsNfsnnnNfnNfnnnNfnNmNfnNfnsnsn-3'(SEQ ID NO: 6356), wherein "Nf" is a 2' fluoro-modified nucleoside, "n"is a2' O-methyl modified nucleoside, and "s"is a phosphorothioate linkage. In some embodiments, the anti sense strand comprises modification pattern 21AS: 5'-nsNfsnnNfnnnnNfnNfnNfnNfnsnsn-3'(SEQ ID NO: 6357), wherein "Nf" is a2' fluoro-modified nucleoside, "n"is a2' O-methyl modified nucleoside, and *s" is a phosphorothioate linkage.

In some embodiments, the composition comprises an oligonucleotide that inhibits the expression of *MST1,* wherein the oligonucleotide comprises an siRNA comprising a sense strand and an antisense strand, wherein the sense strand comprises pattern 1S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21 AS. In some embodiments, the sense strand comprises pattern 2S and the antisense strand comprises pattern 1AS, 2AS, 3AS, 4AS, 5AS, 6AS, 7AS. 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 3S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 4S and the antisense strand comprises pattern 1AS. 2AS, 3AS, 4AS, 5AS, 6AS. 7AS, 8AS, 9AS, 10AS, 11AS, 12AS, 13AS, 14AS, 15AS, 16AS, 17AS, 18AS. 19AS. 20AS, or 21 AS. In some embodiments. the sense strand comprises pattern 5S and the antisense strand comprises pattern 1AS, 2AS, 3AS, 4AS, SAS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 6S and the antisense strand comprises pattern 1AS, 2AS, 3AS, 4AS, 5AS, 6AS, 7AS, 8AS, 9AS, 10AS, 11AS, 12AS, 13AS. 14AS, 15AS, 16AS, 17AS, 18AS, 19AS, 20AS, or 21AS. In some embodiments, the sense strand comprises pattern 7S and the anti sense strand comprises pattern 1AS, 2AS, 3AS, 4AS, 5AS, 6AS, 7AS, 8AS, 9AS, 10AS, 11AS, 12AS, 13AS, 14AS. 15AS, 16AS, 17AS, 18AS, 19AS, 20AS, or 21AS. In some embodiments, the sense strand comprises pattern 8S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 9S and the antisense strand comprises pattern 1AS, 2AS, 3AS, 4AS, 5AS, 6AS, 7AS, 8AS, 9AS, 10AS. 11AS, 12AS, 13AS, 14AS, 15AS, 16AS, 17AS, 18AS, 19AS, 20AS, or 21AS. In some embodiments, the sense strand comprises pattern 10S and the anti sense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 11S and the antisense strand comprises pattern 1AS. 2AS, 3AS, 4AS, 5AS, 6AS, 7AS, SAS, 9AS, 10AS, 11AS, 12AS, 13AS, 14AS. 15AS, 16AS, 17AS, 18AS. 19AS,20AS. or 21AS. In some embodiments, the sense strand comprises pattern 12S and the antisense strand comprises pattern 1AS, 2AS. 3AS, 4AS, 5AS, 6AS. 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 13S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 14S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21 AS. In some embodiments, the sense strand comprises pattern 15S and the antisense strand comprises pattern 1AS, 2AS, 3AS. 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 16S and the anti sense strand comprises pattern 1AS, 2AS, 3AS, 4AS. 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 17S and the antisense strand comprises pattern 1AS, 2AS, 3AS, 4AS, 5AS, 6AS, 7AS, 8AS. 9AS, 10AS, 11AS, 12AS, 13AS, 14AS, 15AS, 16AS, 17AS, 18AS, 19AS, 20AS, or 21AS. In some embodiments, the sense strand comprises pattern 18S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS. or 21AS. In some embodiments, the sense strand comprises pattern 19S and the antisense strand comprises pattern 1AS, 2AS, 3AS, 4AS, SAS, 6AS, 7AS, 8AS, 9AS, 10AS, 11AS, 12AS. 13AS, 14AS, 15AS, 16AS, 17AS, 18AS, 19AS, 20AS, or 21AS. In some embodiments, the sense strand comprises pattern 205 and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 21S and the antisense strand comprises pattern 1AS, 2AS, 3AS, 4AS, 5AS, 6AS. 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 22S and the anti sense strand comprises pattern 1AS, 2AS, 3AS, 4AS, SAS, 6AS. 7AS. 8AS, 9AS, 10AS, 11AS, 12AS, 13AS, 14AS, 15AS, 16AS, 17AS,. 18AS, 19AS, 20AS, or 21AS. In some embodiments, the sense strand comprises pattern 23S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 24S and the antisense strand comprises pattern 1 AS, 2AS, 3AS, 4AS, 5AS, 6AS, 7AS, 8AS, 9AS, 10AS, 11AS, 12AS. 13AS, 14AS, 15AS, 16AS, 17AS, 18AS, 19AS, 20AS, or 21AS. In some embodiments, the sense strand comprises pattern 25S and the anti sense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 26S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 27S and the antisense strand comprises pattern 1AS, 2AS, 3AS, 4AS, 5AS, 6AS. 7AS, 8AS, 9AS, 10AS, 11AS, 12AS, 13AS. 14AS, 15AS, 16AS, 17AS, 18AS, 19AS, 20AS, or 21AS. In some embodiments, the sense strand comprises pattern 28S and the antisense strand comprises pattern 1AS, 2AS, 3AS, 4AS, 5AS, 6AS, 7AS, 8AS, 9AS, 10AS, 11AS, 12AS, 13AS, 14AS. 15AS, 16AS, 17AS, 18AS, 19AS, 20AS, or 21 AS. In some embodiments, the sense strand comprises pattern 29S and the antisense strand comprises pattern 1AS, 2AS, 3AS, 4AS, 5AS, 6AS, 7AS. 8AS, 9AS, 10AS, 11AS, 12AS. 13AS, 14AS, 15AS, 16AS, 17AS, 18AS, 19AS, 20AS, or 21AS. In some embodiments, the sense strand comprises pattern 30S and the antisense strand comprises pattern 1AS, 2AS, 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 31S and the antisense strand comprises pattern 1AS, 2AS, 3AS, 4AS. SAS, 6AS, 7AS, 8AS, 9AS, 10AS, 11AS, 12AS, 13AS, 14AS, 15AS, 16AS, 17AS, 18AS, 19AS, 20AS, or 21 AS. In some embodiments, the sense strand comprises pattern 32S and the antisense strand comprises pattern 1AS, 2AS, 3AS, 4AS, SAS, 6AS, 7AS, 8AS, 9AS, 10AS, 11AS, 12AS, 13AS, 14AS, 15AS, 16AS. 17AS, 18AS, 19AS, 20AS, or 21AS. In some embodiments, the sense strand comprises pattern 33S and the antisense strand comprises pattern 1AS, 2AS, 3AS, 4AS, SAS, 6AS, 7AS, 8AS, 9AS, 10AS, 11AS, 12AS, 13AS, 14AS, 15AS, 16AS, 17AS, 18AS, 19AS, 20AS, or 21AS. In some embodiments, the sense strand comprises pattern 34S and the antisense strand comprises pattern 1AS, 2AS, 3AS, 4AS, SAS, 6AS, 7AS, 8AS, 9AS, 10AS, 11AS, 12AS, 13AS, 14AS, 15AS, 16AS, 17AS, 18AS, 19AS, 20AS, or 21 AS. In some embodiments, the sense strand comprises pattern 35S and the antisense strand comprises pattern 1AS, 2AS, 3AS, 4AS, SAS, 6AS, 7AS, 8AS, 9AS, 10AS, 11AS. 12AS, 13AS, 14AS, 15AS, 16AS, 17AS, 18AS, 19AS, 20AS, or 21AS.

In some embodiments, the sense strand comprises pattern 1S, 2S, 3S, 4S, 5S,6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 1AS. In some embodiments, the sense strand comprises pattern 1S, 2S, 3S. 4S, 5S, 6S, 7S, 8S, 9S, 10S, 11S, 12S, 13S, 14S, 15S. 16S, 17S, 18S, 19S, 20S, 21S, 22S, 23S, 24S, 25S, 26S, 27S, 28S, 29S. 30S, 31S, 32S, 33S, 34S, or 35S and the anti sense strand comprises pattern 2AS. In some embodiments, the sense strand comprises pattern 1S, 2S, 3S, 4S, 5S, 6S, 7S, 8S, 9S, 10S, 11S, 12S, 13S,. 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 3AS. In some embodiments, the sense strand comprises pattern 1S, 2S, 3S, 4S, 5S, 6S, 7S, 8S, 9S. 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 4AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 5AS. In some embodiments, the sense strand comprises pattern 1S, 2S, 3S, 4S, 5S, 6S, 7S, 8S, 95, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 6AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S. 20S, 21S, 22S, 23S, 24S, 25S, 26S, 27S, 28S, 29S, 30S, 31S, 32S, 33S, 34S, or 35S and the antisense strand comprises pattern 7AS. In some embodiments, the sense strand comprises pattern 1S, 2S, 3S, 4S, 5S, 6S, 7S, 8S, 9S, 10S, 11S, 12S. 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 8AS. In some embodiments, the sense strand comprises pattern 1S, 2S, 3S, 4S, 5S, 6S, 7S, 8S. 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 9AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the anti sense strand comprises pattern 10AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the anti sense strand comprises pattern 11AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 12AS. In some embodiments, the sense strand comprises pattern 1S, 2S, 3S, 4S, 5S, 6S, 7S, 8S. 9S. 10S, 11S, 12S, 13S, 14S, 15S, 16S, 17S, 18S, 19S, 20S, 21S, 22S, 23S, 24S, 25S, 26S, 27S, 28S, 298, 30S. 31S, 32S, 33S, 34S, or 35S and the antisense strand comprises pattern 13AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S. 20S, 21S, 22S, 23S, 24S, 25S, 26S, 27S. 28S, 29S, 30S, 31S, 32S, 33S, 34S, or 35S and the antisense strand comprises pattern 14AS. In some embodiments, the sense strand comprises pattern 1S, 2S, 3S, 4S, 5S, 6S, 7S, 8S, 9S, 10S, 11S, 12S. 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 15AS. In some embodiments, the sense strand comprises pattern 1S, 2S, 3S, 4S, 5S, 6S, 7S, 8S, 9S, 10S, 11S, 12S, 13S, 14S, 15S, 16S, 17S. 18S, 19S, 20S, 21S, 22S, 23S, 24S, 25S, 26S, 27S, 28S, 29S, 30S, 31S, 32S, 33S, 34S, or 35S and the antisense strand comprises pattern 16AS. In some embodiments, the sense strand comprises pattern 1S, 2S, 3S, 4S, 5S, 6S, 7S,8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 17AS. In some embodiments, the sense strand comprises pattern JS, 2S, 3S, 4S, 5S, 6S, 7S, 8S, 9S, 10S, 11S, 12S, 13S, 14S, 15S, 16S, 17S, 18S, 19S, 20S, 21S, 22S. 23S, 24S, 25S, 26S, 27S, 28S, 29S, 30S, 31S, 32S, 33S, 34S, or 35S and the antisense strand comprises pattern 18AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S, or 35S and the antisense strand comprises pattern 19AS. In some embodiments, the sense strand comprises pattern 1S, 2S, 3S, 4S, 5S. 6S, 7S, 8S, 9S, 10S, 11S, 12S, 13S, 14S, 15S. 16S, 17S, 18S, 19S. 20S, 21S, 22S, 23S, 24S, 25S, 26S, 27S, 28S, 29S, 30S, 31S, 32S, 33S, 34S, or 35S and the antisense strand comprises pattern 20AS. In some embodiments, the sense strand comprises pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S. or 35S and the antisense strand comprises pattern 21AS.

In some embodiments, the sense strand comprises modification pattern 1AS, 2AS. 3AS, 4AS, 5AS, 6AS, 7AS, 8AS, 9AS, 10AS, 11AS, 12AS, 13AS, 14AS, 15AS, 16AS, 17AS, 18AS, 19AS, 20AS, or 21 AS. In some embodiments, the antisense strand comprises modification pattern 1S, 2S, 3S, 4S, 5S, 6S, 7S, 8S, 9S, 10S, 11S, 12S, 13S, 14S, 15S, 16S, 17S, 18S, 19S, 20S, 21S, 22S, 23S, 24S, 25S, 26S, 27S, 28S, 29S, 30S, 31S, 32S, 33S, 34S. or 35S. In some embodiments, the sense strand or the antisense strand comprises modification pattern ASO1.

In some embodiments, purines of the sense strand comprise 2' fluoro modified purines. In some embodiments, purines of the sense strand comprise 2' methyl modified purines. In some embodiments, purines of the sense strand comprise a mixture of 2' fluoro and 2' methyl modified purines. In some embodiments, all purines of the sense strand comprise 2' fluoro modified purines. In some embodiments, all purines of the sense strand comprise 2' methyl modified purines. In some embodiments, all purines of the sense strand comprise a mixture of 2' fluoro and 2' methyl modified purines.

In some embodiments, pyrimidines of the sense strand comprise 2' fluoro modified pyrimidines. In some embodiments, pyrimidines of the sense strand comprise2' methyl modified pyrimidines. In some embodiments, pyrimidines of the sense strand comprise a mixture of 2' fluoro and 2' methyl modified pyrimidines. In some embodiments, all pyrimidines of the sense strand comprise 2' fluoro modified pyrimidines. In some embodiments, all pyrimidines of the sense strand comprise 2' methyl modified pyrimidines. In some embodiments, all pyrimidines of the sense strand comprise a mixture of 2' fluoro and 2' methyl modified pyrimidines.

In some embodiments, purines of the sense strand comprise 2' fluoro modified purines, and pyrimidines of the sense strand comprise a mixture of 2' fluoro and 2' methyl modified pyrimidines. In some embodiments, purines of the sense strand comprise 2' methyl modified purines, and pyrimidines of the sense strand comprise a mixture of 2' fluoro and 2' methyl modified pyrimidines. In some embodiments, purines of the sense strand comprise 2' fluoro modified purines, and pyrimidines of the sense strand comprise 2' methyl modified pyrimidines. In some embodiments, purines of the sense strand comprise 2' methyl modified purines, and pyrimidines of the sense strand comprise 2' fluoro modified pyrimidines. In some embodiments, pyrimidines of the sense strand comprise 2' fluoro modified pyrimidines, and purines of the sense strand comprise a mixture of 2' fluoro and 2' methyl modified purines. In some embodiments, pyrimidines of the sense strand comprise 2' methyl modified pyrimidines, and purines of the sense strand comprise a mixture of 2' fluoro and 2' methyl modified purines. In some embodiments, pyrimidines of the sense strand comprise 2' fluoro modified pyrimidines, and purines of the sense strand comprise 2' methyl modified purines. In some embodiments, pyrimidines of the sense strand comprise 2' methyl modified pyrimidines, and purines of the sense strand comprise 2' fluoro modified purines.

In some embodiments, al purines of the sense strand comprise 2' fluoro modified purines, and all pyrimidines of the sense strand comprise a mixture of 2' fluoro and 2' methyl modified pyrimidines. In some embodiments, all purines of the sense strand comprise 2⁻ methyl modified purines, and all pyrimidines of the sense strand comprise a mixture of 2' fluoro and 2' methyl modified pyrimidines. In some embodiments, all purines of the sense strand comprise 2' fluoro modified purines, and all pyrimidines of the sense strand comprise 2' methyl modified pyrimidines. In some embodiments, all purines of the sense strand comprise 2' methyl modified purines, and all pyrimidines of the sense strand comprise 2' fluoro modified pyrimidines. In some embodiments, all pyrimidines of the sense strand comprise 2' fluoro modified pyrimidines, and all purines of the sense strand comprise a mixture of 2' fluoro and 2' methyl modified purines. In some embodiments, all pyrimidines of the sense strand comprise 2' methyl modified pyrimidines, and all purines of the sense strand comprise a mixture of 2' fluoro and 2' methyl modified purines. In some embodiments, all pyrimidines of the sense strand comprise 2' fluoro modified pyrimidines, and all purines of the sense strand comprise 2' methyl modified purines. In some embodiments, all pyrimidines of the sense strand comprise 2' methyl modified pyrimidines, and all purines of the sense strand comprise 2⁻ fluoro modified purines.

In some embodiments, purines of the antisense strand comprise 2' fluoro modified purines. In some embodiments, purines of the antisense strand comprise 2' methyl modified purines. In some embodiments, purines of the anti sense strand comprise a mixture of 2' fluoro and 2' methyl modified purines. In some embodiments, all purines of the antisense strand comprise 2' fluoro modified purines. In some embodiments, all purines of the antisense strand comprise 2' methyl modified purines. In some embodiments, all purines of the antisense strand comprise a mixture of 2' fluoro and 2' methyl modified purines.

In some embodiments, pyrimidines of the antisense strand comprise 2' fluoro modified pyrimidines. In some embodiments, pyrimidines of the antise nse strand comprise 2' methyl modified pyrimidines, In some embodiments, pyrimidines of the antisense strand comprise a mixture of 2' fluoro and 2' methyl modified pyrimidines. In some embodiments, all pyrimidines of the antisense strand comprise 2' fluoro modified pyrimidines. In some embodiments, all pyrimidines of the antisense strand comprise 2' methyl modified pyrimidines. In some embodiments, all pyrimidines of the antisense strand comprise a mixture of 2' fluoro and 2' methyl modified pyrimidines.

In some embodiments, purines of the antisense strand comprise 2' fluoro modified purines, and pyrimidines of the antisense strand comprise a mixture of 2⁻ fluoro and 2' methyl modified pyrimidines. In some embodiments, purines of the antisense strand comprise 2' methyl modified purines, and pyrimidines of the antisense strand comprise a mixture of 2' fluoro and 2' methyl modified pyrimidines. In some embodiments, purines of the antisense strand comprise 2' fluoro modified purines, and pyrimidines of the antisense strand comprise 2' methyl modified pyrimidines. In some embodiments, purines of the antisense strand comprise 2' methyl modified purines, and pyrimidines of the anti sense strand comprise 2' fluoro modified pyrimidines. In some embodiments, pyrimidines of the antisense strand comprise 2' fluoro modified pyrimidines, and purines of the antisense strand comprise a mixture of 2' fluoro and 2' methyl modified purines. In some embodiments, pyrimidines of the antisense strand comprise 2⁻ methyl modified pyrimidines, and purines of the antisense strand comprise a mixture of 2' fluoro and 2⁻ methyl modified purines. In some embodiments, pyrimidines of the antisense strand comprise 2' fluoro modified pyrimidines, and purines of the antisense strand comprise 2' methyl modified purines. In some embodiments, pyrimidines of the antisense strand comprise 2' methyl modified pyrimidines, and purines of the antisense strand comprise 2' fluoro modified purines.

In some embodiments, all purines of the antisense strand comprise 2' fluoro modified purines, and all pyrimidines of the antisense strand comprise a mixture of 2' fluoro ard 2' methyl modified pyrimidines. In some embodiments, all purines of the antisense strand comprise 2' methyl modified purines, and all pyrimidines of the antisense strand comprise a mixture of 2' fluoro and 2" methyl modified pyrimidines. In some embodiments, all purines of the antisense strand comprise 2' fluoro modified purines, and all pyrimidines of the antisense strand comprise 2' methyl modified pyrimidines. In some embodiments, all purines of the antisense strand comprise 2' methyl modified purines, and all pyrimidines of the antisensestrand comprise 2' fluoro modified pyrimidines. In some embodiments, all pyrimidines of the antisense strand comprise 2' fluoro modified pyrimidine s, and all purines of the antisense strand comprise a mixture of 2' fluoro and 2' methyl modified purines. In some embodiments, all pyrimidines of the antisense strand comprise 2' methyl modified pyrimidines, and all purines of the antisense strand comprise a mixture of 2' fluoro and 2' methyl modified purines. In some embodiments, all pyrimidines of the antisense strand comprise 2' fluoro modified pyrimidines, and all purines of the antisense strand comprise 2' methyl modified purines. In some embodiments, all pyrimidines of the antisense strand comprise 2' methyl modified pyrimidines, and all purines of the antisense strand comprise 2' fluoro modified purines.

Disclosed herein, in some embodiments are compositions comprising an oligonucleotide that targets *MST1* and when administered to a cell decreases expression of *MST1*, wherein the oligonucleotide comprises a small interfering RNA (siRNA) comprising a sense strand and an antisense strand, wherein the sense strand comprises a sense strand sequence described herein in which at least one internucleoside linkage is modified and at least one nucleoside is modified, or an sense strand sequence comprising 1 or 2 nucleoside substitutions, additions, or deletions of the oligonucleotide sequence in which at least one internucleoside linkage is modified and at least one nucleoside is modified, and wherein the antisense strand comprises an anti sense strand sequence described herein in which at least one internucleoside linkage is modified and at least one nucleoside is modified, or an oligonucleotide sequence comprising 1 or 2 nucleoside substitutions, additions, or deletions of the anti sense strand sequence in which at least one internucleoside linkage is modified and at least one nucleoside is modified. Some embodiments relate to methods that include administering the composition to a subject.

In some embodiments, the siRNA comprises the sense strand and/or the antisense strand sequence of an siRNA in **Table 9,** or a nucleic acid sequence thereof having 3 or 4 nucleoside substitutions, additions, or deletions. In some embodiments, the siRNA comprises the sense strand and/or the antisense strand sequence of an siRNA in **Table 9,** or a nucleic acid sequence thereof having 1 or 2 nucleoside substitutions, additions, or deletions. In some embodiments, the siRNA comprises the sense strand and/or the antisense strand sequence of an siRNA in **Table 9.** The siRNA may include the same internucleoside linkage modifications or nucleoside modifications as those in **Table 9.** The siRNA may include any different internucleoside link age modifications or nucleoside modifications different from those in **Table 9.** The siRNA may include some unmodified internucleoside linkages or nucleosides.

In some embodiments, the siRNA comprises the sense strand and/or the antisense strand sequence of an siRNA in **Table 10,** or a nucleic acid sequence thereof having 3 or 4 nucleoside substitutions, additions, or deletions. In some embodiments, the siRNA comprises the sense strand and/or the antisense strand sequence of an siRNA in **Table 10,** or a nucleic acid sequence thereof having 1 or 2 nucleoside substitutions, additions, or deletions. In some embodiments, the siRNA comprises the sense strand and/or the antisense strand sequence of an siRNA in Table 10. The siRNA may include the same internucleoside linkage modifications or nucleoside modifications as those in **Table 10**. The siRNA may include any different internucleoside linkage modifications or nucleoside modifications different from those in **Table 10.** The siRNA may include some unmodified internucleoside linkages or nucleosides.

In some embodiments, the siRNA comprises the sense strand and/or the antisense strand sequence of an siRNA in **Table 24A,** or a nucleic acid sequence thereof having 3 or 4 nucleoside substitutions, additions, or deletions. In some embodiments, the siRNA comprises the sense strand and/or the antisense strand sequence of an siRNA in **Table 24A,** or a nucleic acid sequence thereof having 1 or 2 nucleoside substitutions, additions, or deletions. In some embodiments, the siRNA comprises the sense strand and/or the antisense strand sequence of an siRNA in **Table 24A.** ThesiRNA may include the same internucleoside linkage modifications or nucleoside modifications as those in **Table 24A.** The siRNA may include any different internucleoside linkage modifications or nucleoside modifications different from those in **Table 24A.** The siRNA may include some unmodified internucleoside linkages or nucleosid es.

In some embodiments, the siRNA comprises the sense strand and/or the antisense strand sequence of an siRNA in **Table 24C,** or a nucleic acid sequence thereof having 3 or 4 nucleoside substitutions, additions, or deletions. In some embodiments, the siRNA comprises the sense strand and/or the antisense strand sequence of an siRNA in **Table 24C,** or a nucleic acid sequence thereof having 1 or **2** moleoside substitutions, additions, or deletions. In some embodiments, the siRNA comprises the sense strand and/or the antisense strand sequence of an siRNA in **Table 24C.** The siRNA may include the same internucleoside link age modifications or nucleoside modifications as those in **Table 24C.** The siRNA may include any different internucleoside linkage modifications or nucleoside modifications different from those in **Table 24C**. The siRNA may include some unmodified internucleoside linkages or nucleosides.

In some embodiments, the siRNA comprises the sense strand and/or the antisense strand sequence of an siRNA in **Table 30,** or a nucleic acid sequence thereof having 3 or 4 nucleoside substitutions, additions, or deletions. In some embodiments, the siRNA comprises the sense strand and/or the anti sense strand sequence of an siRNA in **Table 30,** or a nucleic acid sequence thereof having 1 or 2 nucleoside substitutions, additions, or deletions. In some embodiments, the siRNA comprises the sense strand and/or the antisense strand sequence of an siRNA in **Table 30.** the siRNA may include the same internucleoside link age modifications or nucleoside modifications as those in **Table 30.** The siRNA may include any different internucleoside linkage modifications or nucleoside modifications different from those in **Table 30.** The siRNA may include some unmodified internucleoside linkages or nucleosides.

The siRNA may comprises the sense strand and/or the anti sense strand sequence of an siRNA in any table included herein that includes modifications; or may include a nucleic acid sequence thereof having 1 or 2nucleoside substitutions, additions, or deletions; or may include a nucleic acid sequence thereof having 3 or 4 nucleoside substitutions, additions, or deletions.

In some embodiments, the siRNA comprises a sense strand having a sequence in accordance with SEQ ID NO: 6208. In some embodiments, the sense strand sequence comprises or consists of sequence at least 75% identical to SEQ ID NO: 6208, at least 80% identical to SEQ ID NO: 6208, at least 85% identical to SEQ ID NO: 6208, at least 90% identical to SEQ ID NO: 6208, or at least 95% identical to SEQ ID NO: 6208. In some embodiments, the sense strand sequence comprises or consists of the sequence of SEQ ID NO 6208, or a sense strand sequence thereof having 1, 2, 3, or 4 nucleoside substitutions, additions, or deletions. In some embodiments, the sense strand sequence comprises or consists of the sequence of SEQ ID NO: 6208, or a sense stand sequence thereof having 1 or 2nucleoside substitutions, additions, or deletions. In some embodiments, the sense strand sequence comprises or consists of a sequence 100% identical to SEQ ID NO: 6208. The sense strand may comprise a moiety such as a GalNAc moiety or a lipid moiety. In some embodiments, the siRNA comprises an antisense strand having a sequence in accordance with SEQ ID NO: 6267. In some embodiments, the antisense strand sequence comprises or consists of sequence at least 75% identical to SEQ ID NO: 6267, at least 80% identical to SEQ ID NO: 6267, at least 85% identical to SEQ ID NO: 6267, a least 90% identical to SEQ ID NO: 6267, or at least 95% identical to SEQ ID NO: 6267. In some embodiments, the antisense strand sequence comprises or consists of the sequence of SEQ ID NO 6267, or an antisense strand sequence thereof having 1,2, 3, or 4 nucleoside substitutions, additions, or deletions. In some embodiments, the antisense strand sequence comprises or consists of the sequence of SEQ ID NO: 6267, or an antisense strand sequence thereof having 1 or 2 nucleoside substitutions, additions, or deletions. In some embodiments, the antisense strand sequence comprises or consists of a sequence 100% identical to SEQ ID NO: 6267. The antisense strand may comprise a moiety such as a GalNAc moiety or a lipid moiety.

In some embodiments, the siRNA comprises a sense strand having a sequence in accordance with SEQ ID NO: 6214. In some embodiments, the sense strand sequence comprises or consists of sequence at least 75% identical to SEQ ID NO: 6214, at least 80% identical to SEQ ID NO: 6214, at least 85% identical to SEQ ID NO: 6214, at least 90% identical to SEQ ID NO: 6214, or at least 95% identical to SEQ ID NO: 6214. In some embodiments, the sense strand sequence comprises or consists of the sequence of SEQ ID NO 6214, or a sense strand sequence thereof having 1, 2, 3, or 4 nucleoside substitutions, additions, or deletions. In some embodiments, the sense strand sequence comprises or consists of the sequence of SEQ ID NO: 6214, or a sense strand sequence thereof having 1 or 2 nucleoside substitutions, additions, or deletions. In some embodiments, the sense strand sequence comprises or consists of a sequence 100% identical to SEQ ID NO: 6214. The sense strand may comprise a moiety such as a GalNAc moiety or a lipid moiety. In some embodiments. the siRNA comprises an antisense strand having a sequence in accordance with SEQ ID NO: 6273. In some embodiments, the antisense strand sequence comprises or consists of sequence at least 75% identical to SEQ ID NO: 6273, at least 80% identical to SEQ ID NO: 6273, at least 85% identical to SEQ ID NO: 6273, al least 90% identical to SEQ ID NO: 6273, or at least 95% identical to SEQ ID NO: 6273. In some embodiments, the antisense strand sequence comprises or consists of the sequence of SEQ ID NO 6273, or an antisense strand sequence thereof having 1, 2, 3, or 4 nucleoside substitutions, additions, or deletions. In some embodiments, the antisensestrand sequence comprises or consists of the sequence of SEQ ID NO: 6273, or an anti sense strand sequence thereof having 1 or 2nucleoside substitutions, additions, or deletions. In some embodiments, the anti sense strand sequence comprises or consists of a sequence 100% identical to SEQ ID NO: 6273. The antisense strand may comprise a moiety such as a GalNAc moiety or a lipid moiety.

In some embodiments, the siRNA comprises a sense strand having a sequence in accordance with SEQ ID NO: 6215. In some embodiments, the sense strand sequence comprises or consists of sequence at least 75% identical to SEQ ID NO: 6215, at least 80% identical to SEQ ID NO: 6215, at least 85% identical to SEQ IDNO: 6215, at least 90% identical to SEQ ID NO: 6215, or at least 95% identical to SEQ ID NO: 6215. In some embodiments, the sense strand sequence comprises or consists of the sequence of SEQ ID NO 6215, or a sense strand sequence thereof having 1, 2, 3, or 4 nucleoside substitutions, additions, or deletions. In some embodiments, the sense strand sequence comprises or consists of the sequence of SEQ ID NO 6215, or a sense strand sequence thereof having 1 or 2 nucleoside substitutions, additions, or deletions. In some embodiments, the sense strand sequence comprises or consists of a sequence 100% identical to SEQ ID NO: 6215. The sense strand may comprise a moiety such as a GalNAc moiety or a lipid moiety. In some embodiments, the siRNA comprises an antisense strand having a sequence in accordance with SEQ ID NO: 6274. In some embodiments, the antisense strand sequence comprises or consists of sequence at least 75% identical to SEQ ID NO: 6274, at least 80% identical to SEQ ID NO: 6274, at least 85% identical to SEQ ID NO: 6274, at least 90% identical to SEQ ID NO:6274, or at least 95% identical to SEQ ID NO: 6274. In some embodiments, the antisense strand sequence comprises or consists of the sequence of SEQ ID NO 6274, or an anti sense strand sequence thereof having I, 2, 3, or 4 nucleoside substitutions, additions, or deletions. In some embodiments, the antisense strand sequence comprises or consists of the sequence of SEQ ID NO: 6274, or an antisense strand sequence thereof having 1 or 2 nucleosidesubstitutions, additions, or deletions. In some embodiments, the antisense strand sequence comprises or consists of a sequence 100% identical to SEQ ID NO: 6274. The antisense strand may comprise a moiety such as a GalNAc moiety or a lipid moiety.

In some embodiments, the siRNA comprises a sense strand having a sequence in accordance with SEQ ID NO: 6216. In some embodiments, the sense strand sequence comprises or consists of sequence at least 75% identical to SEQ ID NO: 6216, at least 80% identical to SEQ ID NO: 6216, at least 85% identical to SEQ ID NO: 6216, at least 90% identical to SEQ ID NO: 6216, or at least 95% identical to SEQ ID NO: 6216. In some embodiments, the sense strand sequence comprises or consists of the sequence of SEQ ID NO 6216, or a sense strand sequence thereof having 1, 2, 3, or 4 nucleoside substitutions, additions, or deletions. In some embodiments, the sense strand sequence comprises or consists of the sequence of SEQ ID NO: 6216, or a sense strand sequence thereof having 1 or 2nucleos ide substitutions, additions, or deletions. In some embodiments, the sense strand sequence comprises or consists of a sequence 100% identical to SEQ ID NO: 6216. The sense strand may comprise a moiety such as a GalNAc moiety or a lipid moiety. In some embodiments, the siRNA comprises an antisense strand having a sequence in accordance with SEQ ID NO:6275. In some embodiments. the antisense strand sequence comprises or consists of sequence a least 75% identical to SEQ ID NO: 6275, at least 80% identical to SEQ ID NO: 6275, at least 85% identical to SEQ ID NO: 6275, at least 90% identical to SEQ ID NO: 6275, or at least 95% identical to SEQ ID NO: 6275. In some embodiments, the antisense strand sequence comprises or consists of the sequence of SEQ ID NO 6275, or an antisense strand sequence thereof having 1, 2, 3, or 4 nucleoside substitutions, additions, or deletions. In some embodiments, the antisense strand sequence comprises or consists of the sequence of SEQ ID NO: 6275, or an anti sense strand sequence thereof having 1 or 2 nucleoside substitutions, additions, or deletions. In some embodiments, the antisense strand sequence comprises or consists of a sequence 100% identical to SEQ ID NO: 6275. The antisense strand may comprise a moiety such as a GalNAc moiety or a lipid moiety.

In some embodiments, the siRNA comprises a sense strand having a sequence in accordance with SEQ ID NO: 6229. In some embodiments, the sense strand sequence comprises or consists of sequence at least 75% identical to SEQ ID NO: 6229, at least 80% identical to SEQ ID NO: 6229, at least 85% identical to SEQ ID NO: 6229, at least 90% identical to SEQ ID NO: 6229, or at least 95% identical to SEQ ID NO: 6229.In some embodiments, the sense strand sequence comprises or consists of the sequence of SEQ ID NO 6229, or a sense strand sequence thereof having 1, 2, 3, or 4 nucleoside substitutions, additions. or deletions. In some embodiments, the sense strand sequence comprises or consists of the sequence of SEQ ID NO: 6229, or a sense strand sequence thereof having 1 or 2 nucleoside substitutions, additions, or deletions. In some embodiments, the sense strand sequence comprises or consists of a sequence 100% identical to SEQ ID NO: 6229. The sense strand may comprise a moiety such as a GalNAc moiety or a lipid moiety. In some embodiments, the siRNA comprises an antisense strand having a sequence in accordance with SEQ ID NO: 6288. In some embodiments, the antisense strand sequence comprises or consists of sequence at least 75% identical to SEQ ID NO: 6288, a least 80% identical to SEQ ID NO: 6288, at least 85% identical to SEQ ID NO: 6288, at least 90% identical to SEQ ID NO: 6288, or at least 95% identical to SEQ ID NO: 6288. In some embodiments, the antisense strand sequence comprises or consists of the sequence of SEQ ID NO 6288, or an anti sense strand sequence thereof having 1, 2, 3, or 4 nucleoside substitutions, additions, or deletions. In some embodiments, the anti sense strand sequence comprises or consists of the sequence of SEQ ID NO: 62 88, or an antisense strand sequence thereof having 1 or 2 nucleoside substitutions, additions, or deletions. In some embodiments, the anti sense strand sequence comprises or consists of a sequence 100% identical to SEQ ID NO: 6288. The antisense strand may comprise a moiety such as a GalNAc moiety or a lipid moiety.

In some embodiments, the siRNA comprises a sense strand having a sequence in accordance with SEQ ID NO: 6234. In some embodiments, the sense strand sequence comprises or consists of sequence at least 75% identical to SEQ ID NO: 6234, at least 80% identical to SEQ ID NO: 6234, at least 85% identical to SEQ ID NO: 6234, at least 90% identical to SEQ ID NO: 6234, or at least 95% identical to SEQ IDNO: 6234. In some embodiments, the sense strand sequence comprises or consists of the sequence of SEQ ID NO 6234, or a sense strand sequence thereof having 1, 2, 3, or 4 nucleoside substitutions, additions, or deletions. In some embodiments, the sense strand sequence comprises or consists of the sequence of SEQ ID NO: 6234, or a sense strand sequence thereof having 1 or 2 nucleoside substitutions, additions, or deletions. In some embodiments, the sense strand sequence comprises or consists of a sequence 100% identical to SEQ ID NO: 6234. The sense strand may comprise a moiety such as a GalNAc moiety or a lipid moiety. In some embodiments, the siRNA comprises an antisense strand having a sequence in accordance with SEQ ID NO: 6293. In some embodiments, the anti sense strand sequence comprises or consists of sequence at least 75% identical to SEQ ID NO: 6293, at least 80% identical to SEQ ID NO: 6293, at least 85% identical to SEQ ID NO: 6293, at least 90% identical to SEQ ID NO: 6293, or at least 95% identical to SEQ ID NO: 6293. In some embodiments, the sntisense strand sequence comprises or consists of the sequence of SEQ ID NO 6293, or an anti sense strand sequence thereof having 1, 2, 3, or 4 nucleoside substitutions, additions, or deletions. In some embodiments, the antisense strand sequence comprises or consists of the sequence of SEQ ID NO: 6293, or an antisense strand sequence thereof having 1 or 2nucleoside substitutions, additions, or deletions. In some embodiments, the antisense strand sequence comprises or consists of a sequence 100% identical to SEQ ID NO: 6293. The antisense strand may comprise a moiety such as a GalN Ac moiety or a lipid moiety.

In some embodiments, the siRNA comprises a sense strand having a sequence in accordance with SEQ ID NO: 6238. In some embodiments, the sense strand sequence comprises or consists of sequence at least 75% identical to SEQ ID NO: 6238, at least 80% identical to SEQ ID NO: 6238, at least 85% identical to SEQ ID NO: 6238, at least 90% identical to SEQ ID NO: 6238, or at least 95% identical to SEQ ID NO: 6238. In some embodiments, the sense strand sequence comprises or consists of the sequence of SEQ ID NO 6238, or a sense strand sequence thereof having 1, 2, 3, or 4 nucleoside substitutions, additions, or deletions. In some embodiments, the sense strand sequence comprises or consists of the sequence of SEQ ID NO:6238, or a sense strand sequence thereof having 1 or 2 nucleoside substitutions, additions, or deletions. In some embodiments, the sense strand sequence comprises or consists of a sequence 100% identical to SEQ ID NO: 6238. The sense strand may comprise a moiety such as a GalNAc moiety or a lipid moiety. In some embodiments, the siRNA comprises an anti sense strand having a sequence in accordance with SEQ ID NO: 6297. In some embodiments, the antisense strand sequence comprises or consists of sequence at least 75% identical to SEQ ID NO: 6297, at least 80% identical to SEQ ID NO: 6297, at least 85% identical to SEQ ID NO 6297, at least 90% identical to SEQ ID NO: 6297, or at least 95% identical to SEQ ID NO: 6297. In some embodiments, the antisense strand sequence comprises or consists of the sequence of SEQ ID NO 6297, or an antisense strand sequence thereof having 1, 2, 3, or 4 nucleoside substitutions, additions, or deletions. In some embodiments, the antisense strand sequence comprises or consists of the sequence of SEQ ID NO: 6297, or an anti sense strand sequence thereof having 1 or 2 nucleoside substitutions, additions, or deletions. In some embodiments, the antisense strand sequence comprises or consists of a sequence 100% identical to SEQ ID NO: 6297. The anti sense strand may comprise a moiety such as a GalNAc moiety or a lipid moiety.

In some embodiments, the siRNA comprises a sense strand having a sequence in accordance with SEQ ID NO: 6244. In some embodiments, the sense strand sequence comprises or consists of sequence at least 75% identical to SEQ ID NO: 6244, at least 80% identical to SEQ ID NO: 6244, at least 85% identical to SEQ ID NO: 6244, at least 90% identical to SEQ ID NO: 6244, or at least 95% identical to SEQ ID NO: 6244. In some embodiments, the sense strand sequence comprises or consists of the sequence of SEQ ID NO 6244, or a sense strand sequence thereof having 1, 2, 3, or 4 nucleoside substitutions, additions, or deletions. In some embodiments, the sense strand sequence comprises or consists of the sequence of SEQ ID NO: 6244, or a sense strand sequence thereof having 1 or 2 nucleoside substitutions, additions, or deletions. In some embodiments, the sense strand sequence comprises or consists of a sequence 100% identical to SEQ ID NO: 6244. The sense strand may comprise a moiety such as a GalNAc moiety or a lipid moiety. In some embodiments, the siRNA comprises an antisense strand having a sequence in accordance with SEQ ID NO: 6303. In some embodiments, the antisense strand sequence comprises or consists of sequence at least 75% identical to SEQ ID NO: 6303, at least 80% identical to SEQ ID NO: 6303, at least 85% identical to SEQ ID NO: 6303, al least 90% identical to SEQ ID NO: 6303, or at least 95% identical to SEQ ID NO: 6303. In some embodiments, the antisense strand sequence comprises or consists of the sequence of SEQ ID NO 6303, or an antisense strand sequence thereof having 1,2, 3, or 4 nucleoside substitutions, additions, or deletions. In some embodiments, the antisense strand sequence comprises or consists of the sequence of SEQ ID NO: 6303, or an antisense strand sequence thereofhaving 1 or 2 nucleoside substitutions, additions, or deletions. In some embodiments, the antisense strand sequence comprises or consists of a sequence 100% identical to SEQ ID NO: 6303. The antisense strand may comprise a moiety such as a GalNAc moiety or a lipid moiety.

### 4. ASO modification patterns

In some embodiments, the composition comprises an oligonucleotide that inhibits the expression of *MST1,* wherein the oligonucleotide comprises an antisense oligonucleotide (ASO). In some embodiments, the ASO comprises modification pattern ASOI:
5'-nsnsnsnsnsdNsdNsdNsdNsdNsdNsdNsdNsdNsdNsnsnsnsnsn-3' (SEQ ID NO: 6181), wherein "dN" is any deoxynucleotide, "n" is a 2'O-methyl or 2'O-methox ; ethyl-modified nucleoside, and "s" is a phosphorothioate linkage. In some embodiments, the ASO comprises modification pattern 1S, 2S, 3S, 4S, 5S, 6S, 7S, 8S, 9S, 10S, 11S, 12S, 13S. 14S, 15S, 16S, 17S, 18S, 19S, 20S. 21S, 22S, 23S, 24S, 25S, 26S, 27S, 28S, 29S, 30S. 31S, 32S, 33S, 34S. 35S, 1AS, 2AS, 3AS, 4AS, SAS, 6AS. 7AS, 8AS, 9AS, 10AS, 11AS, 12AS. 12AS, 14AS, 15AS, I6AS. 17AS, 18AS, 19AS, 20AS, or 21AS.

### D. Formulations

In some embodiments, the composition is a pharmaceutical composition. In some embodiments, the composition is sterile. In some embodiments, the composition further comprises a pharmaceutically acceptable carrier.

In some embodiments, the pharmaceutically acceptable carrier comprises water. In some embodiments, the pharmaceutically acceptable carrier comprises a buffer. In some embodiments, the pharmaceutically acceptable carrier comprises a saline solution. In some embodiments, the pharmaceutically acceptable carrier comprises water, a buffer, or a saline solution. In some embodiments, the composition comprises a liposome. In some embodiments, the pharmaceutically acceptable carrier comprises liposomes, lipids, nanoparticles, proteins, protein-antibody complexes, peptides, cellulose, nanogel, or a combination thereof. In some embodiments, the oligonucleotide is combined with lipids, nanoparticles, polymers, liposomes, micelles, or another delivery system.

In some embodiments, the composition is formulated for delivery to a subject's lungs. In some embodiments, the composition is formulated for inhalation. In some embodiments, the composition is formulated for aerosolization. In some embodiments, the composition is formulated for administration by a nebulizer.

### It METHODS AND USES

Disclosed herein, in some embodiments, are methods of administering a composition described herein to a subject. Some embodiments relate to use a composition described herein, such as administering the composition to a subject.

Some embodiments relate to a method of treating a disorder in a subject in need thereof. Some embodiments relate to use of a composition described herein in the method of treatment. Some embodiments include administering a composition described herein to a subject with the disorder. In some embodiments, the administration treats the disorder in the subject. In some embodiments, the composition treats the disorder in the subject.

In some embodiments, the treatment comprises prevention, inhibition, or reversion of the disorder in the subject. Some embodiments relate to use of a composition described herein in the method of preventing, inhibiting, or reversing the disorder. Some embodiments relate to a method of preventing, inhibiting, or reversing a disorder a disorder in a subject in need thereof. Some embodiments include administering a composition described herein to a subject with the disorder. In some embodiments, the administration prevents, inhibits, or reverses the disorder in the subject In some embodiments, the composition prevents, inhibits, or reverses the disorder in the subject.

Some embodiments relate to a method of preventing a disorder a disorder in a subject in need thereof Some embodiments relate to use of a composition described herein in the method of preventing the disorder. Some embodiments include administering a composition described herein to a subject with the disorder, In some embodiments, the administration prevents the disorder in the subject In some embodiments, the composition prevents the disorder in the subject.

Some embodiments relate to a method of inhibiting a disorder a disorder in a subject in need thereof. Some embodiments relate to use of a composition described herein in the method of inhibiting the disorder. Some embodiments include administering a composition described herein to a subject with the disorder. In some embodiments, the administration inhibits the disorder in the subject. In some embodiments, the composition inhibits the disorder in the subject.

Some embodiments relate to a method of reversing a disorder a disorder in a subject in need thereof. Some embodiments relate to use of a composition described herein in the method of reversing the disorder. Some embodiments include administering a composition described herein to a subject with the disorder. In some embodiments, the administration reverses the disorder in the subject. In some embodiments, the composition reverses the disorder in the subject

In some embodiments, the administration is systemic. In some embodiments, the administration is intravenous. In some embodiments, the administration is by injection. In some embodiments, the administration is to a subject's lungs. In some embodiments, the administration is by inhalation. In some embodiments, the administration is performed using a nebulizer.

### A. Disorders

Some embodiments of the methods described herein include treating a disorder in a subject in need thereof. In some embodiments, the disorder is a lung disorder. Non-limiting examples of lung disorders include chronic obstructive pulmonary disease (COPD), acute exacerbation of COPD, emphysema, chronic bronchitis, asthma, status asthmaticus, asthma-COPD overlap syndrome (ACOS), cough, lung cancer, interstitial lung disease, or pulmonary fibrosis. The lung disorder may include an obstructive airway disorder such as COPD or asthma. In some embodiments, the lung disorder includes COPD. In some embodiments, the lung disorder includes acute exacerbation of COPD. In some embodiments, the lung disorder includes emphysema. In some embodiments, the lung disorder includes chronic bronchitis. In some embodiments, the lung disorder includes asthma. In some embodiments, the lung disorder includes status asthmaticus. In some embodiments, the lung disorder includes ACOS. In some embodiments, the lung disorder includes cough In some embodiments, the lung disorder includes lung cancer. In some embodiments, the lung disorder includes interstitial lung disease. In some embodiments, the lung disorder includes pulmonary fibrosis. The lung disorder may result from smoking, or from smoke inhalation.

### B. Subjects

Some embodiments of the methods described herein include treatment of a subject. Non-limiting examples of subjects include vertebrates, animals, mammals, dogs, cats, cattle, rodents, mice, rats, primates, monkeys, and humans. In some embodiments, the subject is a vertebrate. In some embodiments, the subject is an animal. In some embodiments, the subject is a mammal. In some embodiments, the subject is a dog. In some embodiments, the subject is a cat. In some embodiments, the subject is a cattle. In some embodiments, the subject is a mouse. In some embodiments, the subject is a rat. In some embodiments, the subject is a primate. In some embodiments, the subject is a monkey. In some embodiments, the subject is an animal, a mammal, a dog, a cat, cattle, a rodent, a mouse, a rat, a primate, or a monkey. In some embodiments, the subject is a human. In some embodiments, the subject is male. In some embodiments, the subject is female. In some embodiments, the subject is an adult (e.g. at least 18 years old).

### C. Baseline measurements

Some embodiments of the methods described herein include obtaining a baseline measurement from a subject. For example, in some embodiments, a baseline measurement is obtained from the subject prior to treating the subject. Non-limiting examples of baseline measurements include a baseline lung function measurement, a baseline leukocyte measurement, a baseline chronic obstructive pulmonary disease (COPD) exacerbation measurement, a baseline asthma exacerbation measurement, a baseline MSP measurement, or a baseline *MST1* mRNA measurement.

In some embodiments, the baseline measurement is obtained directly from the subject. In some embodiments, the baseline measurement is obtained by observation, for example by observation of the subject or of the subject's tissue. In some embodiments, the baseline measurement is obtained noninvasively using an imaging device.

In some embodiments, the base fine measurement is obtained in a sample from the subject. In some embodiments, the baseline measurement is obtained in one or more histological tissue sections. In some embodiments, the baseline measurement is obtained by performing an assay such as an immunoassay, a colorimetric assay, or a fluorescence assay, on the sample obtained from the subject. In some embodiments, the baseline measurement is obtained by an immunoassay, a colorimetric assay, a fluorescence assay, or a chromatography (e.g. HPLC) assay. In some embodiments, the baseline measurement is obtained by PCR.

In some embodiments, the baseline measurement is a baseline lung function measurement. In some embodiments, the baseline measurement is a baseline spirometry measurement. The baseline spirometry measurement may be obtained using a spirometer. The spirometer may generate a spirogram comprising a volume-time curve or a flow-volume loop. In some embodiments, the baseline spirometry measurement is obtained by having the subject breathe into aspirometer sensor. Examples of baseline spirometry measurements may include a baseline forced expiratory volume in 1 second (FEV1) measurement, a baseline forced expiratory volume in 1 second percent predicted (FEV1pp) measurement, a baseline forced vital capacity (FVC) measurement, a baseline FEVI/FVC ratio, a baseline forced expiratory volume, or a baseline peak expiratory flow measurement. In some embodiments, the baseline measurement includes a baseline forced expiratory volume in 1 second (FEVI) measurement. In some embodiments, the baseline measurement includes a baseline forced expiratory volume in 1 second percent predicted (FEV I pp) measurement. In some embodiments, the baseline measurement includes a baseline forced vital capacity (FVC) measurement. In some embodiments, the baseline measurement includes a baseline FEV1/FVC ratio. The baseline FEV1/FVC ratio may be below 70% or below 80%, in some cases. In some embodiments, the baseline measurement includes a baseline forced expiratory volume. In some embodiments, the baseline measurement includes a baseline peak expiratory flow measurement.

In some embodiments, the baseline measurement includes a baseline leukocyte measurement. In some embodiments, the baseline leukocyte measurement includes a baseline circulating leukocyte measurement. In some embodiments, the baseline leukocyte measurement includes a baseline lung tissue leukocyte measurement. In some embodiments, the baseline leukocyte measurement includes a baseline lung fluid (e.g. bronchoalveolar fluid) leukocyte measurement. In some embodiments, the baseline leukocyte measurement includes a baseline leukocyte count. In some embodiments, the baseline leukocyte measurement includes a baseline leukocyte concentration In some embodiments, the baseline leukocyte measurement includes a baseline leukocyte percentage. The percentage may be in relation to other cells. Examples of leukocytes that may be included in the baseline leukocyte measurement include neutrophils, eosinophils, basophils, monocytes, or lymphocytes. The leukocytes may include neutrophils. The leukocytes may include eosinophils. The leukocytes may include basophils. The leukocytes may include monocytes. The leukocytes may include lymphocytes. In some embodiments, the baseline leukocyte measurement is obtained by an assay such as an immunoassay, a colorimetric assay, or a fluorescence assay. In some embodiments, the baseline leukocyte measurement is high, relative to a control leukocyte measurement. For example, a subject who has not been treated with a composition described herein and who has an inflammatory lung disorder may have a high leukocyte count in the subject's blood or lungs. In some embodiments, the baseine leukocyte measurement is determined in lung tissue or a lung fluid such as bronchoalveolar fluid, and may include a baseline measurement of neutrophils and macrophages.

In some embodiments, the baseline measurement includes a baseline chronic obstructive pulmonary disease (COPD) exacerbation measurement. A COPD exacerbation may include a COPD flare-up such as an acute increase in severity of a respiratory symptom such as difficulty breathing. The baseline COPD exacerbation measurement may include a baseline number of COPD flare-ups, and may be included in a given time frame such as flare-ups per day, week, month, or year. The baseline COPD exacerbation measurement may include a baseline frequency of COPD exacerbations. The baseline COPD exacerbation measurement may include a baseline measurement of worsening of a respiratory symptom, such as increased dyspnea, cough, sputum volume, or sputum purulence. The baseline COPD exacerbation measurement may include a baseline measurement of an event such as when a the subject's conditions change enough to require a change in treatment. The baseline COPD exacerbation measurement may include a baseline peak flow test, a baseline breath nitric oxide measurement, or a baseline blood oxygen level test.

In some embodiments, the baseline measurement includes a baseline asthma exacerbation measurement. An asthma exacerbation may include an asthma attack, for example narrowing of a bronchial tube that causes difficulty breathing. The baseline asthma exacerbation measurement may include a baseline number of number of asthma attack s, and may be included in a given time frame such as flare-ups per day, week, month, or year. The baseline asthma exacerbation measurement may include a baseline frequency of asthma exacerbations. The baseline asthma exacerbation measurement may include a baseline bronchial tube measurement such as a bronchial tube diameter, a bronchial tube circumference, or a bronchial tube area measurement. The baseline asthma exacerbation measurement may include a baseline amount of bronchial tube narrowing, such as a percent constriction. The baseline asthma exacerbation measurement may include a baseline wheezing measurement, a baseline coughing measurement, a baseline chest tightening measurement, a baseline shortness of breath measurement, a baseline agitation measurement, a baseline hyperventilation measurement, a baseline heart rate measurement, a baseline lung function measurement, or a baseline measurement of difficulty speaking or breathing. The baseline asthma exacerbation measurement may include a baseline peak flow test, a baseline breath nitric oxide measurement, or a baseline blood oxygen level test

In some embodiments, the baseline measurement is a baseline MSP measurement. In some embodiments, the baseline MSP measurement comprises a baseline MSP level. In some embodiments, the baseline MSP level is indicated as a mass or percentage of MSP per sample weight. In some embodiments, the baseline MSP level is indicated as a mass or percentage of MSP per sample volume. In some embodiments, the baseline MSP level is indicated as a mass or percentage of MSP per total protein within the sample. In some embodiments, the baseline MSP measurement is a baseline circulating MSP measurement. In some embodiments, the baseline MSP measurement is obtained by an assay such as an immunoassay, a colorimetric assay, or a fluorescence assay.

In some embodiments, the baseline measurement is a baseline *MST1* mRNA measurement. In some embodiments, the baseline *MST1* mRNA measurement comprises a baseline *MST1* mRNA level. In some embodiments, the baseline *MST*1 mRNA level is indicated as an amount or percentage of *MST1* mRNA per sample weight. In some embodiments, the baseline *MST1* mRNA level is indicated as an amount or percentage of *MST1* mRNA per sample volume. In some embodiments, the baseline *MST1* mRNA level is indicated as an amount or percentage of *MST1* mRNA per total mRNA within the sample. In some embodiments, the baseline *MST1* mRNA level is indicated as an amount or percentage of *MST1* mRNA per total nucleic acids within the sample. In some embodiments, the baseline *MST1* mRNA level is indicated relative to another mRNA level, such as an mRNA level of a housekeeping gene, within the sample. In some embodiments, the baseline *MST1* mRNA measurement is obtained by an assay such as a polymerase chain reaction (PCR) assay. In some embodiments, the PCR comprises quantitative PCR (qPCR). In some embodiments, the PCR comprises reverse transcription of the *MST1* mRNA.

Some embodiments of the methods described herein include obtaining a sample from a subject. In some embodiments, the baseline measurement is obtained in a sample obtained from the subject. In some embodiments, the sample is obtained from the subject prior to administration or treatment of the subject with a composition described herein. In some embodiments, a baseline measurement is obtained in a sample obtained from the subject prior to administering the composition to the subject.

In some embodiments, the sample comprises a fluid. In some embodiments, the sample is a fluid sample. For example, the baseline MSP meastrement may be obtained in a fluid sample obtained from the patient. In some embodiments, the baseline *MST1* mRNA measurement is obtained in a fluid sample. In some embodiments, the sample is a blood, plasma, or serum sample. In some embodiments, the baseline *MST1* mRNA measurement is obtained in a fluid sample. In some embodiments, the sample comprises blood. In some embodiments, the sample is a blood sample. In some embodiments, the sample is a whole-blood sample. In some embodiments, the blood is fractionated or centrifuged. In some embodiments, the sample comprises plasma. In some embodiments, the sample is a plasma sample. A blood sample may be a plasma sample. In some embodiments, the sample comprises serum. In some embodiments, the sample is a serum sample. A blood sample may be a serum sample. In some embodiments, the fluid sample includes a lung fluid sample. In some embodiments, the lung fluid srouple includes alveolar fluid. In some embodiments, the lung fluid sample includes bronchial fluid. In some embodiments, the lung fluid sample includes bronchoal veolar fluid. The lung fluid may be obtained via a lavage method such as a bronchoalveolar lavage method. The lavage method may include the use of a bronchoscope.

In some embodiments, the sample comprises a tissue. In some embodiments, the sample is a tissue sample. In some embodiments, the tissue comprises liver, lung, or vascular tissue. For example, the baseline *MST1* mRNA measurement, or the baseline MSP measurement, may be obtained in a lung or liver sample obtaine d from the patient. In some embodiments, the tissue comprises liver tissue. The liver may include hepatocytes. In some embodiments, the tissue comprises lung tissue. The lung may include lung epithelial cells, type I alveolar cells, type 11 alveolar cells, macrophages, alveolar macrophages, goblet cells, club cells, or fibroblasts. In some embodiments, the tissue comprises vascular tissue. The vascular tissue may include vascular endothelial cells. For example, the lung tissue may include vascular endothelial cells.

In some embodiments, the sample includes cells. In some embodiments, the sample comprises a cell. In some embodiments, the cell is a liver cell. In some embodiments, the liver cell is a hepatocyte. In some embodiments, the cell is a lung cell. In some embodiments, the lung cell is a lung epithelial cell. In some embodiments, the lung cell is a type 1 alveolar cell. In some embodiments, the lung cell is a type 11 alveolar cell. In some embodiments, the lung cell is a macrophage. In some embodiments, the lung cell is a alveolar macrophage. In some embodiments, the lung cell is a goblet cell. In some embodiments, the lung cell is a club cell. In some embodiments, the lung cell is a fibroblast. In some embodiments, the cell is a vasculature cell. In some embodiments, the vasculature cell is an endothelial cell.

### D. Effects

In some embodiments, the composition or administration of the composition affects a measurement such as a lung function measurement, a leukocyte measurement, a chronic obstructive pulmonary disease (COPD) exacerbation measurement, an asthma exacerbation measurement, a MSP measurement (for example, circulating or tissue MSP levels), or a *MST1* mRNA measurement, relative to the baseline measurement.

Some embodiments of the methods described herein include obtaining the measurement from a subject. For example, the measurement may be obtained from the subject after treating the subject. In some embodiments, the measurement is obtained in a second sample (such as a fluid or tissue sample described herein) obtained from the subject after the composition is administered to the subject. In some embodiments, the measurement is an indication that the disorder has been treated.

In some embodiments, the measurement is obtained directly from the subject. In some embodiments, the measurement is obtained noninvasively using an imaging device. In some embodiments, the measurement is obtained in a second sample from the subject. In some embodiments, the measurement is obtained in one or more histological tissue sections. In some embodiments, the measurement is obtained by performing an assay on the second sample obtained from the subject. In some embodiments, the measurement is obtained by an assay, such as an assay described herein. In some embodiments, the assay is an immunoassay, a colorimetric assay, a fluorescence assay, a chromatography (e.g. HPLC) assay, or a PCR assay. In some embodiments, the measurement is obtained by an assay such as an immunoassay, a colorimetric assay, a fluorescence assay, or a chromatography (e.g. HPLC) assay. In some embodiments, the measurement is obtained by PCR. In some embodiments, the measurement is obtained by histology. In some embodiments, the measurement is obtained by observation. In some embodiments, additional measurements are made, such as in a 3rd sample, a 4th sample, or a fifth sample.

In some embodiments, the measurement is obtained within 1 hour, within 2 hours, within 3 hours, within 4 hours, within 5 hours, within 6 hours, within 12 hours, within 18 hours, or within 24 hours after the administration of the composition. In some embodiments, the measurement is obtained within 1 day, within 2 days, within 3 days, within 4 days, within 5 days, within 6 days or within 7 days after the administration of the composition. In some embodiments, the measurement is obtained within 1 week, within 2 weeks, within 3 weeks, within 1 month, within 2 months, within 3 months, within 6 months, within 1 year, within 2 years, within 3 years, within 4 years, or within 5 years after the administration of the composition. In some embodiments, the measurement is obtained after 1 hour, after 2 hours, after 3 hours, after 4 hours, after 5 hours, after 6 hours, after 12 hours, after 18 hours, or after 24 hours after the administration of the composition. In some embodiments, the measurement is obtained after 1 day, after 2 days, after 3 days, after 4 days, after 5 days, after 6 days, or after 7 days after the administration of the composition. In some embodiments, the measurement is obtained after 1 week, after 2 weeks, after 3 weeks, after 1 month, after 2 months, after 3 months, after 6 months, after 1 year, after 2 years, after 3 years, after 4 years, or after 5 years, following the administration of the composition.

In some embodiments, the composition reduces the measurement relative to the baseline measurement. For example, an adverse phenotype of a lung disorder may be reduced upon administration of the composition. In some embodiments, the reduction is measured in a second sample obtained from the subject after administering the composition to the subject. In some embodiments, the reduction is measured directly in the subject after administering the composition to the subject In some embodiments, the measurement is decreased by about 2.5% or more, about 5% or more, or about 7.5% or more, relative to the baseline measurement. In some embodiments, the measurement is decreased by about 10% or more, relative to the baseline measurement. In some embodiments, the measurement is decreased by about 20% or more, about 30% or more, about 40% or more, about 50% or more, about 60% or more, about 70% or more, about 80% or more, about 90% or more, relative to the baseline measurement. In some embodiments, the measurement is decreased by no more than about 2.5%, no more than about 5%, or no more than about 7.5%, relative to the baseline measurement In some embodiments, the measurement is decreased by no more than about 10%, relative to the baseline measurement. In some embodiments, the measurement is decreased by no more than about 20%, no more than about 30%, no more than about 40%, no more than about 50%, no more than about 60%, no more than about 70%, no more than about 80%, no more than about 90%, or no more than about 100% relative to the baseline measurement. In some embodiments, the measurement is decreased by 2.5%, 5%, 7.5%, 10%, 20%, 30%, 40%, 50%, 60%, 70%, 80%, 90%, or 100%, or by a range defined by any of the two aforementioned percentages.

In some embodiments, the composition increases the measurement relative to the baseline measurement. For example, a protective lung phenotype may be increased upon administration of the composition. In some embodiments, the increase is measured in a second sample obtained from the subject after administering the composition to the subject In some embodiments, the increase is measured directly in the subject after administering the composition to the subject In some embodiments, the measurement is increased by about 2.5% or more, about 5% or more, or about 7.5%or more, relative to the baseline measureme nl. In some embodiments, the measurement is increased by about 10% or more, relative to the baseline measurement In some embodiments, the measurement is increased by about 20% or more, about 30% or more, about 40% or more, about 50% or more, about 60% or more, about 70% or more, about 80% or more, about 90% or more, relative to the baseline measurement In some embodiments, the measurement is increased by about 100% or more, increased by about 250% or more, increased by about 500% or more, mcreased by about 750% or more, or increased by about 1000% or more, relative to the baseline measurement. In some embodiments, the measurement is increased by no more than about 2.5%, no more than about 5%, or no more than about 7.5%, relative to the baseline measurement. In some embodiments, the measurement is increased by no more than about 10%, relative to the baseline measurement. In some embodiments, the measurement is increased by no more than about 20%, no more than about 30%, no more than about 40%, no more than about 50%, no more than about 60%, no more than about 70%, no more than about 80%, no more than about 90%, or no more than about 100% relative to the baseline measurement. In some embodiments, the measurement is increased by no more than about 100%, increased by no more than about 250%, increased by no more than about 500%, increased by no more than about 750%, or increased by no more than about 1000%, relative to the baseline measurement. In some embodiments, the measurement is increased by 2.5%, 5%, 7.5%, 10%, 20%, 30%, 40%, 50%, 60%, 70%, 80%, 90%, 100%, 250%, 500%, 750%, or 1000%, or by a range defined by any of the two aforementioned percentages.

In some embodiments, the measurement is a lung function measurement. In some embodiments, the measurement is a spirometry measurement. The spirometry measurement may be obtained using a spirometer. The spirometer may generate a spirogram comprising a volume-time curve or a flow-volume loop. In some embodiments, the spirometry measurement is obtained by having the subject breathe into a spirometer sensor. Examples of spirometry measurements may include a forced expiratory volume in 1 second (FEV 1) measurement, a forced expiratory volume in 1 second percent predicted (FEV 1pp) measurement, a forced vital capacity (FVC)measurement, a FEVI/FVC ratio, a forced expiratory volume, or a peak expiratory flow measurement. In some embodiments, the measurement includes a forced expiratory volume in 1 second (FEV 1) measurement. In some embodiments, the measurement includes a forced expiratory volume in 1 second percent predicted (FEV 1pp) measurement. In some embodiments, the measurement includes a forced vital capacity (FVC) measurement. In some embodiments, the measurement includes a FEV 1/FVC ratio. The FEVI/FVC ratio may be below 70% or below 80%, in some cases. In some embodiments, the measurement includes a forced expiratory volume. In some embodiments, the measurement includes a peak expiratory flow measurement.

In some embodiments, the composition increases the lung function measurement relative to the baseline lung function measurement. In some embodiments, the increase is measured directly in the subject after administering the composition to the subject. In some embodiments, the lung function measurement is increased by about 2.5% or more, about 5% or more, or about 7.5% or more, relative to the baseline lung function measurement In some embodiments, the lung function measurement is increased by about 10% or more, relative to the baseline lung function measurement. In some embodiments, the lung function measurement is increased by about 20% or more, about 30% or more, about 40% or more, about 50% or more, about 60% or more, about 70% or more, about 80% or more, about 90% or more, relative to the baseline lung function measurement. In some embodiments, the lung function measurement is increased by about 100% or more, increased by about 250% or more, increased by about 500% or more, increased by about 750% or more, or increased by about 1000% or more, relative to the baseline lung f unction measurement. In some embodiments, the lung function measurement is increased by no more than about 2.5%, no more than about 5%, or no more than about 7.5%, relative to the baseline lung function measurement. In some embodiments, the lung function measurement is increased by no more than about 10%, relative to the baseline lung function measurement. In some embodiments, the lung function measurement is increased by no more than about 20%, no more than about 30%, no more than about 40%, no more than about 50%, no more than about 60%, no more than about 70%, no more than about 80%, no more than about 90%, or no more than about 100% relative to the baseline lung function measurement. In some embodiments, the lung function measurement is increased by no more than about 100%, increased by no more than about 250%, increased by no more than about 500%, increased by no more than about 750%, or increased by no more than about 1000%, relative to the baseline lung function measurement. In some embodiments, the lung function measurement is increased by 2.5%, 5%, 7.5%, 10%, 20%, 30%, 40%, 50%, 60%, 70%, 80%, 90%, 100%, 250%, 500%, 750%, or 1000%, or by a range defined by any of the two aforementioned percentages.

In some embodiments, the measurement includes a leukocyte measurement. In some embodiments, the leukocyte measurement includes a circulating leukocyte measurement. In some embodiments, the leukocyte measureme nt includes a lung tissue leukocyte measurement. In some embodiments, the leukocyte measurement includes a lung fluid (e.g. bronchoalveolar fluid) leukocyte measurement. In some embodiments, the leukocyte measurement includes a leukocyte count. In some embodiments, the leukocyte measurement includes a leukocyte concentration. In some embodiments, the leukocyte measurement includes a leukocyte percentage. The percentage may be in relation to other cells. Examples of leuk ocytes that may be included in the leukocyte measurement include neutrophils, eosinophils, basophils, monocytes, or lymphocytes. The leukocytes may include neutrophils. The leukocytes may include eosinophils. The leukocytes may include basophils. The leukocytes may include monocytes. The leukocytes may include lymphocytes. In some embodiments, the leukocyte measurement is obtained by an assay such as an immunoassay, a colorimetric assay, or a fluorescence assay. In some embodiments, the leukocyte measurement is normal, relative to a control leukocyte measurement. For example, a subject who has been treated with a composition described herein and who has an inflammatory lung disorder may have had a high leukocyte count that is now low or normal. In some embodiments, the leukocyte measurement is determined in lung tissue or a lung fluid such as bronchoalveolar fluid, and may include a measurement of neutrophils and macrophages.

In some embodiments, the composition reduces the leukocyte measurement relative to the baseline leukocyte measurement. In some embodiments, the reduction is measured in a second sample obtained from the subject after administering the composition to the subject. In some embodiments, the leukocyte measurement is decreased by about 2.5% or more, about 5% or more, or about 7.5% or more, relative to the baseline leukocyte measurement. In some embodiments, the leukocyte measurement is decreased by about 10% or more, relative to the baseline leukocyte measurement. In some embodiments, the leukocyte measurement is decreased by about 20% or more, about 30% or more, about 40% or more, about 50% or more, about 60% or more, about 70% or more, or about 80% or more, relative to the baseline leukocyte measurement. In some embodiments, the leukocyte measurement is decreased by no more than about 2.5%, no more than about 5%, or no more than about 7.5%, relative to the baseline leukocyte measurement. In some embodiments, the leukocyte measurement is decreased by no more than about 10%, relative to the baseline leukocyte measurement. In some embodiments, the leukocyte measurement is decreased by no more than about 20%, no more than about 30%, no more than about 40%, no more than about 50%, no more than about 60%, no more than about 70%, or no more than about 80%, relative to the baseline leukocyte measurement. In some embodiments, the leukocyte measurement is decreased by 2.5%, 5%, 7.5%, 10%, 20%, 30%, 40%, 50%, 60%, 70%, 80%, 90%, or 100%, or by a range defined by any of the two aforementioned percentages. In some embodiments, the leukocyte measurement is increased by any of the aforementioned percentages or ranges of percentages, relative to the baseline leukocyte measurement.

In some embodiments, the measurement includes a chronic obstructive pulmonary disease (COPD) exacerbation measurement. A COPD exacerbation may include a COPD flare-up such as an acute increase in severity of a respiratory symptom such as difficulty breathing. The COPD exacerbation measurement may include a number ofCOPD flare-ups, and may be included in a given time frame such as flare-ups per day, week, month, or year. The COPD exacerbation measurement may include a frequency of COPD exacerbations. The COPD exacerbation measurement may include a measurement of worsening of a respiratory symptom, such as increased dyspnea, cough, sputum volume, or sputum purulence. The COPD exacerbation measurement may include a measurement of an event such as when a the subject's conditions change enough to require a change in treatment. The COPD exacerbation measurement may include a peak flow test, a breath nitric oxide measurement, or a blood oxygen level test.

In some embodiments, the composition reduces the COPD exacerbation measurement relative to the baseline COPD exacerbation measurement. In some embodiments, the reduction is measured in a second sample obtained from the subject after administering the composition to the subject In some embodiments, the reduction is measured directly in the subject after administering the composition to the subject In some embodiments, the COPD exacerbation measurement is decreased by about 2.5% or more, about 5% or more, or about 7.5% or more, relative to the baseline COPD exacerbation measurement. In some embodiments, the COPD exacerbation measurement is decreased by about 10% or more, relative to the baseline COPD exacerbation measurement. In some embodiments, the COPD exacerbation measurement is decreased by about 20% or more, about 30% or more, about 40% or more, about 50% or more, about 60% or more, about 70% or more, about 80% or more, about 90% or more, relative to the baseline COPD exacerbation measurement In some embodiments, the COPD exacerbation measurement is decreased by no more than about 2.5%, no more than about 5%, or no more than about 7.5%, relative to the baseline COPD exacerbation measurement. In some embodiments, the COPD exacerbation measurement is decreased by no more than about 10%, relative to the baseline COPD exacerbation measurement. In some embodiments, the COPD exacerbation measurement is decreased by no more than about 20%, no more than about 30%, no more than about 40%, no more than about 50%, no more than about 60%, no more than about 70%, no more than about 80%, no more than about 90%, or no more than about 100% relative to the baseline COPD exacerbation measurement. In some embodiments, the COPD exacerbation measurement is decreased by 2.5%, 5%, 7.5%, 10%, 20%, 30%, 40%, 50%, 60%, 70%, 80%, 90%, or 100%, or by a range defined by any of the two aforementioned percentages.

In some embodiments, the measurement includes an asthma exacerbation measurement. An asthma exacerbation may include an asthma attack, for example narrowing of a bronchi al tube that causes difficulty breathing. The asthma exacerbation measurement may include a number of number of asthma attacks, and may be included in a given time frame such as flare-ups per day, week, month, or year. The asthma exacerbation measurement may include a bronchi al tube measurement such as a bronchial tube diameter, a bronchial tube circumference, or a bronchial tube area measurement. The asthma exacerbation measurement may include an amount of bronchial tube narrowing, such as a percent constriction. The asthma exacerbation measurement may include a wheezing measurement, a coughing measurement, a chest tightening measurement, a shortness of breath measurement, a agitation measurement, a hyperventilation measurement, a heart rate measurement, a lung function measurement or a measurement of difficulty speaking or breathing. The asthma exacerbation measurement may include a peak flow test, a breath nitric oxide measurement, or a blood oxygen level test.

In some embodiments, the composition reduces the asthma exacerbation measurement relative to the baseline asthma exacerbation measurement. In some embodiments, the reduction is measured in a second sample obtained from the subject after administering the composition to the subject. In some embodiments, the reduction is measured directly in the subject after administering the composition to the subject. In some embodiments, the asthma exacerbation measurement is decreased by about 2.5% or more, about 5% or more, or about 7.5% or more, relative to the baseline asthma exacerbation measurement. In some embodiments, the asthma exacerbation measurement is decreased by about 10% or more, relative to the baseline asthma exacerbation measurement In some embodiments, the asthma exacerbation measurement is decreased by about 20% or more, about 30% or more, about 40% or more, about 50% or more, about 60% or more, about 70% or more, about 80% or more, about 90% or more, relative to the baseline asthma exacerbation measurement. In some embodiments, the asthma exacerbation measurement is decreased by no more than about 2.5%, no more than about 5%, or no more than about 7.5%, relative to the baseline asthma exacerbation measurement. In some embodiments, the asthma exacerbation measurement is decreased by no more than about 10%, relative to the baseline asthma exacerbation measurement. In some embodiments, the asthma exacerbation measurement is decreased by no more than about 20%, no more than about 30%, no more than about 40%, no more than about 50%, no more than about 60%, no more than about 70%, no more than about 80%, no more than about 90%, or no more than about 100% relative to the baseline asthma exacerbation measurement. In some embodiments, the asthma exacerbation measurement is decreased by 2.5%, 5%, 7.5%, 10%, 20%, 30%, 40%, 50%, 60%, 70%, 80%, 90%, or 100%, or by a range defined by any of the two aforementioned percentages.

In some embodiments, the measurement is an MSP measurement. In some embodiments, the MSP measurement comprises an MSP level. In some embodiments, the MSP level is indicated as a mass or percentage of MSP per sample weight In some embodiments, the MSP level is indicated as a mass or percentage of MSP per sample volume. In some embodiments, the MSP level is indicated as a mass or percentage of MSP per total protein within the sample. In some embodiments, the MSP measurement is a circulating MSP measurement. In some embodiments, the MSP measurement is obtained by an assay such as an immunoassay, a colorimetric assay, or a fluorescence assay.

In some embodiments, the composition reduces the MSP measurement relative to the baseline MSP measurement. In some embodiments, the composition reduces circulating MSP levels relative to the baseline MSP measurement. In some embodiments, the composition reduces tissue MSP levels relative to the baseline MSP measurement. In some embodiments, the reduced MSP levels are measured in a second sample obtained from the subject after administering the composition to the subject. In some embodiments, the second sample is a blood, serum, plasma, liver, or lung sample. In some embodiments, the MSP measurement is decreased by about 2.5% or more, about 5% or more, or about 7.5% or more, relative to the baseline MSP measurement. In some embodiments, the MSP measurement is decreased by about 10% or more, relative to the baseline MSP measurement In some embodiments, the MSP measurement is decreased by about 20% or more, about 30% or more, about 40% or more, about 50% or more, about 60% or more, about 70% or more, about 80% or more, about 90% or more, or about 100%, relative to the baseline MSP measurement. In some embodiments, the MSP measurement is decreased by no more than about 2.5%, no more than about 5%, or no more than about 7.5%, relative to the baseline MSP measurement. In some embodiments, the MSP measurement is decreased by no more than about 10%, relative to the baseline MSP measurement. In some embodiments, the MSP measurement is decreased by no more than about 20%, no more than about 30%, no more than about 40%, no more than about 50%, no more than about 60%, no more than about 70%, no more than about 80%, no more than about 90%, or no more than about 100% relative to the baseline MSP measurement. In some embodiments, the MSP measurement is decreased by 2.5%, 5%, 7.5%, 19%, 20%, 30%, 40%, 50%, 60%, 70%, 80%, 90%, or 100%, or by a range defined by any of the two aforementioned percentages.

In some embodiments, the measurement is an *MST1* mRNA measurement. In some embodiments, the *MST1* mRNA measurement comprises an *MST1* mRNA level. In some embodiments, the *MST1* mRNA level is indicated as an amount or perce ntage of *MST1* mRNA per sample weight. In some embodiments, the *MST1* mRNA level is indicated as an amount or percentage of *MST1* mRNA per sample volume. In some embodiments, the *MST1* mRNA level is indicated as an amount or percentage of *MST1* mRNA per total mRNA within the sample. In some embodiments, the *MST1* mRNA level is indicated as an amount or percentage of *MST1* mRNA per total nucleic acids within the sample. In some embodiments, the *MST1* mRNA level is indicated relative to another mRNA level, such as an mRNA level of a housekeeping gene, within the sample. In some embodiments, the *MST1* mRNA measurement is obtained by an assay such as a PCR assay. In some embodiments, the PCR comprises qPCR. In some embodiments, the PCR comprises reverse transcription of the*MST1* mRNA.

In some embodiments, the composition reduces the *MST1* mRNA measurement relative to the basel ine *MST1* mRNA measurement. In some embodiments, the *MSTI* mRNA measurement is obtained in a second sample obtained from the subject after administering the composition to the subject. In some embodiments, the composition reduces *MST1* mRNA levels relative to the baseline *MST1* mRNA levels. In some embodiments, the reduced *MST1* mRNA levels are measured in a second sample obtained from the subject after administering the composition to the subject. In some embodiments, the second sample is a lung sample. In some embodiments. the second sample is a liver sample. In some embodiments, the *MST1* mRNA measurement is reduced by about 2.5% or more, about 5% or more, or about 7.5%or more, relative to the baseline *MST1* mRNA measurement In some embodiments, the *MST1* mRNA measurement is decreased by about 10% or more, relative to the baseline *MST1* mRNA measurement. In some embodiments, the *MST1* mRNA measurement is decreased by about 20% or more, about 30% or more, about 40% or more, about 50% or more, about 60% or more, about 70% or more, about 80% or more, about 90% or more, or about 100%, relative to the baseline *MST1* mRNA measurement. In some embodiments, the *MST1* mRNA measurement is decreased by no more than about 2.5%, no more than about 5%, or no more than about 7.5%, relative to the baseline *MST1* mRNA measurement. In some embodiments, the *MST1* mRNA measurement is decreased by no more than about 10%, relative to the baseline *MST1* mRNA measurement. In some embodiments, the *MST1* mRNA measurement is decreased by no more than about 20%, no more than about 30%, no more than about 40%, no more than about 50%, no more than about 60%, no more than about 70%, no more than about 80%, no more than about 90%, or no more than about 100%, relative to the baseline *MST1* mRNA measurement. In some embodiments, the *MST1* mRNA measurement is decreased by 2.5%, 5%, 7.5%, 10%, 20%, 30%, 40%, 50%, 60%, 70%, 80%, 90%, or 100% or by a range defined by any of the two aforementioned percentages.

### III. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used in the specification and claims, the singular forms "a", "an" and "the" include plural references unless the context clearly dictates otherwise. For example, the term "a sample" includes a plurality of samples, including mixtures thereof.

The terms "determining," "measuring," "evaluating," "assessing," "assaying," and "analyzing" are often used interchangeably herein to refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

The terms "subject," and "patient" may be used interchangeably herein. A "subject" can be a biological entity containing expressed genetic materials. The biological entity can be a plant, animal, or microorganism, including, for example, bacteria, viruses, fungi, and protozoa. The subject can be a mammal. The mammal can be a human. The subject may be diagnosed or suspected of being at high risk for a disease. In some cases, the subject is not necessarily diagnosed or suspected of being at high risk for the disease.

As used herein, the term "about" a number refers to that number plus or minus 10% of that number. The term "about" a range refers to that range minus 10% of its lowest value and plus 10% of its greatest value.

As used herein, the terms "treatment" or "treating" are used in reference to a pharmaceutical or other intervention regimen for obtaining beneficial or desired results in the recipient. Beneficial or desired results include but are not limited to a therapeutic benefit and/or a prophylactic benefit. A therapeutic benefit may refer to eradication or amelioration of symptoms or of an underlying disorder being treated. Also, a therapeutic benefit can be achieved with the eradication or amelioration of one or more of the physiological symptoms associated with the underlying disorder such that an improvement is observed in the subject, notwithstanding that the subject may still be afflicted with the underlying disorder. A prophylactic effect includes delaying, preventing, or eliminating the appearance of a disease or condition, delaying or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

Some embodiments refer to nucleic acid sequence information. It is contemplated that in some embodiments, thymine (T) may be interchanged with uracil (U), or vice versa. For example, some sequences in the sequence listing may recite Ts, but these may be replaced with Us in some embodiments. In some oligonucleotides with nucleic acid sequences that include uracil, the uracil may be replaced with thymine. Similarly, in some oligonucleotides with nucleic acid sequences that include thymine, the thymine may be replaced with uracil. In some embodiments, an oligonucleotide such as an siRNA comprises or consists of RNA. In some embodiments, the oligonucleotide may comprise or consist of DNA. For example, an ASO may include DNA.

The section headings used herein are for organizational purposes only and are not to be construed as limiting the subject matter described.

### VI. EXAMPLES

### Example1: Variants in MST1 demonstrate protective associations for obstructive lung diseases and related traits

Variants in *MST1* were evaluated for associations with lung diseases and related pulmonary and leukocyte traits in approximately 382,000 individuals with genotype data from the UK Biobank cohort. Variants evaluated included: (1) rs142690032, a low-frequency (AAF=0.02) *MST1* stop-gained variant (Arg651Ter; R651Ter) which prematurely terminates the *MST1* protein at amino acid 651, (2) rs3197999, a common (AAF=0.29) *MST1* missense variant (Arg703Cys; R703C) which has been experimentally characterized as a *MST1* hy pomorph variant and is also a *MST1* ↓ pQTL, and (3) rs3020779, a common (AAF=0.82) *MST1* synonymous variant (Prol 53Pro; PI53P) which is a liver and lung *MST1* sQTL and a lung ↓ eQTL for *MST1R,* the gene encoding MSP's receptor. All three variants were considered hy pomorphic or loss-of-function variants that resulted in a decrease in the abundance or activity of the *MST1* gene product.

Analyses used a logistic or linear regression model with age, sex and the first ten principal components of genetic ancestry as covariates. The analyses resulted in identification of associations for the individual *MST1* variants (**Table 2A, 2B, 2C**, and **2D**). For example, there were protective associations with multiple lung-disease-related traits. The evaluated variants were associated with protection from COPD, asthma and lower risk of inhaled betaagonist prescription (**Table 2A** and **2B**). Additionally, the evaluated variants were associated with increased lung function (FEV1 and FVC) and decreased circulating neutrophil counts (**Table 2C** and **2D**).

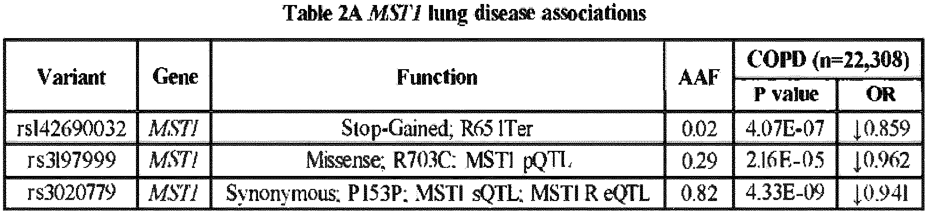

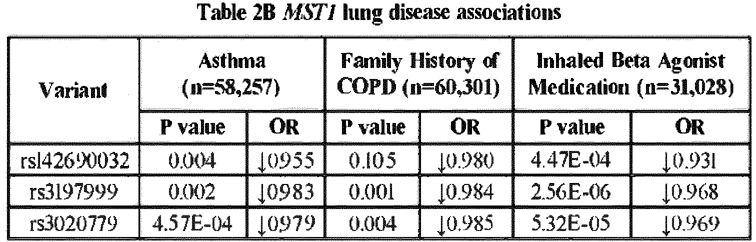

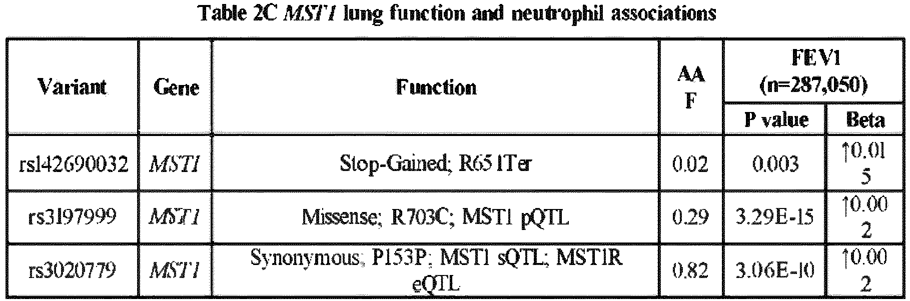

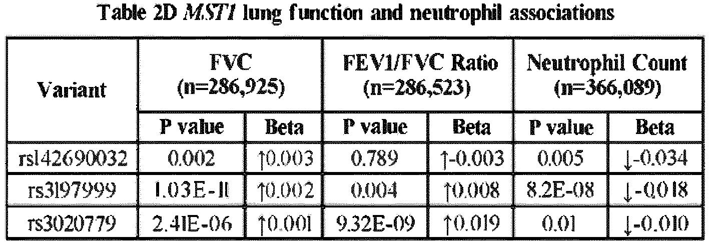

These results indicate that loss-of-function of *MST1* results in protection from COPD and asthma, improved lung function and lower circulating neutrophils, which are an important proinflammatory cell type in obstructive airways disease. These results further indicate that therapeutic inhibition of *MST1* may result in similar disease-protective effects.

### Protective variants in MST1 result in loss of MST1 protein

Pre-mRNA expression constructs encoding for wild type and R651Ter (Arg651Ter; rsl42690032) proteins were generated. The pre-mRNA of the protein coding transcript (ENST00000449682) of MST1, containing the exons, introns, and 5' and 3' UTRs, was cloned into a pcDNA3.1(+) vector driven by a CMV promoter. Empty vector was used as control. For R651 Ter expression constructs, the A allele replaced the G allele at DNA sequence position chr3:49684379 (human genome build 38). This created an R651Ter premature stop codon.

Transfections of Huh7 cells were optimized. Huh7 cells were plated in a T75 flask in complete growth media and grown for48 hours followed by a media change. Cells were then transfected with 15 µg of plasmid DNA and 45 µl of TransIT-2020. Cells were incubated for 48 hours, and then harvested.

Cell lysales from transfected cells were assayed to evaluate intracellular MST1 protein by western blot (**FIG. 1**). In empty vector transfected Huh7 cells, MST1 was not detectable by western blot. In cells transfected with the wild type construct, MST1 was detected by western blot as a band around 80 kDa. In cells transfected with the R651Ter construct, MST1 was substantially reduced by western blot compared with wild type, suggesting the premature stop codon results in loss of MST1 via nonsense mediated decay or degradation at the protein level.

These data provide experimental verification that MST1 gene variants associated with protection from COPD and asthma, improved lung function and lower circulating neutrophils, result in loss or MST1 protein abundance or function. Accordingly, in some cases therapeutic inhibition or modulation or MST1 may be an effective genetically-informed method of treatment for these diseases and measures.

### Example 2: Bioinformatic selection of sequences in order to identify therapeutic siRNAs to downmodulate expression of the MST1 mRNA

Screening sets were defined based on bioinformatic analysis. Therapeutic siRNAs were designed to target human *MST1,* and the *MST1* sequence of at least one toxicology-relevant species, in this case, the non-human primates (NHP) rhesus and cynomolgus monkeys. Drivers for the design of the screening set were predicted specificity of the siRNAs against the transcriptome of the relevant species as well as cross-reactivity between species. Predicted specificity in human, rhesus monkey, cynomolgus monkey, mouse and rat was determined for sense (S) and antisense (AS) strands. These were assigned a "specificity score" which considered a likelihood of unintended downregulation of any other transcript by full or partial complementarity of an siRNA strand (up to 4 mismatches within positions 2-18) as well as the number and positions of mismatches. Thus, off-target(s) for antisense and sense strands of each siRNA were identified. In addition, the number of potential off-targets was used as an additional specificity factor in the specificity score. As identified, siRNAs with high specificity and a low number of predicted off-targets provide a benefit of increased targeting specificity.

In addition to selecting siRNA sequences with high sequence specificity to *MST1* mRNA, siRNA sequences within the seed region were analyzed for similarity to seed regions of known miRN As. siRNAs can f unction in a miRNA like manner via base-pairing with complementary sequences within the 3'-UTR of mRNA molecules. The complementarity typically encompasses the 5'-bases at positions 2-7 of the miRNA (seed region). To circumvent siRN As to act via functional miRNA binding sites, siRNA strands containing natural miRNA seed regions were avoided. Seed regions identified in miRNAs from human, mouse, rat, rhesus monkey, dog, rabbit and pig are referred to as "conserved". Combining the "specificity score" with miRNA seed analysis yielded a "specificity category" This is divided into categories 1-4, with 1 having the highest specificity and 4 having the lowest specificity. Each strand of the siRNA is assigned to a specificity category.

Specificity and species cross-reactivity was assessed for human, cynomolgus monkey, rhesus monkey, mouse and rat*MST1*. The analysis was based on a canonical siRNA design using 19 bases and 17 bases (without considering positions 1 and 19) for cross-reactivity. Full match as well as single mismatch analyses were included.

Analysis of the human Single Nucleotide Polymorphism (SNP) database (NCBI-DB-SNP) to identify siRNAs targeting regions with known SNPs was also carried out to identify siRNAs that may be non-functional in individuals containing the SNP. Infonnation regarding the positions of SNPs within the target sequence as well as minor allele frequency (MAF) in case data was obtained in this analysis.

Initial analysis of relevant *MST1* mRNA sequence revealed few sequences that fulfil the specificity parameters and at the same time target *MST1* mRNA in all of the analyzed relevant species. Therefore, it was decided to design independent screening subsets for the therapeutic siRN As.

The siRNAs in these subsets recognize the human, cynomolgus monkey, rhesus monkey *MST1* sequences. Therefore, the siRNAs in these subsets can be used to target human *MST1* in a therapeutic setting.

The number of siRNA sequences that can be derived from human *MST1* mRNA (NM_020998.4 SEQ ID NO: 6185) without consideration of specificity or species cross-reactivity was 3024 (sense and antisense strand sequences included in SEQ ID NOS: 1-6048).

Prioritizing sequences for target specificity, species cross-reactivity, miRNA seed region sequences and SNPs as described above yields subset A. Subset A contains 231 siRNAs whose base sequences are shown in Table 3.

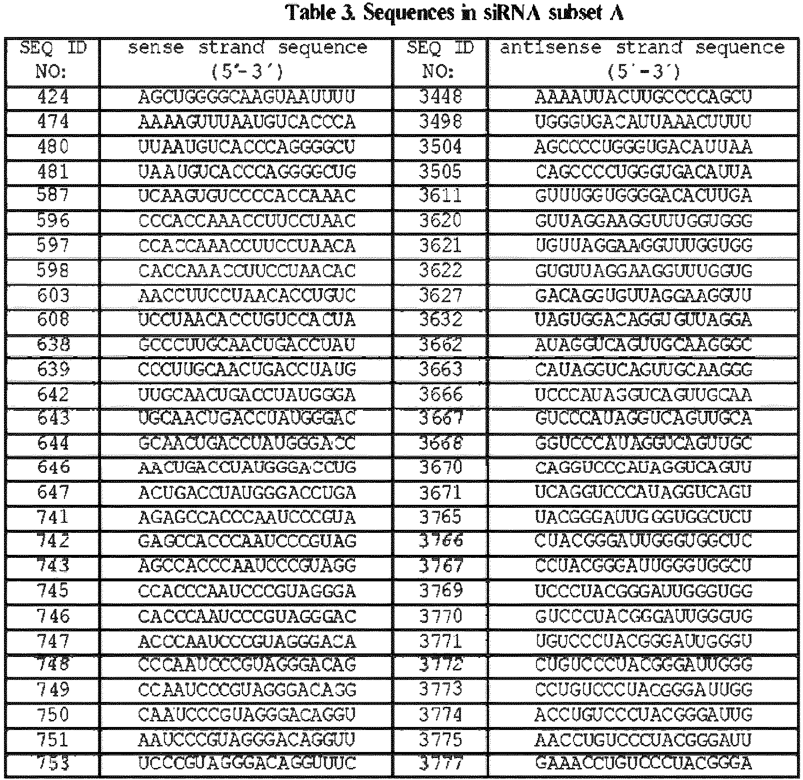

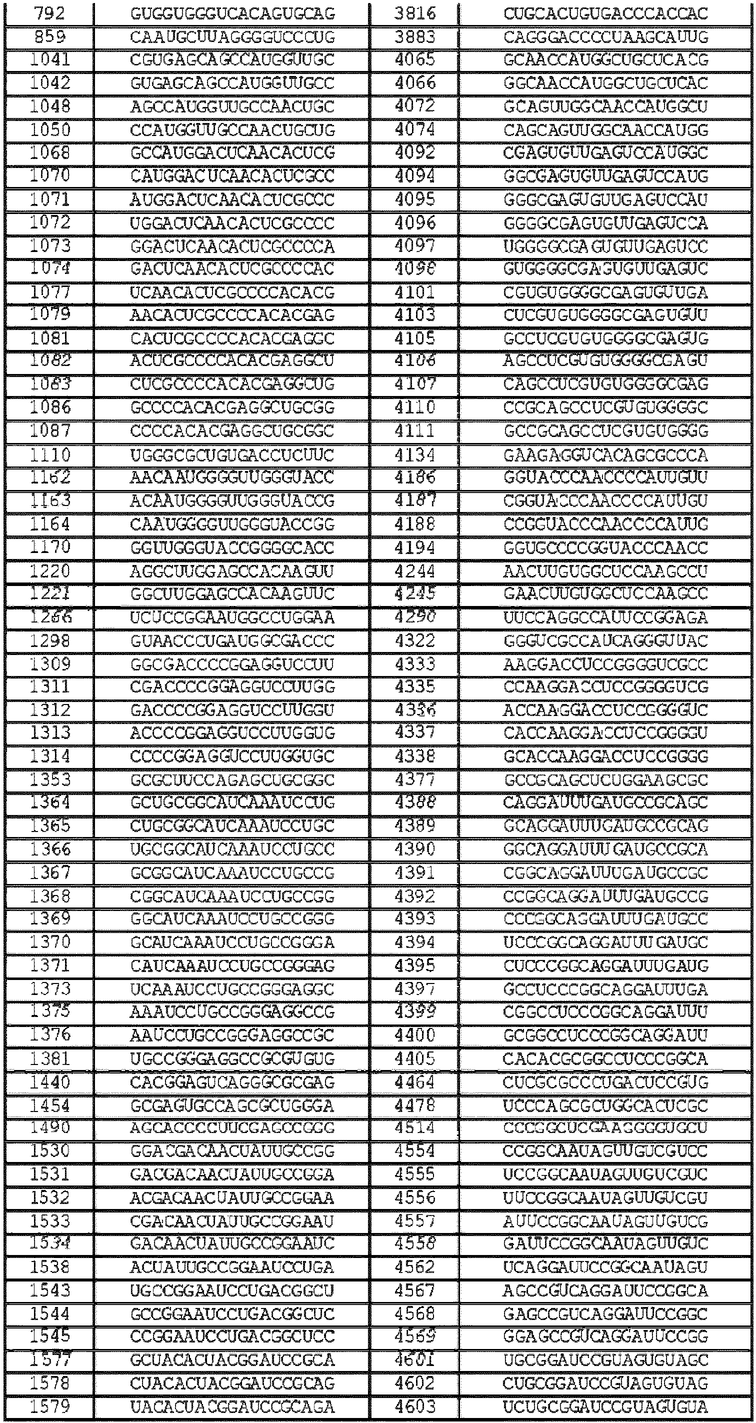

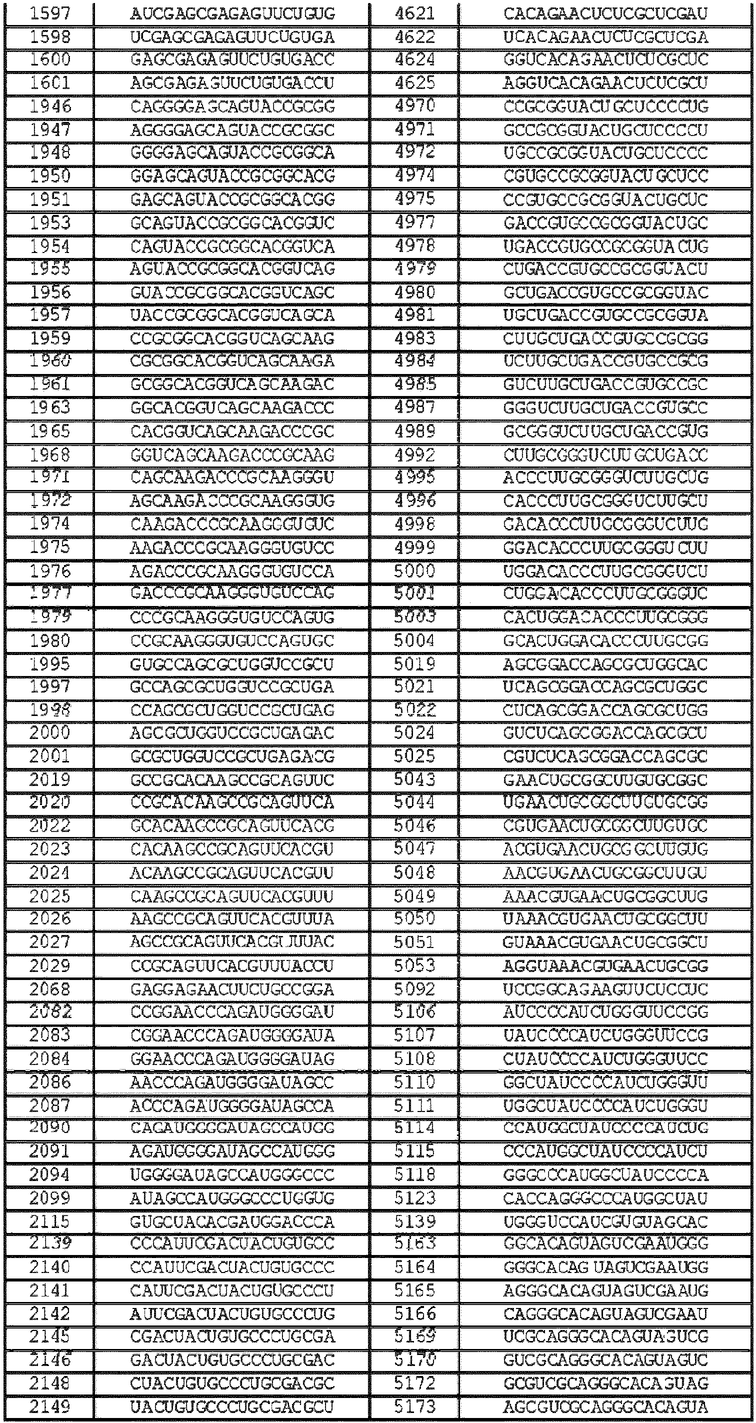

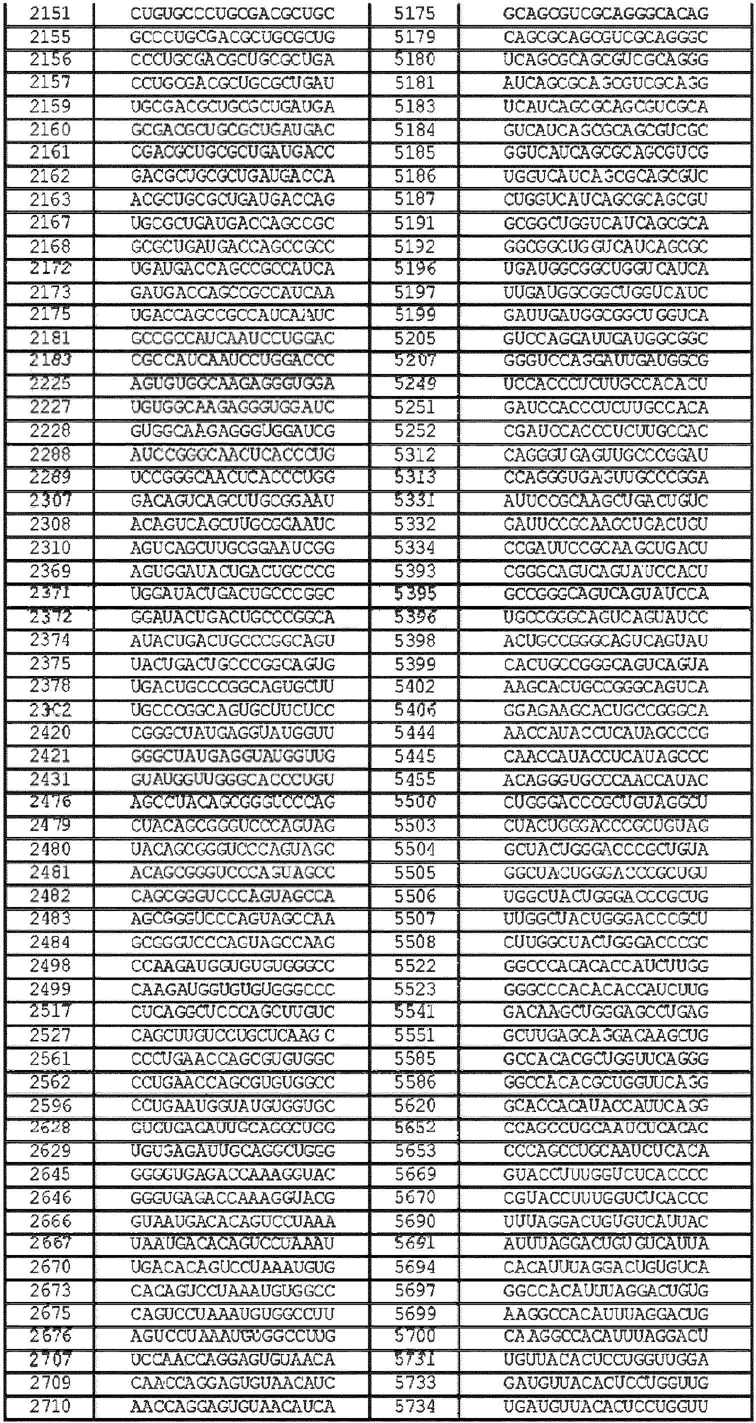

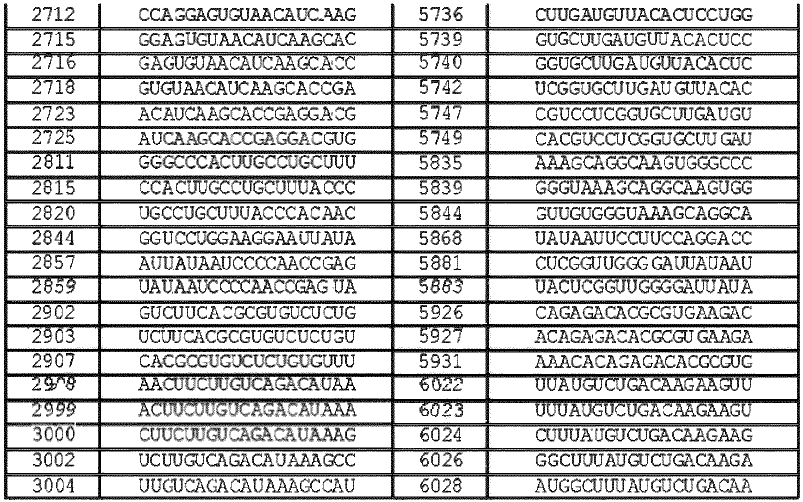

The siRNAs in subset A have the following characteristics:
- Cross-reactivity: With 19mer in human *MST1* mRNA, with 17mer'19merin NHP *MST1*
- Specificity category: For human and NTIP: AS2 or better, SS3 or better
- miRNA seeds: AS+SS strand: seed region not conserved in human, mouse, and rat and not present in >4 species
- Off-target frequency: ≤20 human off-targets matched with 2 mismatches in antisense strand
- SNPs: siRNA target sites do not harbor SNPs with a MAF ≥ 1% (pos. 2-18)

The siRNA sequences in subset A were selected for more stringent specificity to yield subset B. Subset B includes 197 siRNAs whose base sequences are shown in **Table 4.**

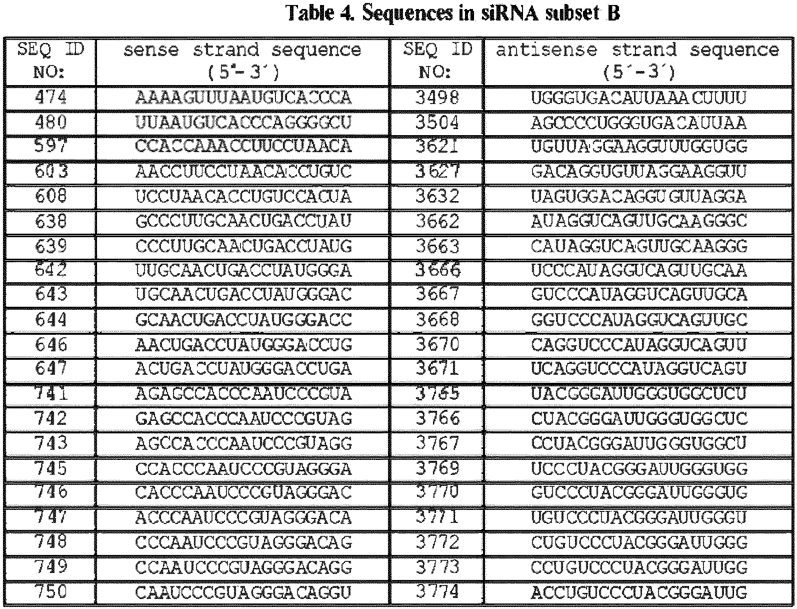

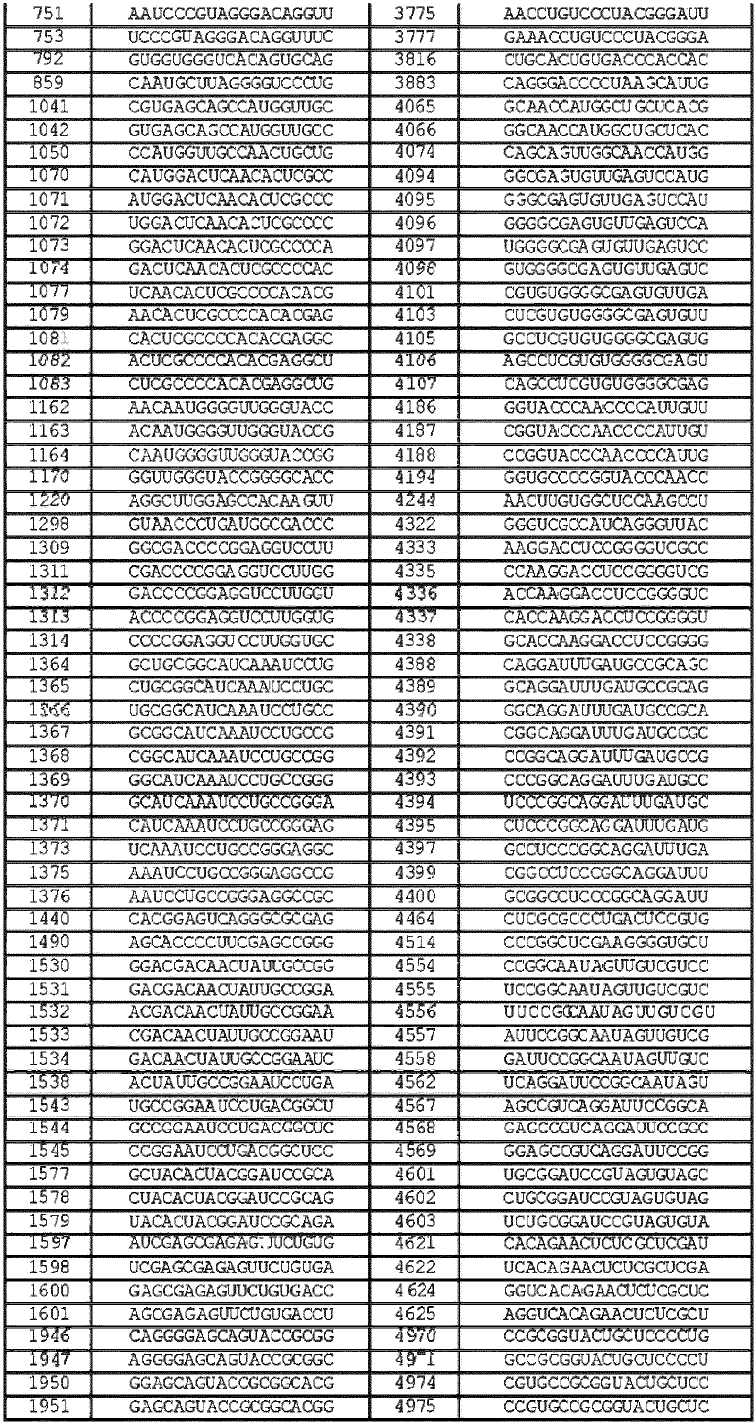

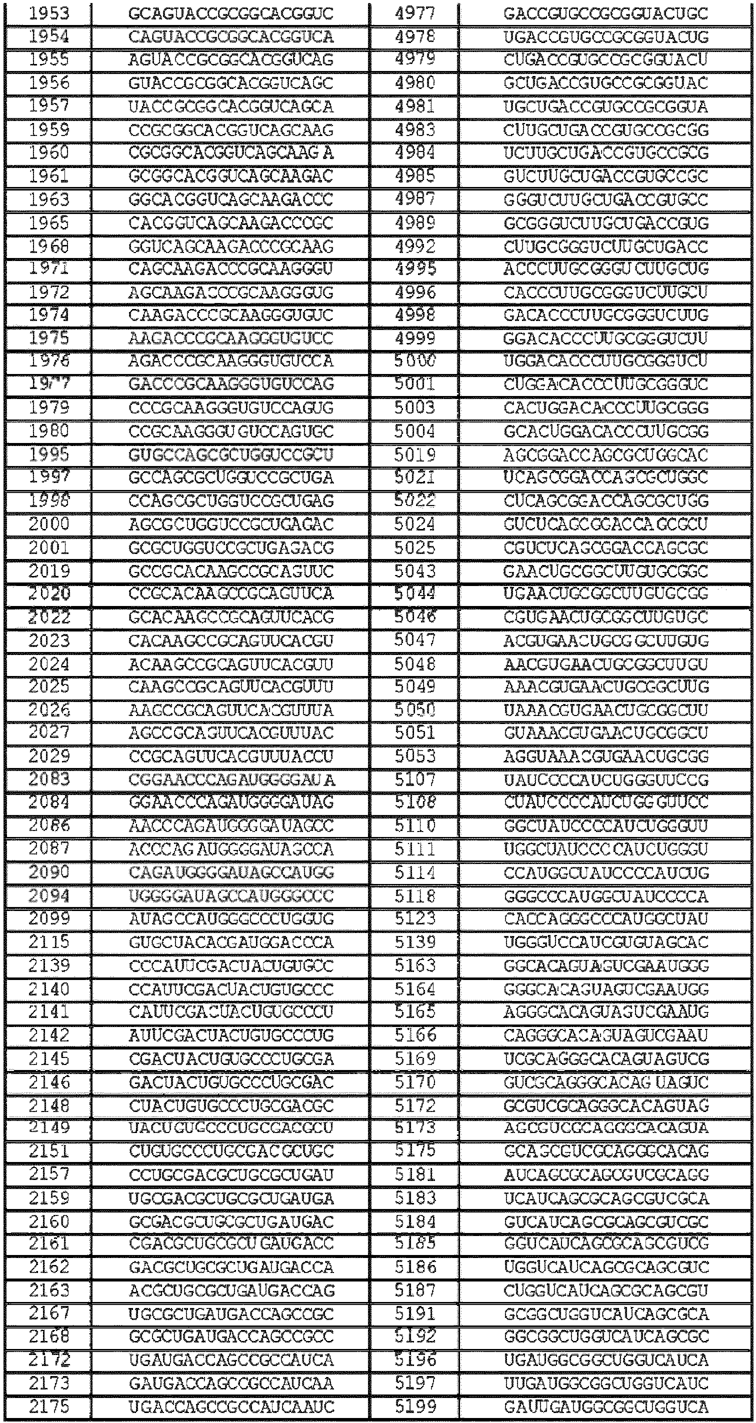

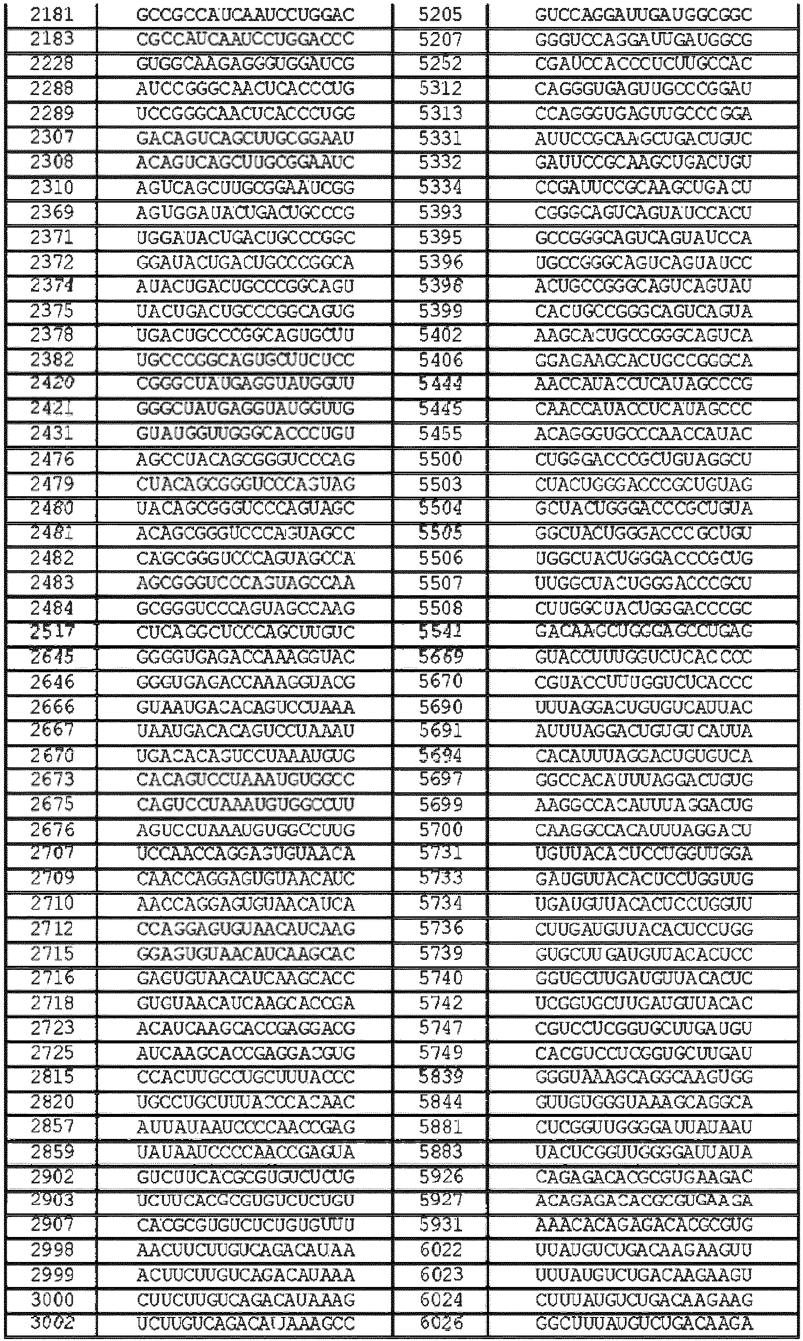

The siRNAs in subset B have the following characteristics:
- Cross-reactivity: With 19mer in human MST1 mRNA. with 17mer/19mer in NHP MST1
- Specificity category: For human and NHP: AS2 or better, SS3 or better
- miRNA seeds: AS+SS strand: seed region not conserved in human, mouse, and rat and not present in >4 species
- Off-target frequency: ≤15 human off-targets matched with 2 mismatches in antisense strand
- SNPs: siRNA target sites do not harbor SNPs with a MAF ≥ 1% (pos. 2-18)

The siRNA sequences in subset B were further selected for absence of seed regions in the AS strand that are identical to a seed region of known human miRNA to yield subset C. Subset C includes 140 siRNAs whose base sequences are shown in **Table 5.**

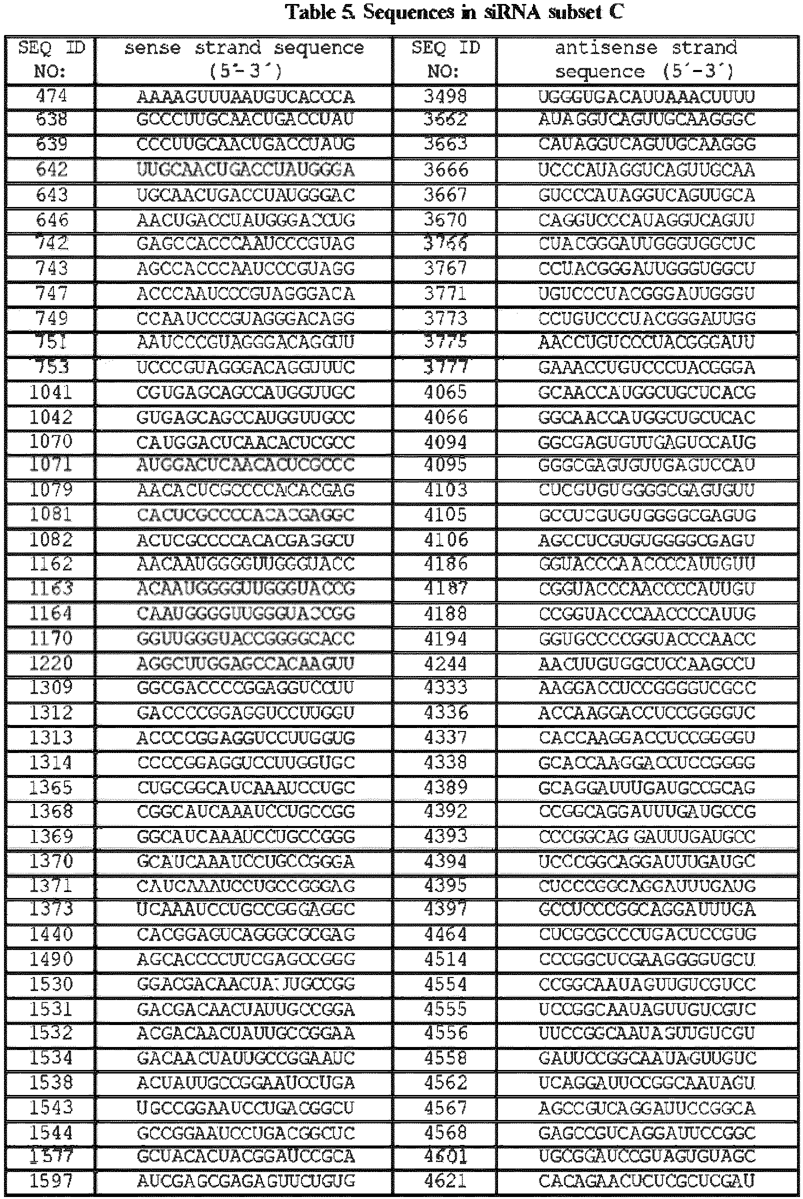

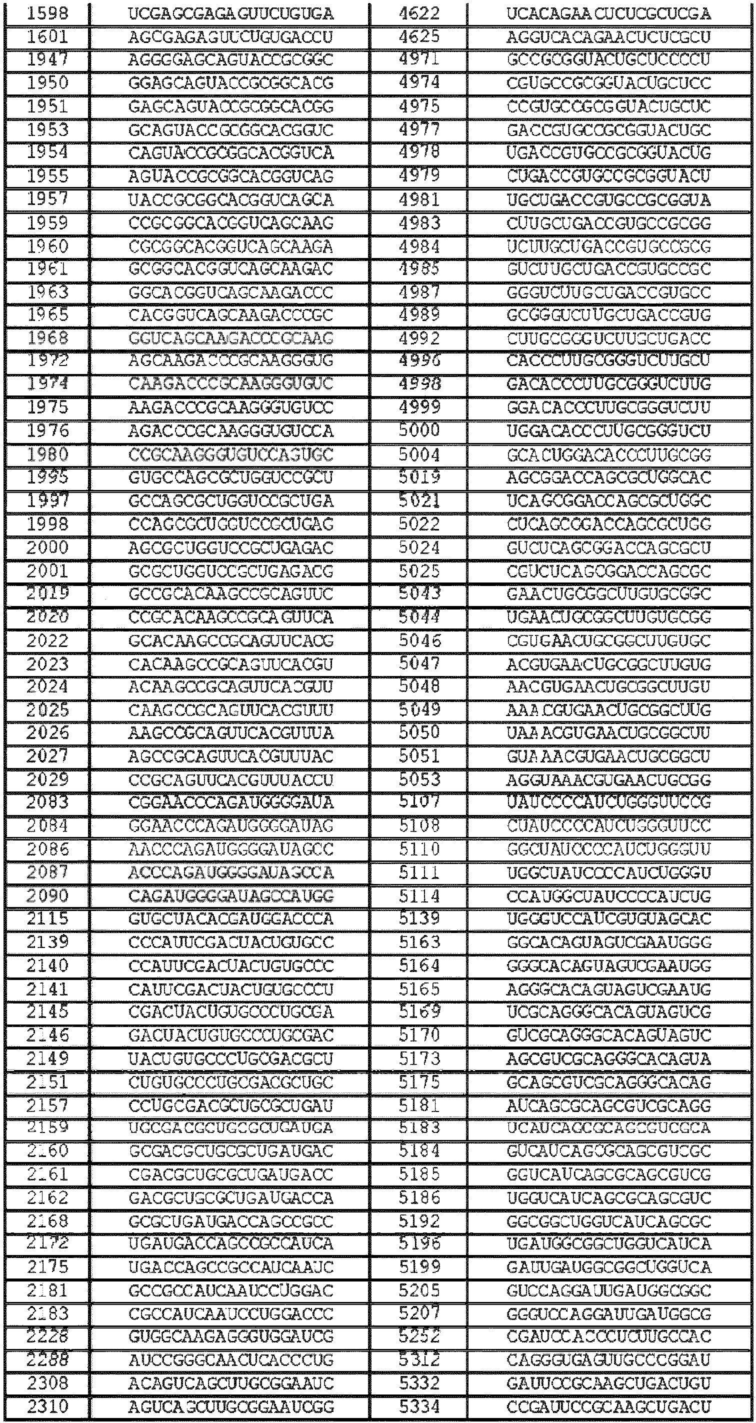

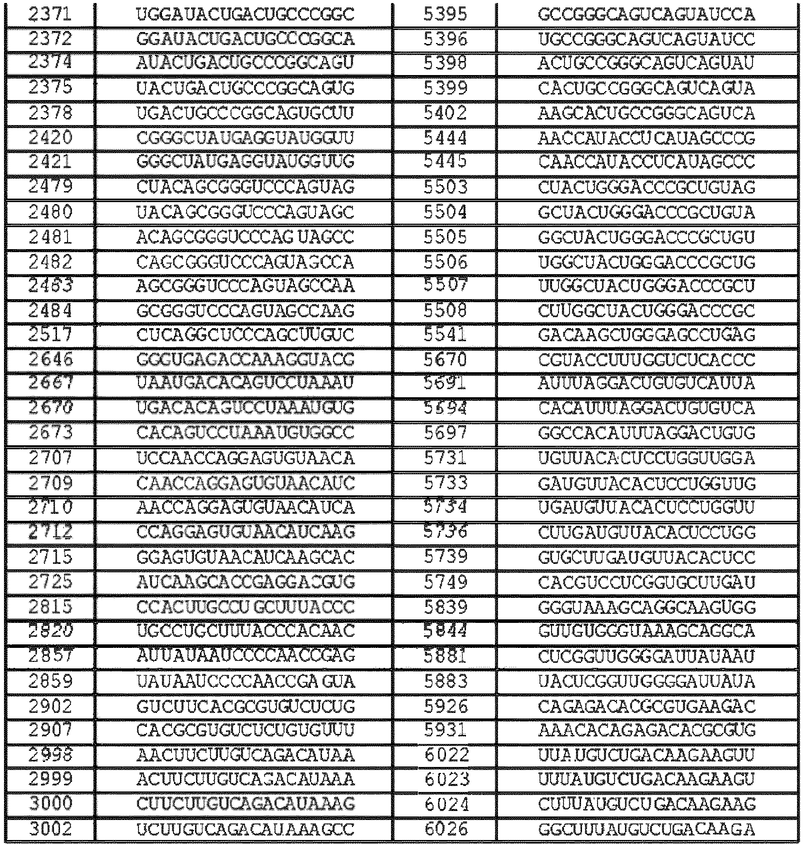

The siRNAs in subset C have the following characteristics:
- Cross-reactivity: With 19mer in human MST1 mRNA, with 17mer/19mer in NHP MST1
- Specificity category: For human and NHP: AS2 or better, SS3 or better
- miRNA seeds: AS+SS strand: seed region not conserved in human, mouse, and rat and not present in >4 species. AS strand: seed region not identical to seed region of known human miRNA
- Off-target frequency: ≤15 human off-targets matched with 2 mismatches by antisense strand
- SNPs: siRNA target sites do not harbor SNPs with a MAF ≥ 1% (pos. 2-18)

The siRNA sequences in subset C were also selected for absence of seed regions in the AS or S strands that are identical to a seed region of known human miRNA to yield subset D. Subset D includes 102 siRNAs whose base sequences are shown in **Table 6.**

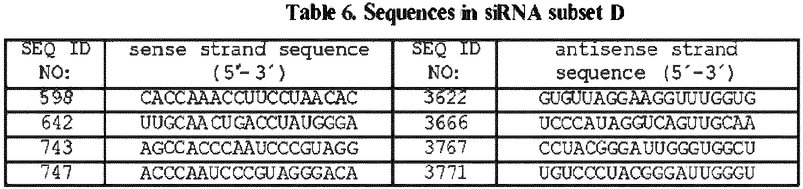

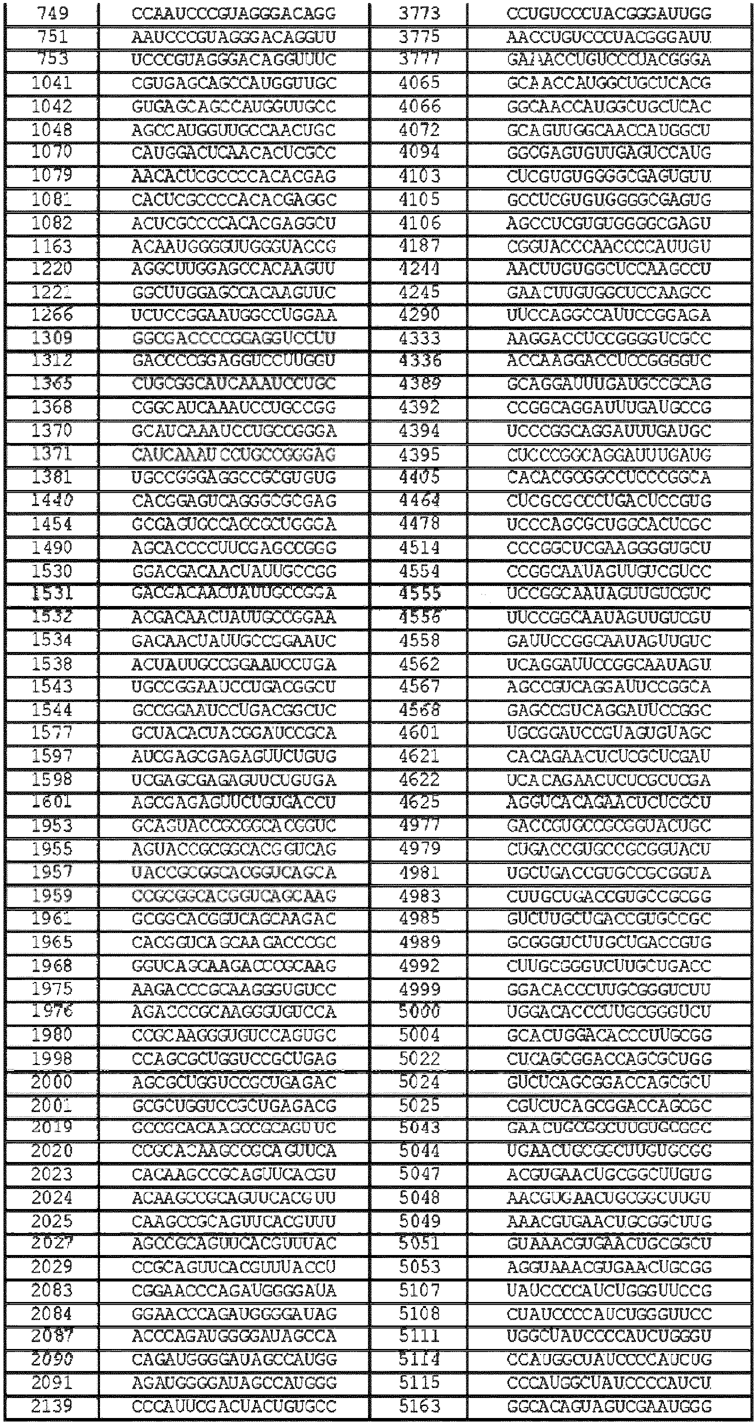

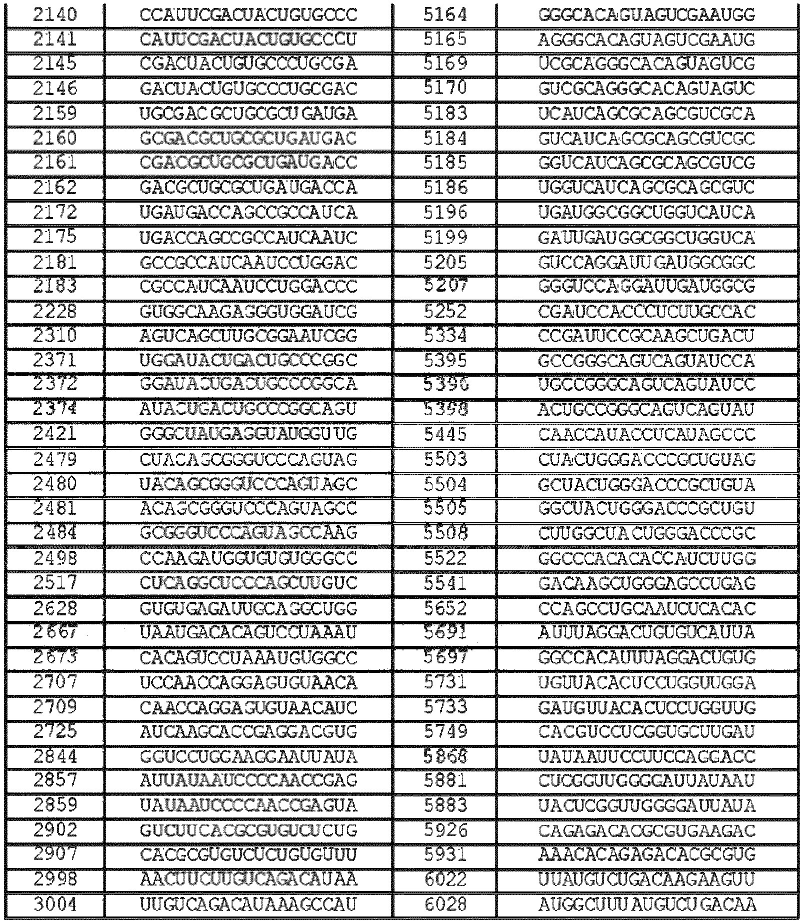

The siRNAs in subset D have the following characteristics:
- Cross-reactivity: With 19mer in human MST1 mRNA, with 17mer/19mer in NHP MST1
- Specificity category: For human and NHP: AS2 or better, SS3 or better
- miRNA seeds: AS+SS strand: seed region not conserved in human, mouse, and rat and not present in >4 species. AS+SS strand: seed region not identical to seed region of known human miRNA
- Off-target frequency. ≤20 human off-targets matched with 2 mismatches by antisense strand
- SNPs: siRNA target sites do not harbor SNPs with a MAF ≥ 1% (pos. 2-18)

The siRNA sequences in subset D were further selected for more stringent specificity to yield subset F. Subset E includes 91 siRNAs whose base sequences are shown in **Table7.**

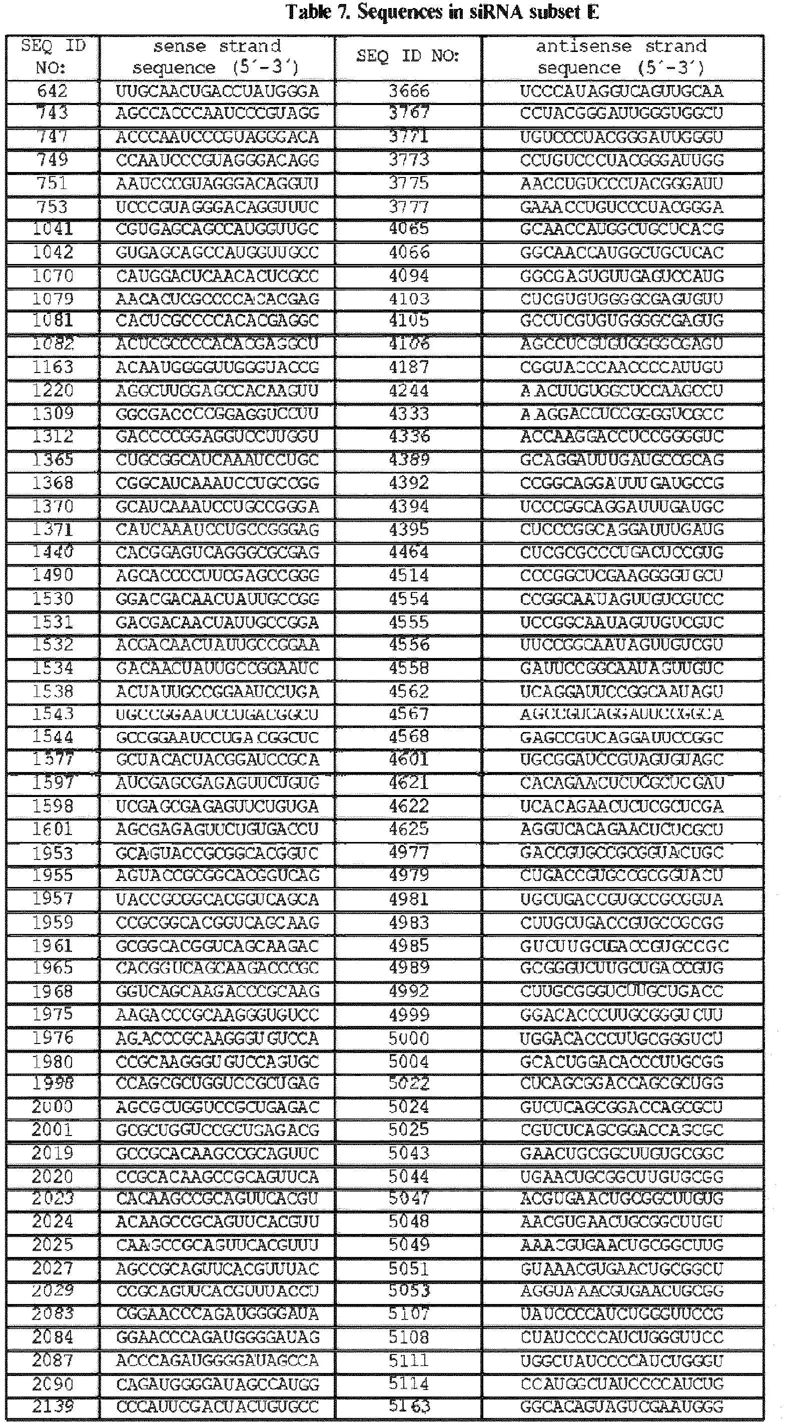

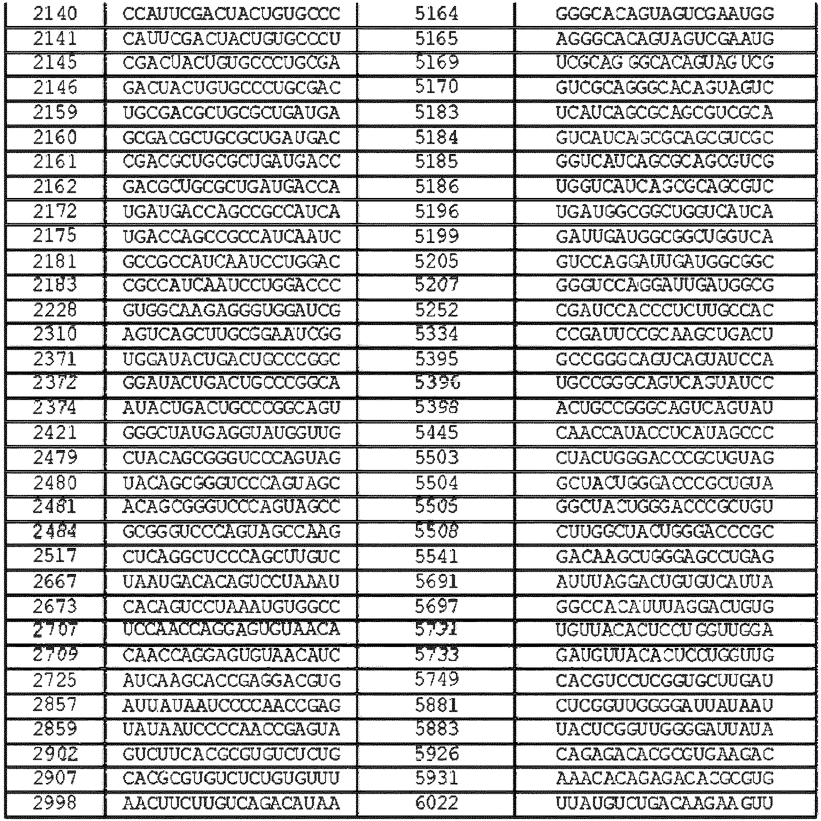

The siRNAs in subset E have the following characteristics:
- Cross-reactivity: With 19mer in human MST1 mRNA, with 17mer/19mer in NHP MST1
- Specificity category: For human and NHP: AS2 or better, SS3 or better
- miRNA seeds: AS+SS strand: seed region not conserved in human, mouse, and rat and not present in >4 species. AS+SS strand: seed region not identical to seed region of known human miRNA
- Off-target frequency: ≤15 human off-targets matched with 2 mismatches by antisense strand
- SNPs: siRNA target sites do not harbor SNPs with a MAF ≥ 1% (pos. 2-18)

Subset F includes 38 siRNAs. The siRNAs in subset F include siRNAs from subset A and are included in **Table 8.** In some cases, the sense strand of any of the siRNAs of subset F comprises modification pattern 6S (**Table 9**). In some cases, the antisense strand of any of the siRNAs of subset F comprises modi fication pattern 7AS (**Table9**). In some cases, the sense strand of any of the siRNAs of subset F contains an alternative modification pattern (**Table 10**). In some cases, the antisense strand of any of the siRNAs of subset **F** comprises modification pattern 7AS (**Table 10**). The siRNAs in subset **F** may comprise any other modification pattern(s). In **Table9** and **Table 10,** Nf (e.g. Af, Cf, Gf, Tf, or Uf) is a2' fluoro-modified nucleoside, n(e.g. a, c, g, t, or u) is a2⁻ O-methyl modified nucleoside, and "s" is a phosphorothioate linkage.

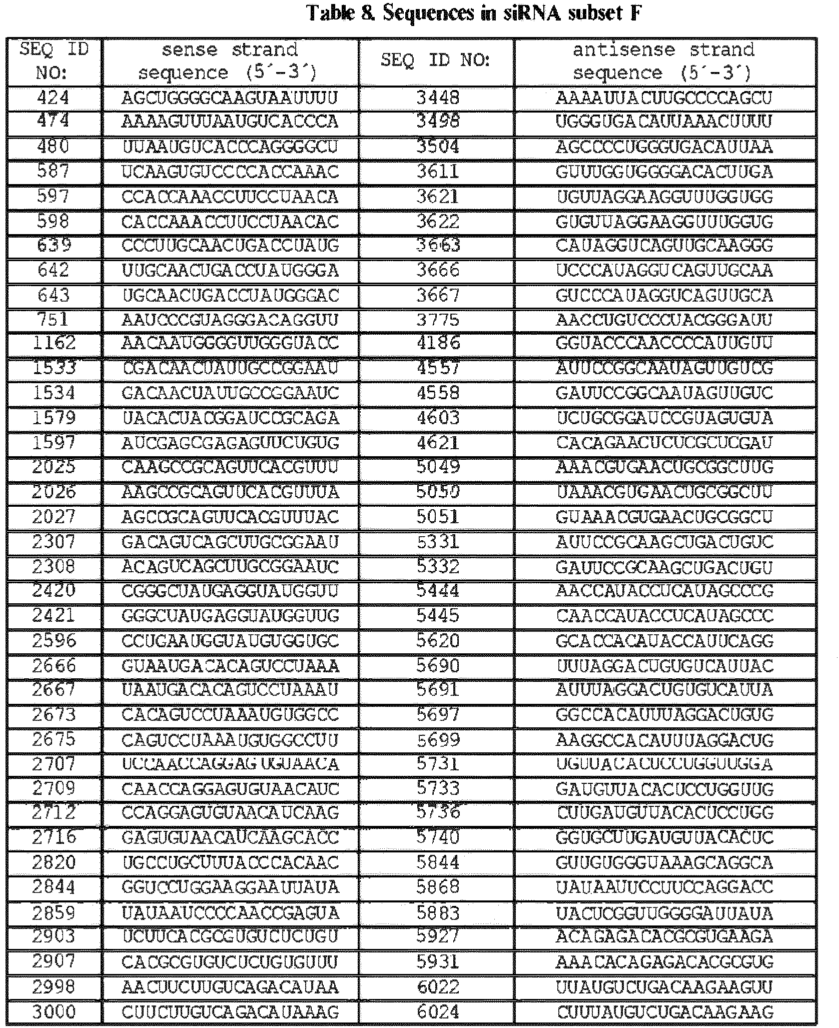

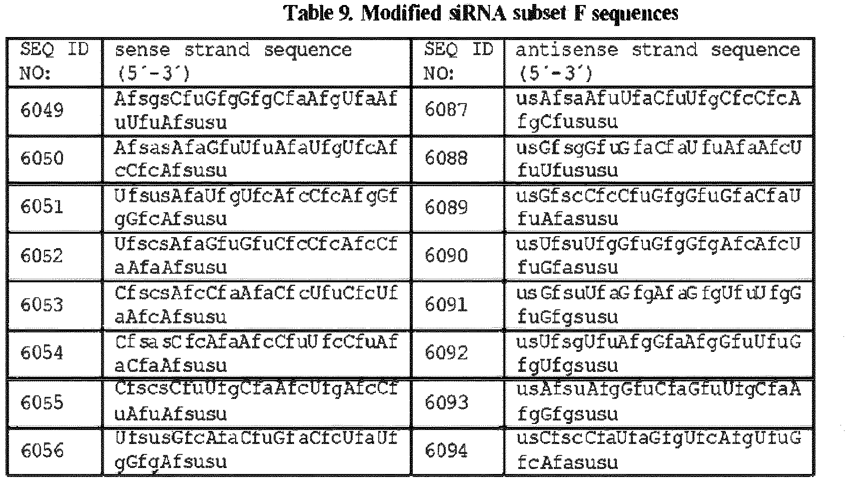

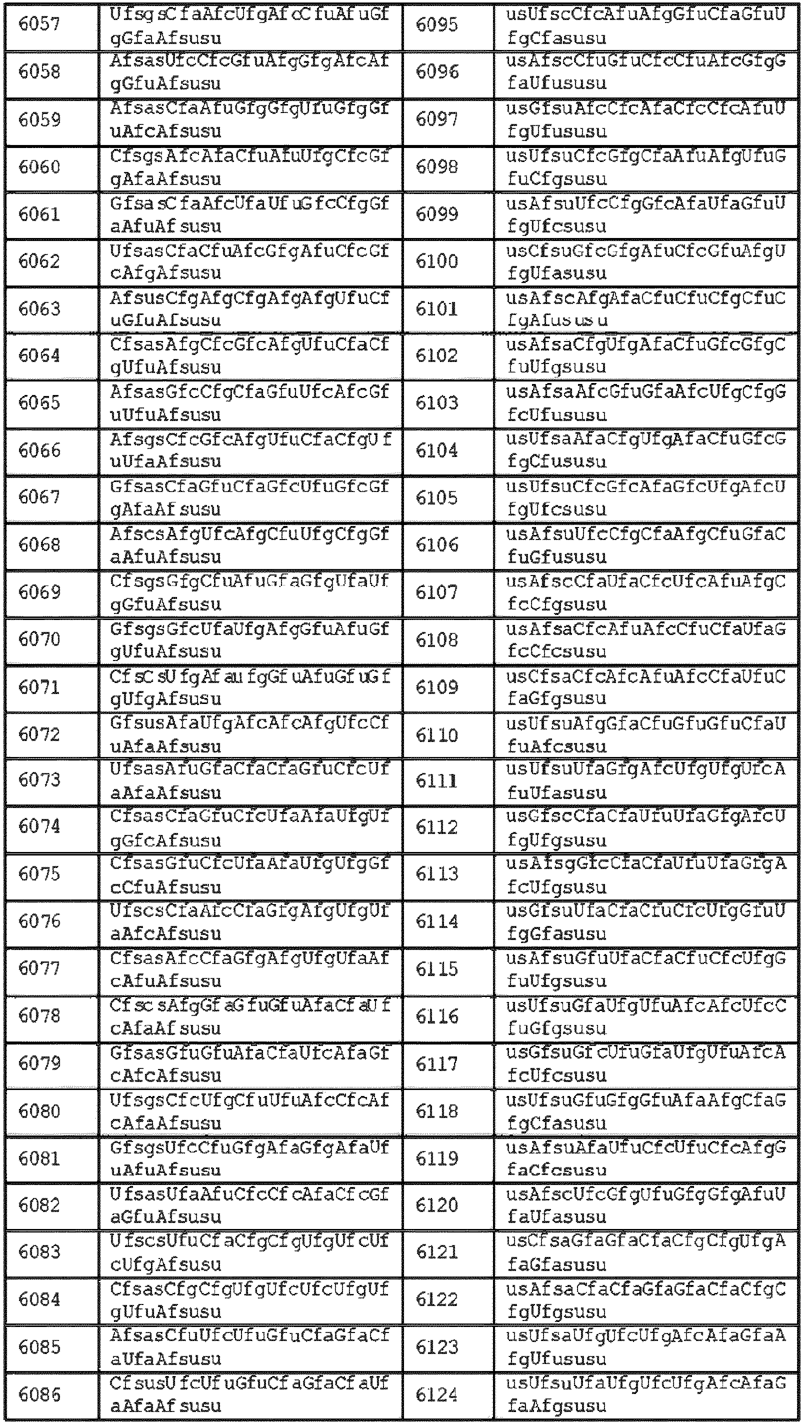

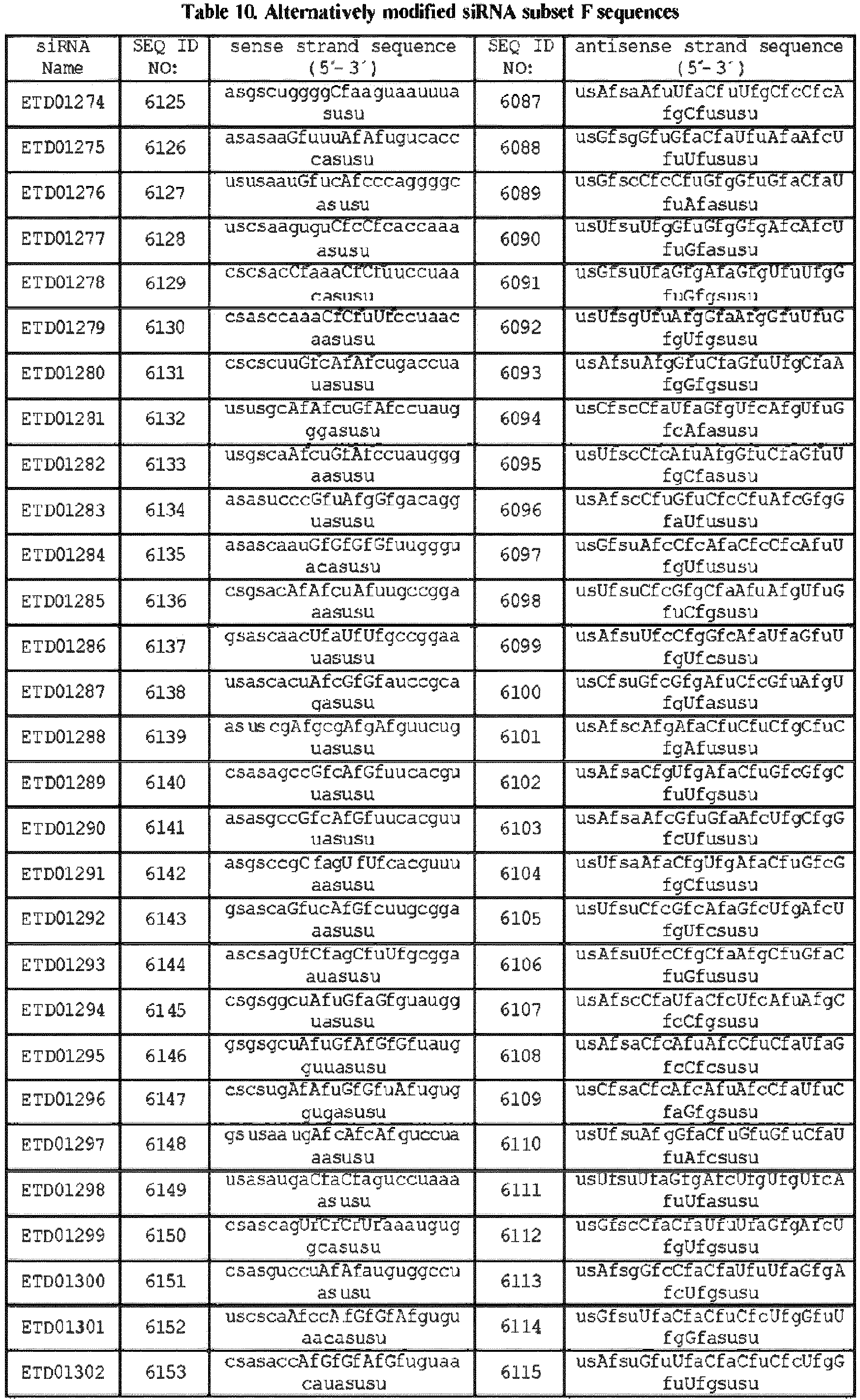

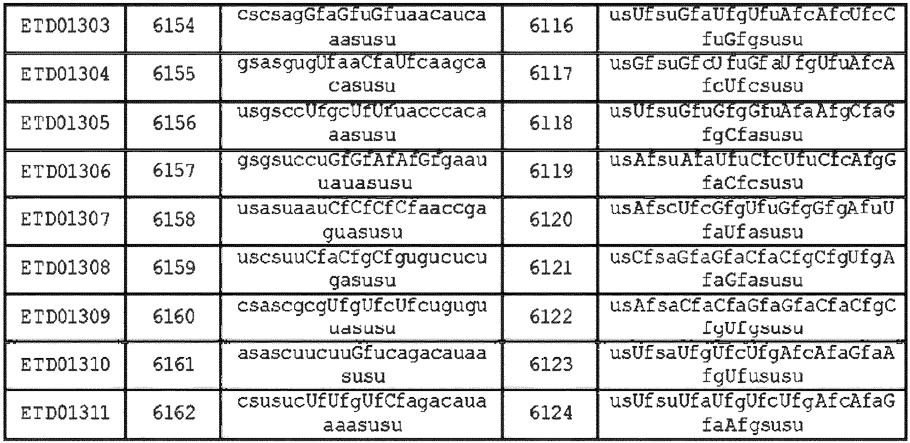

Any siRNA among any of subsets A-H may comprise any modification pattern described herein. If a sequence is a different number of nucleotides in length than a modification pattern, the modification pattern may still be used with the appropriate number of additional nucleotides added 5'or 3' to match the number of nucleotides in the modification pattern. For example, if a sense or anti sense strand of the siRNA among any of subsets A-F comprises 19 nucleotides, and a modification pattern comprises 21 nucleotides, UU may be added onto the 5'end of the sense or antisense strand.

### Example 3: Screening MSTlsiRNAs for activity in human cells in culture

Chemically modified MST1 siRNAs cross reactive for human and non-human primate and derived from sequences in siRNA subset F (**Table 8**) and shown in **Table 10** were assayed for MSTI mRNA knockdown activity in cells in culture. Hep 3B21-7 cells (ATCC^{®} HB-8064^{™}) were seeded in 96-well tissue culture plates at a cell density of 7,500 cells per well in EMEM(ATCC Catalog No. 30-2003) supplemented with 10% fetal bovine serum and incubated overnight in a water-jacketed, humidified incubator at 37°C in an atmosphere composed of air plus 5% carbon dioxide. The MST1 siRNAs were individually transfected into Hep 3B2.1-7 cells in duplicate wells at 10 nM final concentration using 0.15 µL Lipofectamine RNAiMax (Fisher) per well. Silencer Select Negative Control #1 (ThermoFisher, Catalog# 4390843) was transfected at 10 nM final concentration as a control. Silencer Select human MST1 (ThermoFisher, Catalog# 4427037, ID: s8994)) was transfected at 10 nM final concentration and used as a positive control. After incubation for 48 hours at 37°C, total RNA was harvested from each well and cDNA prepared using TaqMan^{®} Fast Advanced Cells-to-CT^{™} Kit (ThermoFisher, Catalog# A35374) according to the manufacturer's instructions. The level of MST1 mRNA from each well was measured in triplicate by real-time qPCR on a QuantStudio^{™} 6 Pro Real-Time PCR System using TaqMan Gene Expression Assay for human MST1 (ThermoFisher, assay# Hs003606g4_ml). The level of PPIA mRNA was measured using TaqMan Gene Expression Assay (ThermoFisher, assay# Hs99999904_m1) and used to determine relative MSTI mRNA levels in each well using the delta-delta Ct method. All data was normalized to relative MST1 mRNA levels in untreated Hep 3B2.1-7 cells. The results are shown in **Table 11.** The siRNAs ETD01290, ETD01274, ETD01298, ETD01299, ETD01296, ETD01297, ETD01281, ETD01303, ETD01308, ETD01289, ETD01302, ETD01305 and ETD01306 reduced MST1 levels by greater than 50% when transfected at 10 nM.

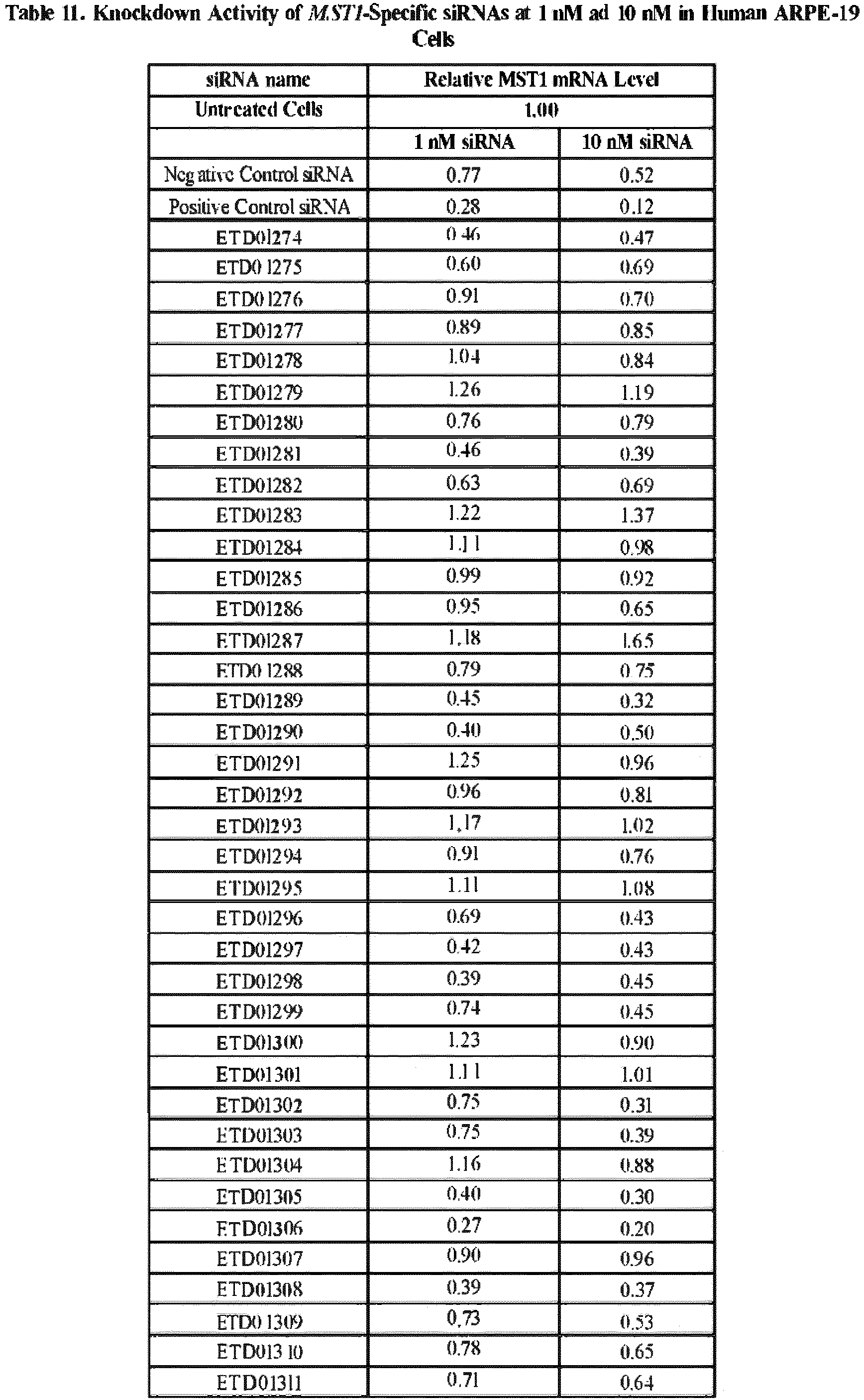

### Example 4: Determining the IC50 of MST1siRNAs

The IC50 values for knock down of *MST1* mRNA by select MST1 siRNAs will be determined in Hep 3B2.1-7 cells (ATCC^{®} HB-8064^{™}) cells. The siRNAs will be assayed individually at 30 nM, 10 nM,3 nM, 1 nM and 0.3 nM, or 3 nM, 1nM, 0.3 nM. 0.1 nM and 0.03 nM, or 30nM, 10nM, 3nM, 1 nM, 0.3nM. 0.1 nM and 0.03 nM. The HepG2 cells will be seeded in 96-well tissue culture plates a a cell density of 7,500 cells per well in EMEM(ATCC Catalog No. 30-2003) supplemented with 10% fetal bovine serum and incubated overnight in a water-jacketed, humidified incubator at 37°C in an atmosphere composed of air plus 5% carbon dioxide. The MST1 siRNAs will be individually transfected into HepG2 cells in triplicate wells using 0.15 µL Lipofectamine RNAMax (Fisher) per well. After incubation for 48 hours at 37°C, total RNA will be harvested from each well and cDNA prepared using TaqMan^{®} Fast Advanced Cells-to-CT^{™} Kit (ThermoFisher, Catalog# A35374) according to the manufacturer's instructions. The level of *MST1* mRNA from each well will be measured in triplicate by real-time qPCR on a QuantStudio^{™} 6 Pro Real-Time PCR System using TaqMan Gene Expression Assay for human *MST1* (ThermoFisher, assay# Hs00360684_ml). The level of PPIA mRNA will be measured using TaqMan Gene Expression Assay (ThermoFisher, assays Hs99999904_m1) and used to determine relative *MST1* mRNA levels in each well using the delta-delta Ct method. All data will be normaliz ed to relative *MST1* mRNA levels in untreated HepG2 cells. Curve fit will be accomplish using the [inhibitor| vs. response (three parameters) function in GraphPad Prism software.

### Example 5: siRNA-mediated knockdown of MSTlin Hep3B2.1-7cell line

siRNAs targeting *MST1* mRNA may downregulate levels of *MST1* mRNA and MSP, leading to a decrease in MSP secretion, when administered to the cultured human hepatocyte cell line, Hep 3B2.1-7 cells (ATCC^{®} HB-806 4^{™}). Accordingly, these results will demonstrate that siRNAs targeting MST1 mRNA *in vivo* will also downregulate levels of *MST1* mRNA and MSP. leading to a decrease in MSP secretion into the bloodstream. The accompanying decrease in circulating MSP levels may improve lung conditions, particularly in subjects with lung disorders.

On Day 0, Hep 3B2, 1-7 cells are to be seeded at 150,000 cells/mL into a Falcon 24-well tissue culture plate (ThermoFisher Cat. No. 353047) at 0.5 mL per well

On Day 1, MST1 siRNA and negative control siRNA master mixes are prepared. The MST1 siRNA master mix contains 350 µL of Opti-MEM (ThermoFisher Cat No. 4427037 - s1288 Lot No. AS02B02D) and 3.5 µL of a mix ture of the two MST1 siRNAs (10 µM stock). The negative control siRNA master mix contains 350 µL of Opti-MEM and 3.5 µL of negative control siRNA (ThermoFisher Cat. No. 4390843, 10 µM stock). Next, 3 µL of Trans1T-X2 (Mirus Cat. No. MIR-6000) is added to each master mix. The mixes are incubated for 15 minutes to allow transfection complexes to form, then 51 µL of the appropriate master mix+ TransIT-X2 is added to duplicate wells of HEPG2 cells with a final siRNA concentration of 10 nM.

On Day 3, 48 hours post transfection, media is collected and mixed with protein lysis buffer containing protease and phosphatase inhibitors, and the cells are lysed using the Cells-to-Ct kit according to the manufacturer's protocol (ThermoFisher Cat. No. 4399002). For the Cells-to-Ct, cells are washed with 50 µL using cold 1X PBS and lysed by adding 49.5 µL of Lysis Solution and 0.5 µL DNase 1per well and pipetting up and down 5 times and incubating for 5 minutes at room temperature. The Stop Solution (5 µL/well) is added to each well and mixed by pipetting up and down five times and incubating at room temperature for 2 minutes. The reverse transcriptase reaction is performed using 22.5 µL of the lysate according to the manufacturer's protocol. Samples are stored at -80°C until real-time qPCR is performed in triplicate using TaqMan Gene Expression Assays (Applied Biosystems FAM/MST1 using a BioRad CFX96 Cat. No. 1855195). For the protein quantification, equivalent quantities (30-50 µg) of protein are separated by 10% SDS polyacrykimide gels and transferred to polyvinylidene fluoride membranes. Membranes are blocked with 5% nonfat milk and incubated overnight wih the appropriate primary antibody at dilutions specified by the manufacturer. Next, the membranes are washed three times in TBST and incubated with the corresponding horseradish peroxidase conjugated secondary antibody at 1:5000 dilution for 1 hr. Bound secondary antibody is detected using an enhanced chemiluminescence system. The primary immunoblotting antibody is an anti-MSP antibody (Abcam, Cambridge, UK).

A decrease in *MST1* mRNA and MSP expression in the Hep 3B2.1-7 cells is expected afler transfection with the MST1 siRNAs compared to *MST1* mRNA and MSP levels in HEPG2 cells transfected with the non-specific control siRNA 48 hours after transfection. There is an expected decrease in the amount of *MST1* mRNA and secreted MSP, measured by quantifying the amount of *MST1* mRNA and MSP in media of Hep 3B2.1-7 cells transfected with the MST1 siRNAs relative to the amount of *MST1* mRNA and MSP in media of Hep 3B2.1-7 cells transfected with anon-specific control siRNA 48 hours after transfection. These results are expected to show that the MST1 siRNAs elicit knockdown of *MST1* mRNA in Hep 3B2.1-7 cells and that the decrease in *MST1* expression may correspond with a decrease in *MST1* mRNA and MSP secretion.

### Example 6: ASO-mediated knockdown of MST1 in Hep 3B2.1-7 cell line

ASOs targeting *MST1* mRNA may downregulate levels of *MST1* mRNA and MSP, leading to a decrease in MSP secretion, when administered to the cultured human hepatocyte cell line, Hep 3B2.1-7. Accordingly, these results will demonstrate that siRNAs targeting *MST1* mRNA *in vivo* will also downregulate levels of *MST1* mRNA and MSP, leading to a decrease in MSP secretion into the bloodstream. The accompanying decrease in circulating MSP levels may improve lung conditions, particularly in subjects with lung disorders.

On Day 0, Hep 3B2.1-7 cells are to be seeded at 150,000 cells/mL into a Falcon 24-well tissue culture plate (ThermoFisher Cat. No. 353047) at 0.5 mL per well.

On Day 1, MST1 ASO and negative control ASO master mixes are prepared. The MST1 ASO master mix contains 350 µL of Opti-MEM (ThermoFisher Cat. No. 4427037- s1288 Lot No. AS02B02D) and 3.5 µL of a mixture of the two MST1 ASOs (10 µM stock). The negative control ASO master mix contains 350 µL of Opti-MEM and 3.5 µL of negative control ASO(ThermoFisherCat. No. 4390843, 10 µM stock). Next, 3 µL of TransIT-X2 (Mirus Cat. No. MIR-6000) is added to each master mix. The mixes are incubated for 15 minutes to allow transfection complexes to form, then 51 µL of the appropriate master mix + Trans1 T-X2 is added to duplicate wells of HEPG2 cells with a final ASO concentration of 10nM.

On Day 3, 48 hours post transf action, media is collected and mixed with protein lysis buffer containing protease and phosphatase inhibitors, and the cells are lysed using the Cells-to-Ct kit according to the manufacturer's protocol (ThermoFisher Cat. No. 4399002). For the Cells-to-Ct, cells are washed with 50 µL using cold 1X PBS and lysed by adding 49.5 µL of Lysis Solution and 0.5 µL DNase I per well and pipetting up and down 5 times and incubating for 5 minutes at room temperature. The Stop Solution (5 µL/well) is added to each well and mixed by pipetting up and down five times ard incubating at room temperature for 2 minutes. The reverse transenptase reaction is performed using 22.5 µL of the lysate according to the manufacturer's protocol. Samples are stored at -80 °C until real-time qPCR is performed in triplicate using TaqMan Gene Expression Assays (Applied Biosystems FAM/MST1 using a BioRad CFX96 Cat No. 1855195). For the protein quantification, equivalent quantities (30-50 µg) of protein are separated by 10% SDS polyacrylamide gels and transferred to poly vinylidene fluoride membranes. Membranes are blocked with 5% nonfat milk and incubated overnight with the appropriate primary antibody at dilutions specified by the manufacturer. Next, the membranes are washed three times in TBST and incubated with the corresponding horseradish peroxidase conjugated secondary antibody at 1:5000 dilution for 1 hr. Bound secondary antibody is detected using an enhanced chemiluminescence system. The primary immunoblotting antibody is an anti-MSP antibody (Abcam, Cambridge, UK).

A decrease in *MST1* mRNA and MSP expression in the Hep 3B2.1-7 cells is expected after transfection with the MSTI ASOs compared to *MST1\* mRNA levels in Hep 3B2.1-7 cells transfected with the non-specific control ASO 48 hours after transfection. There is an expected decrease in the amount of *MST1* mRNA and secreted MSP, measured by quantifying the amount of *MST1* mRNA and MSP in media of Hep 3B2. 1-7 cells transfected with the MST1 ASOs relative to the amount of *MST1* mRNA and MSP in media of Hep 3B2. 1-7 cells transfixted with anon-specific control ASO 48 hours after transfection. These results are expected to show that the MST1 ASOs elicit knockdown of *MST1* mRNA and MSP in HEPG2 cells and that the decrease in *MST1* expression may correspond with a decrease in *MST1* mRNA and MSP secretion.

### Example7: Inhibition of MST1 inaMouseModel of Lung Inflammation ViaAcute Cigarette Smoke Exposure Using MST1 siRNAs or ASOs

In this experiment, a mouse model of lung inflammation induced by acute cigarette smoke exposure is used to evaluate the effect of siRNA or ASO inhibition of *MST1.* In this cigarette smoke induced model, mice are exposed to cigarette smoke for 3 hours which will result in a transient inflammatory response. Lung inflammation is assessed by measuring neutrophils and macrophages in bronchoalveolar lavage fluid and lung tissue.

Briefly, mice are divided into six groups: Group 1 - a group treated with non-targeting control siRNA and cigarette smoke inhalation, Group 2 - a group treated with non-targeting control ASO and cigarette smoke inhalation, Group 3 - a group treated with MST1 siRNA1 and cigarette smoke inhalation, Group 4 - a group treated with MST1 ASO1 and cigarette smoke inhalation, Group 5 - a group treated with vehicle and cigarette smoke inhalation, Group 6 - a group treated with vehicle and not receiving cigarette smoke stimulus. Each group contains eight mice (4 males, 4 females).

Administration of siRNA or ASO is achieved with a 200 µL subcutaneous injection of siRNA or ASO resuspended in PBS at concentration of 10 µM. At Time 0, Group 1 mice are injected subcutaneously with non-targeting control siRNA, Group 2 mice are injected subcutaneously with non-targeting control ASO, Group 3 mice are injected subcutaneously with siRNA1 targeting mouse *MST1*, Group 4 mice are injected subcutaneously with ASO1 targeting mouse *MST1,* and Group 5 and 6 mice are injected subcutaneously with vehicle.

24 hours alter the smoke inhalatio n treatment, bronchoalveolar lavage fluid is collected and the mice are sacrificed by cervical dislocation following an intraperitoneal injection of 0.3 ml Nembutal (5 mg/ml) (Sigma Cat. No. 1507002). Final blood samples are collected, and livers and lungs are removed, and a section placed in RN Alater for mRNA isolation.

mRNA is isolated from tissue placed in RNAlater solution using the PureLink kit according to the manufacturer's protocol (ThermoFisher Cat. No. 12183020). The reverse transcriptase reaction is performed according to the manufacturer s protocol Samples are stored at -80 °C until real-time qPCR is performed in triplicate using TaqMan Gene Expression Assays (Applied Biosystems FAM/MSTI using a BioRad CFX96 Cat. No. 1855195). A decrease in *MST1* mRNA and MSP expression in the liver tissue and circulating MSP in the blood from mice dosed with the MST1 siRNA1 or ASO1 is expected compared to *MST1* mRNA or MSP expression in the liver tissue and circulating MSP in the blood from mice dosed with the non-specific controls. There is an expected decrease in neutrophil and macrophage counts in the bronchoal veolar lavage fluid in cigarette smoke exposed mice that receive the MST1 siRNA or ASO compared to the neutrophil and macrophage counts in the bronchoalveolar lavage fluid in cigarette smoke exposed mice that receive the non-specific control. These results are expected to show that the MST1 siRNA or ASO elicits knockdown of *MST1* mRNA and MSP in liver tissue and reduces circulating MSP, and that the decrease in *MST1* mRNA and MSP expression may correspond with a decrease in neutrophil and macrophage counts in the bronchoalveolar lavage fluid in mice exposed to cigarette smoke.

### Example 8: Inhibition of MST1 in a Mouse Model of COPD Using MST1 siRNAs or ASOs

In this experiment, a mouse model of cigarette smoke induced COPD is to be used to evaluate the effect of siRNA or ASO inhibition of *MST1*. In the cigarette smoke induced COPD model, mice are exposed to cigarette smoke for 6 months to mimic patients with a substantial history of cigarette smoking. Lung inflammation is assessed by measuring neutrophil and macrophage in bronchoalveolar lavage fluid and lung tissue. Lung function is also assessed by measuring tidal volume, resistance and dynamic compliance. Additionally, lung morphology and air space enlargement is assessed by fixing and staining the lungs and measuring structural parameters such as air space, septal wall thickness and mean linear intercept.

Briefly, mice are divided into six groups: Group 1 - a group treated with non-targeting control siRNA and cigarette smoke inhalation, Group 2 - a group treated with non-targeting control ASO and cigarette smoke inhalation, Group 3 - a group treated with MST1 siRNA1 and cigarette smoke inhalation, Group 4 - a group treated with MST1 ASOI and cigarette smoke inhalation, Group 5 - a group treated with vehicle and cigarette smoke e inhalation, Group 6-a group treated with vehicle and not receiving cigarette smoke stimulus. Each group contains eight mice (4 males, 4 females).

Administration of siRNA or ASO is achieved with a 200 µL subcutaneous injection of siRNA or ASO resuspended in PBS at concentration of 10 µM. On Study Day 0, Group 1 mice are injected subcutaneously with non-targeting control siRNA, Group 2 mice are injected subcutaneously with non-targeting control ASO, Group 3 mice are injected subcutaneously with siRNA1 targeting mouse *MST1.* Group 4 mice are injected subcutaneously with ASO1 targeting mouse *MST1,* and Group 5 and 6 mice are injected subcutaneously with vehicle. Every 14 days after the first injection animals from each group will be dosed for a total of 12 injections.

24 hours after the final smoke inhalation treatment, bronchoalveolar lavage fluid is collected and the mice are sacrificed by cervical dislocation following an intraperitoneal injection of 0.3 ml Nembutal (5 mg/ml) (Sigma Cat. No. 1507002). Final blood samples are collected, and livers and lungs are removed, and a section placed in RNAlater for mRNA isolation or fixed with paraformaldehyde and then embedded in paraffin for tissue sectioning.

mRNA is isolated from tissue placed in RNAlater solution using the PureLink kit according to the manufacturer's protocol (ThermoFisher Cat. No. 12183020). The reverse transcriptase reaction is performed according to the manufacturer's protocol. Samples are stored at -80 °C until real-time qPCR is performed in triplicate using TaqMan Gene Expression Assays (Applied Biosystems FAM/MSTI using a BioRad CFX96 Cat. No. 1855195). A decrease in *MST1* mRNA and MSP expression in the liver tissue and circulating MSP in the blood from mice dosed with the MST1 siRNA1 or ASOI is expected compared to *MST1* mRNA and MSP expression in the liver tissue and circulating MSP in the blood from mice dosed with the non-specific controls. There is an expected decrease in neutrophil and macrophage counts in the bronchoal veolar lavage fluid in cigarette smoke exposed mice that receive the MST1 siRNA or ASO compared to the neutrophil and macrophage counts in the bronchoalveolar lavage fluid in cigarette smoke exposed nuce that receive the non-specific control There is also an expected decrease in air space and mean linear intercept and an increase in septal wall thickness in cigarette smoke exposed mice that receive the MST1 siRNA or ASO compared to the air space, mean linear intercept and septal wall thickness in cigarette smoke exposed mice that receive the non-specific control. Additionally, there is also an expected decrease in compliance and tidal volume and an increase in resistance in cigarette smoke exposed mice that receive the MST1 siRNA or ASO compared to the compliance, tidal volume and resistance in cigarette smoke exposed mice that receive the non-specific control. These results will show that an MST1 siRNA or ASO may elicit knockdown of *MST1* mRNA and MSP in liver tissue and reduce circulating MSP, and that the decrease in *MST1* mRNA and MSP expression may correspond with a decrease in neutrophil and macrophage counts in the bronchoalveolar lavage fluid and increased lung function and decreased pathology in mice exposed to cigarette smoke.

### Example 9: Screening siRNAs targeting human MST1 mRNA in mice transfected with AAV8-TBG-h-MST1

Several siRN As targeting human *MST1* mRNA were tested for activity in mice following transfection with an adeno-associated viral vector. The siRNAs were attached to the GalNAc ligand ETL1 followed by a phosphorothioate linkage at the 5' end of the sense strand. The siRNAs used in this Example are included in **Table 24A.**

Six to eight week old female mice (C57Bl/6) were injected with 10 uL of a recombinant adeno-associated virus 8 (AAV8) vector (1.5 x 10E13 genome copies/mL) by the retroorbital route. The recombinant AAV8 contained the open reading frame and the majority of the 3'UTR of the human *MST1* mRNA sequence (NM_020998.4) under the control of the human thyroxine binding globulin promoter in an AAV2 backbone packaged in AAV8 capsid (AAV8-TBG-h-MST1). On Day 14 after infection, serum was collected and the level of human MSP protein in each mouse was measured using the Human MSP/ MST1 / Macrophage Stimulating Protein ELIS A Kit PicoKine^{™} from Boster Bio (Catalog# EK0814) according to the manufacturer's instructions using a serum sample dilution of 1:25 in PBS. Recombinant MSP included in the kit was used to generate a standard curve of 10,000 pg/mL to 0 pg/mL MSP. The optical density of the plate was read at 450 nm using a PerkinElmer Envision multi mode plate reader. The concentration of MSP in each mouse serum sample was calculated from the standard curve by interpolation using least squares fit (Prism version 9, Software MacKiev).

Mice were allocated into groups (n=3) such that the groups had similar serum levels of human MSP and then given a subcutaneous injection of a single 100 ug dose of a GalN Ac-conjugated siRNA or PBS as vehicle control. On Days 0, 4 and 13 after injection, serum was collected to assess serum MSP concentrations by ELISA using the methods described above. The MSP serum concentration at each timepoint was made relative to the level of MSP in the Day 0 sample for each individual mouse. The results are shown in Table 12. Mice injected with ETD01723 had the greatest reduction in serum MSP of the siRNAs tested, with lower levels on Day 13 than on Day 4 relative to Day 0. Mice injected with ETD01728, ETD0 1725 and ETD0 1729 also showed substantial reduction of serum MSP. Note that ETD01724 did not have its target sequence in the AAV8-TBG-h-MST1 construct and therefore functioned as a negative control siRNA in this study.

Mice were sacrificed on Day 13 and a liver sample from each was collected and placed in RN Alater (ThermoFisher Catalog# AM7020) until processing. Total liver RNA was prepared by homogenizing the liver tissue in homogenization buffer (Maxwell RSC simply RNA Tissue Kit) using a Percellys 24 tissue homogenizer (Bertin Instruments) set at 5000 rpm for two 10 second cycles. Total RNA from the lysate was purified on a Maxwell RSC 48 platform (Promega Corporation) according to the manufacturer's recommendations. Preparation of cDNA was performed using Quanta qScript cDNA SuperMix (VWR, Catalog# 95048-500) according to the manufacturer's instructions. The relative levels of liver *MST1* mRNA were assessed by RT-qPCR in triplicate on a QuantStudio^{™} 6 Pro Real-Time PCR System using TaqMan assays for human *MST1* (ThermoFisher, assay# Hs00360684_ml) and the mouse housekeeping gene PPIA (ThermoFisher, assay# Mm02342430_gl) and PerfeCTa^{®} qPCR FastMix^{®}, Low ROX^{™}(VWR, Catalog# 101419-222). Data were normalized to the level in animals receiving PBS. Results are shown in **Table 13**. Mice injected with ETD01723, ETD01728, ETD01725, ETD01729 and ETD01731 had substantially lower levels in mean liver human *MST1* mRNA on Day 13 relative to mice receiving PBS.

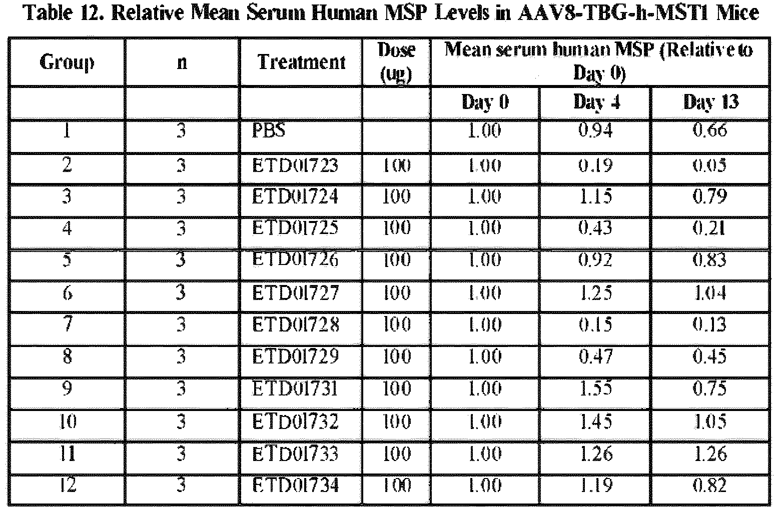

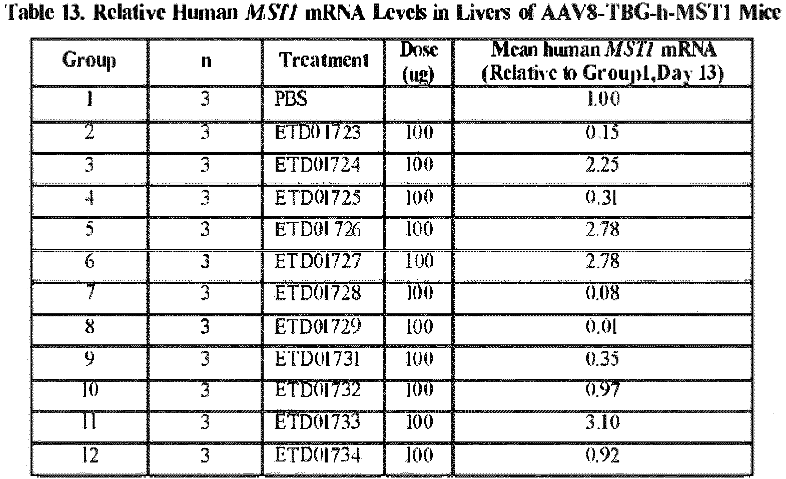

### Example10: Screening of additionalsiRNAs targeting human MST1 mRNA in mice transfected withAAV8-TBG-h-MST1 and confirmation of the activity of ETD01723, ETD01725, ETD01728 and ETD01729

Additional siRNAs targeting human *MST1* mRNA (ETD01 795, ETD01 798, ETD01 799, ETD01800) were tested for activity in mice following transfection with an adeno-associated viral vector. The siRNAs were attached to the GalNAc ligand ETL17 followed by a phosphorothioate linkage at the 5' end of the sense strand. Confirmation of the activities of ETD01723, ETD01725, ETD01728 and ETD01729 from the Example above was also performed. The siRNAs used in this Example are included in **Table 24A.**

Six to eight week old female mice (C57Bl/6) were injected with 10 uL of a recombinant adeno-associated virus 8 (AAV8) vector (1.5 x 10E13 genome copies/mL) by the retroorbital route. The recombinant AAV8 contained the open reading frame and the majority of the 3'UTR of the human MST1 sequence (NM_020998.4) under the control of the human thyroxine binding globulin promoter in an AAV2 backbone packaged in AAV8 capsid (AAV8-TBG-h-MST1). On Day 14 after infection, serum was collected and the level of human MSP in each mouse was measured using the Human MSP/MST1 DuoSet ELISA from R&D (Catalog# DY352). The manufacturer's instructions regarding all reagent preparations for buffers and solutions was followed. A serum sample dilution of 1:250 was utilized for all test samples. Recombinant MSP included in the kit was used to create a standard curve of 10,000 pg/mL to 0 pg/mL. The optical density of the plate was read a 450 nm using a Perk inElmer Envision multimode plate reader. The concentration of MSP in each mouse serum sample was calculated from the standard curve by interpolation using least squares fit (Prism version 9, Software MacKiev).

Mice were allocated into groups (n=3) such that the groups had similar serum levels of human MSP and then given a subcutaneous injection of a single 100 ug dose of a GalN Ac-conjugated siRNA or PBS as vehicle control On Days 0, 4 and 10 after injection, serum was collected to assess serum MSP concentrations by ELISA using the methods described above. The MSP serum concentration a each timepoint was made relative to the level of MSP in the Day 0 sample for each individual mouse. The results are shown in **Table 14**. Mice injected with ETD1799 or ETD01800 had the greatest reduction in serum MSP of the additional siRNAs tested, with lower levels on Day 10 than on Day 4 relative to Day 0. The activities of ETD01723, ETD01 725, ETD01728 and ETD01 729 was confirmed with treatment of mice with ETD01723 and ETD01 728 yielding the greatest reduction in serum MSP. Of the additional siRNA targeting *MST1* mRNA (ETD01795, ETD01798, ETD01 799, ETD01800), ETD01 799 and ETD01800 gave the largest reduction in serum MSP. Replacement of the ETL1 ligand on ETD01723 with the ETL17 ligand on the same sequence (ETD01823) resulted in a greater reduction in MSP.

Mice were sacrificed on Day 10 and a liver sample from each was collected and placed in RNAlater (ThermoFisher Cat# AM7020) until processing. Total liver RNA was prepared by homogenizing the liver tissue in homogenization buffer (Maxwell RSC simplyRNA Tissue Kit) using a Percellys 24 tissue homogenizer (Bertin Instruments) set at 5000 rpm for two 10 second cycles. Total RNA from the lysate was purified on a Maxwell RSC 48 platform (Promeg a Corporation) according to the manufacturer's recommendations. Preparation of cDNA was performed using Quanta qScript cDNA SuperMix (VWR, Catalog# 95048-500) according to the manufacturer's instructions. The relative levels of liver *MST1* mRNA were assessed by RT-qPCR in triplicate on a QuantStudio^{™} 6 Pro Real-Time PCR System using TaqMan assays for human *MST1* (ThermoFisher, assay# Hs00360684_ml) and the mouse housekeeping gene PP1A (ThermoFisher, assay# Mm02342430_g1) and PerfeCTa^{®} qPCR FastMix^{®}, Low ROX^{™} (VWR, Catalog# 101419-222). Data were normalized to the level in animals receiving PBS. Results are shown in Table 15. Mice receiving siRNAs targeting *MST1* had substantially lower levels in mean liver human *MST1* mRNA on Day 10 relative to mice receiving PBS. The activities of ETD0I723, ETD01725, ETD01728 and ETD01729 was confirmed with treatment of mice with ETD01723 and ETD01728 yielding the greatest reduction in liver *MST1* mRNA. Of the additional siRNA targeting *MST1* mRNA (ETD01 795, ETD01 798, ETD01799, ETD01800), ETD01799 and ETD01800 gave the largest reduction in in liver *MST1* mRNA.

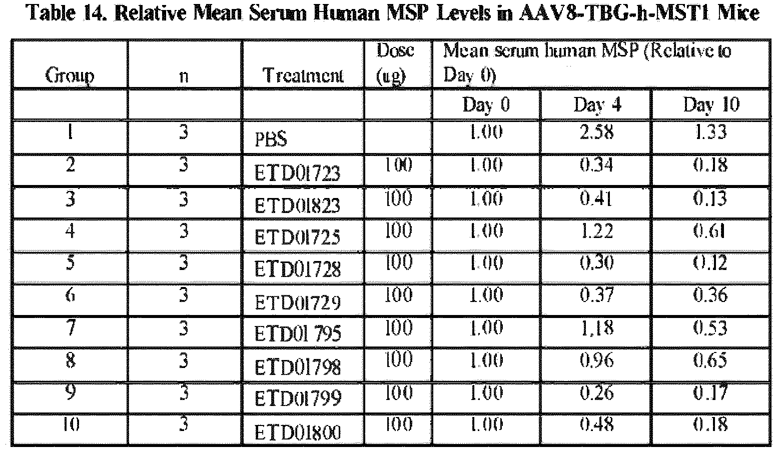

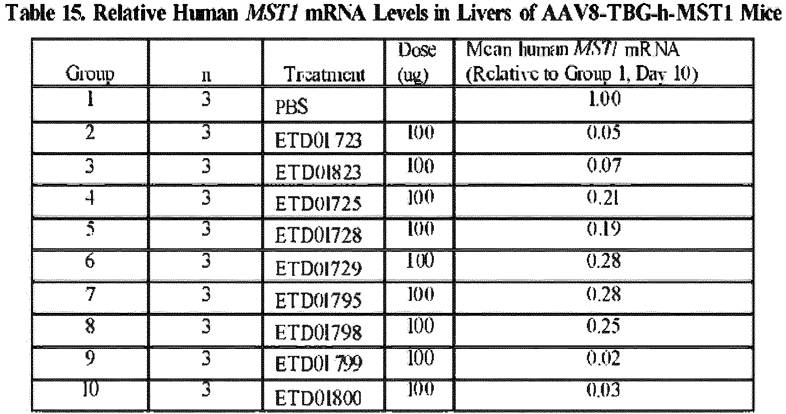

### Example 11: Screening of additional siRNAs targeting human MST1 in mice transfected with AAV8-TBG-h-MST1 and testing theactivity of siRNAs containing alternative modification patterns of ETD01723 and ETD01728.

Additional siRN As targeting human *MST1* mRNA (ETD01789 and ETD01794) were tested for activity in mice following transfection with an adeno-associated viral vector. The siRNAs were attached to the GalNAc ligand ETL1 followed by a phosphorothioate linkage at the 5' end of the sense strand. The activities of siRN As with alternative modification patterns of ETD01723 (ETD01827-ETD0 1831) and ETD01 728 (ETD01832-ETD01837) were also assessed The siRNAs were attached to the GalNAc ligand ETL1 7 followed by a phosphorothioate linkage at the 5'end of the sense strand The activities of ETD01827-ETD01831 were compared to ETD01823 which had the identical sequence and modification pattern to ETD01723 but attached to ETL17. The activities of ETD01832-ETD01837 were compared to ETD01821 which had the identical sequence and modification pattern to ETD01728 but attached to ETL17. The siRNAs used in this Example are included in **Table 24A.**

Six to eight week old female mice (C57Bl/6) were injected with 10 uL of a recombinant adeno-associated virus 8 (AAV8) vector (2.4 x 10E13 genome copies/mL) by the retroorbital route. The recombinant AAV8 contained the open reading frame and the majority of the 3"UTR of the human *MST1* sequence (NM_020998.4) under the control of the human thyroxine binding globulin promoter in an AAV2 backbone packaged in AAV8 capsid (AAV8-TBG-h-MST1). On Day 13 after infection, serum was collected and the level of human MSP in each mouse was measured using the Human MSP/MST1 DuoSet ELISA from R&D (Catalog# DY352). The manufacturer's instructions regarding all reagent preparations for buffers and solutions was followed. A serum sample dilution of 1:250 was utilized for all test samples. Recombinant MSP included in the kit was used to create a standard curve of 10,000 pg/mL to 0 pg/mL. The optical density of the plate was read at 450 nm using a PerkinElmer Envision multi mode plate reader. The concentration of MSP in each mouse serum sample was calculated from the standard curve by interpolation using least squares fit (Prism version 9, Software MacKiev).

Mice were allocated into groups (n=3) such that the groups had similar serum levels of human MSP and then given a subcutaneous injection of a single 60 ug dose of a GalNAc-conjugated siRNA or PBS as vehicle control. On Days 0 and 11 after injection, serum was collected lo assess serum MSP concentrations by ELISA using the methods described above. The MSP serum concentration at each timepoint was made relative to the level of MSP protein in the Day 0 sample for each individual mouse. The results are shown in **Table 16.** Mice injected with ETD01 789 or ETD01 794 did not have greater reductions in serum MSP than ETD01823 or ETD01821 on Day 11. The activities of siRN As with alternative modification patterns of ETD01723 and ETD01823, namely ETD01824-ETD01831 were comparable to ETD01823, with ETD01828 and ETD01831 showing the greatest level serum MSP reduction on Day 11. The activities of siRNAs with alternative modification patterns of ETD01 728 and ETD01821, namely ETD01832-ETD01837 were comparable to ETD01823, with ETD01834, ETD0 1835 and ETD01836 showing the greatest level serum MSP reduction on Day 11.

Mice were sacrificed on Day 11 and a liver sample from each was collected and placed in RNAlater (ThermoFisher Cat#AM7020) until processing. Total liver RNA was prepared by homogeniz ing the liver tissue in homogenization buffer (Maxwell RSC simplyRNA Tissue Kit) using a Percellys 24 tissue homogenizer (Bertin Instruments) set at 5000 rpm for two 10 second cycles. Total RNA from the lysate was purified on a Maxwell RSC 48 platform (Promega Corporation) according to the manufacturer's recommendations. Preparation of cDNA was performed using Quanta qScript cDNA SuperMix (VWR, Catalog# 95048-500) according to the manufacturer's instructions. The relative levels of liver *MST1* mRNA were assessed by RT-qPCR in triplicate on a QuantStudio^{™} 6 Pro Real-Time PCR System using TaqMan assays for human *MST1* (ThermoFisher, assy# Hs00360684_ml) and the mouse housekeeping gene *PP1A* (ThermoFisher, assay# Mm02342430_gl) and PerfeCTa^{®} qPCR FastMix^{®}, Low ROX^{™} (VWR, Catalog# 101419-222). Data were normalized to the level in animals receiving PBS. Results are shown in **Table 17.** Mice injected with ETD01789 or ETD01794 did not have greater reductions in liver *MST1* mRNA than ETD01823 or ETD01821 on Day 11. The mRNA reduction activities of mice receiving siRNAs with alternative modification patterns of ETD01723 and ETD01823, namely ETD01824-ETD01831, were comparable to ETD01823 relative to mice receiving PBS. The activities of siRNAs with alternative modification patterns of ETD01728 and ETD01821, namely ETD01832-ETD01837, were comparable to ETD01821.

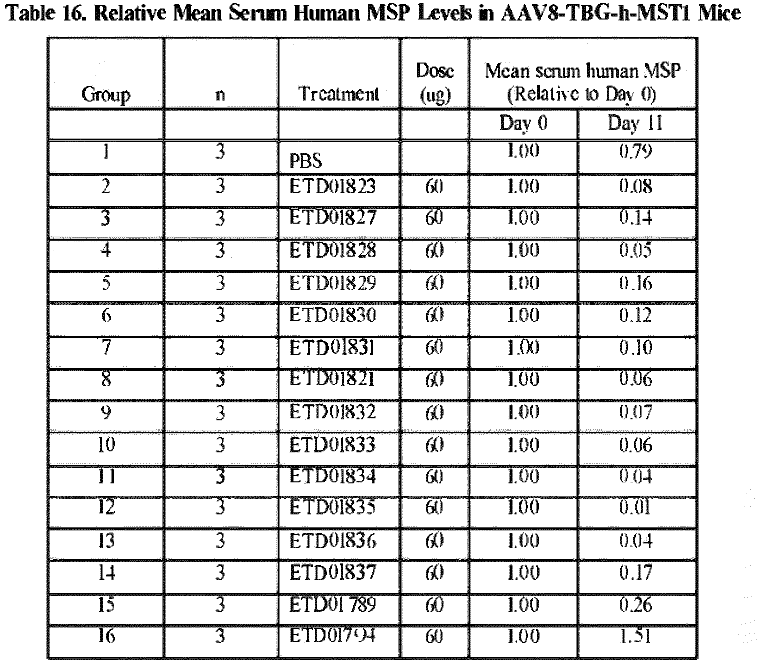

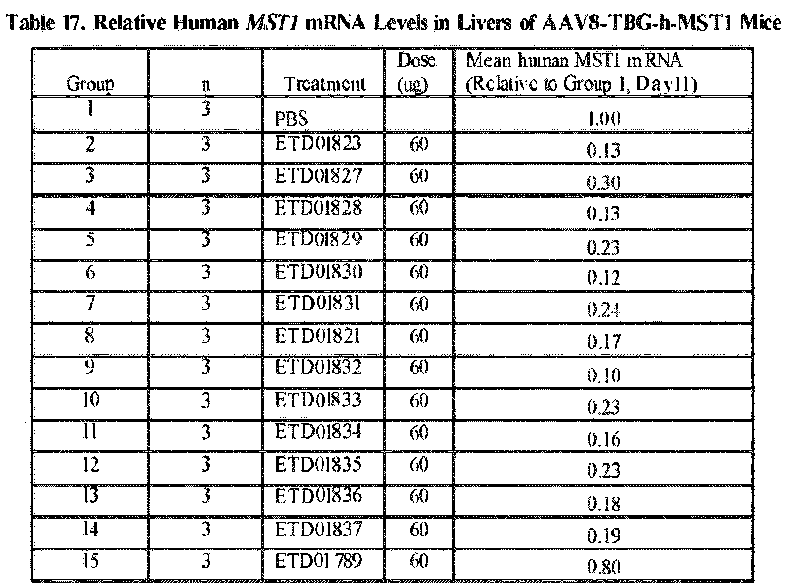

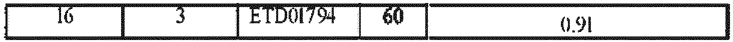

### Example 12: Screening of additional siRNAs targeting human MST1 in mice transfected with AAV8-TBG-h-MST1

Additional siRNAs targeting human *MST1,* namely ETD1860-ETD01868, were tested for activity in mice following transfection with an adeno-associated viral vector. The siRNAs were attached to the GalNAc ligand ETL17 followed by a phosphorothioate linkage at the 5' end of the sense strand The siRNAs ETD01823 and ETD01800 were included as positive controls. The siRNAs used in this Example are included in **Table 24A.**

Six to eight week old female mice (C57Bl/6) were injected with 10 uL of a recombinant adeno-associated virus 8 (AAV8) vector (2.4 x 10E13 genome copies/mL) by the retroorbital route. The recombinant AAV8 contained the open reading frame and the majority of the 3'UTR of the human *MST1* sequence (NM_020998.4) under the control of the human thyroxine binding globulin promoter in an AAV2 backbone packaged in AAV8 capsid (AAV8-TBG-h-MST1). On Day 14 after infection, serum was collected and the level of human MSP in each mouse was measured using the Human MSP/MST1 DuoSet ELISA from R&D (Catalog# DY352). The manufacturer's instructions regarding all reagent preparations for buffers and solutions were followed. A serum sample dilution or 1: 50 was utilized for all test samples. Recombinant MSP included in the kit was used to create a standard curve of 10,000 pg/mL to 0 pg/ml. The optical density of the plate was read at 450 nm using a PerkinElmer Envision multi mode plate reader. The concentration of MSP in each mouse serum sample was calculated from the standard curve by interpolation using least squares fit (Prism version 9, Software MacKiev).

Mice were allocated into groups (n=3) such that the groups had similar serum levels of MSP and then given a subcutaneous injection of a single 100 ug dose of a GalNAc-conjugated siRNA or PBS as vehicle control. On Days 0 and 10 after injection, serum was collected lo assess serum MSP concentrations by ELISA using the methods described above. The MSP serum concentration at each timepoint was made relative to the level of MSP in the Day 0 sample for each individual mouse. The results are shown in Table 18. Mice injected with ETD01867 or ETD01868 had the greatest reduction in serum MSP of the additional siRNAs tested. The magnitude of the reduction was comparable to ETD01823 and ETD01800.

Mice were sacrificed on Day 10 and a liver sample from each was collected and placed in RNAlater (ThermoFisher Cat# AM7020) until processing. Total liver RNA was prepared by homogenizing the li ver tissue in homogenization buffer (Maxwell RSC simply RNA Tissue Kit) using a Percellys 24 tissue homogenizer (Bertin Instruments) set at 5000 rpm for two 10 second cycles. Total RNA from the lysate was purified on a Maxwell RSC 48 platform (Promega Corporation) according to the manufacturer' s recommendations. Preparation of cDNA was performed using Quanta qScript cDNA SuperMix (VWR, Catalog# 95048-500) according to the manufacturer's instructions. The relative levels of liver *MST1* mRNA were assessed by RT-qPCR in triplicate on a QuantStudio^{™} 6 Pro Real-Time PCR System using TaqMan assays for human *MST1* (ThermoFisher, assay# Hs00360684_ml) and the mouse housekeeping gene PPIA (ThermoFisher, assay# Mm02342430_gl) and PerfeCTa^{®} qPCR FastMix^{®}, Low ROX^{™}(VWR, Catalog# 101419-222). Data were normalized to the level in animals receiving PBS. Results are shown in **Table 19**. Of the additional sRNAs tested in this Example, ETD01867 and ETD01868 had the greatest *MST1* mRNA reduction activity, which was comparable to the *MST1* mRNA reduction activity observed with ETD01823 and ETD01800.

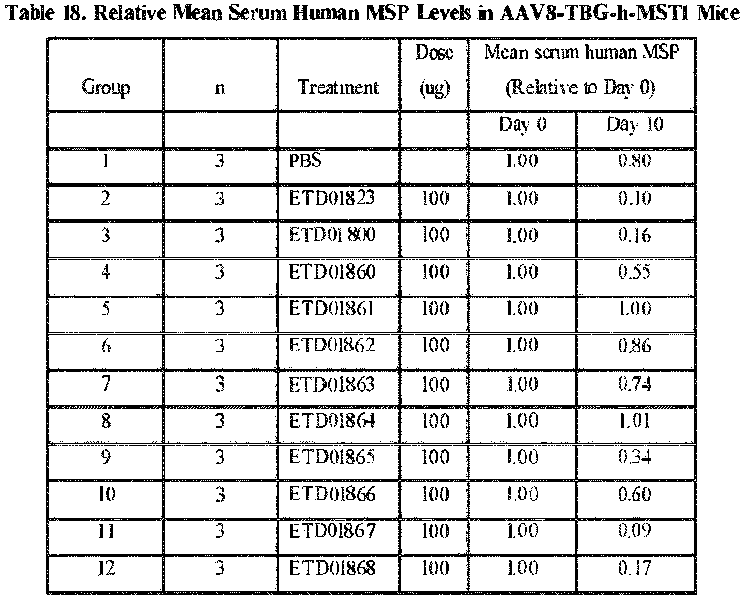

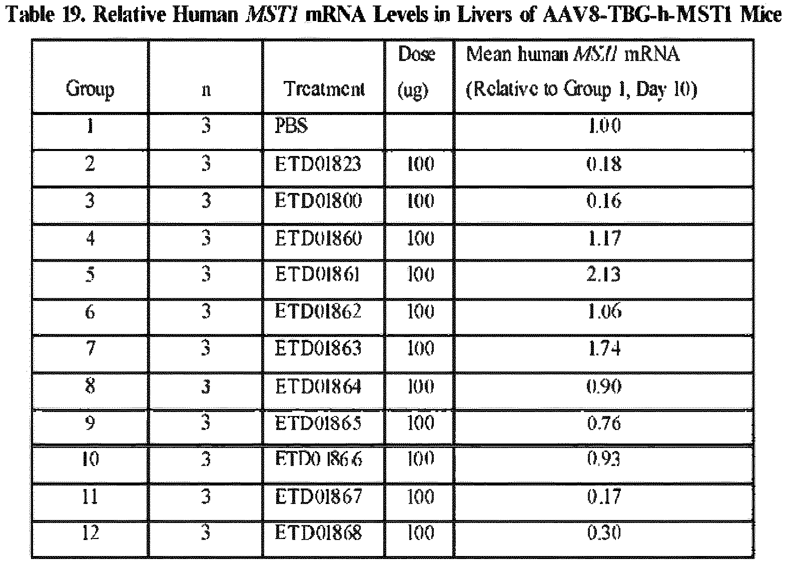

### Example13: Testing the activity of siRNAs containing alternative modification patternsof ETD01800 targeting human MST1 in mice transfected with AAV8-TBG-h-MST1.

The activities of siRNAs with alternative modification patterns of ETD01800, namely ETD01871-ETD01878 were assessed. The siRNAs with alternative modifications were attached to the GalNAc ligand ETL17 followed by a phosphorothioate linkage at the 5' end of the sense strand. The activities of ETD01871-ETD01878 were compared to ETD01800. The siRNAs used in this Example are included in **Table 24A.**

Six to eight week old female mice (C57Bl/6) were injected with 10 uL of a recombinant adeno-associated virus 8 (AAV8) vector(2.7 x 10E13 genome copies/mL) by the retroorbital route. The recombinant AAV8 contained the open reading frame and the majority of the 3'UTR of the human *MST1* sequence (NM- 020998.4) under the control of the human thyroxine binding globulin promoter in an AAV2 backbone pack aged in AAV8 capsid (AAV8-TBG-h-MST1). On Day 13 after infection, serum was collected and the level of human MSP in each mouse was measured using the Human MSP/MST1 DuoSet ELISA from R&D (Catalog# DY352). The manufacturer's instructions regarding all reagent preparations for buffers and solutions was followed. A serum sample dilution of 1:100 was utilized for at test samples. Recombinant MSP included in the kit was used to create a standard curve of 10,000 pg/mL to 0 pg/mL. The optical density of the plate was read at 450 nm using a PerkinElmer Envision multi mode plate reader. The concentration of MSP in each mouse serum sample was calculated from the standard curve by interpolation using least squares fit (Prism version 9, Software MacKiev).

Mice were allocated into groups (n=3) such that the groups had similar serum levels of MSP and then given a subcutaneous injection of a single 60 ug dose of a GalNAc-conjugated siRNA or PBS as vehicle control. On Days 0 and 10 after injection, serum was collected to assess serum MSP concentrations by ELISA using the methods described above. The MSP serum concentration at each timepoint was made relative to the level of MSP in the Day 0 sample for each individual mouse. The results are shown in **Table 20.** The activities of siRNAs with alternative modification patterns of ETD01800, namely ETD01871-ETD01878 were comparable to ETD01800, with ETD01873 and ETD01878 showing the greatest level serum MSP reduction on Day 10.

Mice were sacrificed on Day 10 and a liver sample from each was collected and placed in RNAlater (ThermoFisher Cat# AM7020) until processing. Total liver RNA was prepared by homogenizing the liver tissue in homogenization buffer (Maxwell RSC simply RNA Tissue Kit) using a Percellys 24 tissue homogenizer (Bertin Instruments) set a 5000 rpm for two 10 second cycles. Total RNA from the lysate was purified on a Maxwell RSC 48 platform (Promega Corporation) according to the manufacturer' s recommendations. Preparation of cDNA was performed using Quanta qScript cDNA SuperMix (VWR, Catalog# 95048-500) according to the manufacturer's instructions. The relative levels of liver *MST1* mRNA were assessed by RT-qPCR in triplicate on a QuantStudio^{™} 6 Pro Real-Time PCR System using TaqMan assays for human *MST1* (ThermoFisher, assay#Hs00360684_m1) and the mouse housekeeping gene PPIA (ThermoFisher, assay# Mm02342430_gl) and PerfeCTa^{®} qPCR FastMix^{®}, Low ROX^{™}(VWR, Catalog# 101419-222). Data were normalized to the level in animals receiving PBS. Results are shown in **Table 21.** Mice receiving ETD0 1800 had substantially lower liver *MST1* mRNA on Day 10 relative to mice receiving PBS. Mice receiving any of the alternatively modified siRNAs targeting MST1 also had substantially lower levels in mean liver human *MST1* mRNA on Day 10 relative to mice receiving PBS.

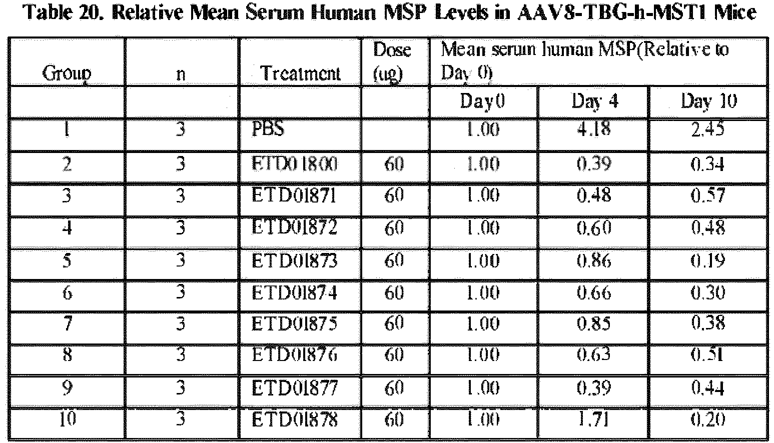

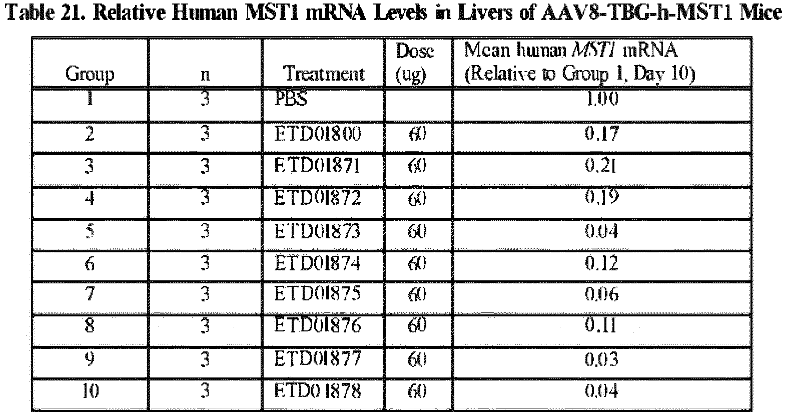

### Example 14: Testing the activity of MST1 siRNAs containing alternative modification patterns of ETD01867 and ETD01868 in in mice transfiected with AAV8-TBG-h-MST1.

The activities of siRNAs with alternative modification pattems of ETD01867, namely ETD01963-ETD01966, and siRNAs with alternative modification patterns of ETD01868, namely ETD0 1967-ETD01972. were assessed. The siRNAs were attached to the GalNAc ligand ETL 17 followed by a phosphorothioate linkage at the 5⁻ end of the sense strand. The siRNAs used in this Example are included in **Table 24A.**

Six to eight week old female mice (C57B1/6) were injected with 5 uL of a recombinant adeno-associated virus 8 (AAV8) vector (2.7 x 10E 13 genome copies/mL) by the retroorbital route. The recombinant AAV8 contained the open reading frame and the majority of the 3'UTR of the human *MST1* sequence (NM_020998.4) under the control of the human thyroxine binding globulin promoter in an AAV2 backbone pack aged in AAV8 capsid (AAV8-TBG-h-MST1). On Day 13 after infection, serum was collected and the level of human MSP in each mouse was measured using the Human MSP/MSTI DuoSet ELISA from R&D (Catalog# DY352). The manufacturer's instructions regarding all reagent preparations for buffers and solutions was followed. A serum sample dilution of 1:50 was utilized for all test samples. Recombinant MSP included in the kit was used to create a standard curve of 10,000 pg/mL to 0pg/mL. The optical density of the plate was read at 450 nm using a PerkinElmer Envision multimode plate reader. The concentration of MSP in each mouse serum sample was calculated from the standard curve by interpolation using least squares fit (Prism version 9, Software MacKiev).

Mice were allocated into groups (n=3) such that the groups had similar serum levels of MSP and then given a subcutaneous injection of a single 60 ug dose of a GalNAc-conjugated siRNA or PBS as vehicle control. On Days 0, 4 and 12 after injection, serum was collected to assess serum MSP concentrations by ELISA using the methods described above. The MSP serum concentration at each timepoint was made relative to the level of MSP in the Day 0 sample for each individual mouse. The results are shown in **Table 22.** The activities of siRNAs with alternative modification patterns of ETD01867. namely ETD01963-ETD01966, were comparable to ETD01867. with ETD01964 and ETD01966 showing the greatest level serum MSP reduction on Day 12. The activities of siRNAs with alternative modification patterns of ETD01868. namely ETD0196 7-ETD01972. were comparable to ETD01868, with ETD01972 showing the greatest level serum MSP reduction on Day 12.

Mice were sacrificed on Day 12 and a liver sample from each was collected and placed in RNAlater (ThermoFisher Cat#AM7020) until processing. Total liver RNA was prepared by homogenizing, the liver tissue in homogenization buffer (Maxwell RSC simplyRNA Tissue Kit) using a Percellys 24 tissue homogenizer (Bertin Instruments) set at 5000 rpm for two 10 second cycles. Total RNA from the lysate was purified on a Maxwell RSC 48 platform (Promega Corporation) according to the manufacturer's recommendations. Preparation of cDNA was performed using Quanta qScript cDNA SuperMix (VWR, Catalog# 95048-500) according to the manufacturer's instructions. The relative levels of liver *MST1* mRNA were assessed by RT-qPCR in triplicate on a QuantStudio^{™}6 Pro Real-Time PCR System using TaqMan asays for human *MST1* (ThermoFisher, assay # Hs00360684_ml) and the mouse housekeeping gene PPIA (ThermoFisher, assy # Mm02342430_gl) and PerdieCTa^{®} qPCR FastMix^{®}, Low ROX^{™} (VWR. Catalog# 101419-222). Data were normalized to the level in animals receiving PBS. Results are shown in **Table 23.** The activities of siRNAs with alternative modification patterns of ETD01867, namely ETD01965 and ETD01966, showed similar or slightly better activity than the parent for MST1 mRNA reduction on Day 12. The activities of siRNAs with alternative modification patterns of ETD01868, namely ETD01967 and ETDOI 972, showed the highest MST1 mRNA reduction on Day 12.

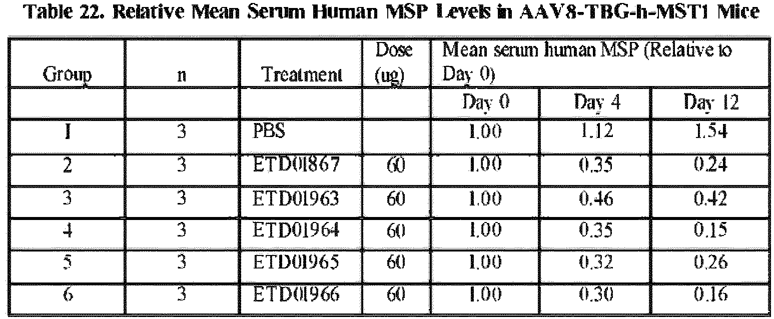

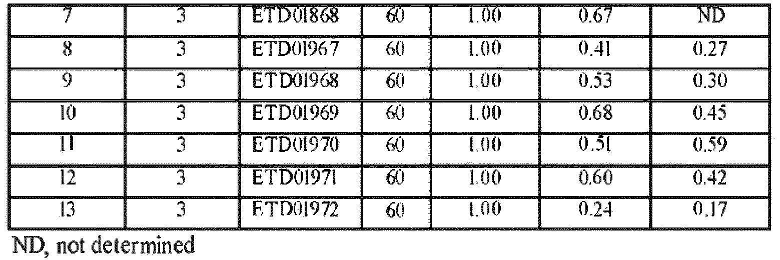

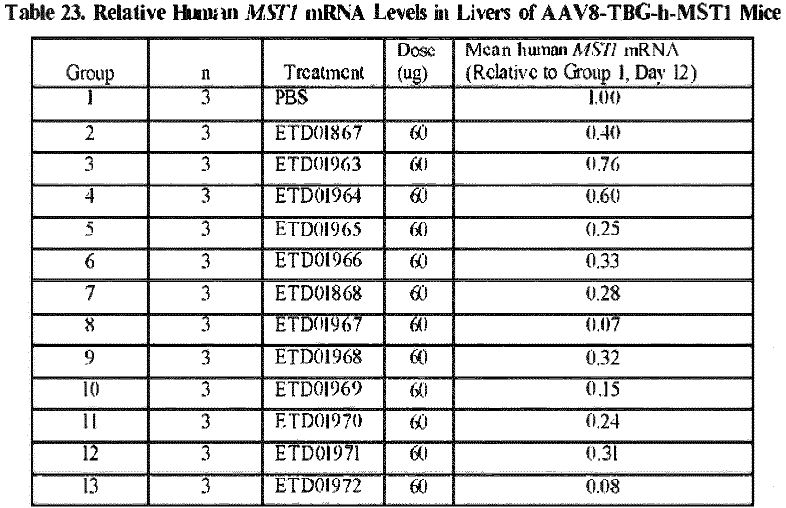

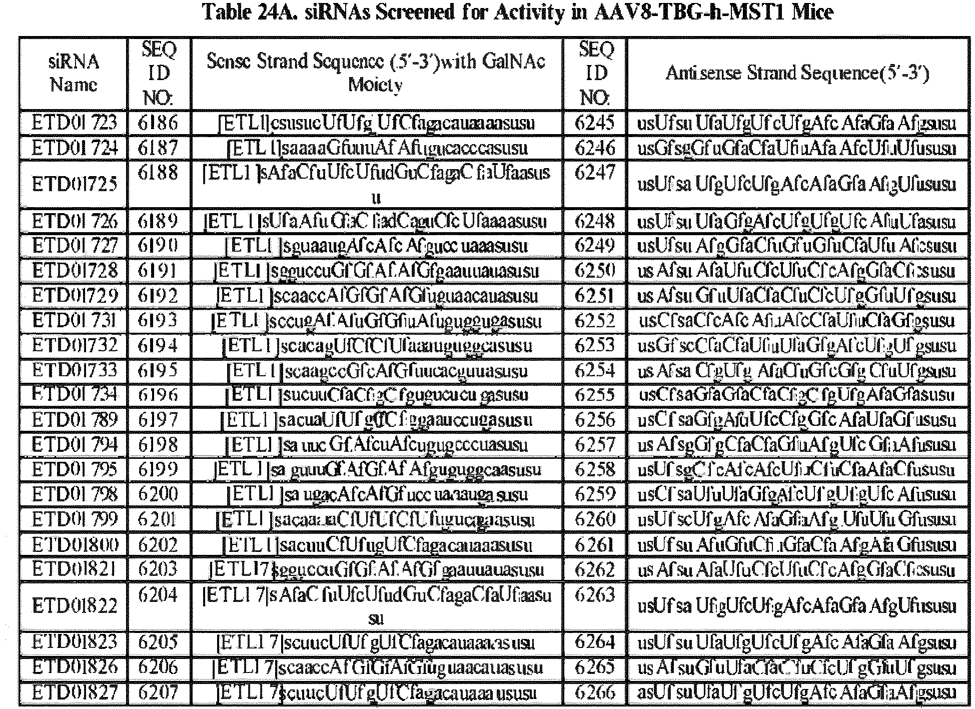

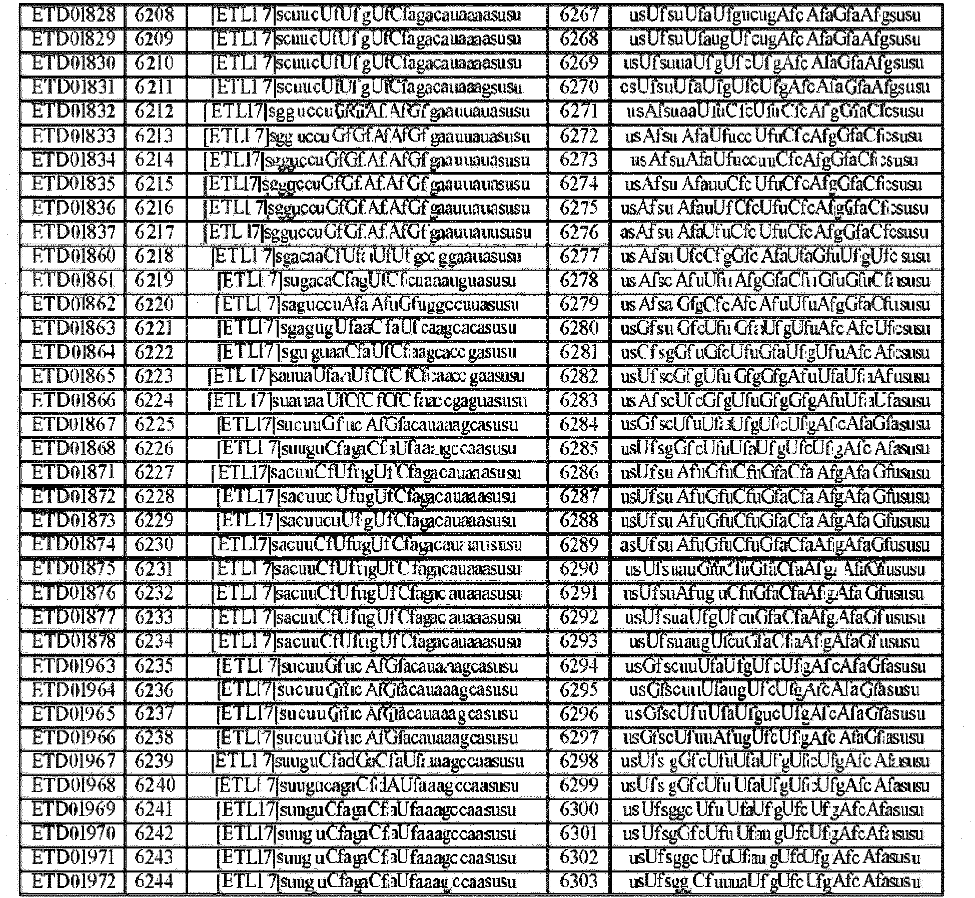

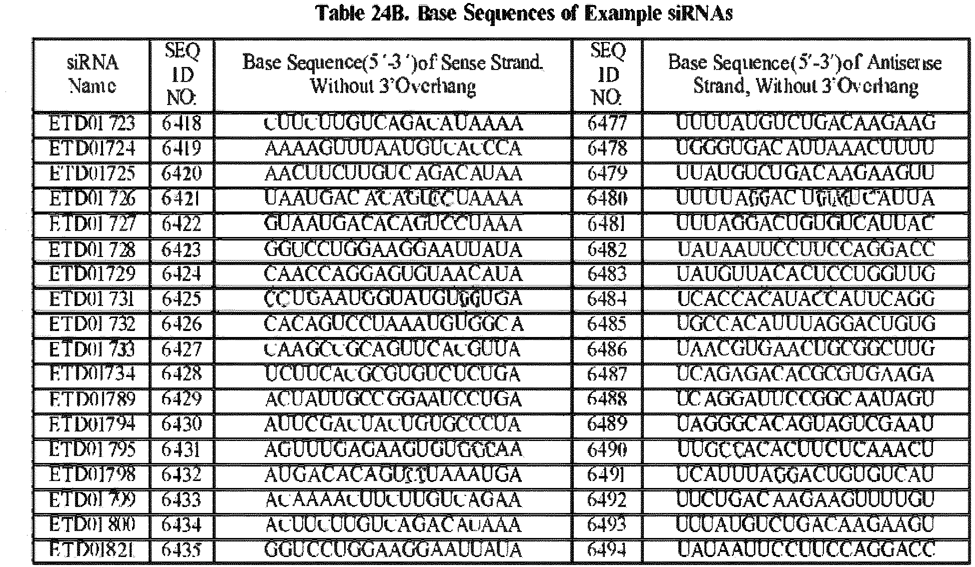

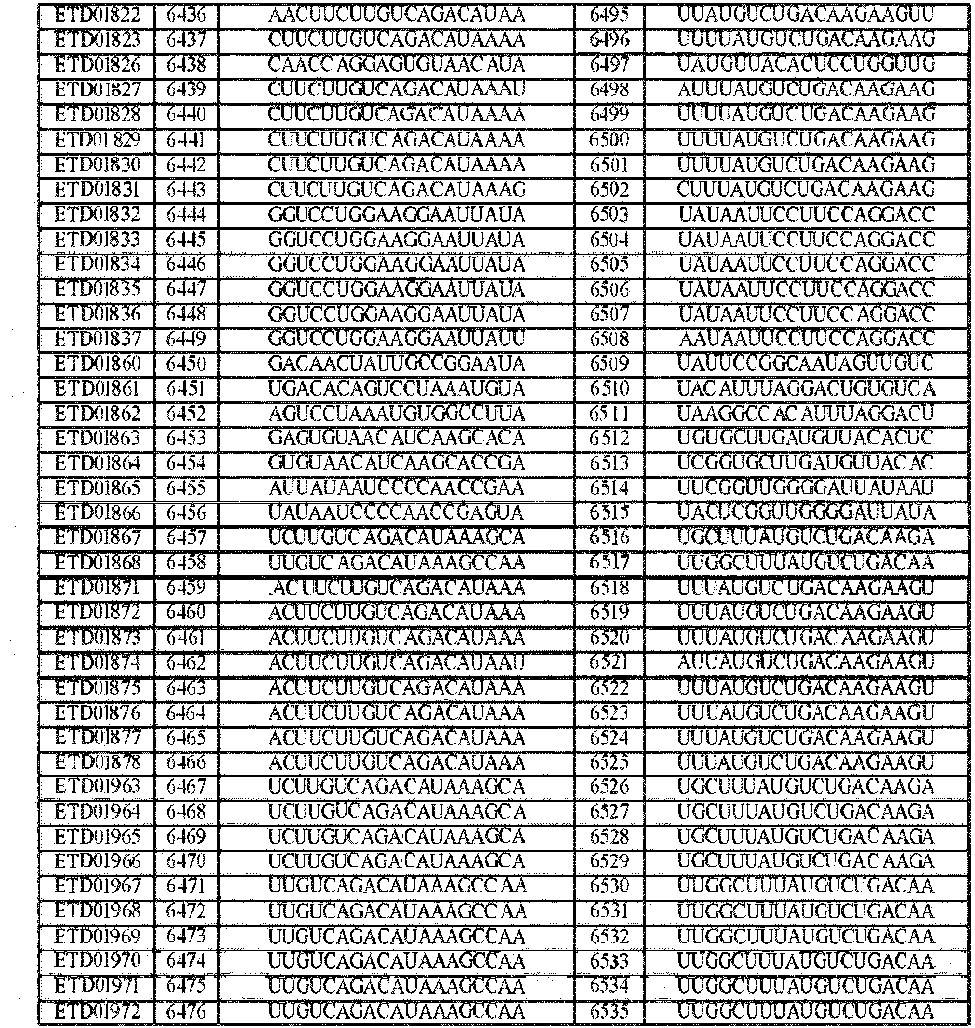

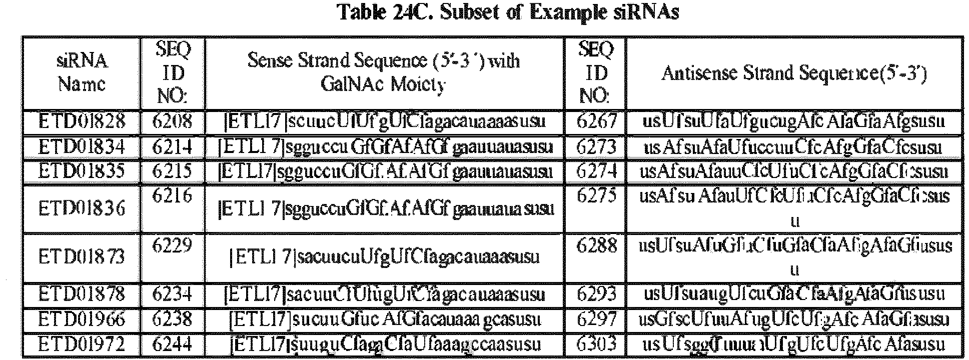

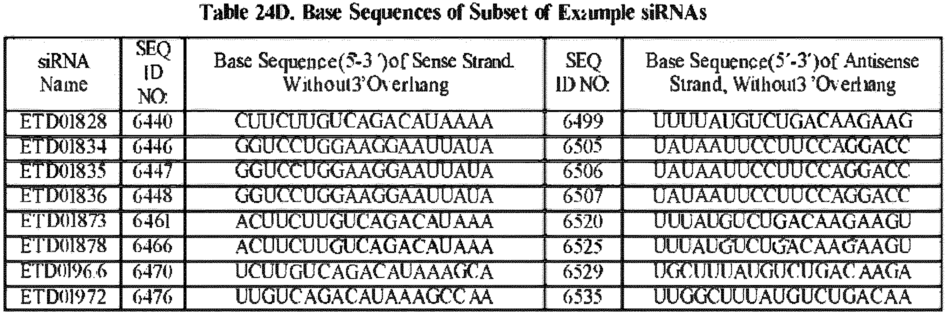

The sense strands of the example siRNAs in **Table 24A** each include a GalNAc moiety as indicated. In **Table 24A** and **Table 24C,** Nf(e.g. Af, Cf, Gf, Tf, or Uf) is a2' fluoro-modified nucleoside, dN(e.g. dA, dC, dG, dT, or dU) is a2' deoxy-modified nucleoside, n(e.g. a. c, g, l or u)is a2' O-methyl modified nucleoside, and "s" is a phosphorothioate linkage.

### Example 15: Inhibition of MST linaMouse Model of Lung Inflammation ViaLPS ExposureUsing MST1 siRNAs

In this experiment a mouse model of lung inflammation induced by acute LPS exposure was used to evaluate the effect of siRNA inhibition of *MST1.* In this LPS induced model, mice were exposed to LPS for 6 hours which will resulted in a transient inflammatory response. Lung inflammation was assessed by measuring neutrophils, macrophages, eosinophils, lymphocytes and cytok ines in bronchoalveolar lavage fluid and lung tissue.

Briefly, mice were divided into five groups. Group 1 - a group treated with vehicle and saline intratracheal instillation, Group 2 - a group treated with vehicle and LPS intratracheal instillation, Group 3 - a group treated with low dose MSTI siRNA ETD01218 (50ug) and LPS intratracheal instillation, Group 4- agroup treated with high dose MSTI siRNA ETD01218 (150ug) and LPS intratracheal instillation, Group 5 - a group treated with Betamethasone and LPS intratracheal instillation. Each group contained twelve mice (male). The sequence of ETD01218 is shown in **Table 29.**

Administration of siRNA was achieved with a 100 µL subcutaneous injection of siRNA resuspended in PBS at concentrations of 0.5mg/ml or 1.5mg/ml. Administration of 3mg/kg Betamethasone was achieved via oral gavage with Betamethasone resuspended in PBS at a concentration of 0.3mg/ml. At days-21, -14, and -7, Group 1 mice were injected subcutaneously with vehicle, Group 2 mice were injected subcutaneously with vehicle, Group 3 mice were injected subcutaneously with low dose MSTI siRNA ETD01218 targeting mouse *MST1,* Group 4 mice were injected subcutaneously with high dose MST1 siRNA ETD01218 targeting mouse *MST1*, and Group 5 mice were injected subcutaneously with vehicle. On Day 1, 30 minutes prior to LPS administration, Group 5 mice were dosed with Betamethasone via oral gavage.

On Day 1, 6 hours after LPS administration, bronchoalveolar lavage fluid was collected and the mice were euthanized by isoflurane inhalation and exsanguination of abdominal aorta. Final blood samples were collected, and livers and lungs are removed, and a section placed in RNAker for mRNA isolation.

Mice were sacrificed on Day 1, 6 hours after LPS administration, and a liver and lung samples from each was collected and placed in RNAlater (ThermoFisher Cat# AM7020) until processing. Total liver RNA was prepared by homogenizing the liver tissue in homogenization buffer (Maxwell RSC simply RNA Tissue Kit) using a Percellys 24 tissue homogenizer (Bertin Instruments) set at 5000 rpm for two 10 second cycles. Total RNA from the lysate was purified on a Maxwell RSC 48 platform (Promega Corporation) according to the manufacturer's recommendations. Preparation of cDNA was performed using Quanta qScript cDNA Super Mix (VWR. Catalog# 95048-500) according to the manufacturer's instructions. The relative levels of liver MSTI mRNA were assessed by RT-qPCR in triplicate on a QuantStudio^{™} 6 Pro Real-Time PCR Sy stemusing TaqMan assay s for mouse MSTI (ThermoFisher, assay# Mm01229834_ml) and the mouse housekeeping gene PPIA (ThermoFisher, assay# Mm02342430_gl) and PerfeCTa^{®} qPCR FastMix^{®}, Low ROX^{™}(VWR Catalog# 101419-222), Data were normalized to the tevel in animals receiving vehicle and LPS intratracheal instillation. *MST1* mRNA expression in the liver tissue from mice dosed with the MST1 siRNA and administered LPS was reduced by 85% and 98%, low and high dose MST1 siRNA respectively, compared to *MSTI* mRNA expression in the liver tissue from mice dosed with the vehicle and administered LPS (Table 25). *MSP* protein in the liver tissue from mice dosed with the MST1 siRNA and administered LPS was reduced from 8.94 ng/ml to 1.71 ng/ml in the low dose MSTI siRNA and to 0.53 ng/ml in the high dose MST1 siRNA. This equates to an 81% and 94% reduction, low and high dose MST1 siRNA respectively, compared to *MSP* protein in the liver tissue from mice dosed with the vehicle and administered LPS (**Table 25).** *MSP* protein in the serum from mice dosed with the MST1 siRNA and administered LPS was reduced by ~80% and ~90%, low and high dose MST1 siRNA respectively, compared to *MSP* protein in the serum from mice dosed with the vehicle and administered LPS (**Table 26).** The high dose MSTI siRNA decreased in neutrophil, eosinophils and lymphocytes counts, 45%, 30%, and 48%, respectively, in the bronchoal veolar lavage fluid in LPS exposed mice compared to the neutrophil, eosinophils and lymphocytes counts in the bronchoalveolar lavage fluid in LPS exposed mice that receive the vehicle control **(Table 27).** Whereas, the low dose MST1 siRNA is able to decrease in neutrophil, eosinophils and lymphocytes counts. 42%, 40%, and 14%, respectively, in the bronchoalveolar lavage fluid in LPS exposed mice compared to the neutrophil, eosinophils and lymphocytes counts in the bronchoalveolar lavage fluid in LPS exposed mice that receive the vehicle control **(Table 27).** The ability of the MST1 siRNAs to lower the neutrophil, eosinophils and lymphocytes counts was comparable to the positive control Betamethasone which is able to decrease in neutrophil, eosinophils and lymphocytes counts, 48%, 32%, and 41%, respectively, in the bronchoalveolar lavage fluid in LPS exposed mice compared to the neutrophil, eosinophils and lymphocytes counts in the bronchoalveolar lavage fluid in LPS exposed mice that receive the vehicle control (Table 27). Additionally, MST1 siRNA, as well as the positive control Betamethasone, was able to reduce the pro-inflammatory cytokines, IL-1b, IL-6.KC-GRO, MCP-1 and TNF-a(Table 28). These results show that the MSTI siRNA elicited knockdown of *MST1* mRNA and MSP in liver tissue and reduced circulating MSP in serum, and that the decrease in *MSTI* mRNA and MSP expression corresponds with a decrease in neutrophil, eosinophils and lymphocytes counts and associated cytokines in the bronchoalveolar lavage fluid in mice exposed to LPS.

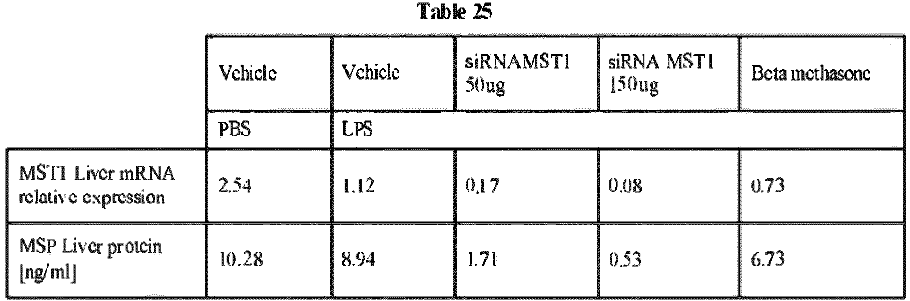

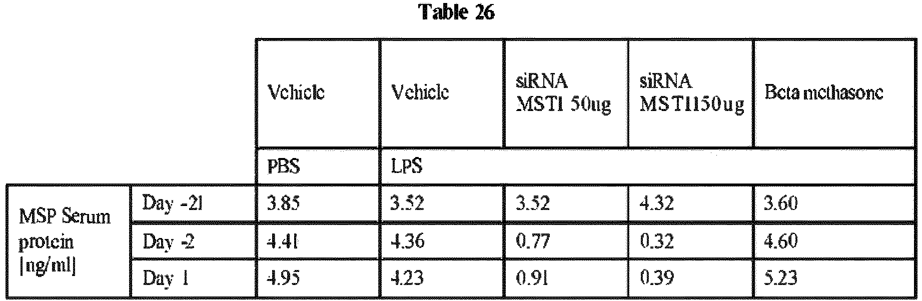

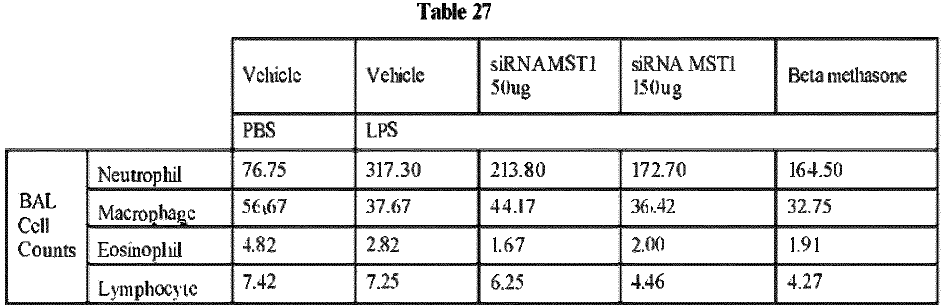

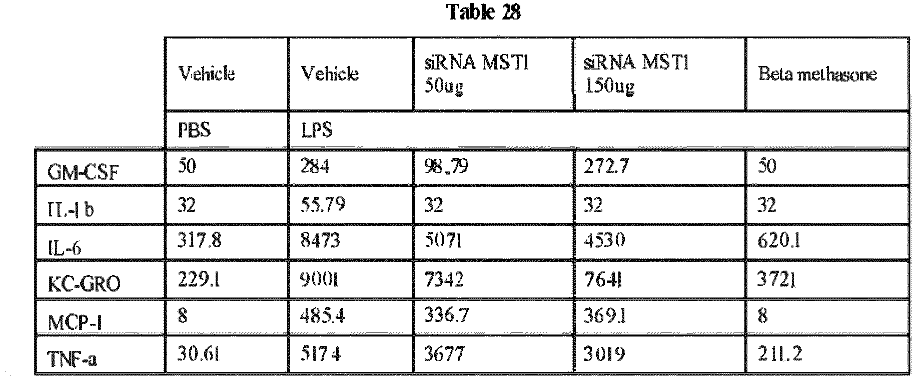

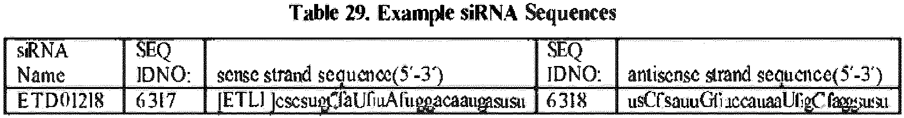

### Example 16: Inhibition of MST1 in a Non-human Primates Using MSTI siRNAs

In this experiment, non-human primates will be used to evaluate the efficacy of siRNA inhibition of *MST1.*

Briefly, cynomolgus monkeys, will be divided into 4 groups: Group 1- this group will be treated with siRNA ETD01821, Group 2 - this group will be treated with siRNA ETD01822. Group 3 - this group treated with siRNA ETD01823, and Group 4 - this group will be treated with siRNA ETD01826. These siRNAs are shown in **Table 30.** Their sequences are included in **Table 24A,** and these siRNAs were derivatives of ETD01728, ED01 725, ETD01723 and ETD01729, respectively. Each group will contain three cynomolgus monkeys (males).

Administration of siRNA will be achieved with a 1 mL subcutaneous injection of siRNA resuspended in PBS at concentration of 25mg/ml. At Day 0, Group 1 cynomologus monkeys will be injected subcutaneously with siRNA ETD01723, Group 2 cynomologus monkeys will be injected subcutaneously with siRNA ETD01725, Group 3 cynomologus monkeys will be injected subcutaneously with siRNA ETD01728, and Group 4 cynomologus monkeys will be injected subcutaneously with siRNA ETD01729.

2 days prior to siRNA administration, liver biopsies will be collected along with serum samples. On Day 28, final liver biopsies and blood samples will be collected and the livers sections placed in RNAlater for mRNA isolation.

Total liver RNA will be isolated from tissue and placed in RN Alater solution using the PureLink kit according to the manufacturer's protocol (ThermoFisher Cat. No. 12183020). The reverse transcriptase reaction is performed according to the manufacturer's protocol. Samples are stored at -80°C until real-time qPCR is performed in triplicate using TaqMan Gene Expression Assays TaqMan assays for cynomolgus *MST1* (ThermoFisher, assay# Mf02878573_g1) and the cynomolgus housekeeping gene GAPDH (ThermoFisher, assay# Mf04392546_g1). A decrease in *MSTI* mRNA in the liver tissue and circulating MSP in the serum from cynomologus monkeys dosed with the MSTI siRNAI is expected compared to *MST1* mRNA or MSP expression in the liver tissue and circulating MSP in the blood from samples taken prior to dosing. These results are expected to show that the MST1 siRNA elicits knock down of *MST1* mRNA and and reduces circulating MSP in non-human primates.

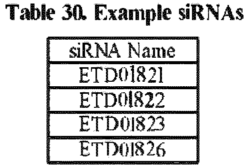

### Example 18: GalNAcligand for hepatocyte targeting of oligonucleotides

Without limiting the disclosure to these individual methods, there are at least two general methods for attachment of multivalent N-acetylgalactosamine (GalNAc) ligands to oligonucleotides: solid or solution-phase conjugations. GalNAc ligands may be attached to solid phase resin for 3'conjugation or at the 5' terminus using GalNAc phosphoramidite reagents. GalNAc phosphoramidites may be coupled on solid phase as for other nucleosides in the oligonucleotide sequence at any position in the sequence. Reagents for GalNAc conjugation to oligonucleotides are shown in **Table 31.**

### Example 17: Oligonucleotide Syn thesis

Oligonucleotides such as siRNAs may be synthesized according to phosphoramidite technology on a solid phase. For example, a K&A oligonucleotide synthesizer may be used Syntheses may be performed on a solid support made of controlled pore glass (CPG, 500 A or 600 Å, obtained from AM Chemicals, Oceanside, CA, USA). All 2'-OMe and 2'-F phosphoramidites may be purchased from Hongene Biotech (Union City, CA, USA). All phosphoramidites may be dissolved in anhydrous acetonitrile (100 mM) and molecular sieves (3 A) may be added. 5-Benzylthio-1H-tetrazole (BTT. 250 mM in acetonitrile) or 5-Ethylthio-1H-tetrazole (ETT, 250 nM in acetonitrile) may be used as activator solution. Coupling times may be 9-18 min (e. g. with a GalNAc such as E TL17), 6 min (e.g. with 2'OMe and 2'F). In order to introduce phosphorothioate linkages, a 100 mM solution of 3-phenyl 1,2,4-dithiazoline-5-one (POS, obtained from PolyOrg. Inc., Leominster, Mass., USA) in anhydrous acetonitrile may be employed

After solid phase synthesis, the dried solid support may be treated with a 1:1 volume solution of 40 wt % methylamine in water and 28% ammonium hydroxide solution (Aldrich) for two hours at 30° C. The solution may be evaporated and the solid residue may be reconstituted in water and purified by anionic exchange HPLC using a TKSgel SuperQ-5PW 13u column. Buffer A may be 20 mM Tris, 5 mM EDTA, pH 9.0 and contained 20% Acetonitrile and buffer B may be the same as buffer A with the addition of 1 M sodium chloride. UV traces at 260 nm may be recorded. Appropriate fractions troy be pooled then desalted using Sephadex G-25 medium.

Equimolar amounts of sense and antisense strand may be combined to prepare a duplex. The duplex solution may be prepared in 0.1 ×PBS (Phosphate-Buffered Saline, 1 × , Gibco). The duplex solution may be annealed at 95° C. for 5 min, and cooled to room temperature slowly. Duplex concentration may be determined by measuring the solution absorbance on a UV-Vis spectrometer at 260 nm in0.1xPBS. For some experiments, a conversion factor may be calculated from an experimentally determined extinction coefficient.

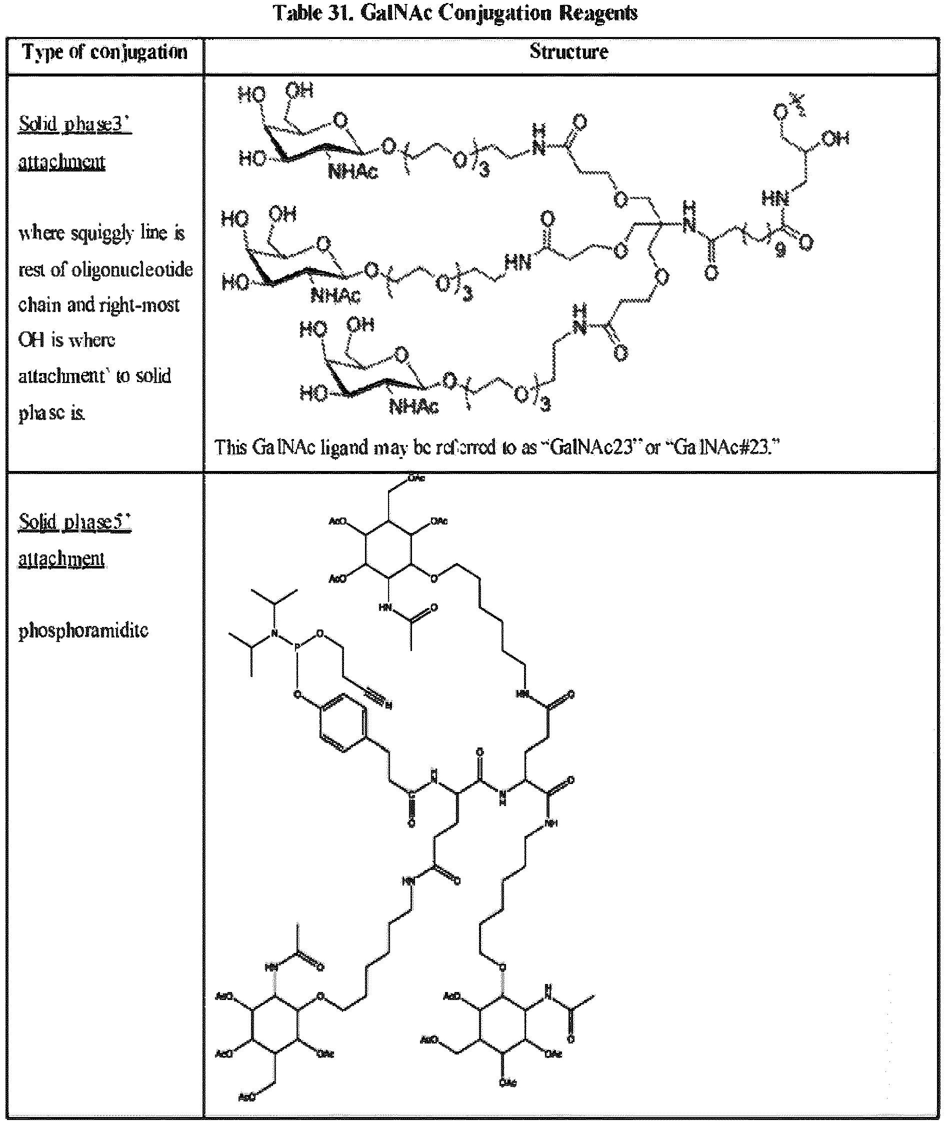

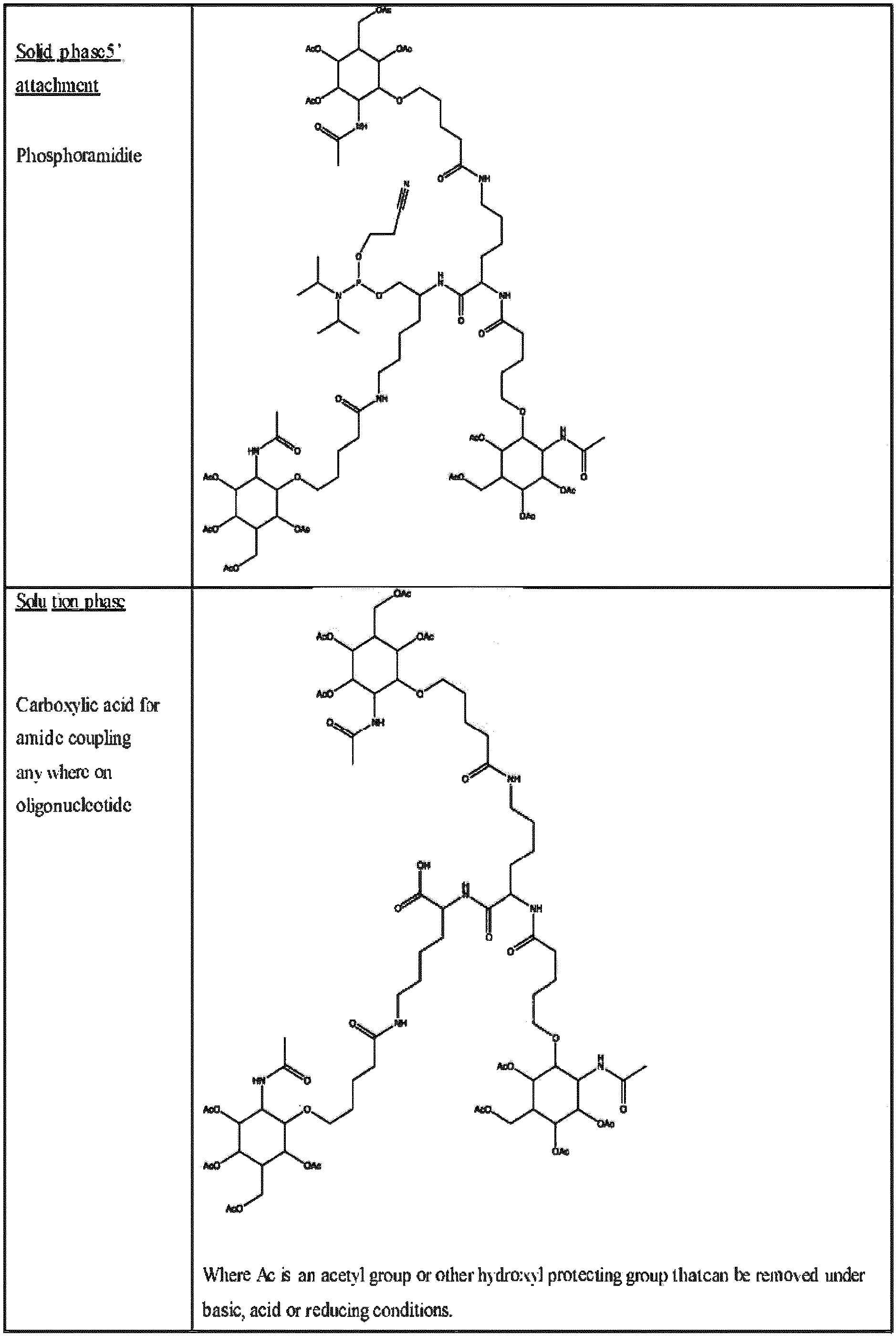

In solution phase conjugation, the oligonucleotide sequence-including a reactive conjugation site-is formed on the resin. The oligonucleotide is then removed from the resin and GalNAc is conjugated to the reactive site.

The carboxy GalNAc derivatives may be coupled to amino-modified oligonucleotides. The peptide coupling conditions are known to the skilled in the art using a carbodiimide coupling agent like DCC (N,N'-Dicyclohexycarbodiimide), EDC (N-(3-dimethy laminopropyl)-N'-ethylcarbodiimide) or EDC. HCI (N-(3-dimethylaminopropyl)-N'-ethylcarbodiimide hydrochloride and an additive like HOBt(1-hydroxy benztriazole), HOSu (N-hydroxysuccinimide), TBTU (N,N,N',N'-Tetramethyl-O-(benzotriazol-1-yl)uromum tetrafluoroboraie, HBTU (2-(1H-ben_{z}etriazol-1-yl)-1,1,3,3-tetramethyluronium hexafluorophosphate) or HOAt (1-Hydroxy-7-azabenzotriazole and common combinations thereof such as TBTU/HOBt or HBTU/HOAt to form activated amine-reactive esters.

Amine groups may be incorporated into oligonucleotides using a number of known, commercially available reagents at the 5' terminus, 3'terminus or any where in between.

Non-limiting examples of reagents for oligonucleotide synthesis to incorporate an amino group include:
- 5' attachment:
- 6-(4-Monomethoxytritylamino)hexyl-(2-cyanoethyl)-(N,N-diisopropyl)-phosphoramidite CAS Number:114616-27-2
- 5'-Amino-Modifier TEG CE-Phosphoramidite
- 10-(O-trifluoroacetamido-N-ethyl)-triethyleneglycol-1-[(2-cyanoethyl)-(N,N-diisopropyl)]-phosphoramidite
- 3' attachment:
- 3'-Amino-Modifier Serinol CPG
- 3-Dimethoxytrityloxy-2-(3-(fluorenylmethoxycarbonylamino)propanamido)propyl-1-O-succinyl-long chain alkylamino-CPG (where CPG stands for controlled-pore glass and is the solid support)
- Amino-Modifier Serinol Phosphoramidite
- 3-Dimethoxytrityloxy-2-(3-(fluorenylmethoxycarbonylamino)propanamido)propyl-1-O-(2-cyanoethyl)-(N,N-diisopropyl)-phosphoramidite

Internal (base modified):
- Amino-Modifier C6 dT
- 5'-Dimethoxytrity-5-[N-(trifluoroacetylaminohexyl)-3-acrylimidol-2'-deoxyUridine,3'-[(2-cyanoethyl)-(N,N-diisopropyl)[-phosphoramidite. CAS Number: 178925-21-8

Solution phase conjugations may occur after oligonucleotide synthesis via reactions between non-nucleosidic nucleophilic functional groups that are attached to the oligonucleotide and electrophilic GalNAc reagents. Examples of nucleophilic groups include amines and thiols, and examples of electrophilic reagents include activated esters (e.g. N-hydroxysuccinimide, pentafluorophenyl) and maleimides.

### Example 19: GalNAcligands for hepatocyte targeting of oligonucleotides

Without limiting the disclosure to these individual methods, there are at least two general methods for attachment of multivalent N-acetylgalactosamine (GalNAc) ligands to oligonucleotides: solid or solution-phase conjugations. GalNAc ligands may be attached to solid phase resin for 3' conjugation or the 5' terminus using GalN Ac phosphoramidite reagents. GalNAc phosphoramidites may be coupled on solid phase as for other nucleosides in the oligonucleotide sequence at any position in the sequence. A non-limiting example of a phosphoramidite reagent for GalNAc conjugation to a 5' end oligonucleotide is shown in **Table 32.**

**Table 32. GalNAc Conjugation Reagent**

| Type of conjugation | Structure |
|---|---|
| Solid phase 5' attachment phosphoramidite | 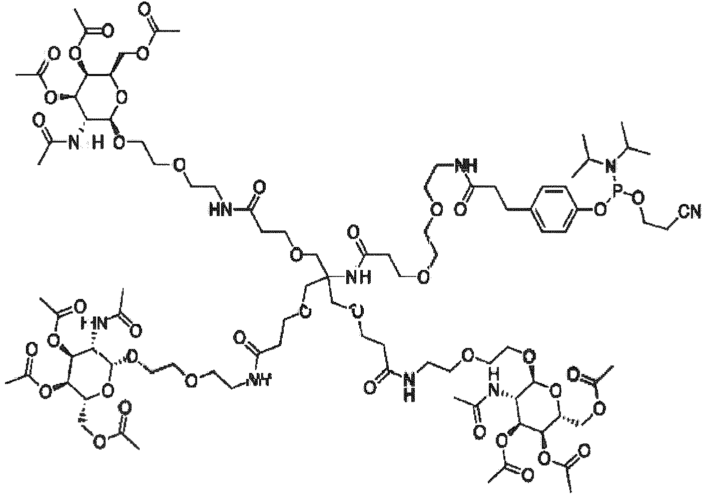 |

The following includes examples of synthesis reactions used to create a GalNAc moiety:

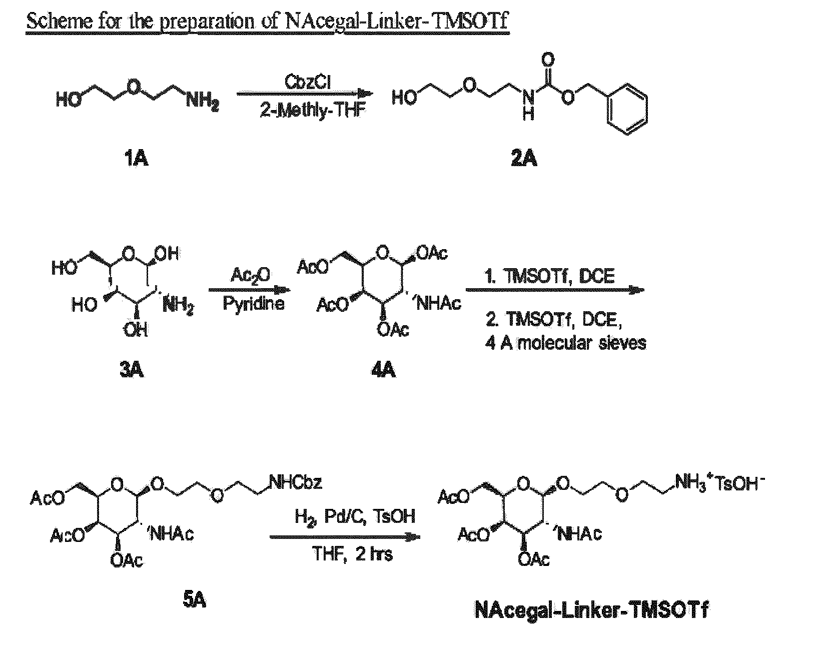

### General procedure for preparation of Compound 2A

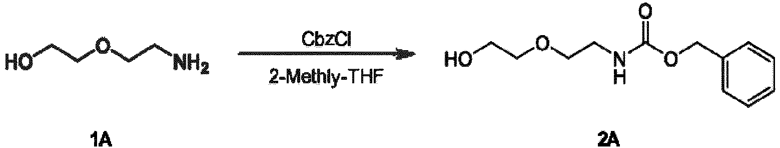

To a solution of Compound 1A (500 g, 4.76 mol, 476 mL) in 2-Methly-THF (2.00 L) is added CbzCl (406 g, 2.38 mol, 338 mL) in 2-Methyl-THF (750mL) dropwise at 0°C. The mixture is stirred at 25 °Cf ar 2 hrs under N₂ atmosphere. TLC (DCM: MeOH =20: 1, PMA) may indicate CbzCl is consumed completely and one new spot (R_{f} = 0.43) formed. The reaction mixture is added HCl/EtOAc (1 N, 180 mL) and stirred for 30 mins, white solid is removed by filtration through celite, the filtrate is concentrated under vacuum to give Compound 2A (540 g 2.26 mol, 47.5%yield) as a pale yellow oil and used into the next step without further purification. ¹H NMR: δ7.28-7.41 (m, 5H),5.55 (brs, 1H), 5.01 - 5.22(m, 2H), 3.63-3.80 (m, 2H), 3.46-3.59(m, 4H), 3.29-3.44 (m, 2H), 2.83-3.02 (m, 1H).

### General procedure for preparation of Compound 4A

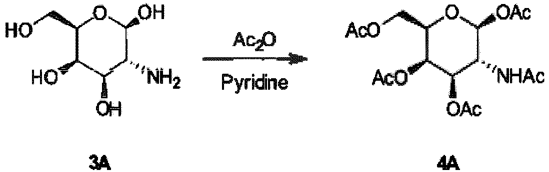

To a solution of Compound 3A (1.00 kg, 4.64 mol, HCl) in pyridine (5.00 L) is added acetyl acetate (4.73 kg, 46.4 mol, 4.34 L) dropwise at 0 °C under N₂ atmosphere. The mixture is stirred at 25 °C for 16 hrs under N₂ atmosphere. TLC (DCM: MeOH =20: 1, PMA) indicated Compound 3A is consumed completely and two new spots (R_{f}= 0.35) formed. The reaction mixture is added to cold water (30.0L) and stirred at 0°C for 0.5 hr, white solid formed, fillered and dried to give Compound 4A (1.55 kg, 3.98 mol, 85.8% yield) as a white solid and used in the next step without further purification. ¹HNMR: δ7.90 (d. *J* - 9.29Hz1H), 5.64(d,*J* - 8.78Hz, 1H), 5.26(d,*J* - 3.01Hz, 1H), 5.06 (dd,J - 11.2 9,3.26 Hz, 1H), 4.22(1, *J* =6.15 Hz, 1H), 3.95-4.16 (m, 3H), 2.12(s, 3H),2.03 (s, 3H), 1,99(s, 3H), 1.90(s,3 H), 1.78(s. 3H).

### General procedure of preparation of Compound 5A

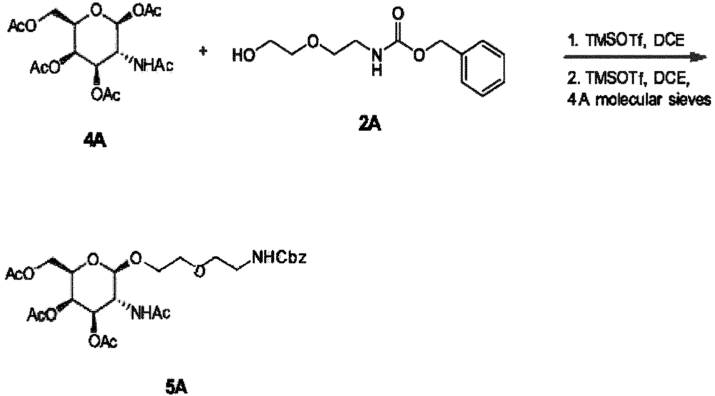

To a solution of Compound 4A (300 g, 771 mmol) in DCE (1.50 L) is added TMSOTf(257 g, 1.16 mol, 209 mL) and stirred for 2 hrs at 60 °C, and then stirred for 1 hr at 25 °C. Compound 2A (203 g, 848 mmol) is dissolved in DCE (1.50L) and added 4 Å powder molecular sieves (150 g) stirrirg for 30 mins under N₂ atmosphere. Then the solution of Compound 4A in DCE is added dropwise to the mixture at 0 °C. The mixture is stirred a 25 °C for 16 hrs under N₂ atmosphere. TLC (DCM: MeOH = 25:1, PMA) indicated Compound 4A is consumed completely and new spot (R_{f}= 0.24) formed. The reaction mixture is filtered and washed with sat. NaHCO₃ (2.00 L), water (2.00 L) and sat. brine (2.00L). The organic layer is dried over anhydrous Na₂SO4, filtered and concentrated under reduced pressure to give a residue. The residue is triturated with 2-Me-THE/heptane (5/3, v/v, 1.80 L) for 2 hrs, filtered and dried to give Compound 5A (225 g, 389 mmol, 50.3% yield, 98.4% purity) as a white solid. ¹H NMR: δ7.81(d.*J* = 929Hz, 1H), 7.20-7.42 (m, 6H), 5.21(d,*J* = 3.26 Hz, 1H), 4 92-5.05 (m, 3H), 4,55(d,*J* = 828 Hz. 1H), 3.98-4.07(m, 3H), 3.82-3.93 (m, 1H),3.71-3.81(m, 1H), 3.55 -3.62(m, 1H), 3.43-3.53 (m, 2H), 3.37-3.43 (m, 2H), 3.14(q,*J* - 5.77Hz, 2H), 2.10(s, 3H), 1.99(s, 3H), 1.89(s, 3H), 1.77 (s,3 H).

### General procedure for preparation of N. Acegal-Linker-Tosylate salt

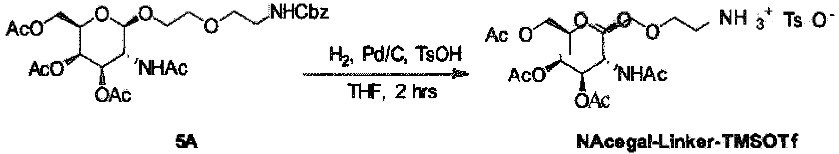

To a solution of Compound 5A (200 g, 352 mmol) in THF (1.0 L) is added dry Pd/C(15.0g, 10% purity) and TsOH (60.6 g, 352 mmol) under N₂ atmosphere. The suspension is degassed under vacuum and purged with H₂ several times. The mixture is stirred at 25 °C for 3 hrs under H₂ (45 psi) atmosphere. TLC (DCM: MeOH = 10: 1, PMA) indicated Compound 5A is consumed completely and one new spot (R_{f}= 0.04) is formed. The reaction mixture is filtered and concentrated (≤40 °C) under reduced pressure to give a residue. Diluted with anhydrous DCM (500 mL, dried overnight with 4 A molecular sieves (dried at 300 °C for 12 hrs)) and concentrate to give a residue and run Karl Fisher (KF) to check for water content. This is repeated 3 times with anhydrous DCM (500 mL) dilutions and concentration to give NAcegal-Linker-TMSOTf (205 g 95.8% yield, TsOH salt) as a foamy white solid. ¹H NMR: 67.91 (d,*J* =9.0311z, 1H), 7.53-7.86 (m, 211), 7.49(d,*J* =8.0311z, 2H), 7.13(d,*J* = 8.03Hz, 2H), 5.22 (d, J=3.26Hz, 1H), 4.98 (dd,J= 11.29. 3.26 Hz, 1H), 4.57(d,J=8.53 Hz, 1H), 3.99-4.05 (m. 3 H). 3.87-3.94(m, 1H), 3.79-3.85(m, 1H), 3.51-3.62 (m, 5H), 2.96(brt,*J* = 5.14Hz, 2H), 2.29(s,3 H), 2.10(s, 3H), 2.00(s, 3H), 1.89(s. 3H), 1.78(s, 3H).

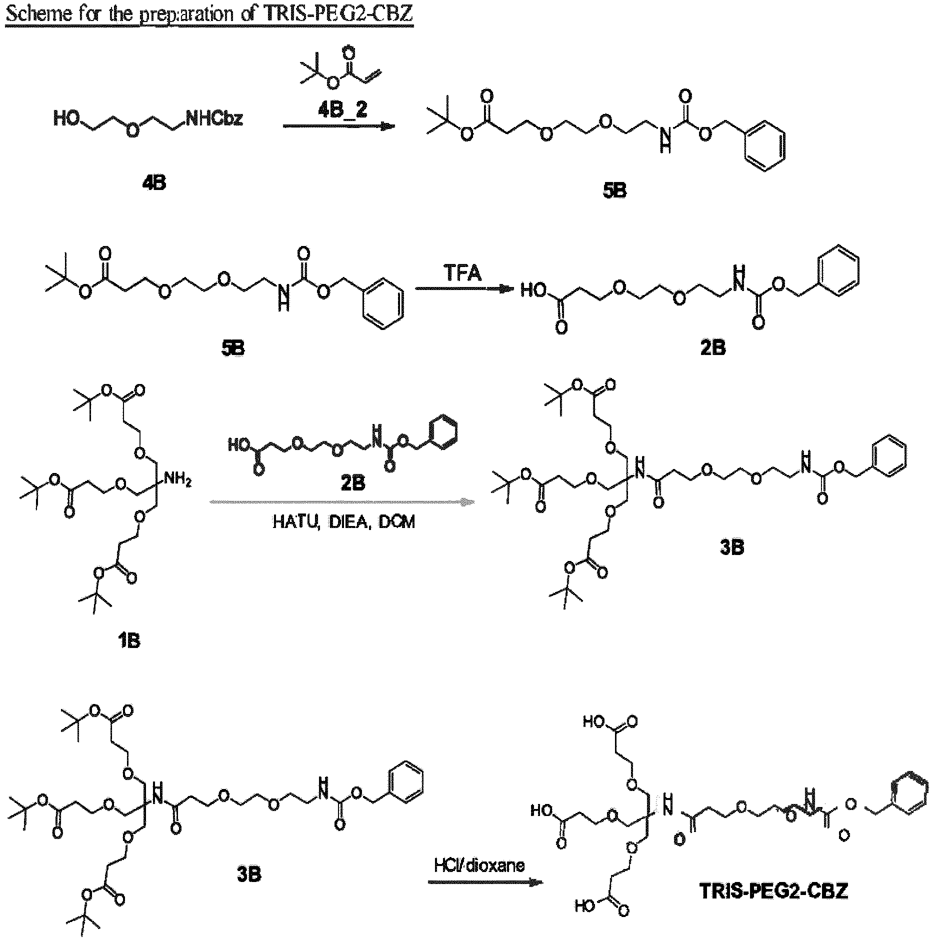

### General procedure for preparation of Compound 5B

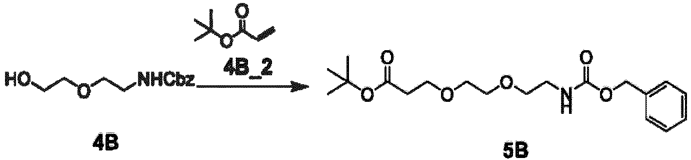

To a solution of Compound 4B (400 g, 1.67 mol, 1.00 *eq)* and NaOH (10 M, 16.7 mL, 0.10 *eq)* in THF (2.00 L) is added Compound 4B_ 2(1.07 kg, 8.36 mol, 1.20 L. 5.00 *eq),* the mixture is stirred at 30 °C for 2hrs. LCMS showed the desired MS is given. Five batches of solution are combined to one batch, then the mixture is diluted with water (6.00 L), extracted with ethyl acetate (3.00 L*3), the combined organic layer is washed with brine (3.00 L), dried over Na₂SO₄, filtered and concentrated under vacuum. The crude is purified by column chromatography (SiO₂, petroleum ether : ethyl acetate=100:1-10:1, R_{f}=0.5) to give Compound 5B (2.36kg, 6.43 mol, 76.9% yield) as light yellow oil. HNMR: δ7.31-7.36 (m, 5 H), 5.38(s, 1H), 5.11-5.16(m,2H), 3.75(t, J= 6.4Hz) 3.54-3.62 (m, 6H), 3.39(d,*J*= 5.2 Hz), 2.61 (t,*J*=6.0Hz).

### General procedure for preparation of 3-oxo-1-phenyl-2,7,10-trioxa-1-azatridecan-13-oic acid (Compound 2B below)

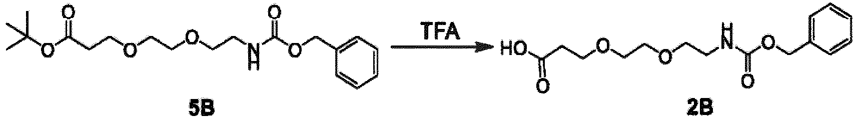

To a solution of Compound 5B (741 g 2.02 mol, 1.00 *eq*) in DCM (2.80 L) is added TFA (1.43kg, 12.5 mol, 928 mL, 6.22 *eq),* the mixture is stirred at 25 °C for 3 hrs. LCMS showed the desired MS is given. The mixture is diluted with DCM (5.00 L), washed with water (3.00 L*3), brine (2.00 L), the combined organic layer is dried over Na₂ SO₄, filtered and concentrated under vacuum to give Compound 2B (1800 g, crude) as light yellow oil. HNMR: δ9.46(s, 5H), 7.27-7.34 (m, 5H), 6.50-6.65 (m 1H), 5.71(s, 1H), 5.10-5.15 (m, 2H), 3.68-3.70 (m, 14H), 3.58-3.61(m, 6H), 3.39(s, 2H), 2.55 (s, 6 H), 2.44 (s, 2 H).

### General procedure for preparation of Compound 3B

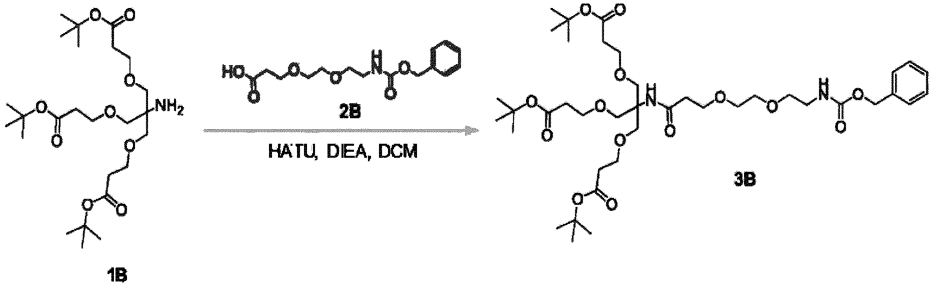

To a solution of Compound 2B (375 g, 999 mmol, 83.0% purity, 1.00 *eq)* in DCM(1.80 L) is added HATU (570 g, 150 mol, 1.50 *eq)* and DIEA (258 g, 2.00 mol, 348 mL, 2.00*eq*) a10°C, the mixture is stirred at 0 °C for 30 min, then Compound 1B (606 g, 1.20 mol, 1.20 *eq)* is added, the mixture is stirred at 25 °Cfor 1 hr. LCMS showed desired MS is given. The mixture is combined to one batch, then the mixture is diluted with DCM (5.00 L), washed with 1 N HCl aqueous solution (2.00 L*2), then the organic layer is washed with saturated Na₂CO₃ aqueous solution (2.00L *2) and brine (2.00 L), the organic layer is dried over Na₂SO₄, filtered and concentrated under vacuum to give Compound 3B (3.88 kg, crude) as yellow oil.

### General procedure for preparation of TRIS-PEG2-CBZ.

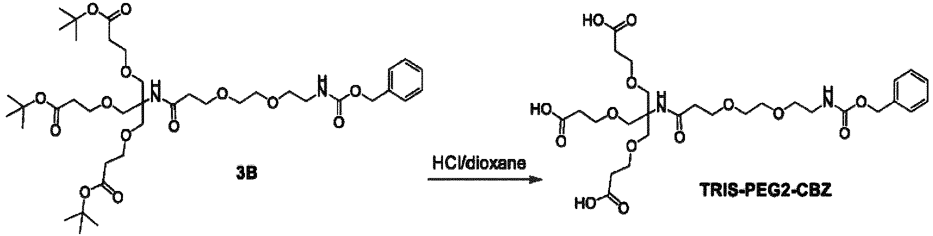

A solution of Compound 3B (775 g, 487 mmol, 50.3% purity, 1.00 *eq*) in HCl/dioxane (4 M, 2.91L, 23.8*eq*) is stirred at 25 °C for 2hrs. LCMS showed the desired MS is given. The mixture is concentrated under vacuum to give a residue. Then the combined residue is diluted with DCM(5.00 L). adjusted to pH=8 with 2.5 M NaOH aqueous solution, and separated. The aqueous phase is extracted with DCM (3.00 L) again, then the aqueous solution is adjusted to pH=3 with 1 N HCl aqueous solution, then extracted with DCM (5.00L*2), the combined organic layer is washed with brine (3.00 L), dried over Na₂SO₄, filtered and concentrated under vacuum. The crude is purified by column chromatography (SiO₂, DCM:MeOH=0:1-12:1,0.1% HOAc, R_{f}=0.4). The residue is diluted with DCM (5.00 L), adjusted to pH=8 with 2.5 M NaOH aqueous solution, separated, the aqueous solution is extracted with DCM (3.00 L) again, then the aqueous solution is adjusted to pH=3 with 6 N HCl aqueous solution, extracted with DCM:MeOH=10:1 (5.00 L*2), the combined organic layer is washed with brine (2.00 L), dried over Na₂SO₄, filtered and concentrated under vacuum to give a residue. Then the residue is diluted with MeCN (5.00 L), concentrated under vacuum, repeat this procedure twice to remove water to give TRIS-PEG2-CBZ (1.25 kg, 1.91 mol, 78.1%yield, 95.8% purity) as light yellow oil. ¹H NMR: 400 MHz, MeOD, δ 7.30-7.35 (5 H), 5.07(s, 2H), 3.65-3.70 (m, 16H), 3.59(s, 4H). 3.45(1, *J*= 5.6Hz), 2.51(t, *J*=6.0Hz), 2.43(t, 6.4Hz).

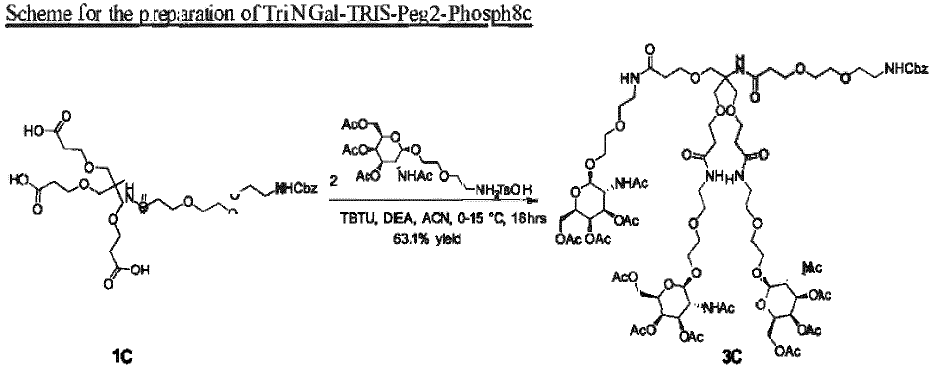

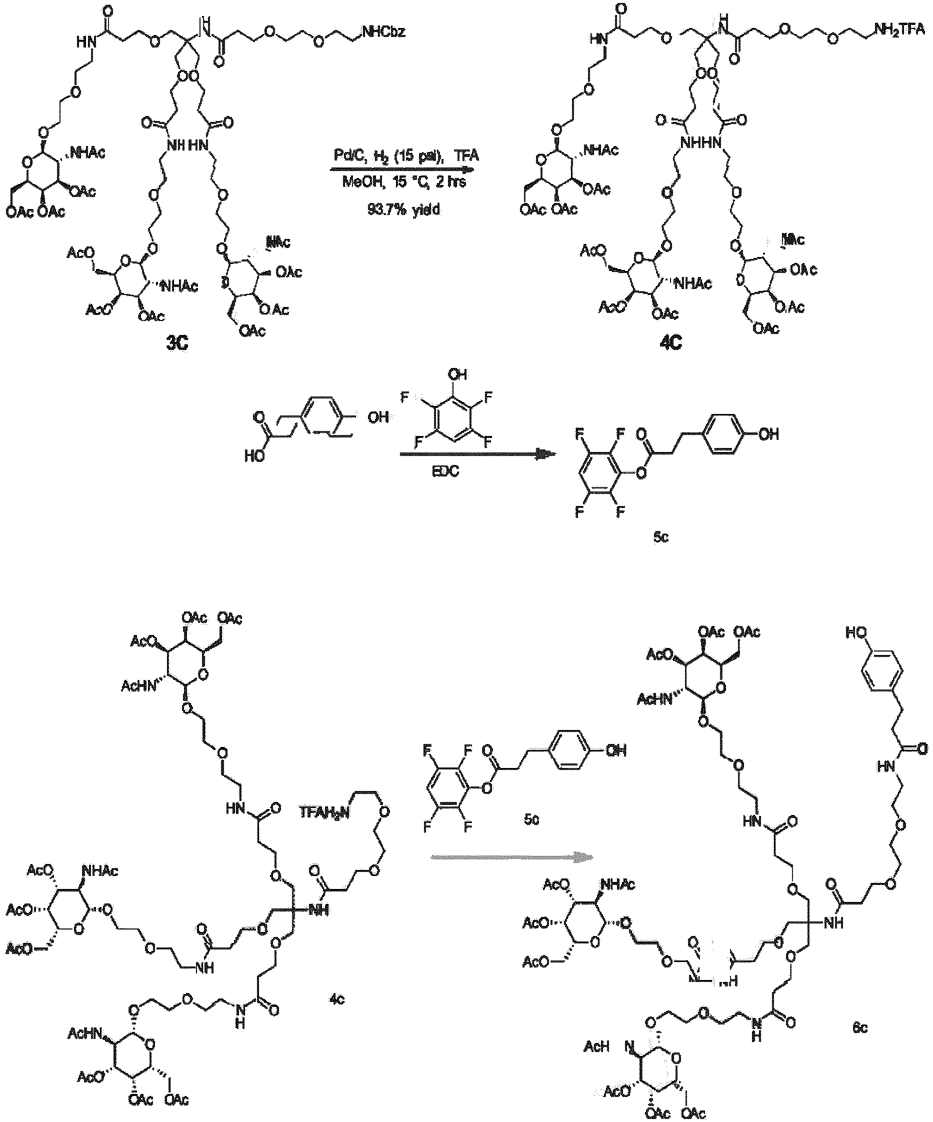

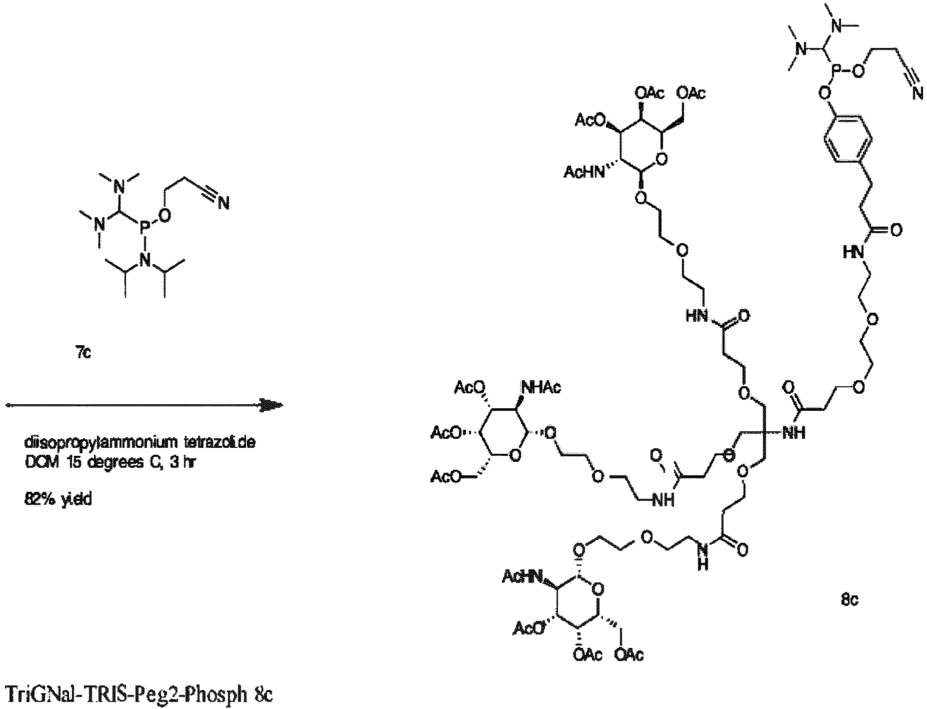

### General procedure for preparation of Compound 3C

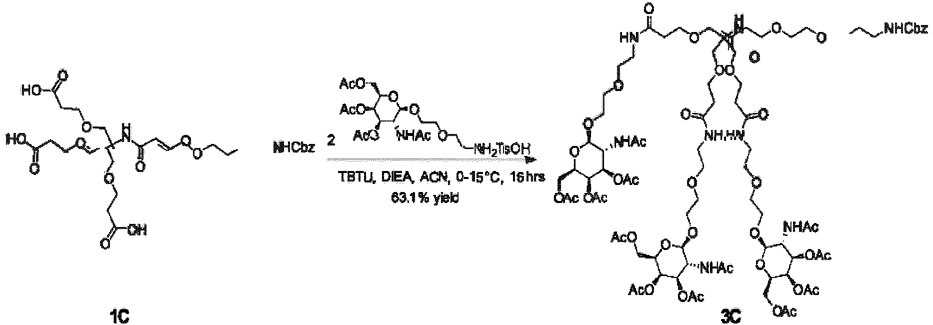

To a solution of Compound 1C (155 g, 245 mmol, 1.00 *eq*) in ACN (1500 mL) is added TBTU (260 g, 811 mmol, 3.30 *eg*), DIEA (209 g, 1.62 mol, 282 mL, 6.60 *eq)* and Compound 2C (492 g 811 mmol, 3.30 *eq,* TsOH) at 0°C, the mixture is stirred at 15 °C for 16 hrs. LCMS showed the desired MS is given. The mixture is concentrated under vacuum to give a residue, then the mixture is diluted with DCM (2000 mL), washed with 1 N HCl aqueous solution (700 mL * 2), then saturated NaHCO₃ aque ous solution (700 mL *2) and concentrated under vacuum The crude is purified by column chromatography to give Compound 3C (304 g, 155 mmol, 63.1% yield, 96.0% purity) as a yellow solid.

### General procedure for preparation of Compound 4C

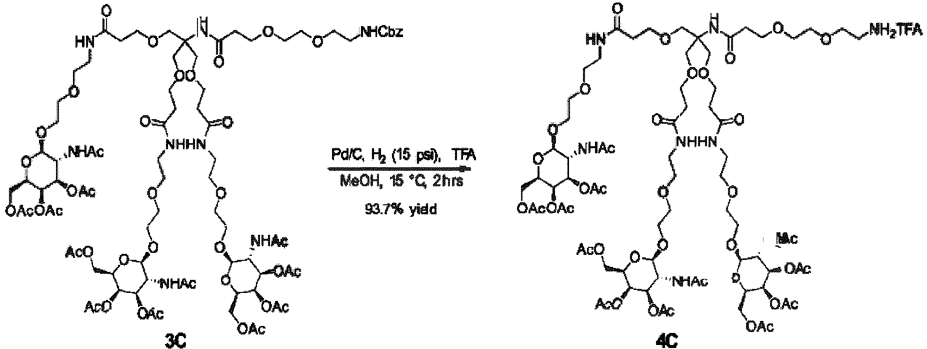

Two batches solution of Compound 3C (55.0 g, 29.2 mmol, 1.00 *eq*) in MeOH (1600 mL) is added Pd/C (6.60 g, 19.1 mmol, 10.0 % purity) and TFA (3.34g, 29.2 mmol, 2.17 mL, 1.00*eq*), the mixture is degassed under vacuum and purged with H₂. The mixture is stirred under H₂ (15 psi) at 15 °C for 2 hours. LCMS showed the desired MS is given. The mixture is filtered and the filtrate is concentrated under vacuum to give Compound 4C (106 g, 54.8 mmol, 93.7%yield, 96.2% purity, TFA) as a white solid.

### General procedure for preparation of compound 5C

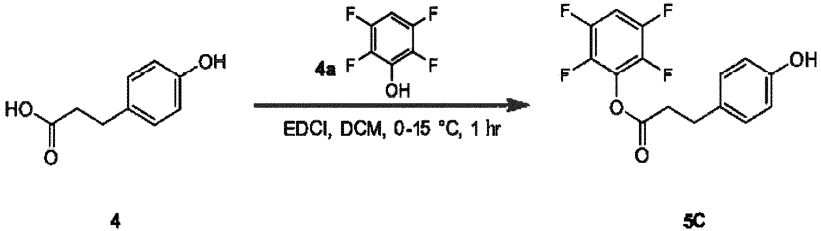

Two batches in parallel. To a solution of EDC1 (28.8 g, 150 mmol, 1.00 eq) in DCM (125 mL) is added compound **4a** (25.0 g, 150 mmol, 1.00 eq) dropwise at 0°C, then the mixture is added to compound 4 (25.0g,1 50 mmol, 1.00 eq) in DCM(125 mL) at 0°C, then the mixture is stirred at 25 °C for 1 hr. TLC (Petroleum ether: Ethyl acetate= 3 : 1, R_{f} = 0.45) showed the reactant is consumed and one new spot is formed. The reaction mixture is diluted with DCM (100 mL) then washed with aq. NaHCO₃ (250 mL * 1) and brine (250 mL), dried over Na₂SO₄, filtered and concentrated under reduced pressure to give a residue. The residue is purified by column chromatography (SiO₂, Petroleum ether : Ethyl acetate = 100: 1 to 3: 1), TLC (SiO₂, Petroleum ether : Ethyl acetate =3:1), R_{f}= 0.45, then concentrated under reduced pressure to give a residue. Compound **5C**(57.0 g, 176 mmol, 58.4% yield, 96.9% purity) is obtained as colorless oil and confirmed **¹H NMR:** EW33072-2-P1A, 400 MHz, DMSO *δ* 9.21(s, 1H), 7.07-7.09 (m, 2H), 6.67-6.70(m, 2H), 3.02-3.04(m, 2H), 2.86-2.90(m, 2 H)

### General procedure for preparation of compound 6

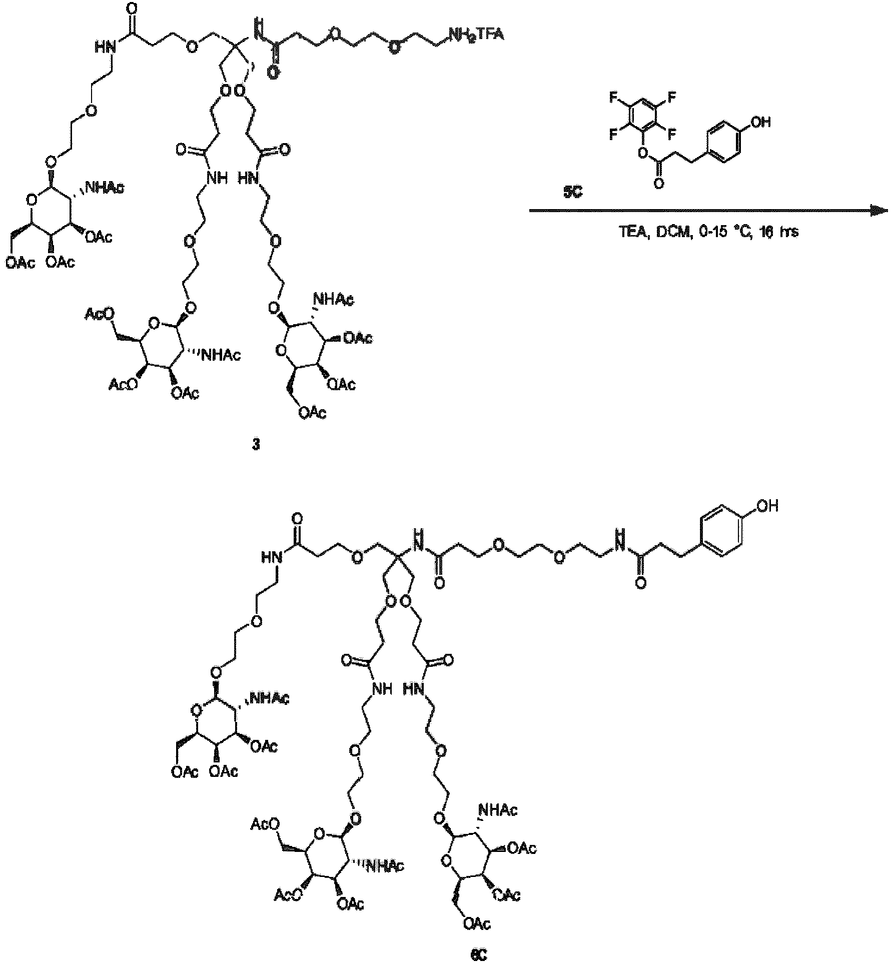

To a mixture of compound **3** (79.0 g, 41.0 mmol, 96.4% purity, 1.00 eq, TFA) and compound **6C**(14.2 g, 43.8 mmol, 96.9%purity, 1.07 eq) in DCM (800 mL) is added TEA (16.6 g, 164 mmol, 22.8 mL, 4.00 eq) dropwise at 0 °C, the mixture is stirred at 15°C for 16 hrs. LCMS (EW33072-12-P1B,Rt= 0.844 min) showed the desired mass is detected. The reaction mixture is diluted with DCM (400 mL) and washed with aq. NaHCO₃ (400 mL * 1) and brine(400 mL * 1), then the mixture is diluted with DCM (2.00L) and washed with 0.7 M Na₂CO₃ (1000 mL * 3) and brine(800 mL * 3), dried over Na₂SO₄, filtered and concentrated under reduced pressure to give a residue. The residue is used to next step directly without purification. Compound 6 (80.0 g, crude) is obtained as white solid and confirmed via **¹HN MR:** EW33072-12-P1A, 400 MHz, MeOD *δ* 7.02-7.04 (m, 2H), 6.68- 6.70 (m, 2H), 5.34-5.35 (s, 3H), 5.07-5.08(d, *J* = 4.00Hz, 3H), 4.62-4.64(d, *J* = 8.00Hz, 3H), 3.71-4.16(m, 16H), 3.31-3.70(m, 44H), 2.80-2.83 (m, 2H), 2.68 (m, 2H), 2.46-2.47 (m, 10H), 2.14(s,9H), 2.03 (s, 9H), 1.94 - 1.95(d, *J* = 4.00Hz, 18 H).

### General procedure for preparation of TriGNal-TRIS-Peg2-Plusph 8c

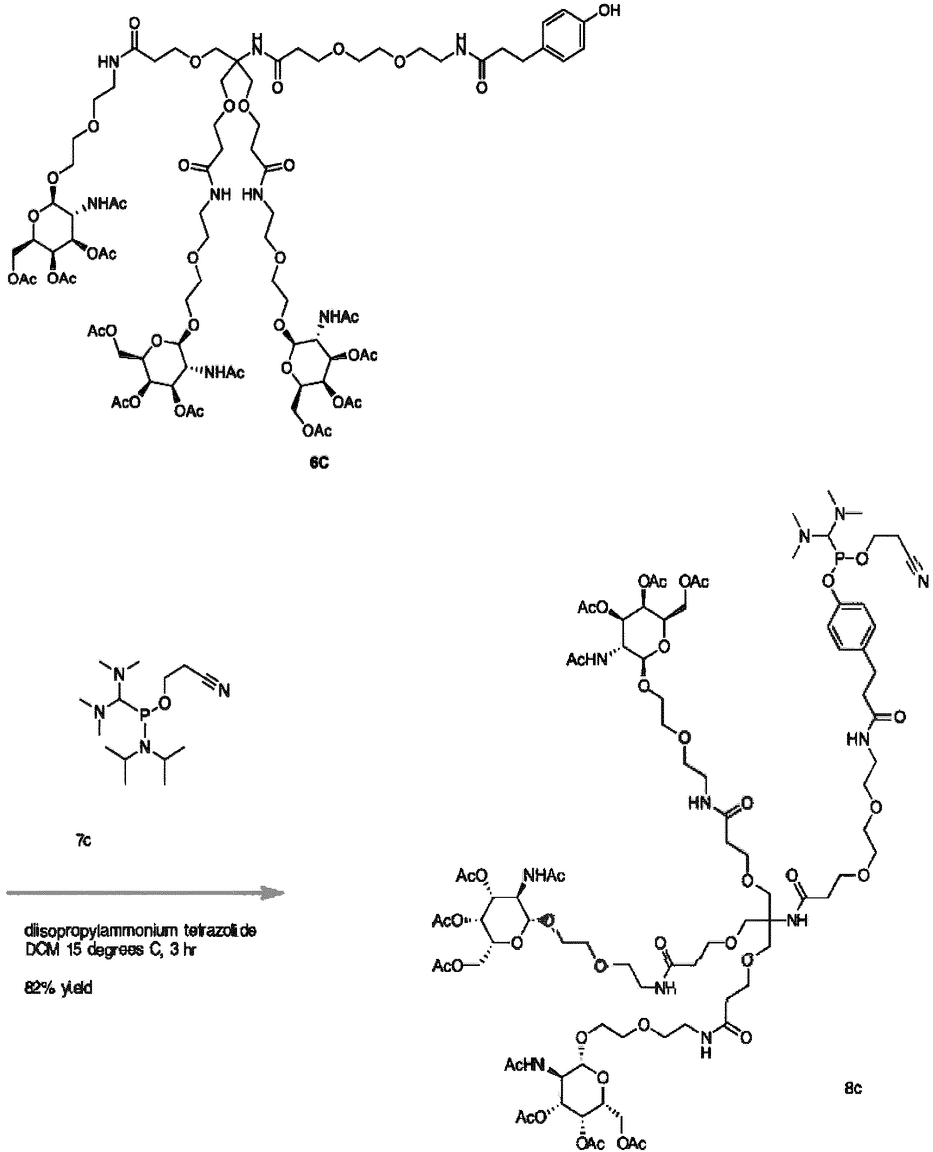

Two batches are synthesized in parallel. To a solution of compound **6C** (40.0 g, 21.1 mmol, 1.00 eq in DCM (6.00 mL) is added diisopropylammonium tetrazolide (3.62 g, 21.1 mmol, 1.00 eq) and compound 7c (6.37 g 21.1 mmol, 6.71 mL. 1.00 *eq*) in DCM (8.00 mL) drop-wise, the mixture is stirred at 30 °C for 1 hr, then added compound 7c (3.18 g, 10.6 mmol, 3.35 mL, 0.50 eq) in DCM (8.00 mL) drop-wise, the mixture is stirred at 30 °C for 30 mins, then added compound **7c** (3.18 g, 10.6 mmol, 3.35 mL, 0.50 eq) in DCM (8.00 mL) drop-wise, the mixture is stirred at 30 °C for 1.5 hrs. LCMS (EW33072-17-PIC1, Rt= 0.921 min) showed the desired MS+1 is detected. LCMS (EW33072-17-PIC2, Rt=0.919 min) showed the desired MS+ 1 is detected. Two batches are combined for work -up. The mixture is diluted with DCM (1.20 L), washed with saturated NaHCO₃ aqueous solution (1.60 L * 2), 3% DMF in H₂O(1.60L * 2),H₂O(1.60L * 3), brine (1.60 L), dried over Na₂SO₄, filtered and concentrated under reduced pressure to give a residue. The residue is purified by column chromatography (SiO₂, DCM: MeOH: TEA = 100: 3:2) TLC (SiO₂, DCM: MeOH = 10:1, R_{f}= 0.45), then concentrated under reduced pressure to give a residue. **Compound** 8C(76.0 g, 34.8 mmol, 82. 5%yield, 96.0% purity) is obtained as white solid and confirmed via **¹HNMR:** EW33072-19-PIC, 400 MHz, MeOD *δ* 7.13-7.15(d, *J* =8.50Hz, 2H), 6.95-6.97(dd, *J* =8.38, 1.13 Hz, 2H), 5.34(d, *J* =2.88 Hz, 3H), .09 (dd, *J*=11.26, 3.38 Hz, 3H), 4.64(d, *J* =8.50Hz, 3H), 3.99-4.20 (m, 12 H), 3.88-3.98 (m, 5H), 3.66-3.83 (m, 20 H), 3.51-3.65 (m, 17H), 3.33-3.50(m.9H), 2.87(t, *J* =7.63Hz, 2H), 2.76(t, *J* =5.94Hz, 2H), 2.42-2.50(m, 10H), 2.14 (s, 9H), 2.03 (s, 9H), 1.94-1.95 (d, *J*=6.13 Hz, 18 H), 1.24-1.26 (d,*J* =675 Hz, 6H), 1.18-1.20(d, *J* =6.75Hz, 6 H)

### Example 20: Modification motif 1

An example MST1 siRNA includes a combination of the following modifications:
- Position 9(from 5' to 3') of the sense strand is2' F.
- If position 9 is a pyrimidine then all purines in the Sense Strand are 2'OMe, and 1-5 pyrimidines between positions 5 and 11 are2' F provided that there are never three 2'F modifications in a row.
- If position 9 is a purine then all pyrimidines in the Sense Strand are 2'OMe, and 1-5 purines between positions 5 and 11 are 2' F provided that there are never three 2'F modifications in a row.
- Antisense strand odd-numbered positions are 2'OMe and even-numbered positions are a mixture of 2'F,2' OMeand2' deoxy.

### Example21:Modification motif2

An example MST1 siRNA includes a combination of the f allowing modifications:
- Position9(from 5' to 3') of the sense strand is 2' deoxy.
- Sense strand positions 5, 7 and 8 are2' F.
- All pyrimidines in positions 10-21 are 2' OMe, and purines are a mixture of 2' OMe and 2' F. Alternatively, all purines in positions 10-21 are 2' OMe and all pyrimidines in positions 10-21 are a mixture of2' OMe and2' F.
- Antisense strand odd-numbered positions are 2'OMe and even-numbered positions are a mixture of 2'F,2' OMe and2' deoxy.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and compositions within the scope of these claims and their equivalents be covered thereby.

### IV. SEQUENCE INFORMATION

Some embodiments include one or more nucleic acid sequences in the following tables:

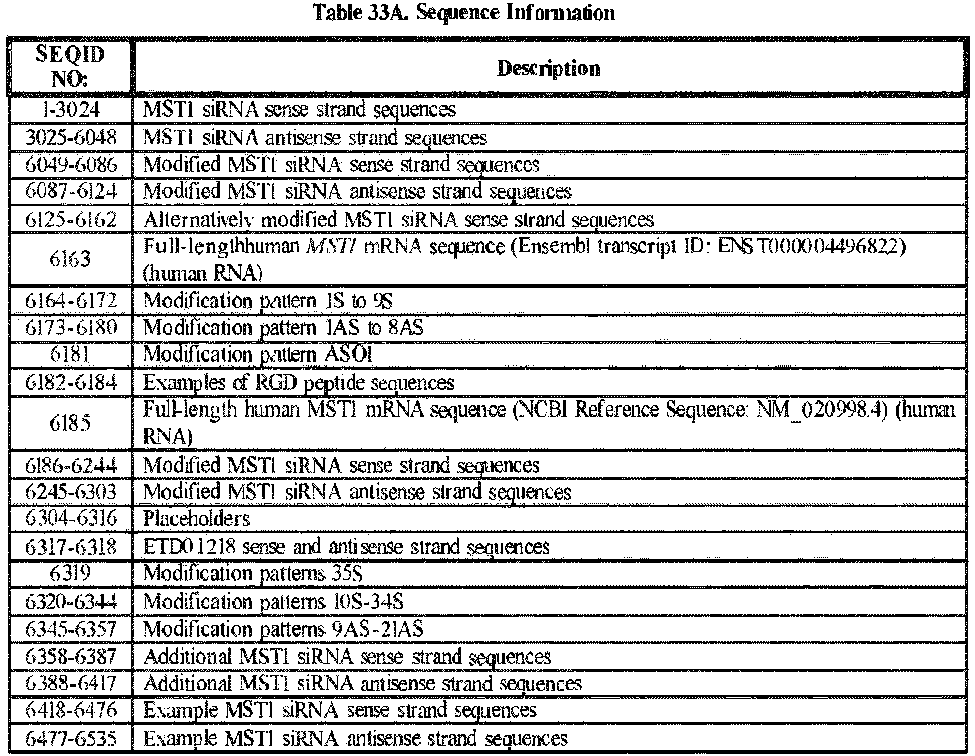

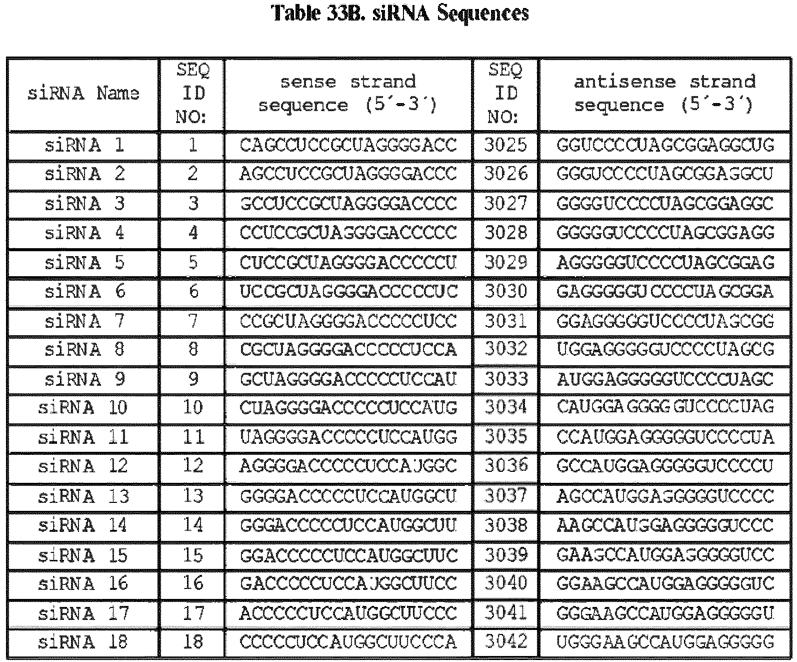

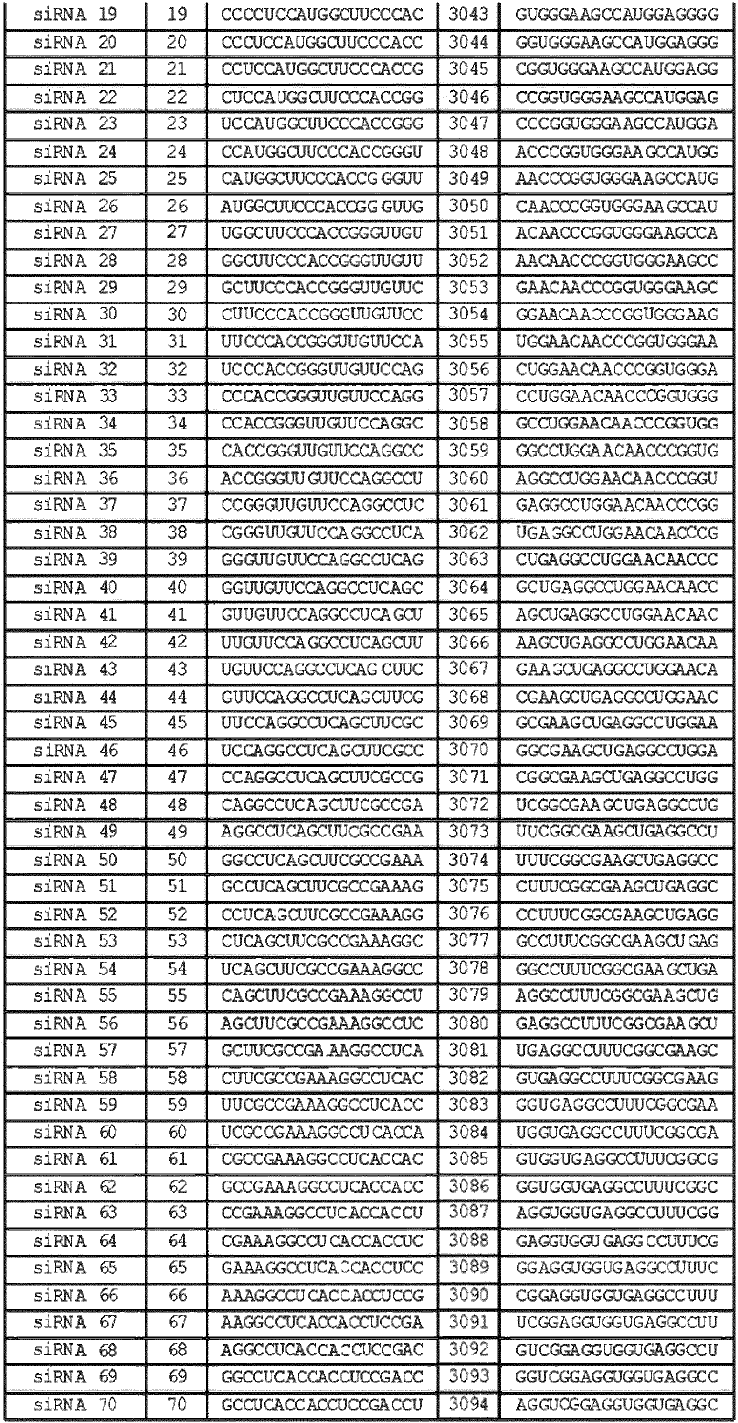

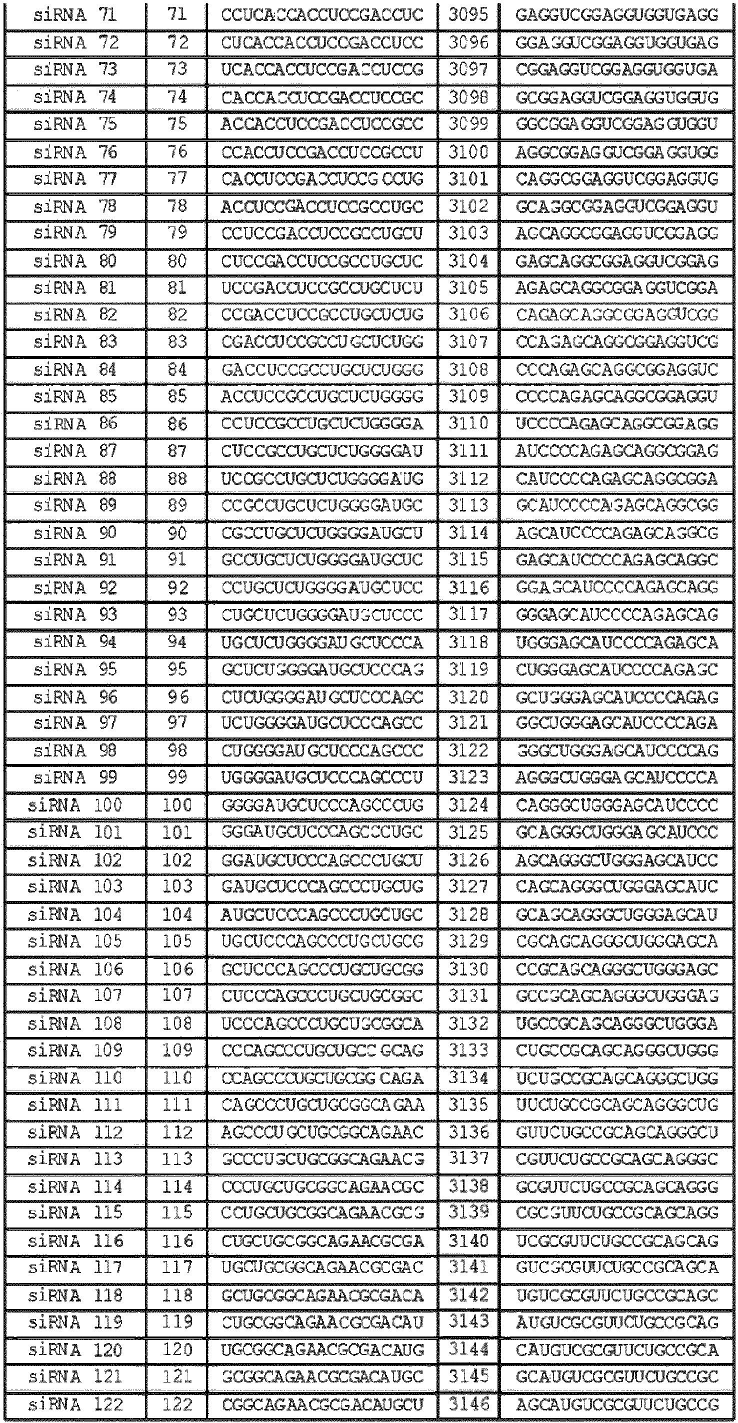

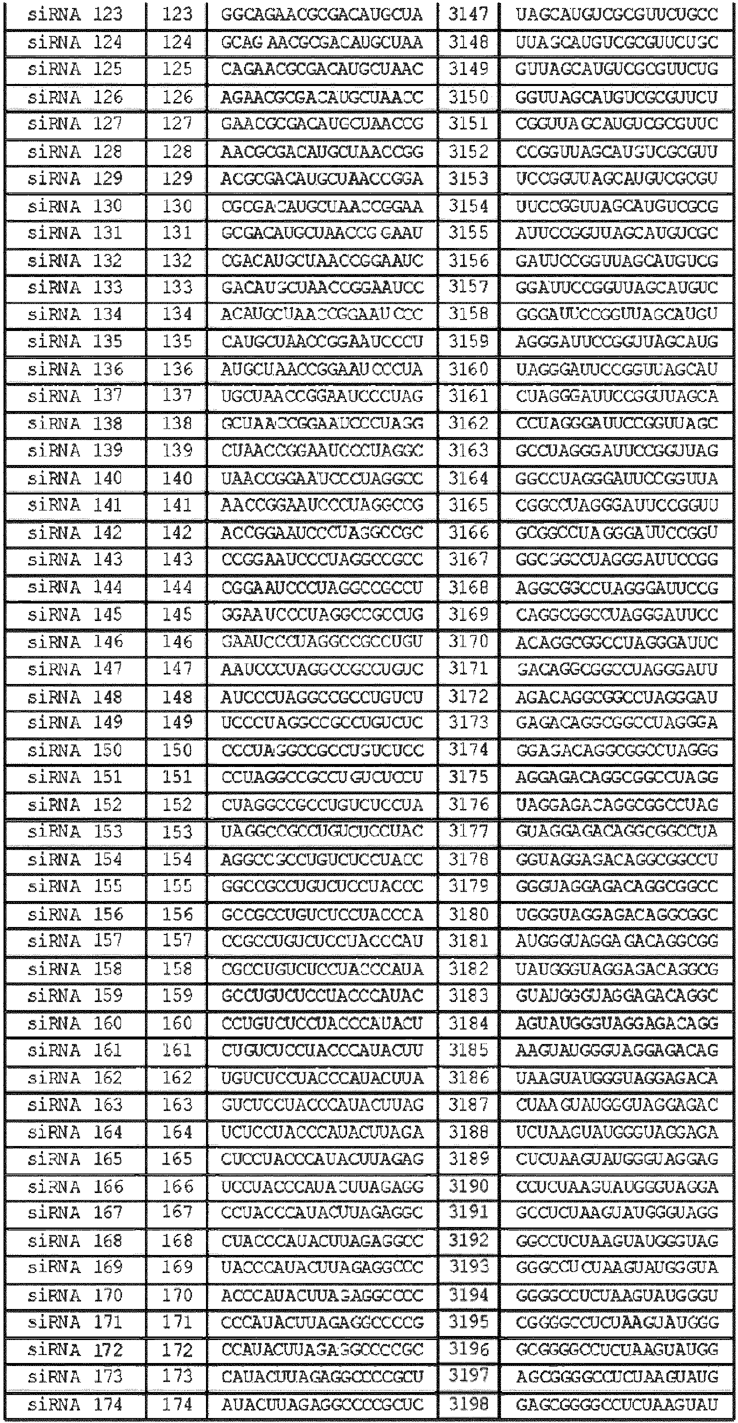

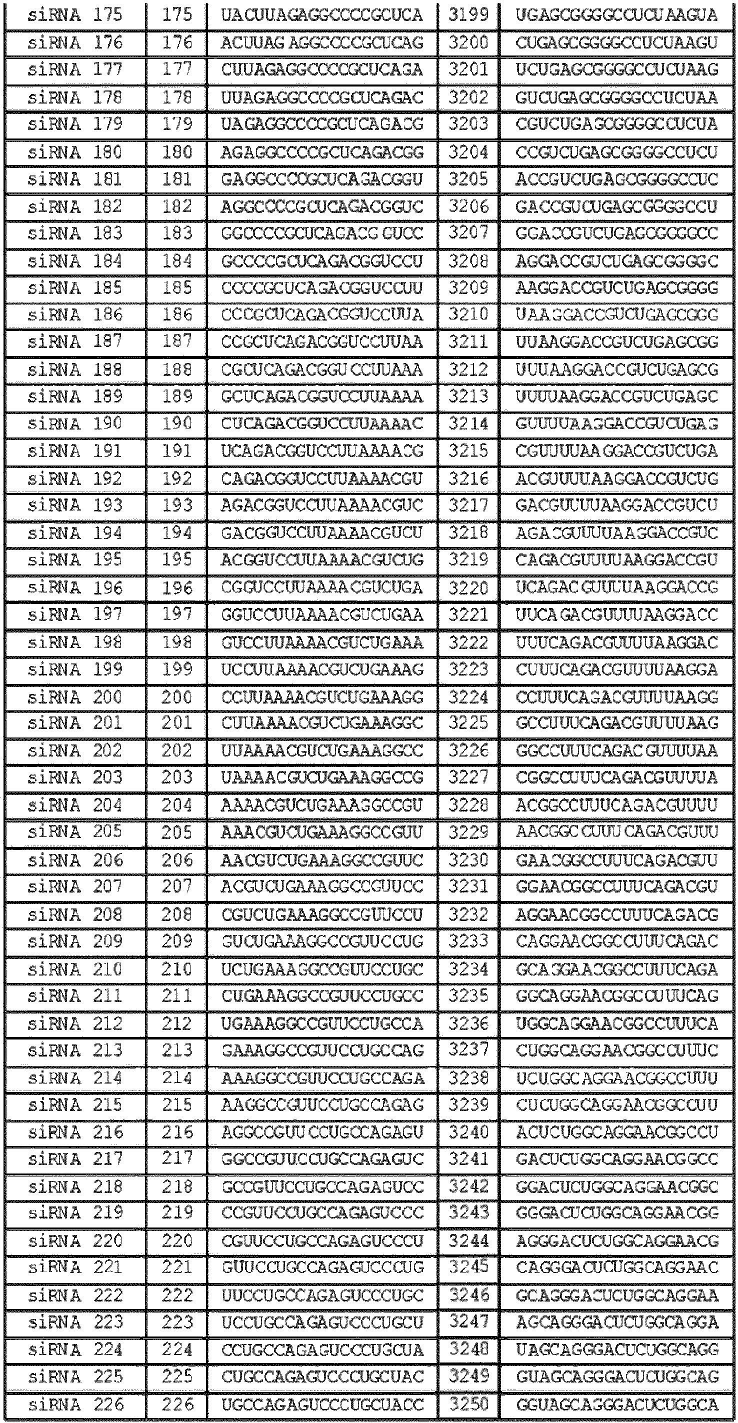

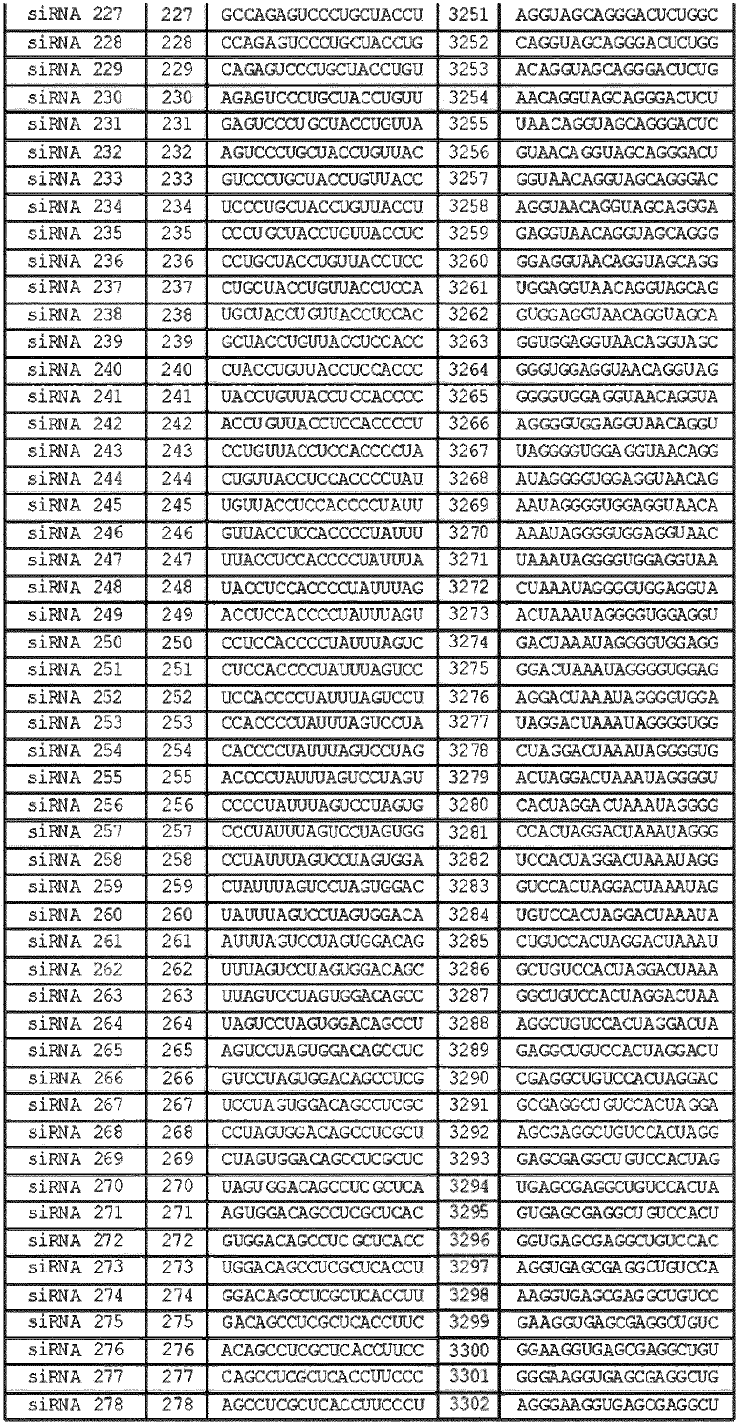

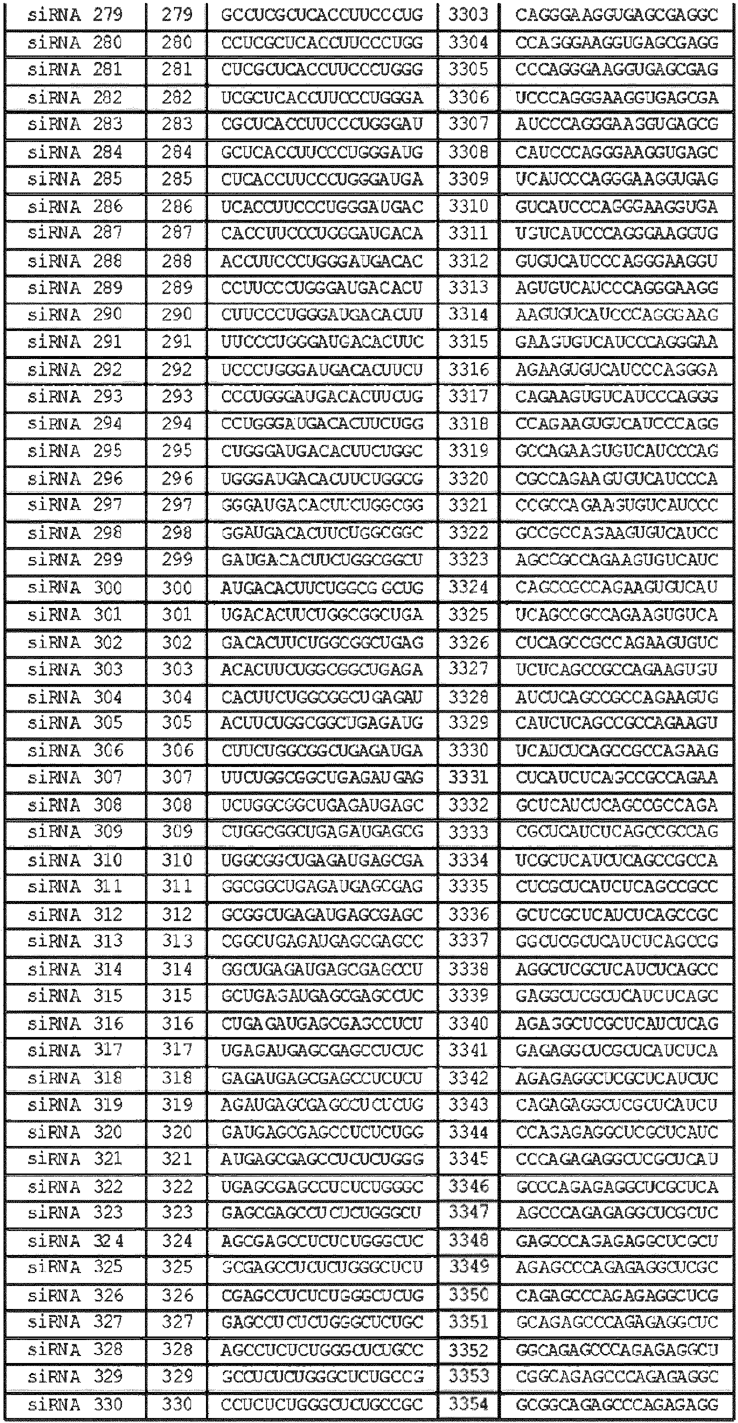

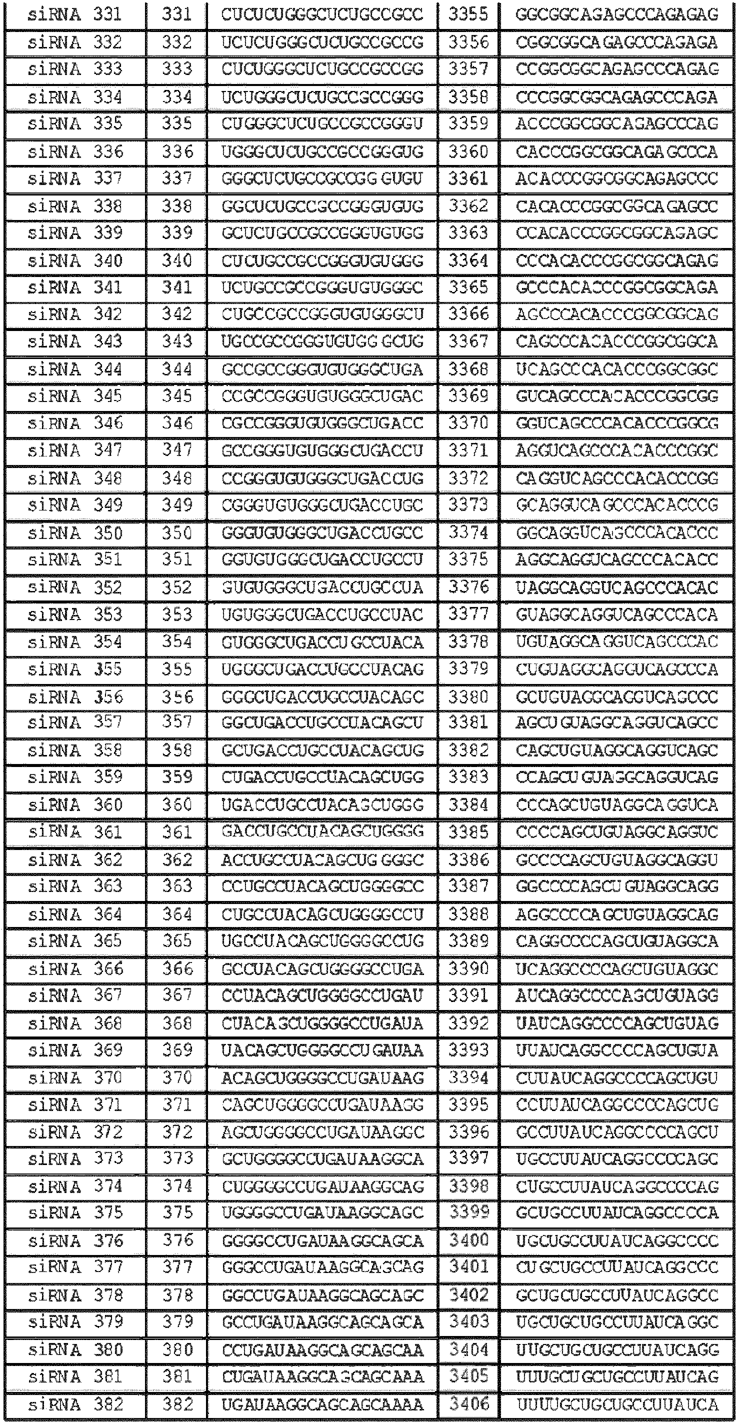

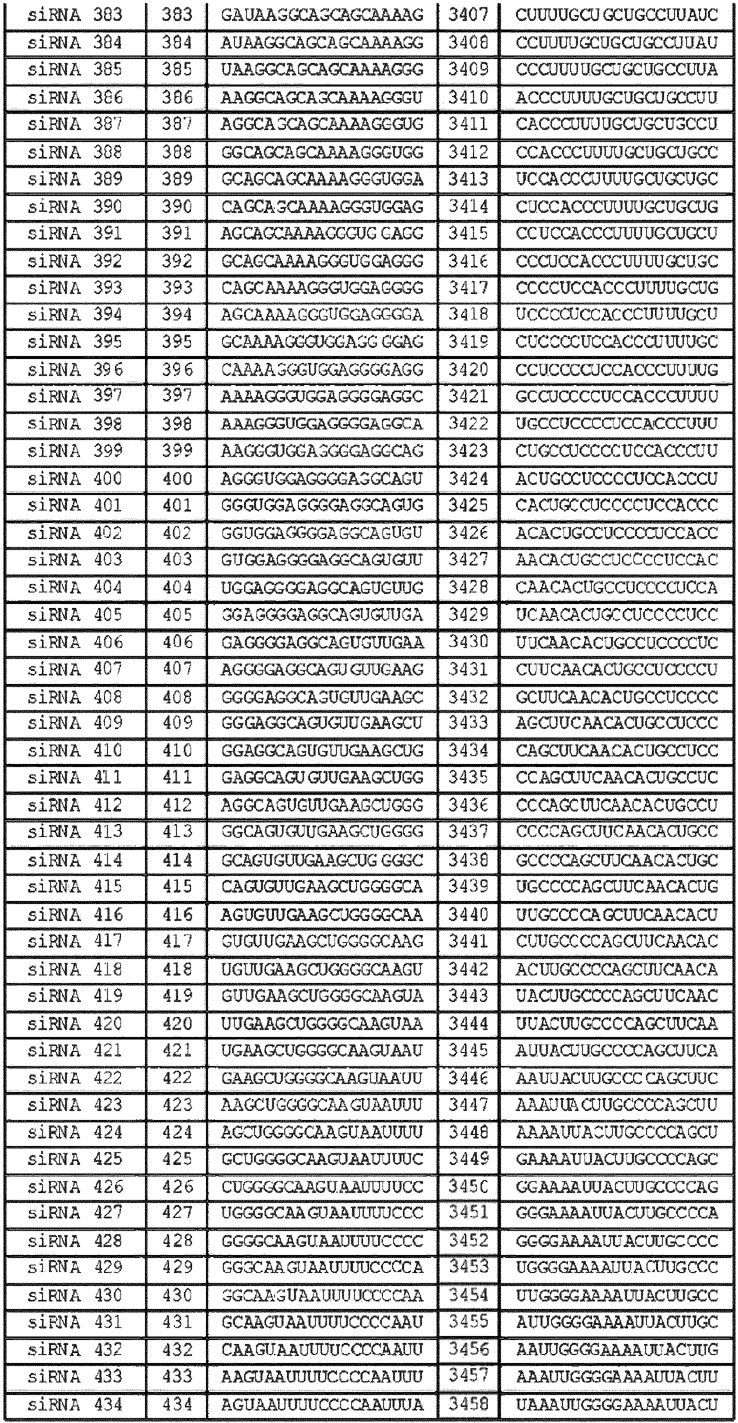

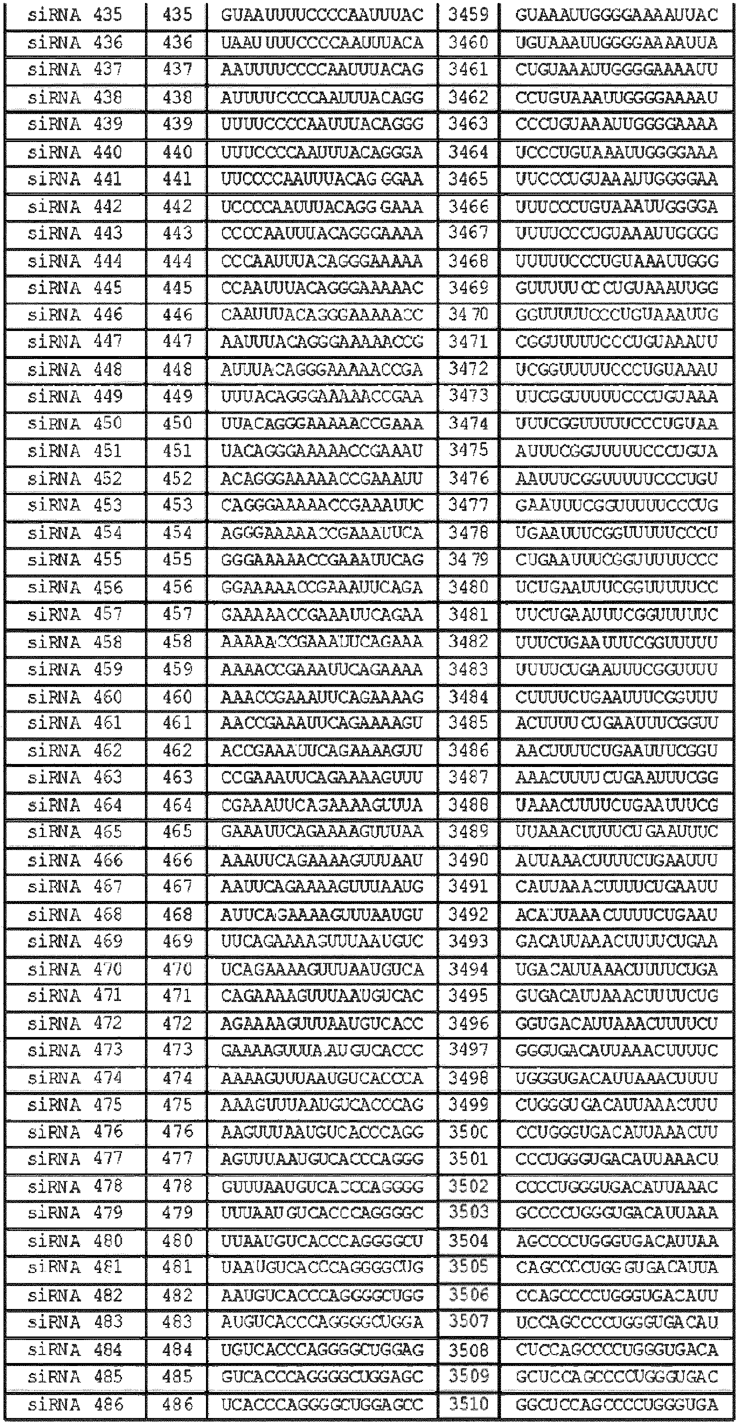

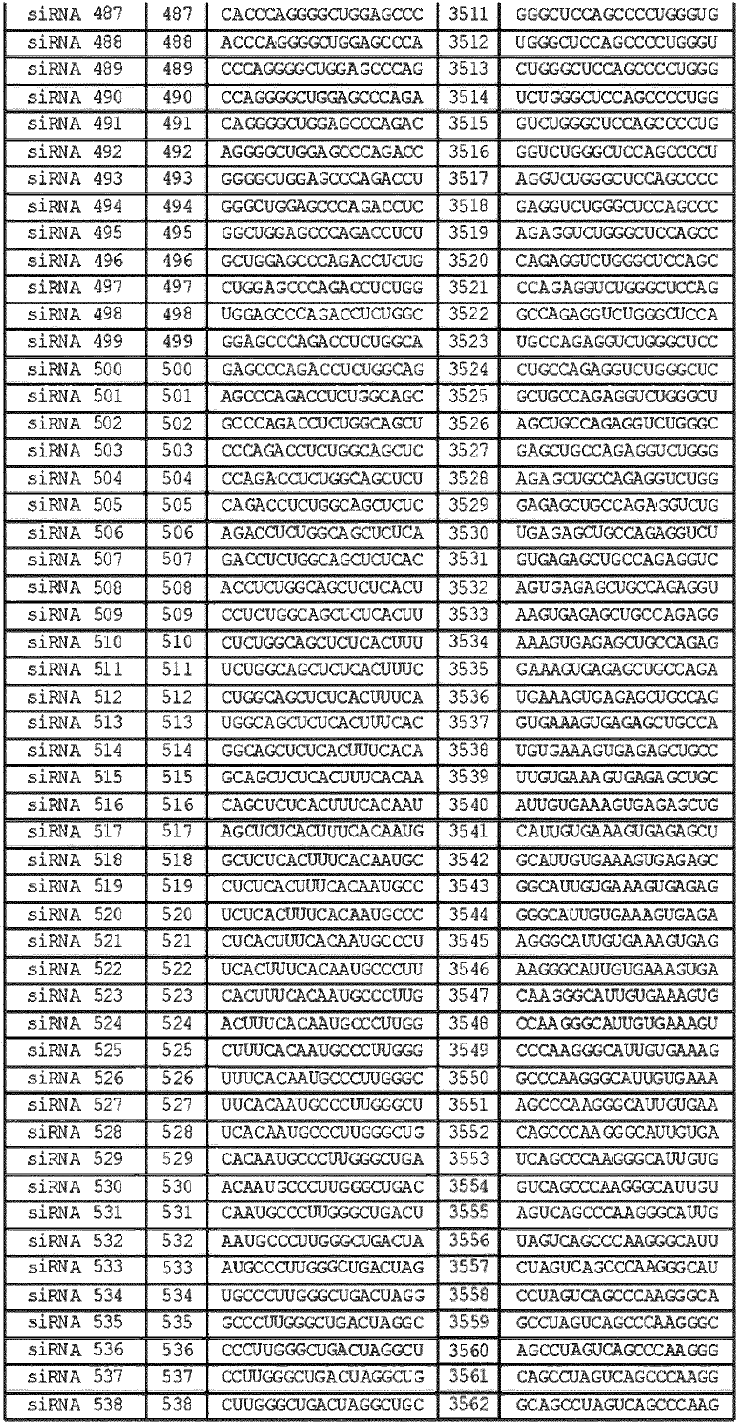

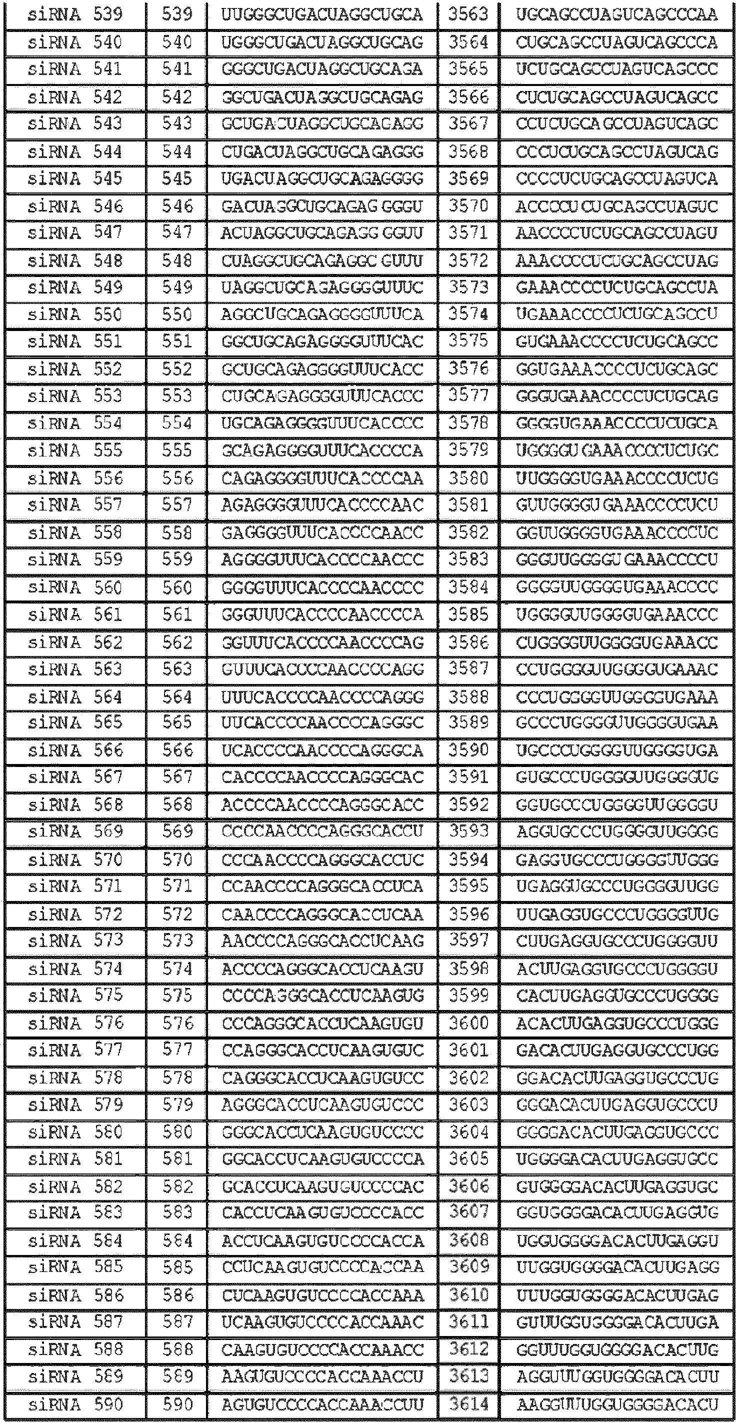

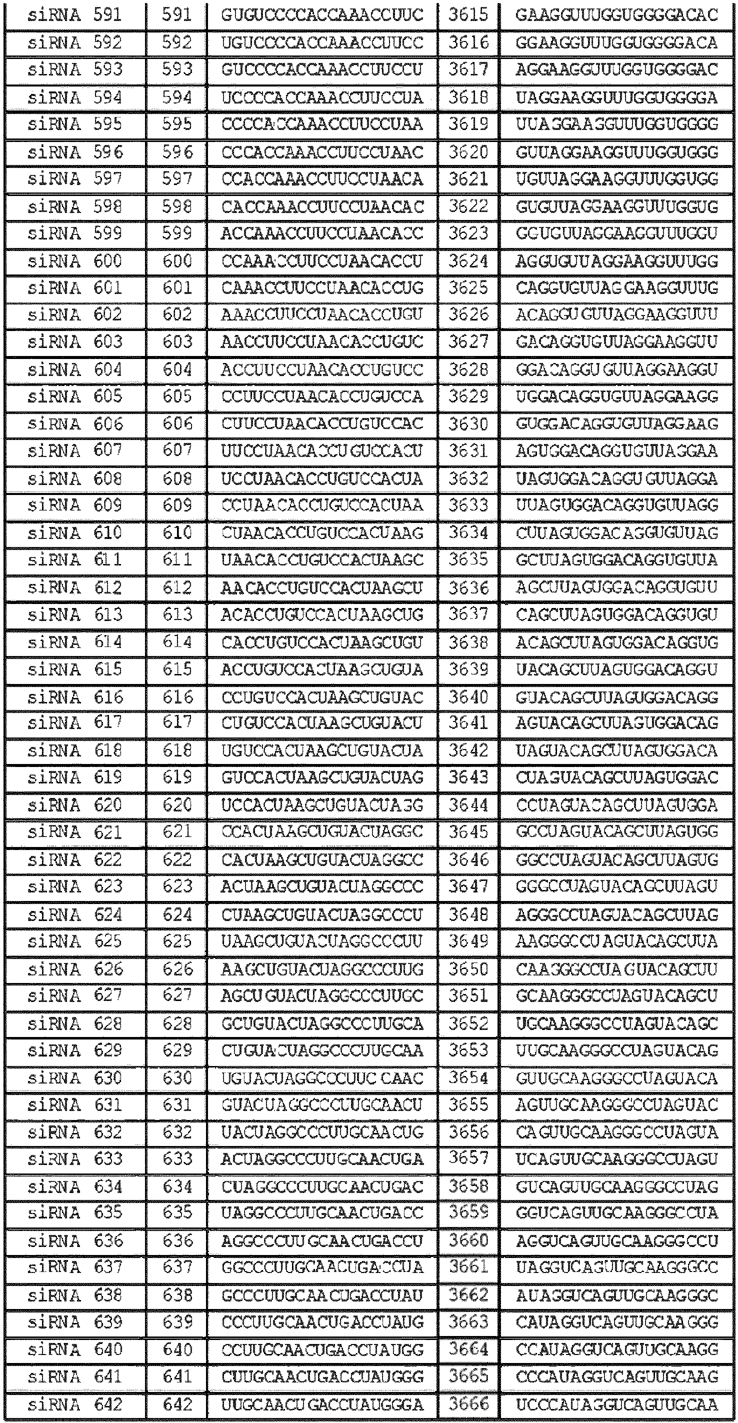

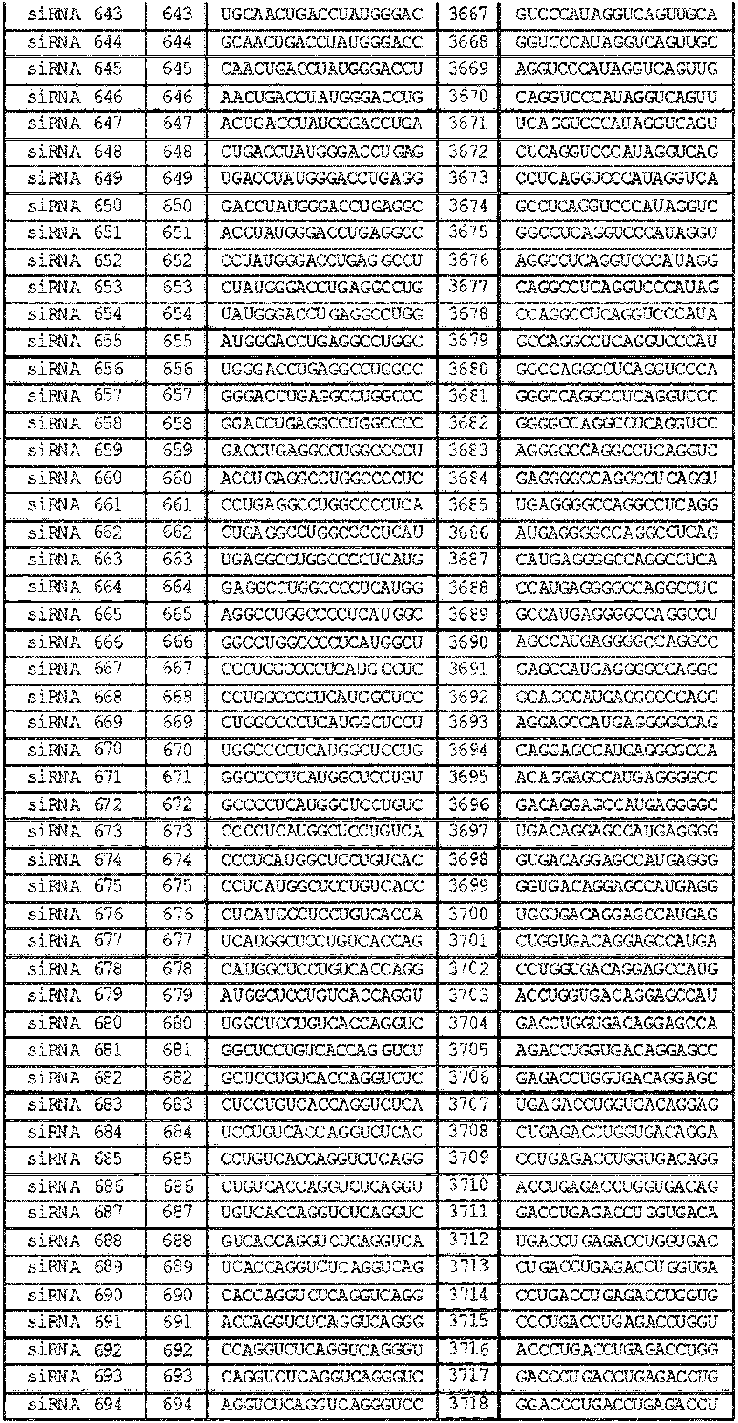

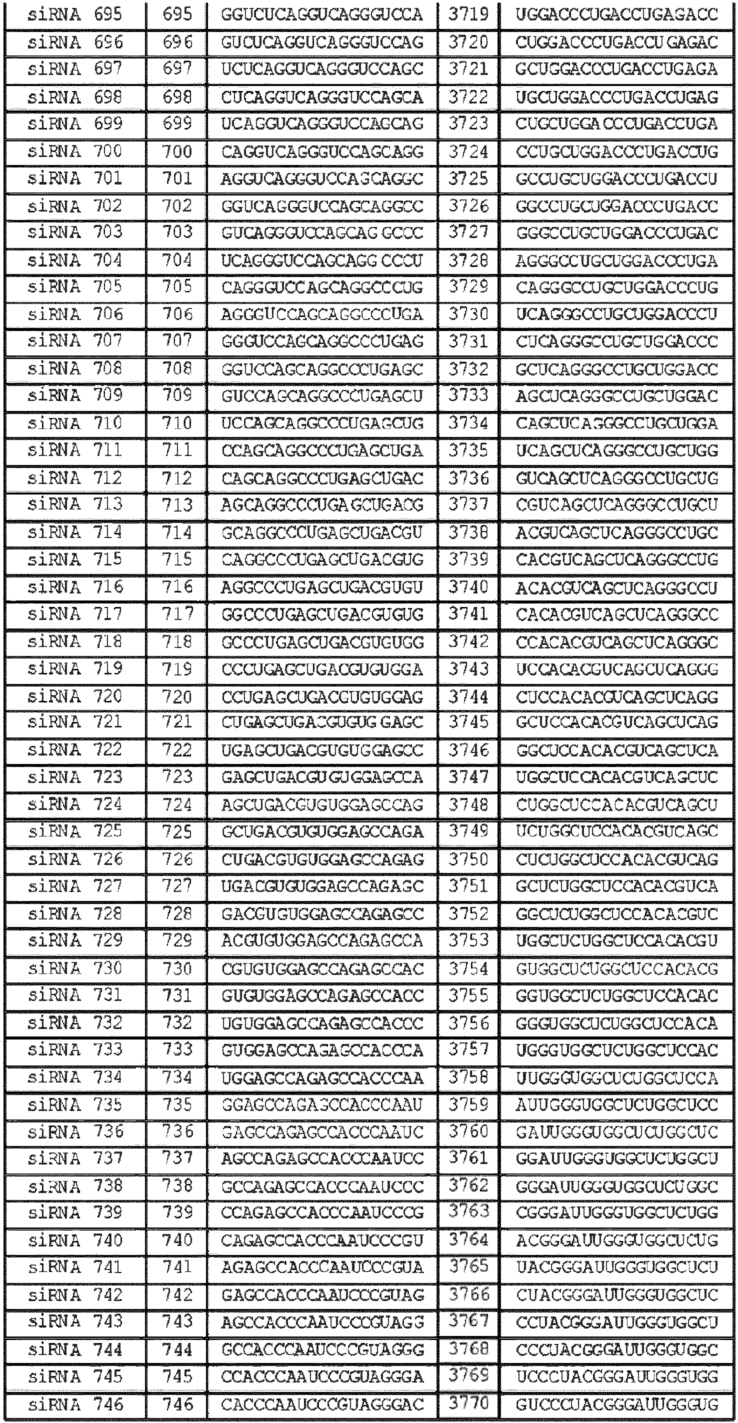

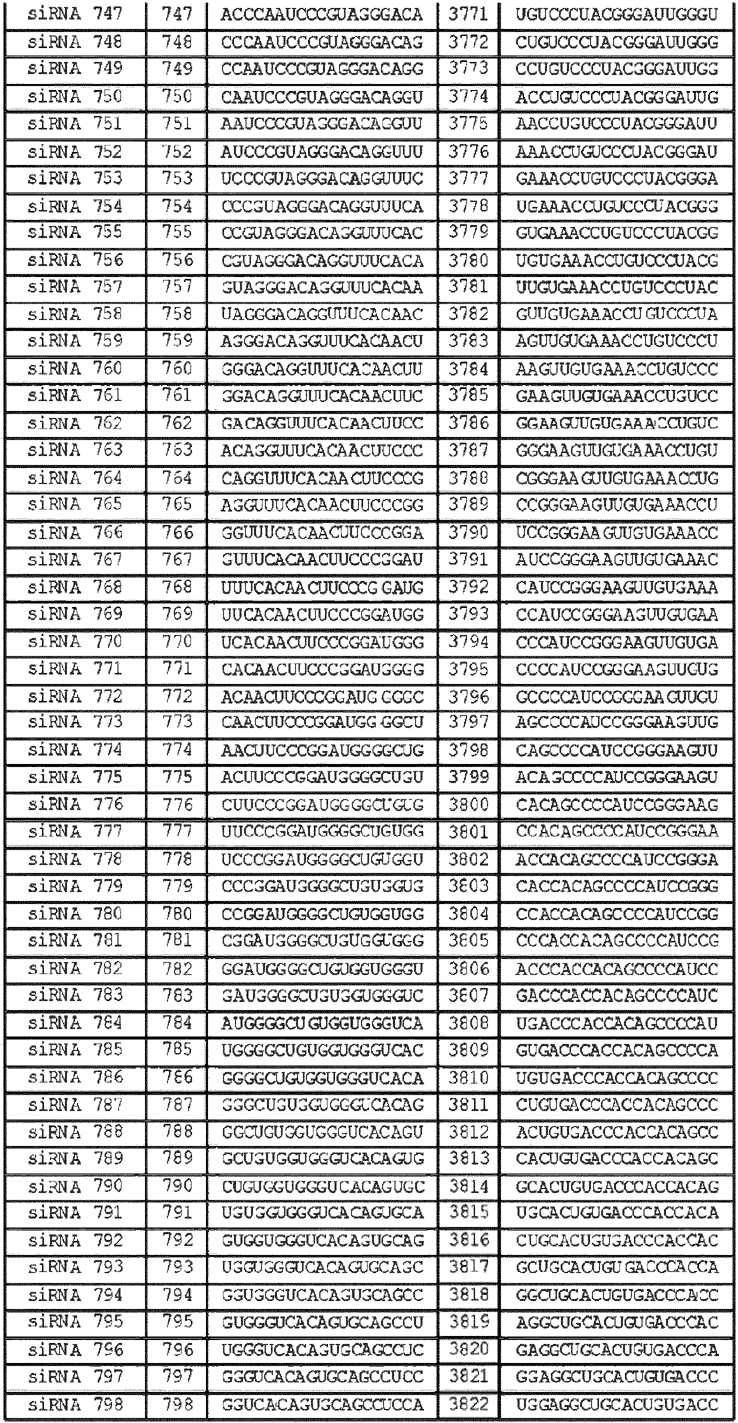

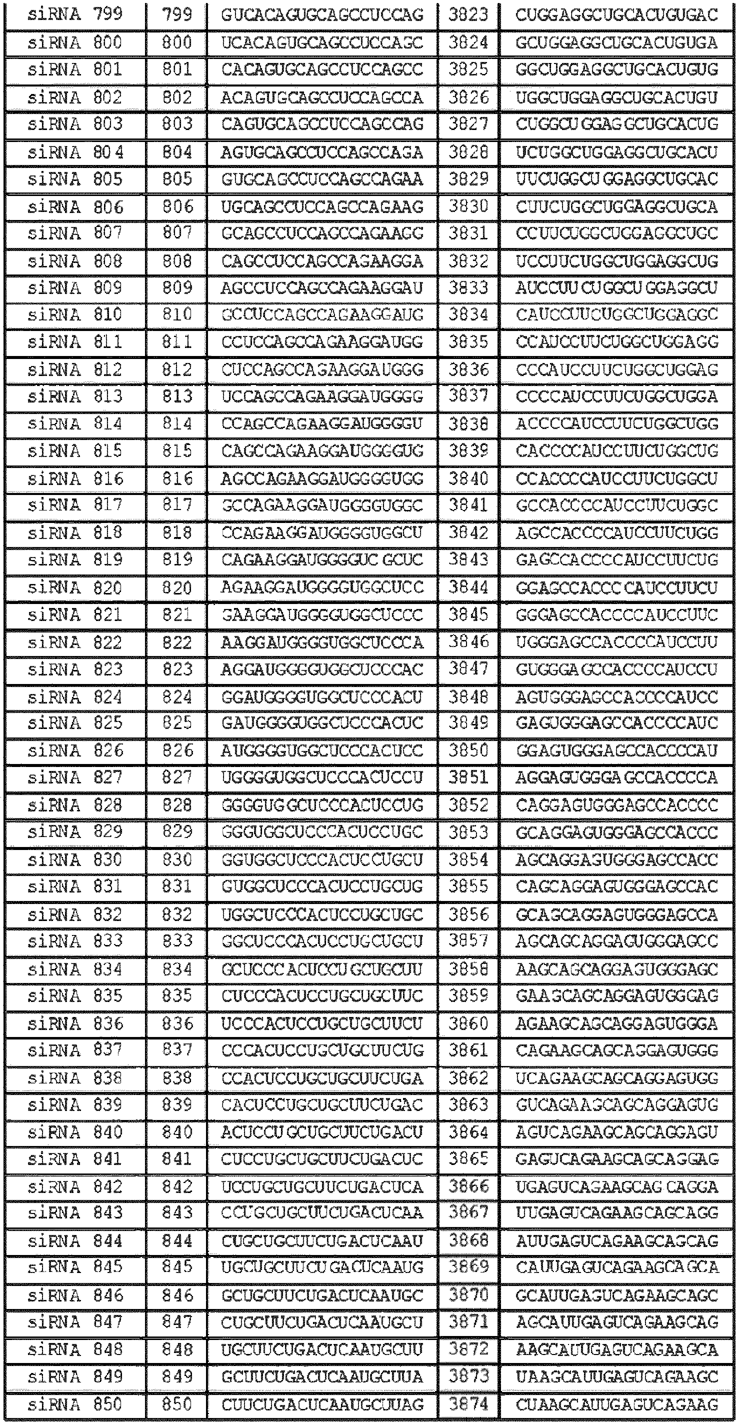

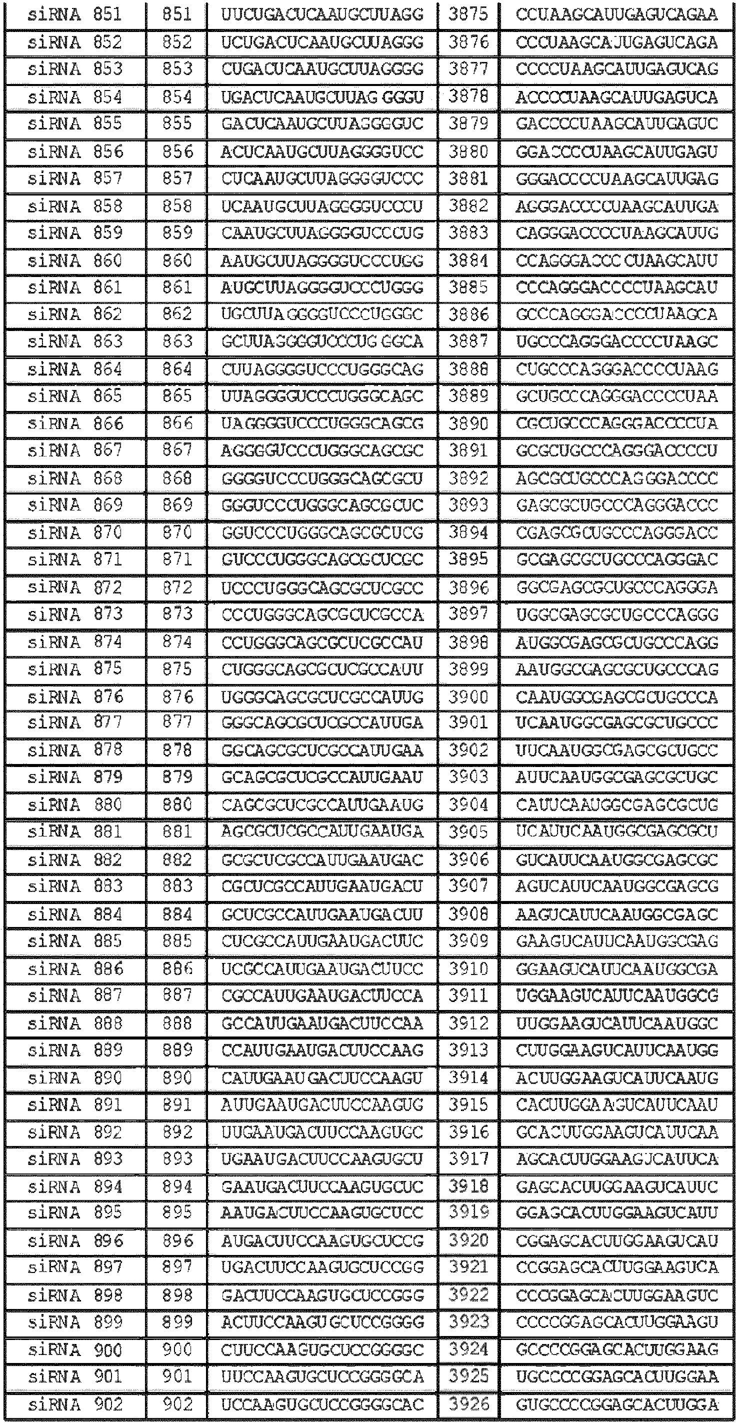

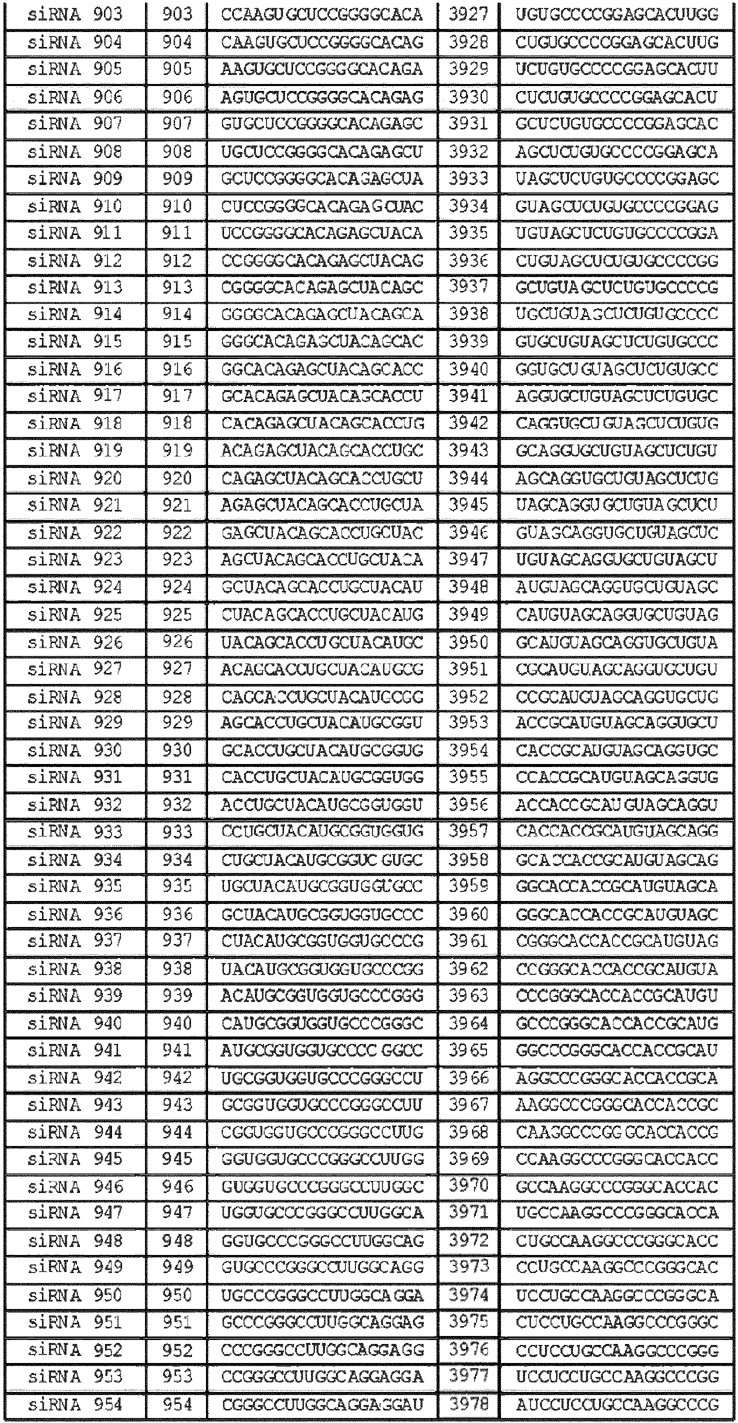

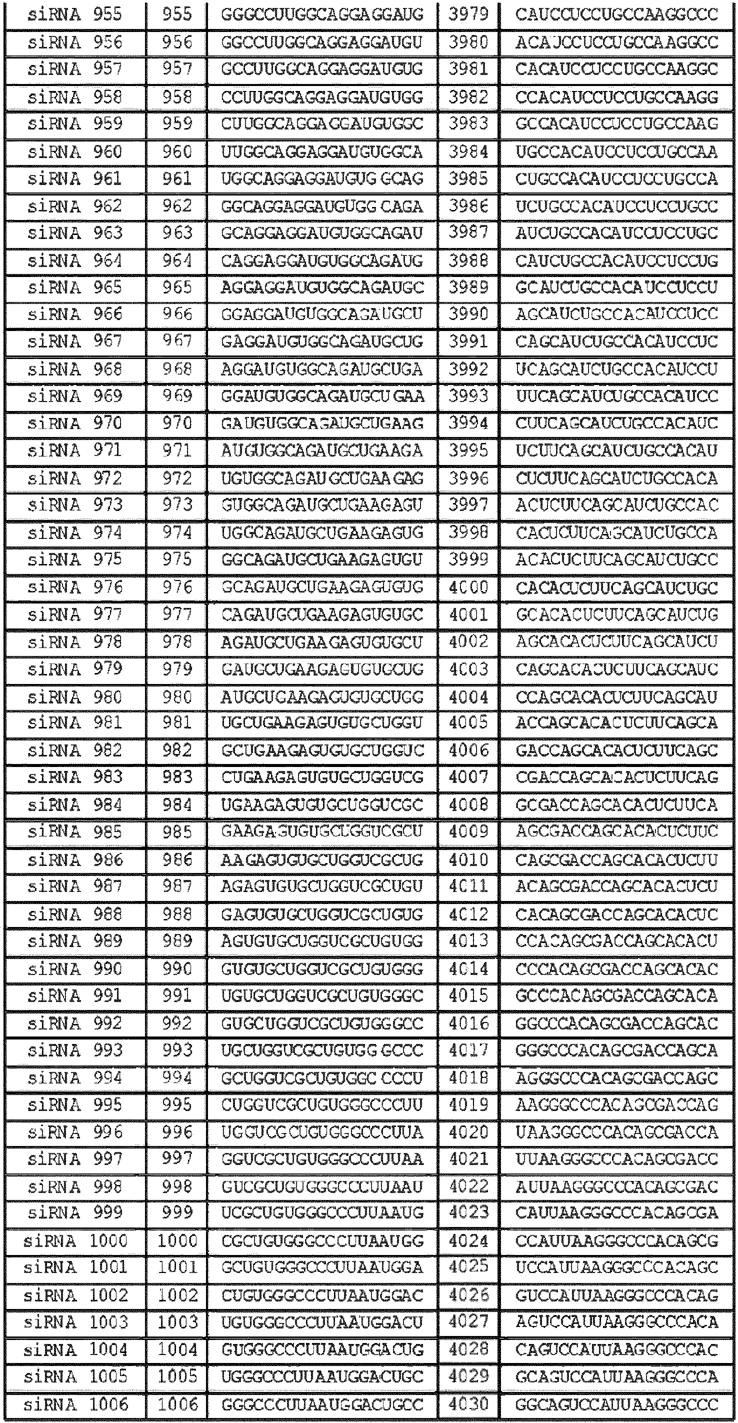

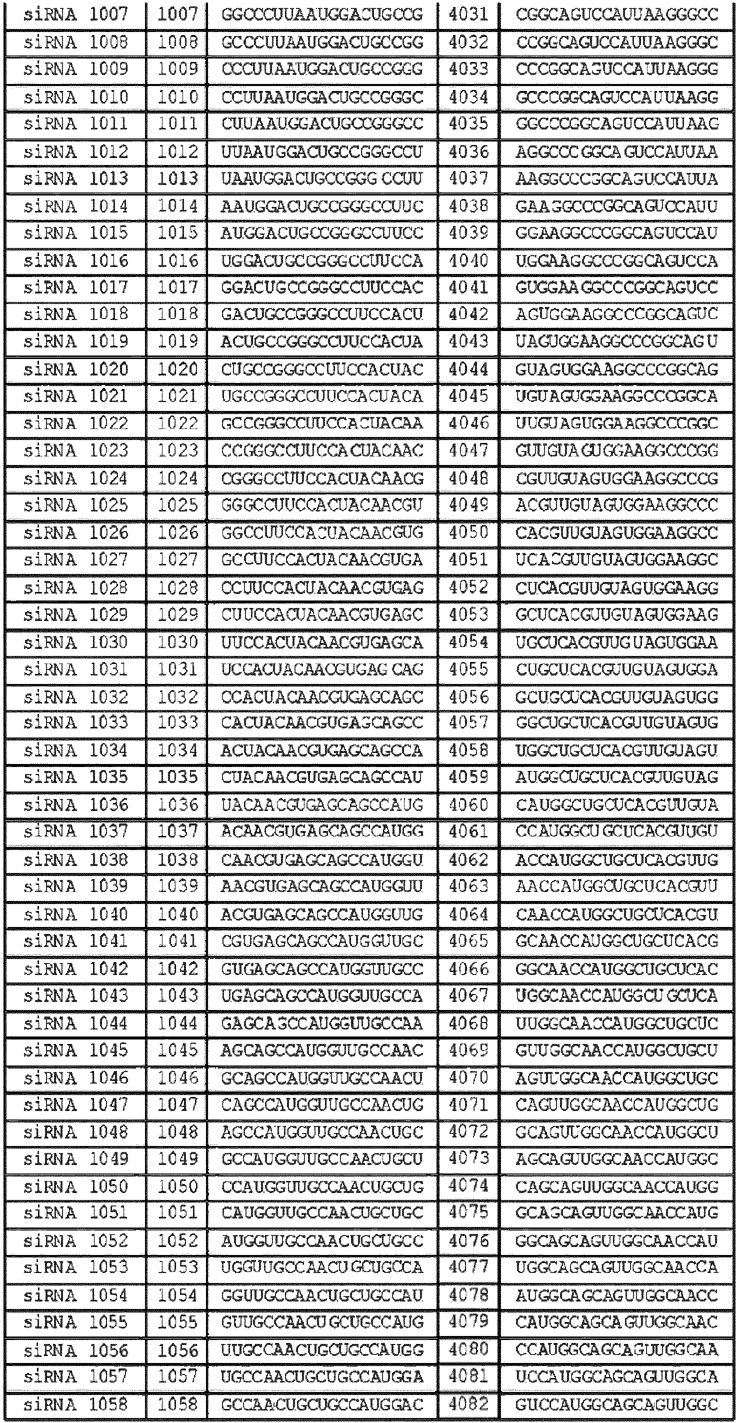

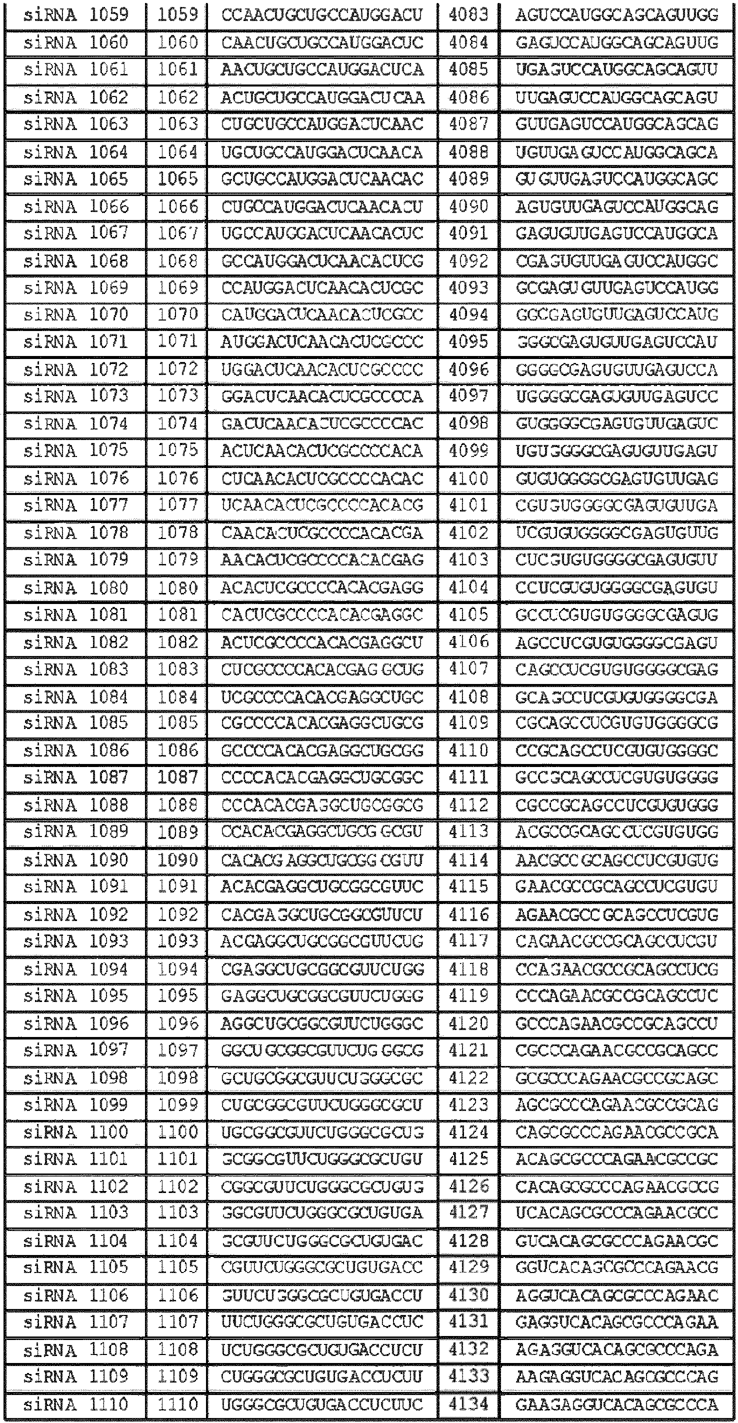

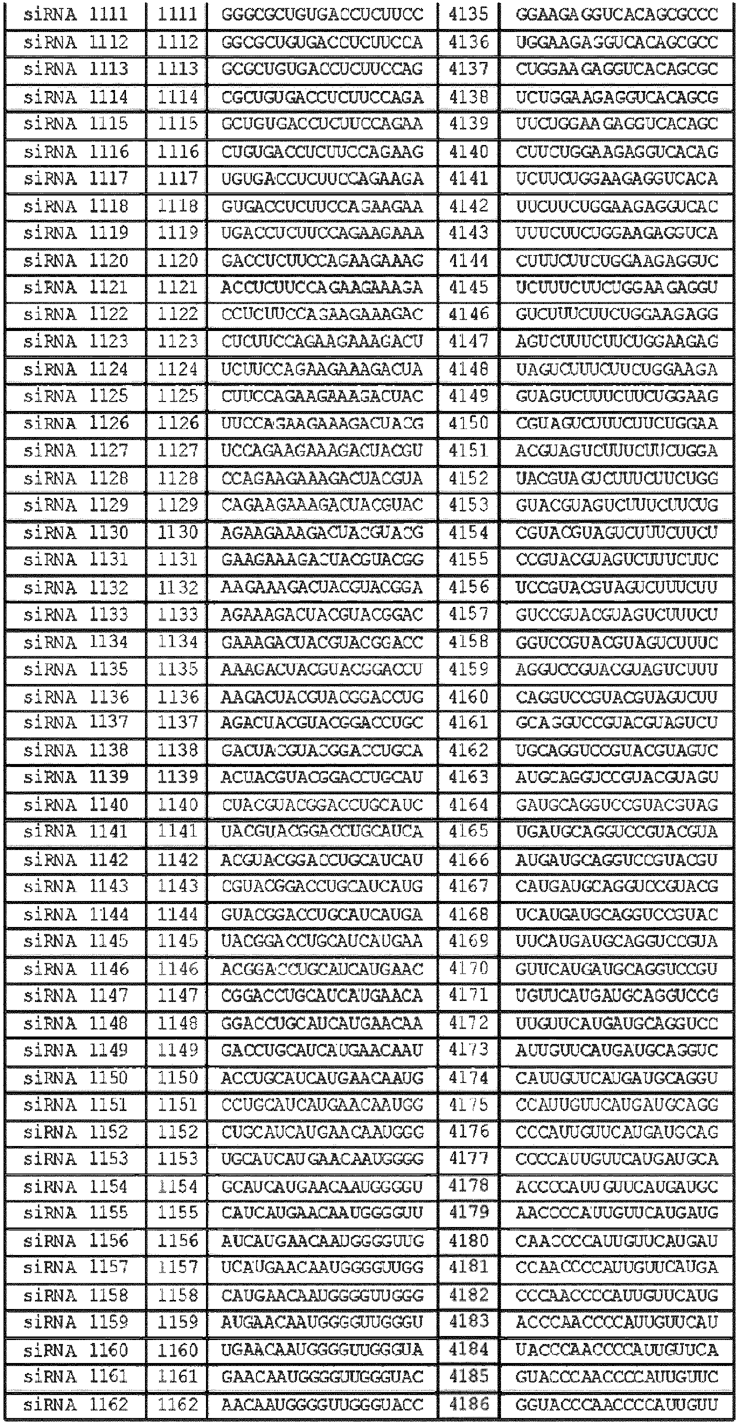

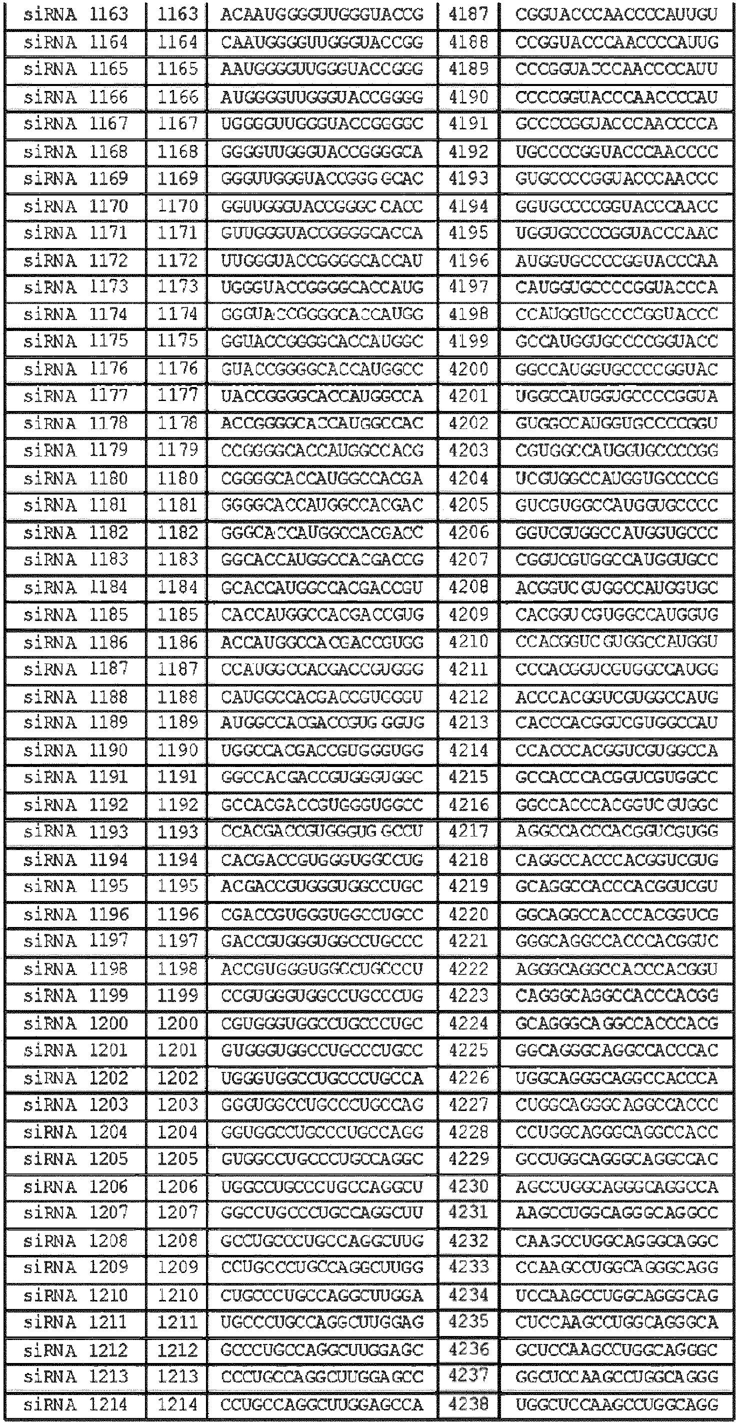

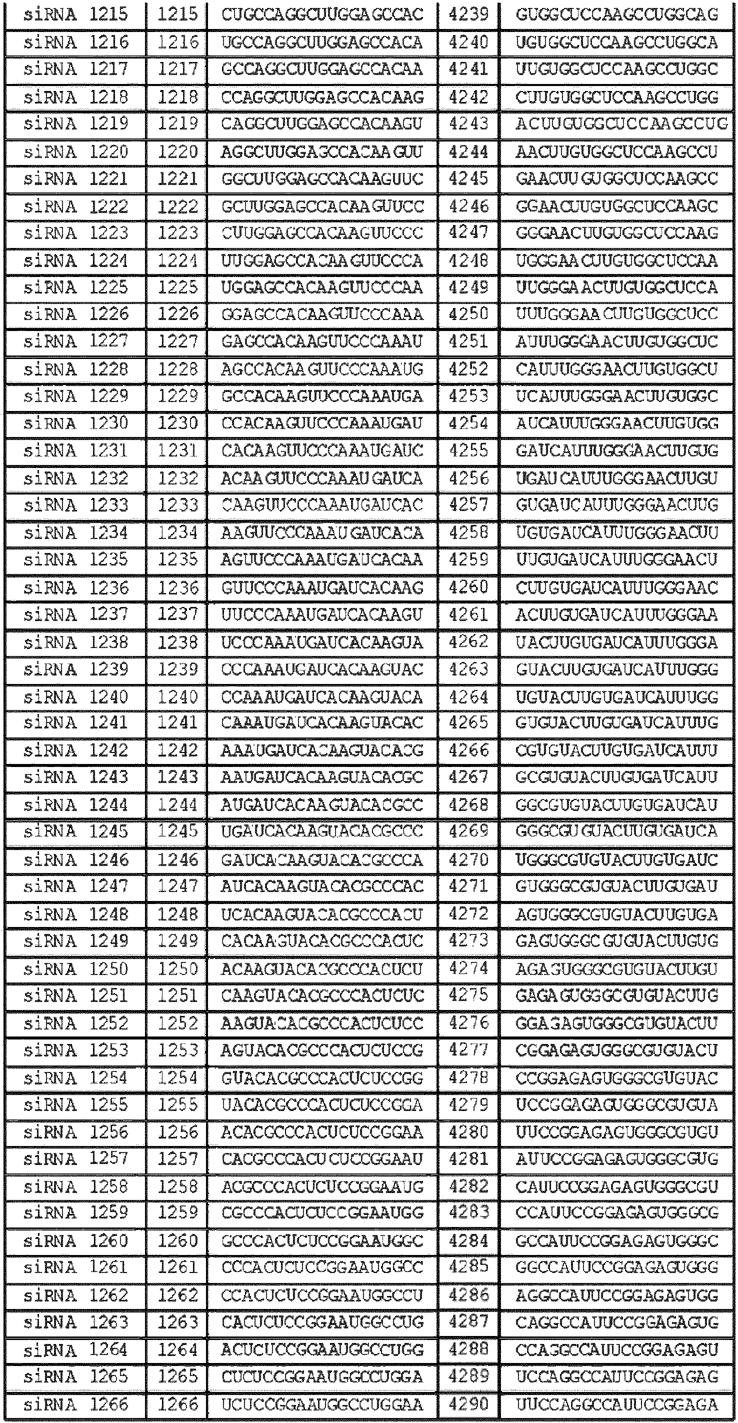

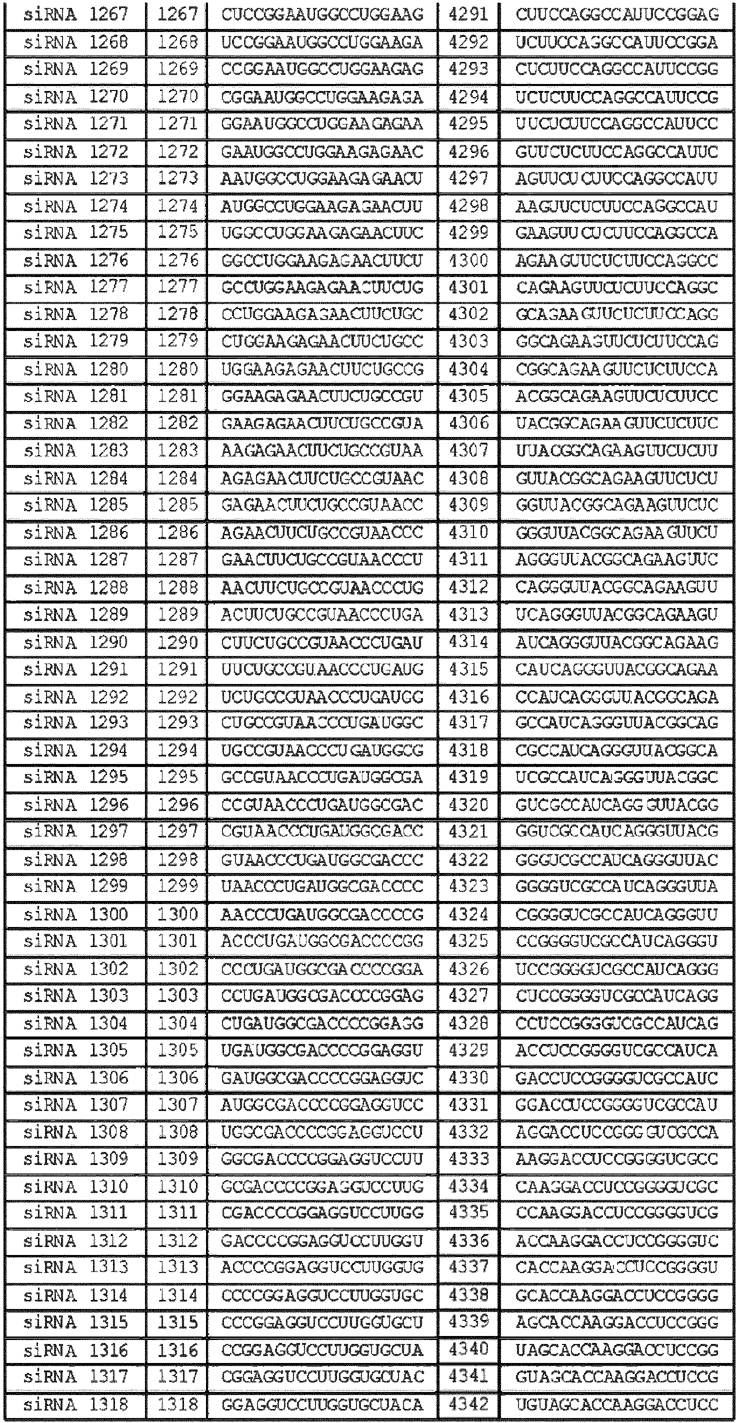

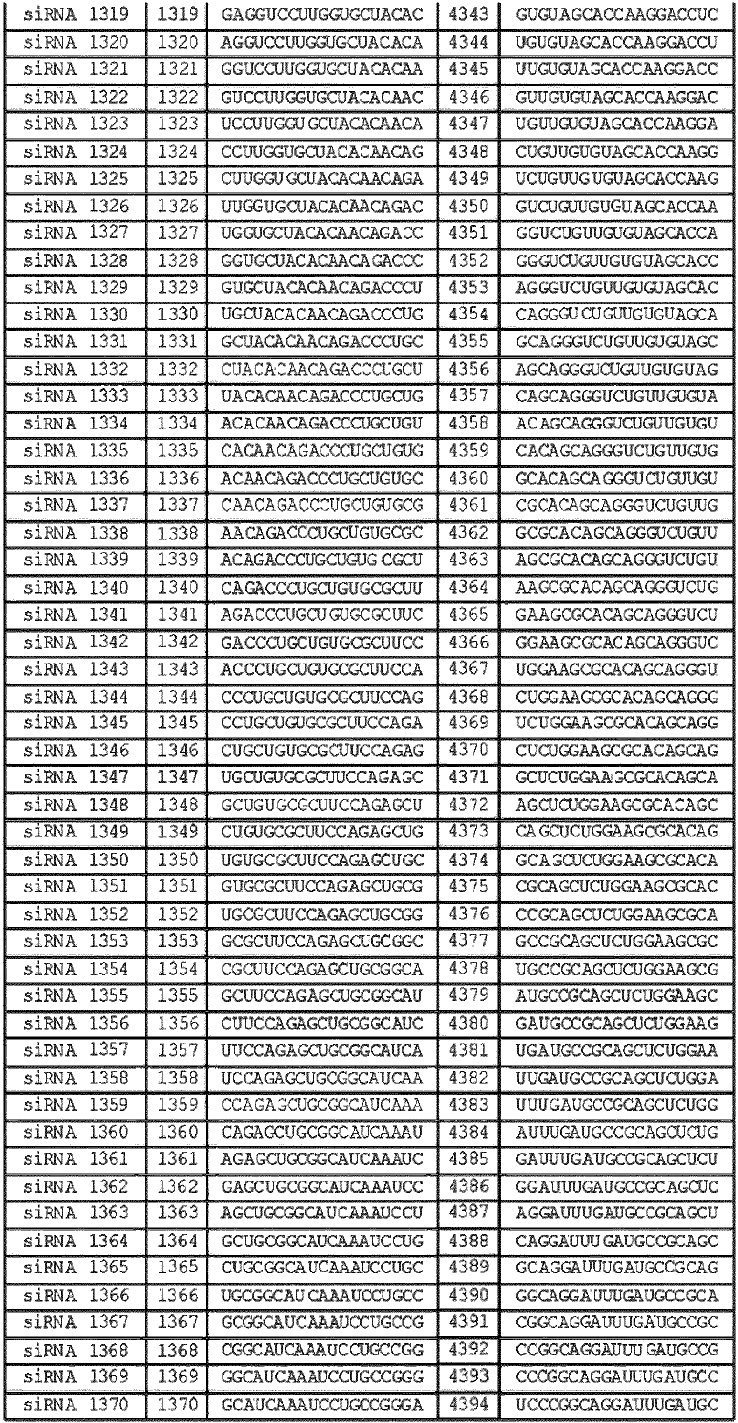

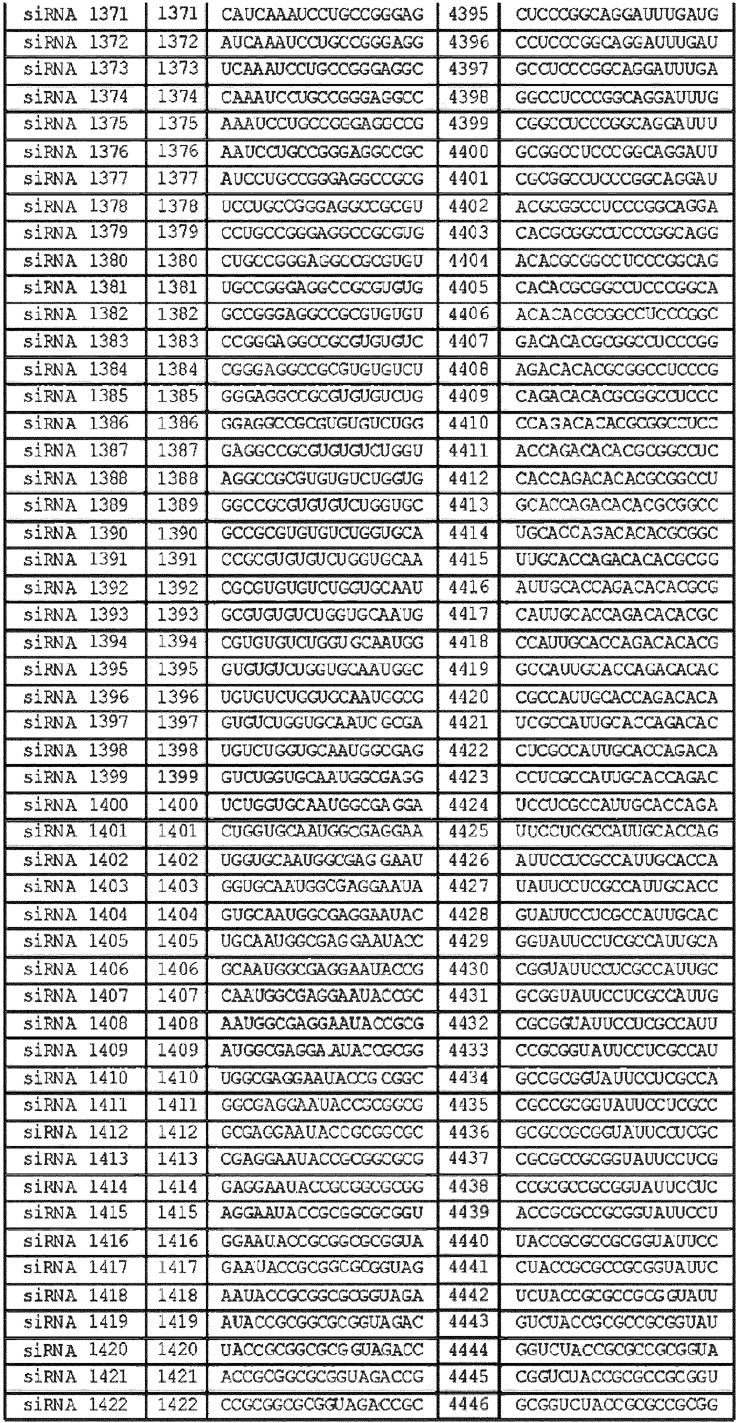

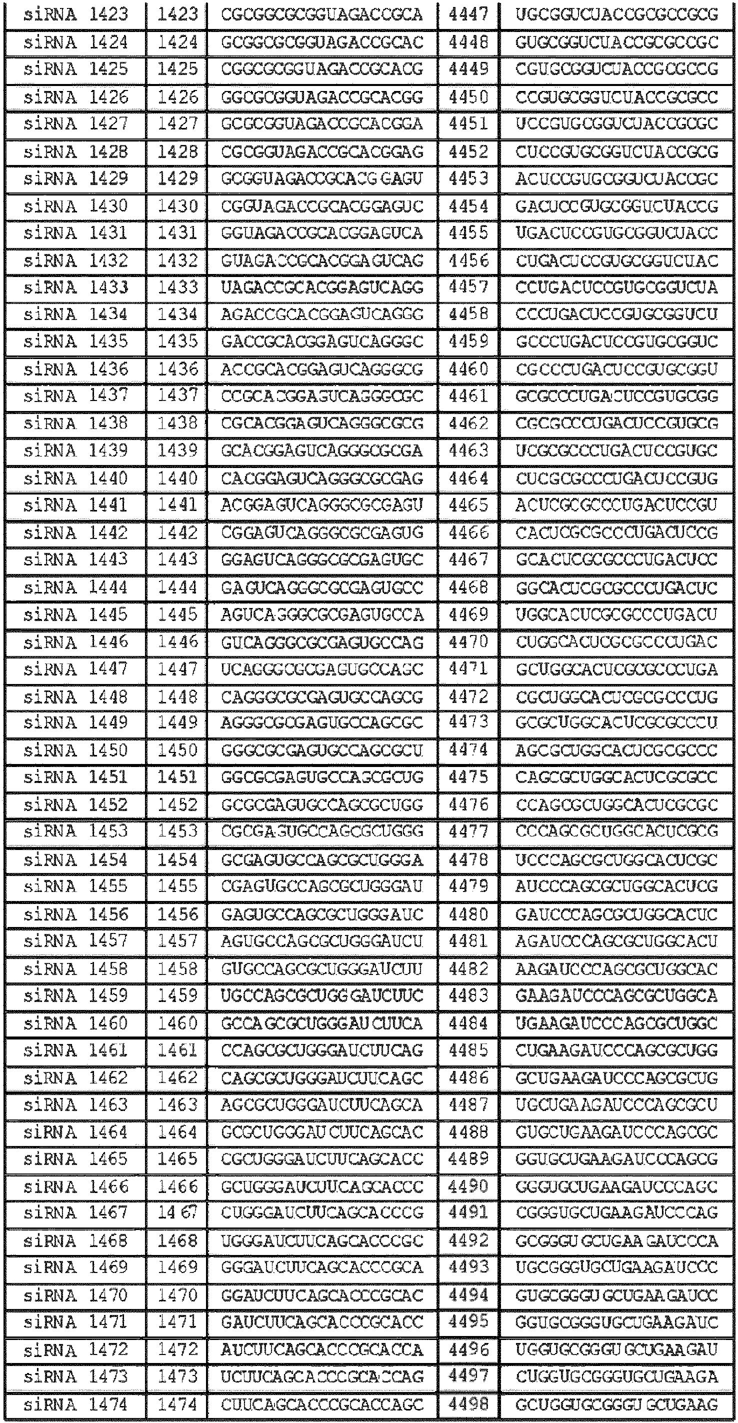

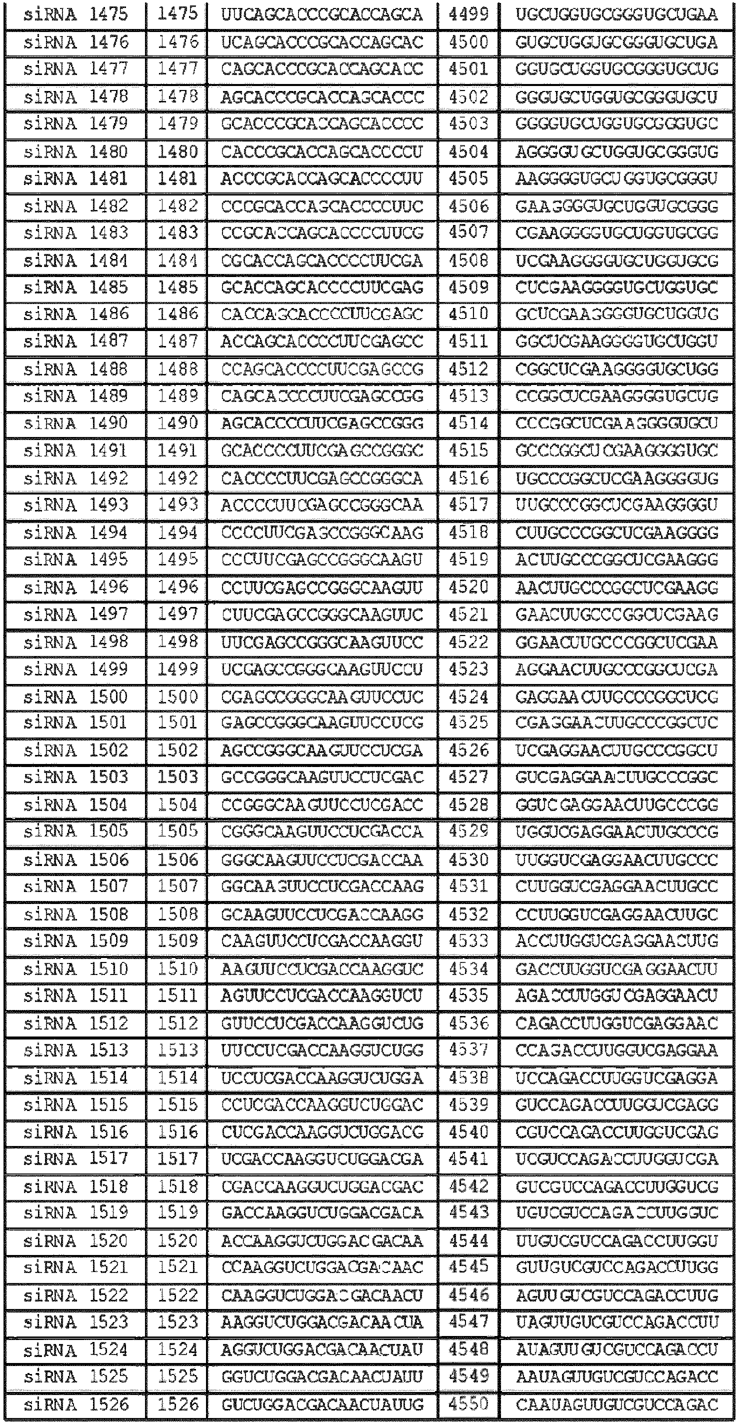

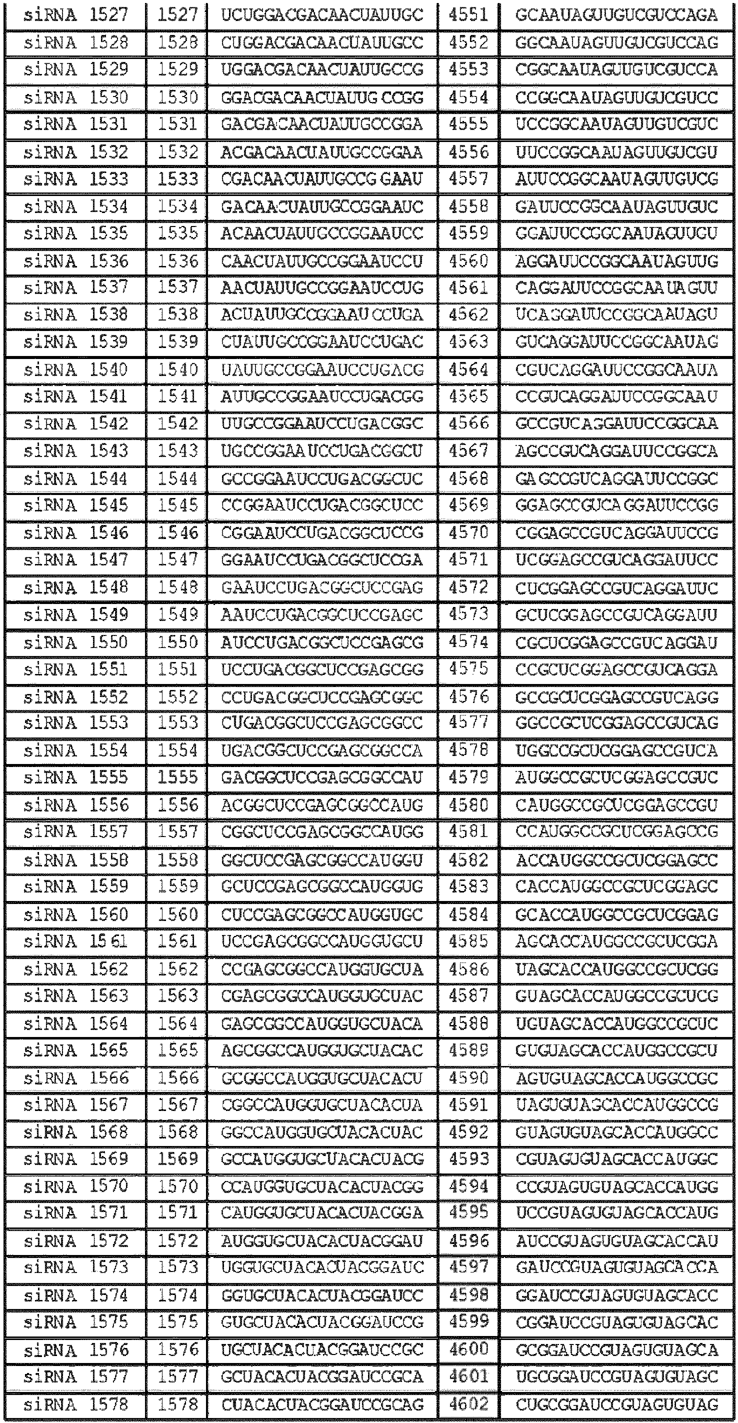

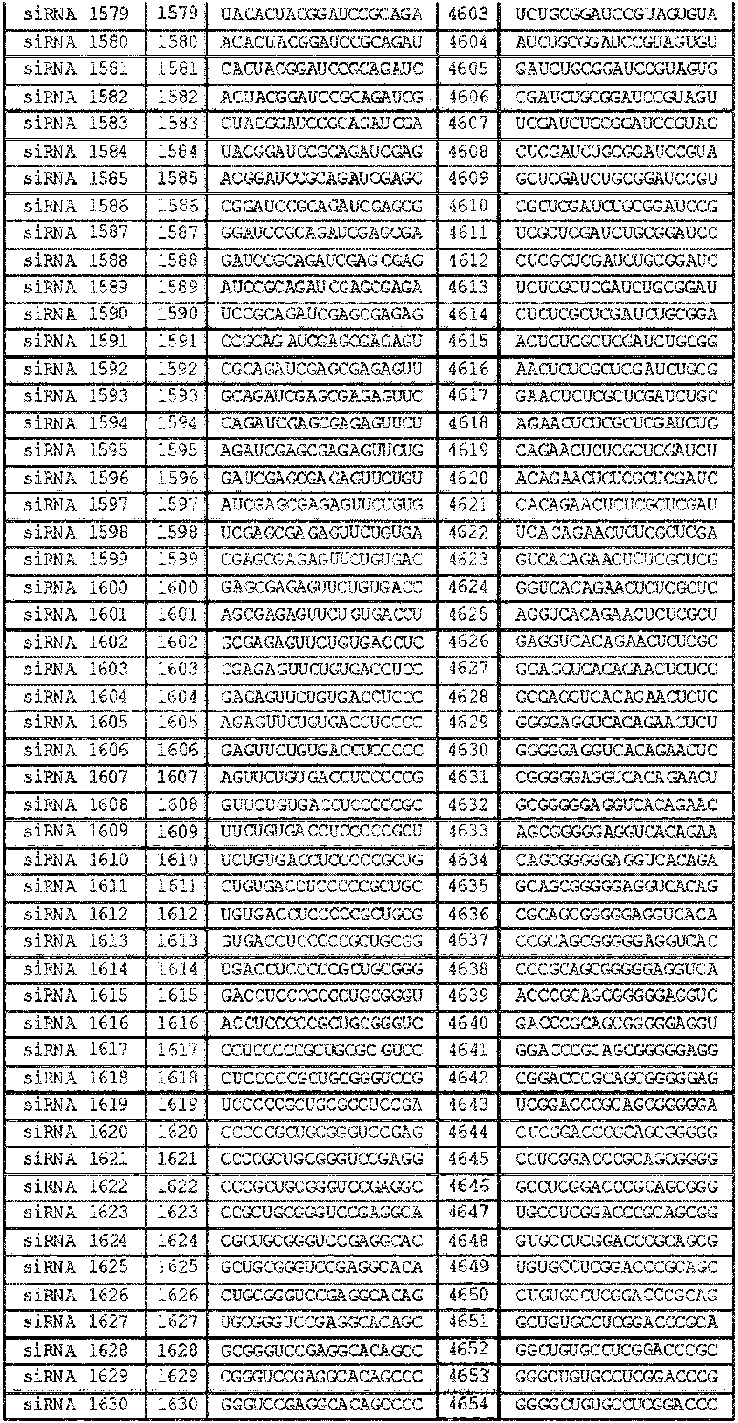

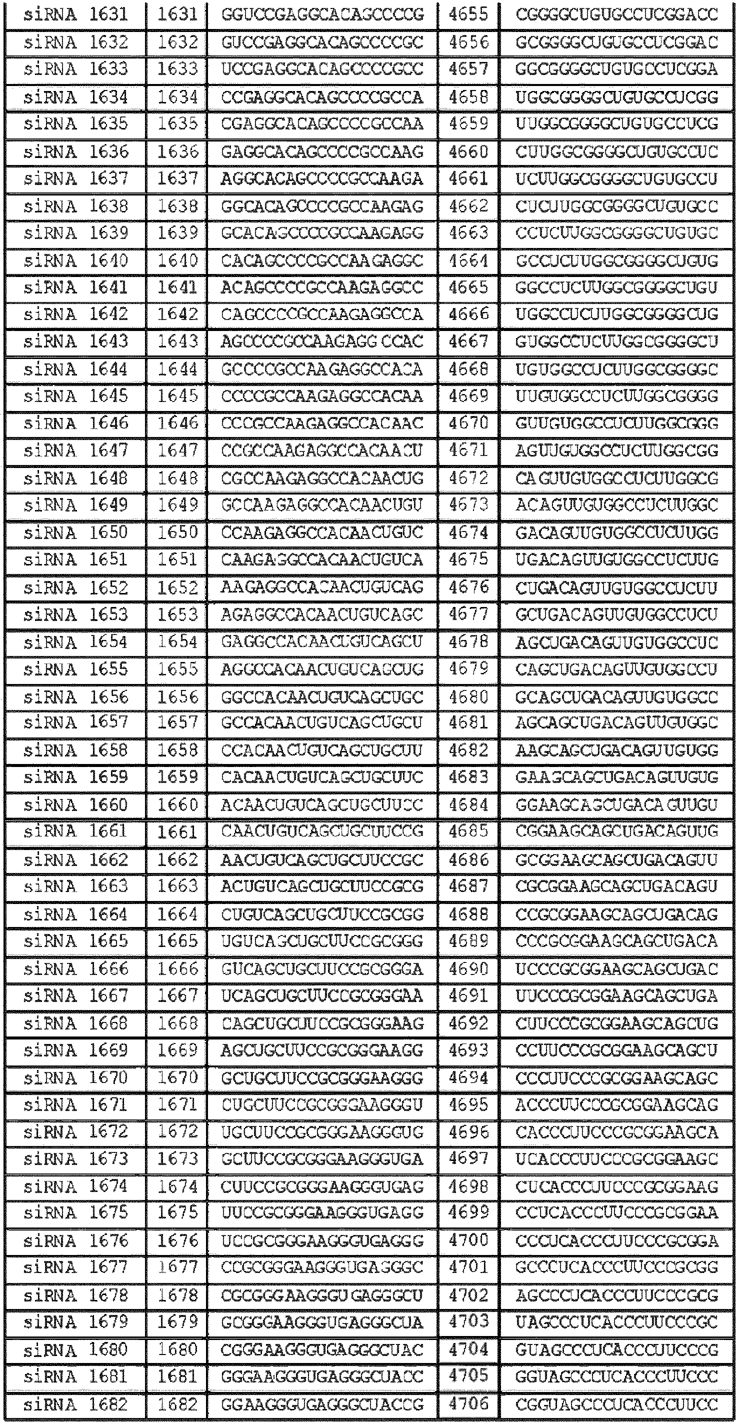

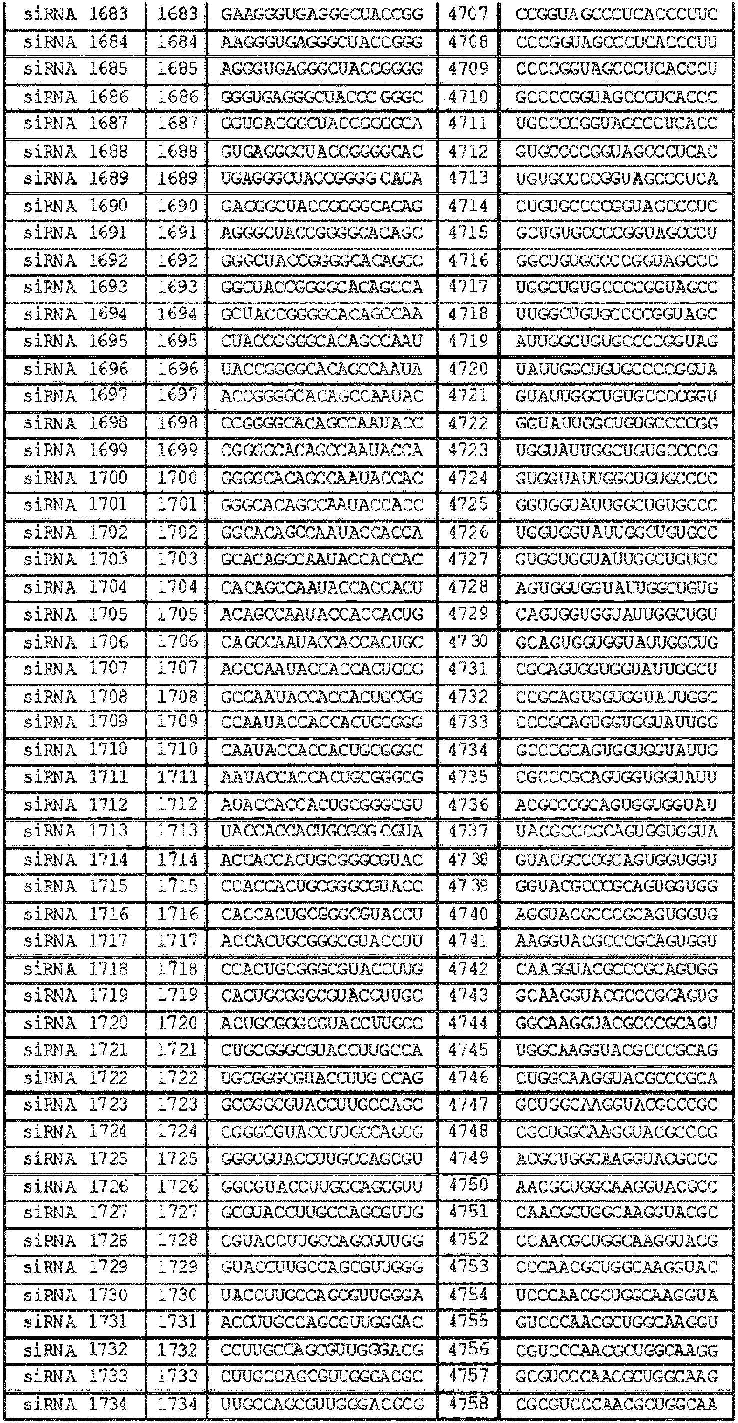

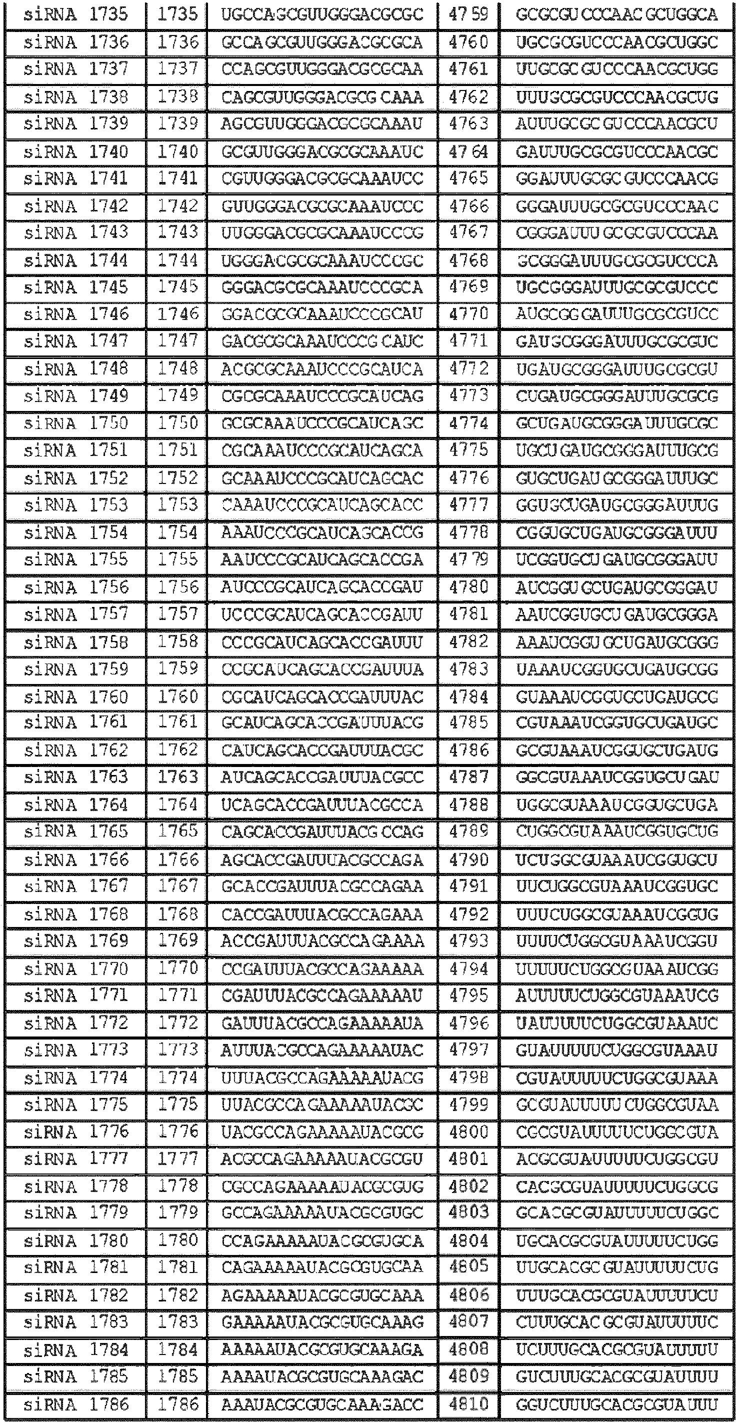

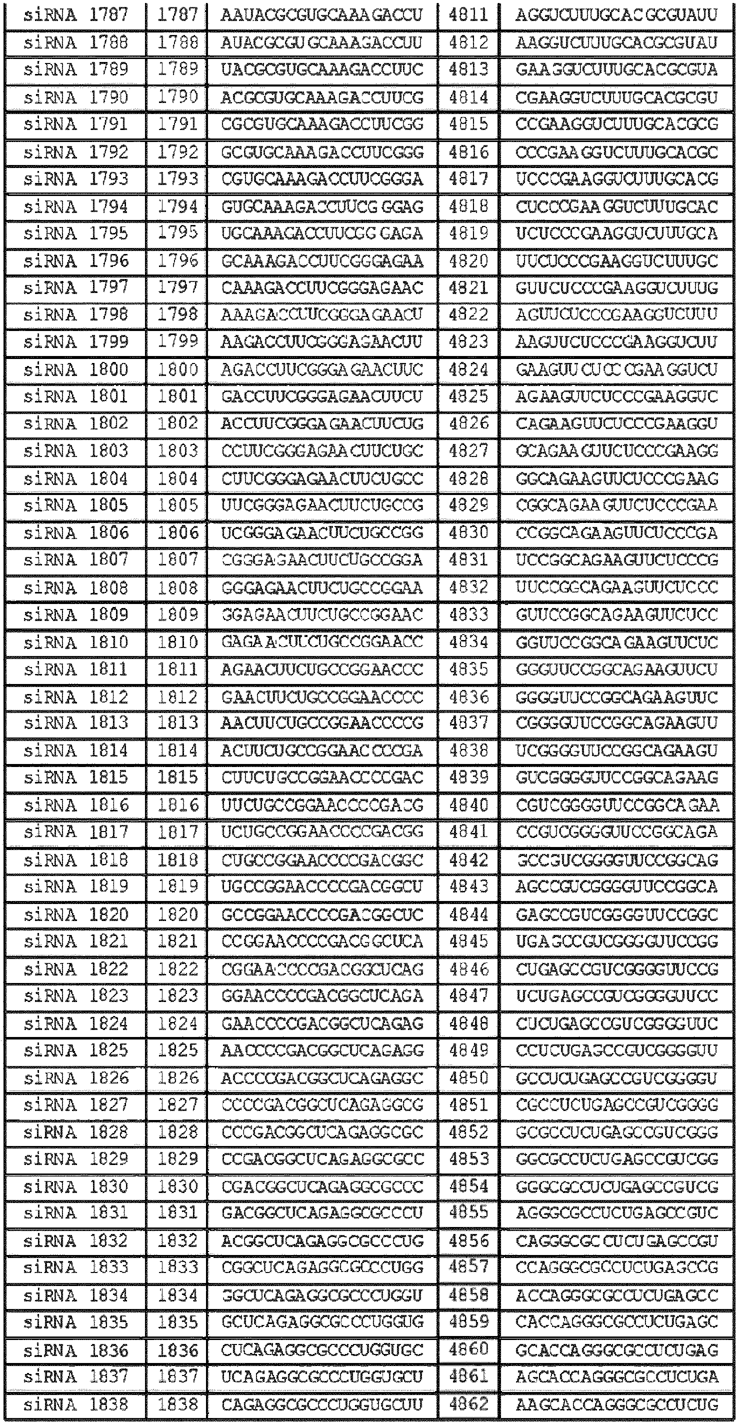

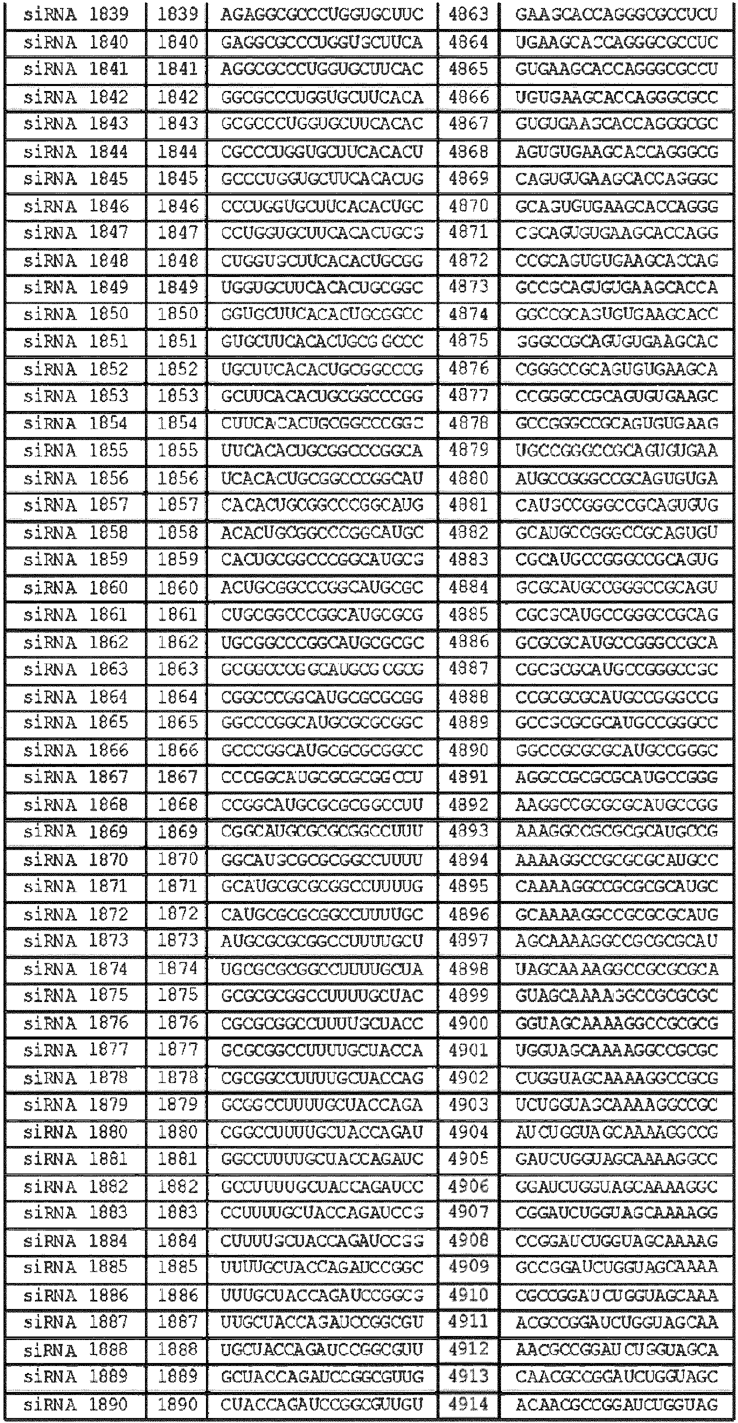

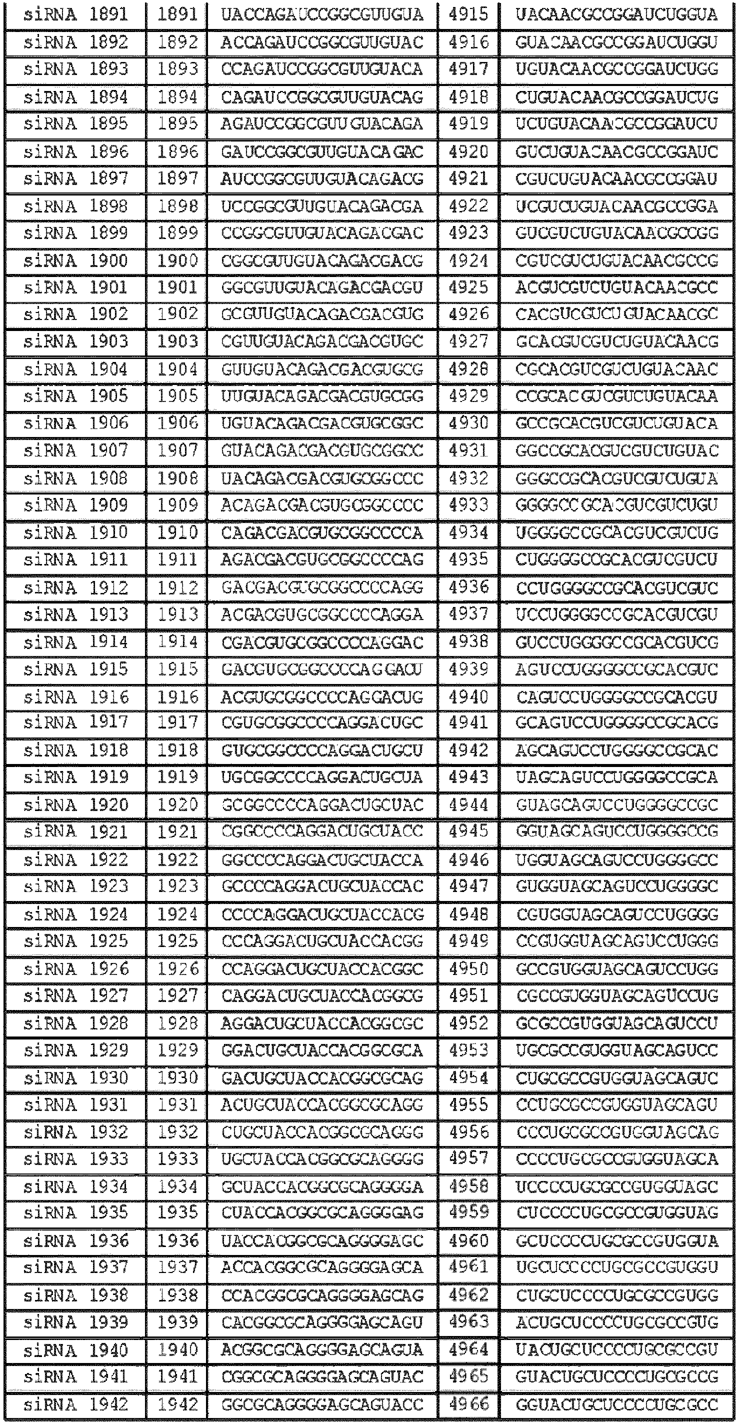

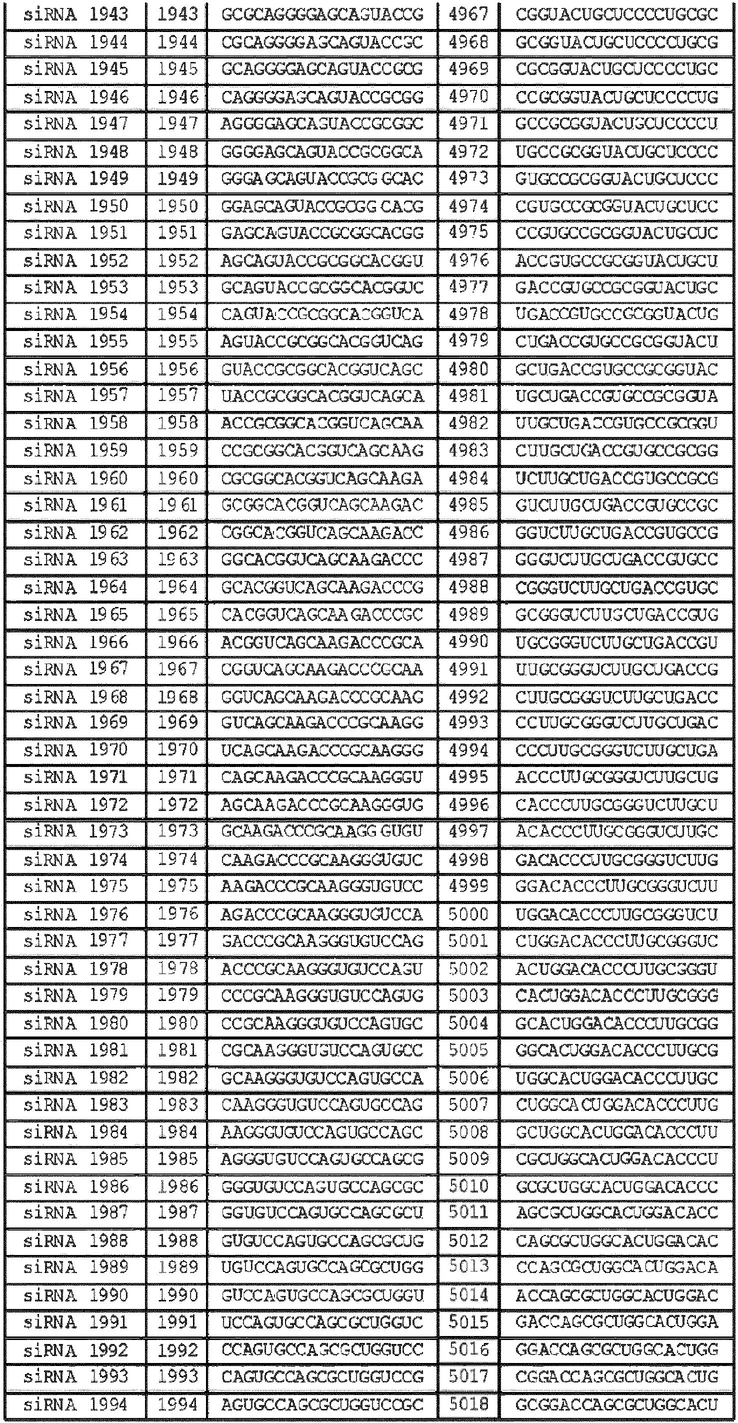

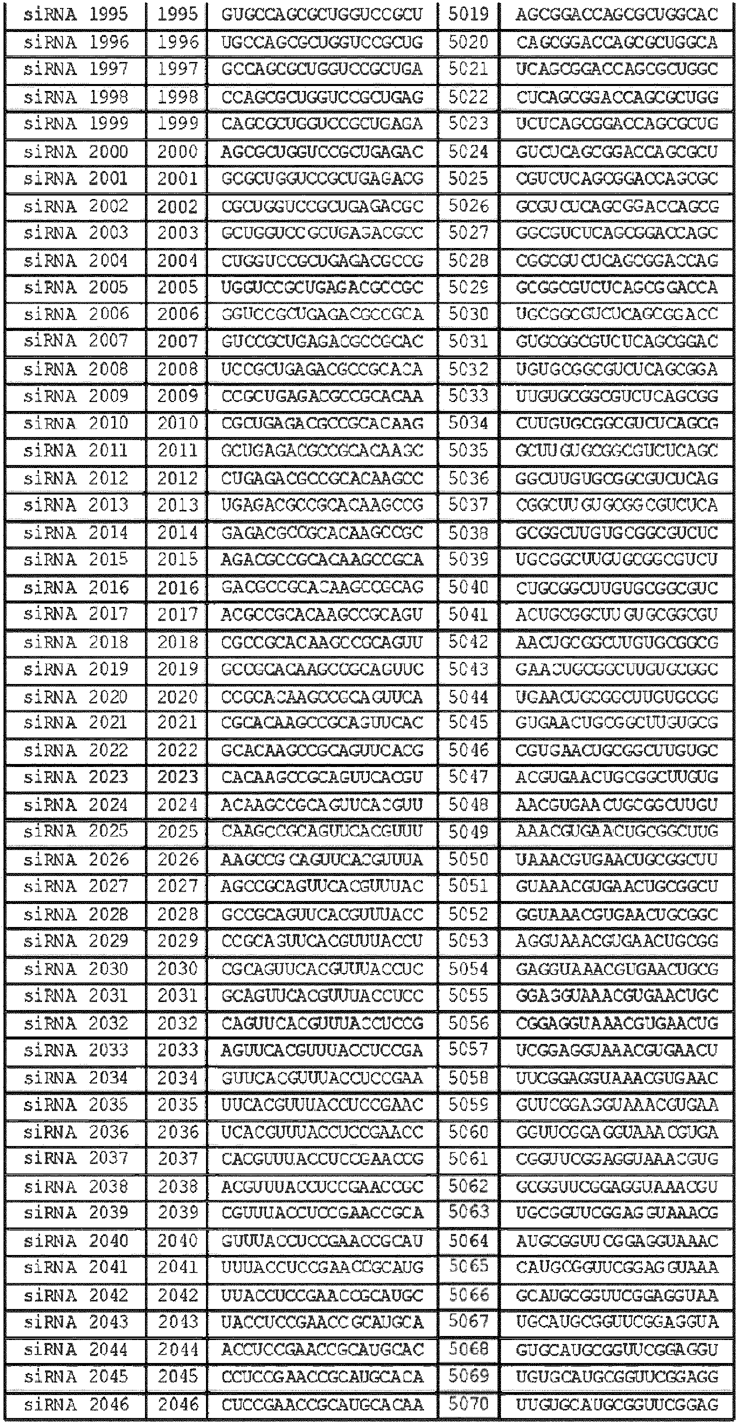

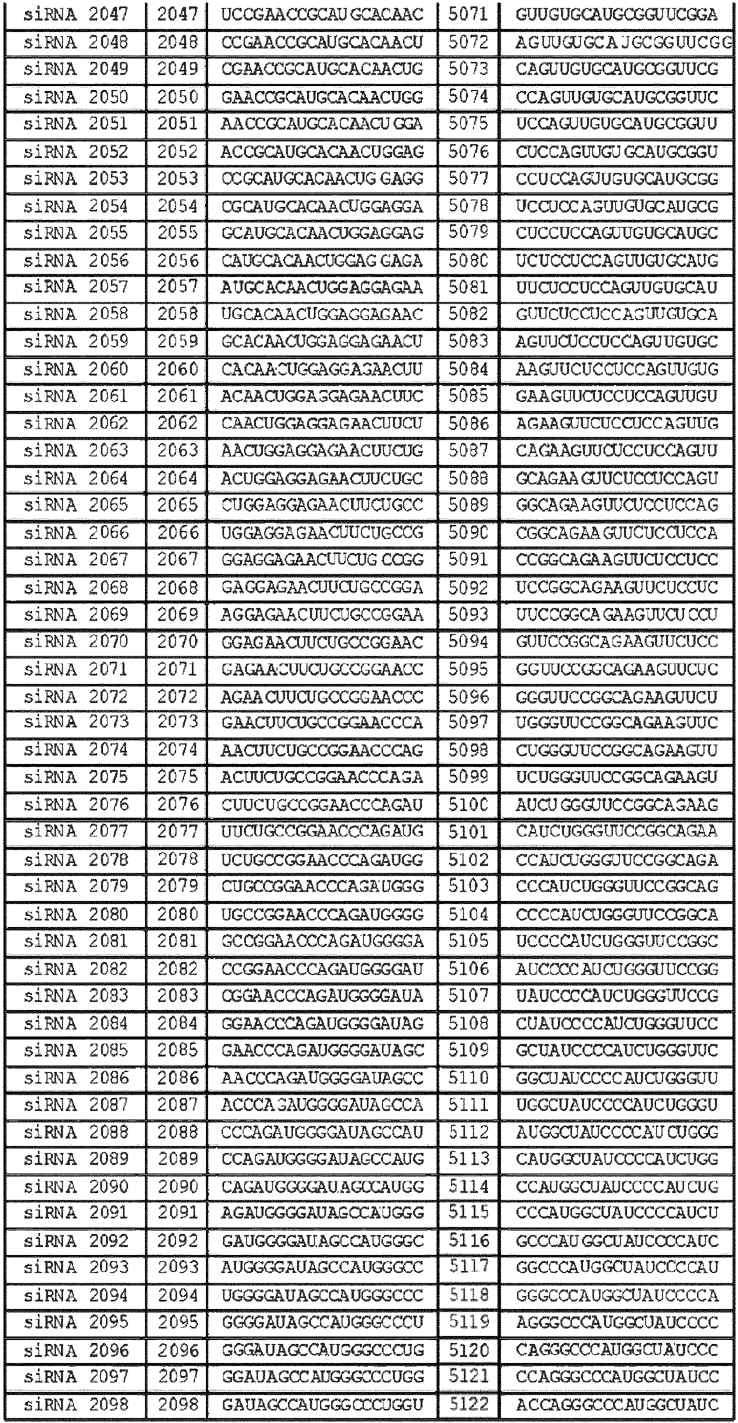

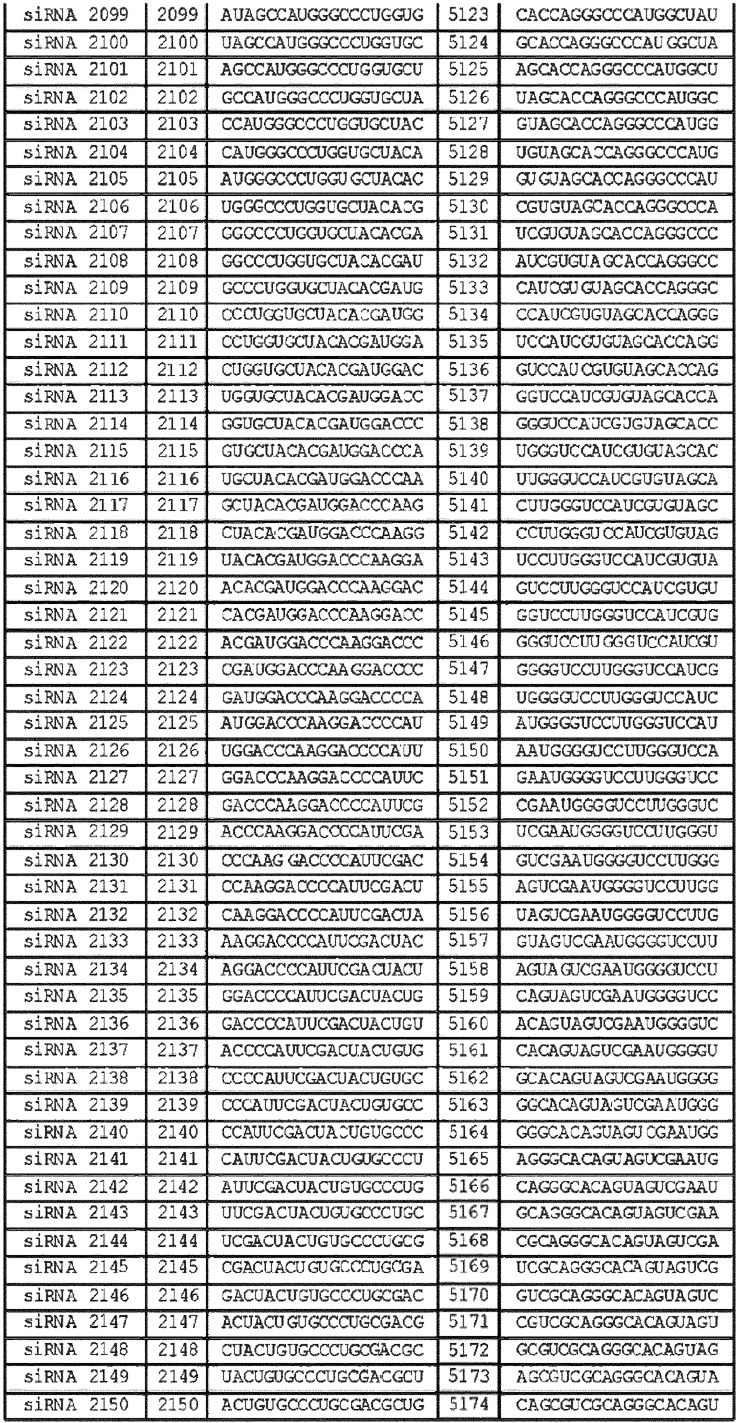

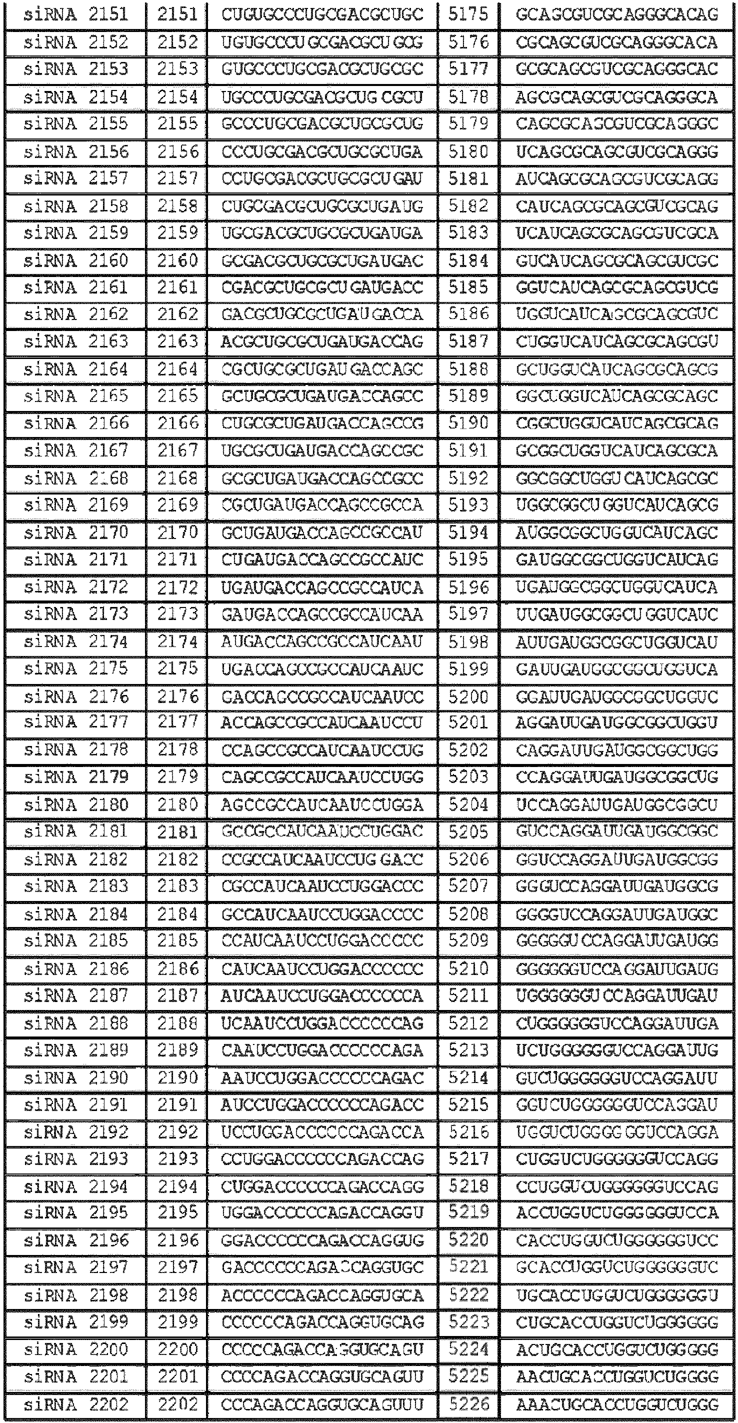

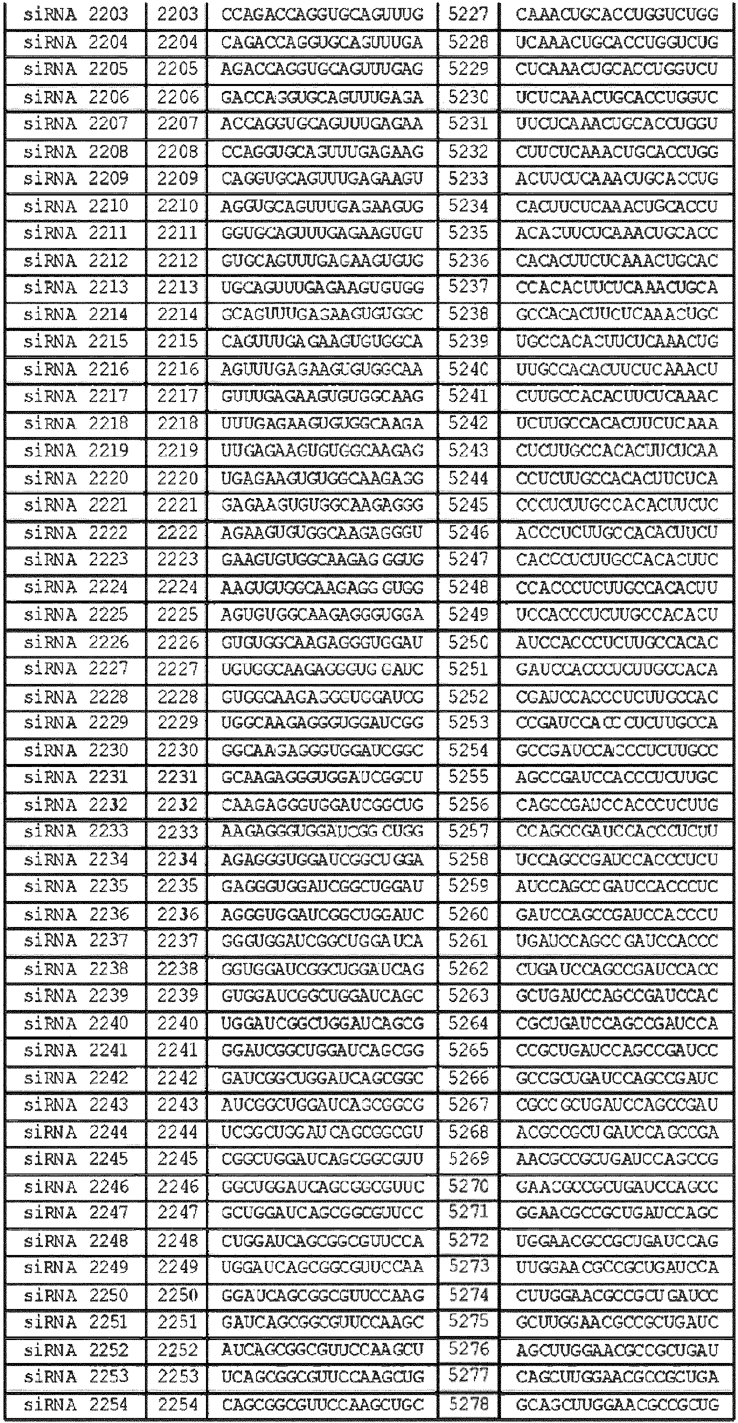

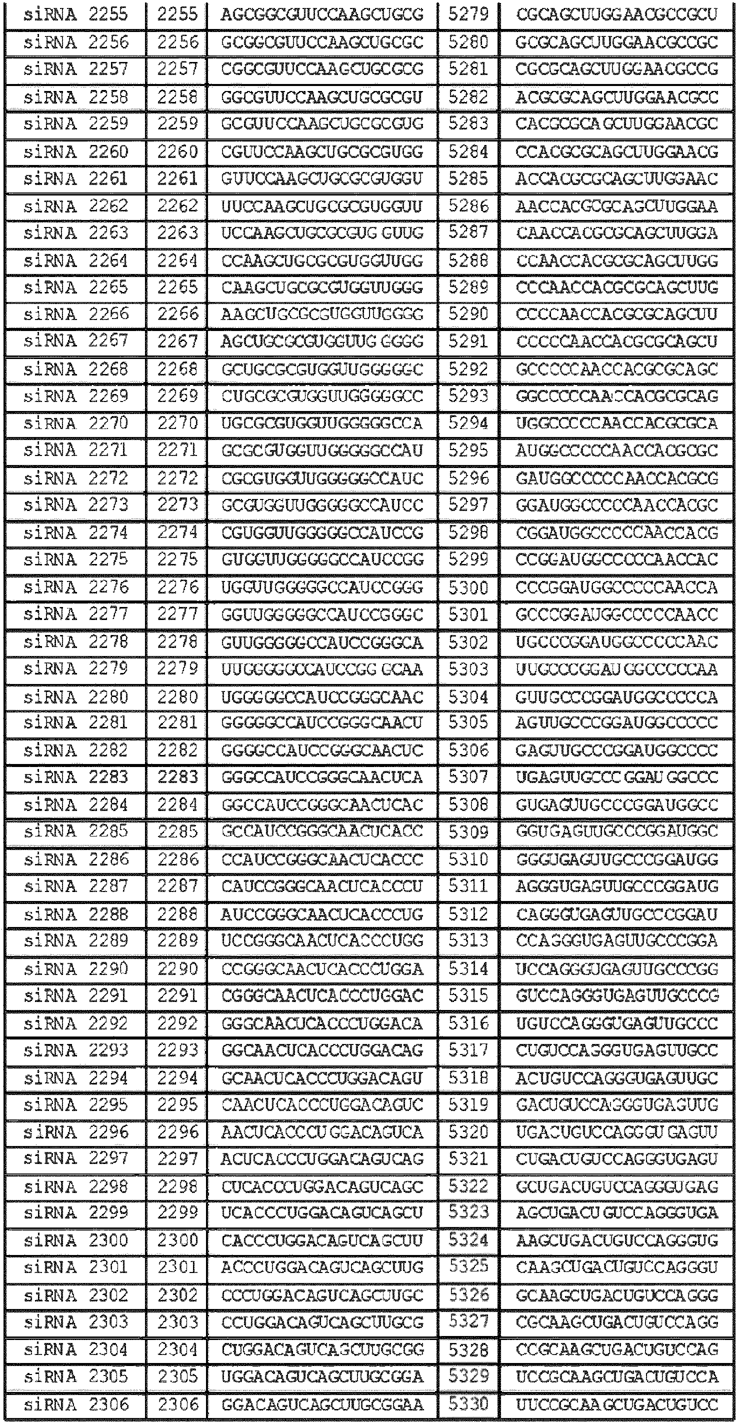

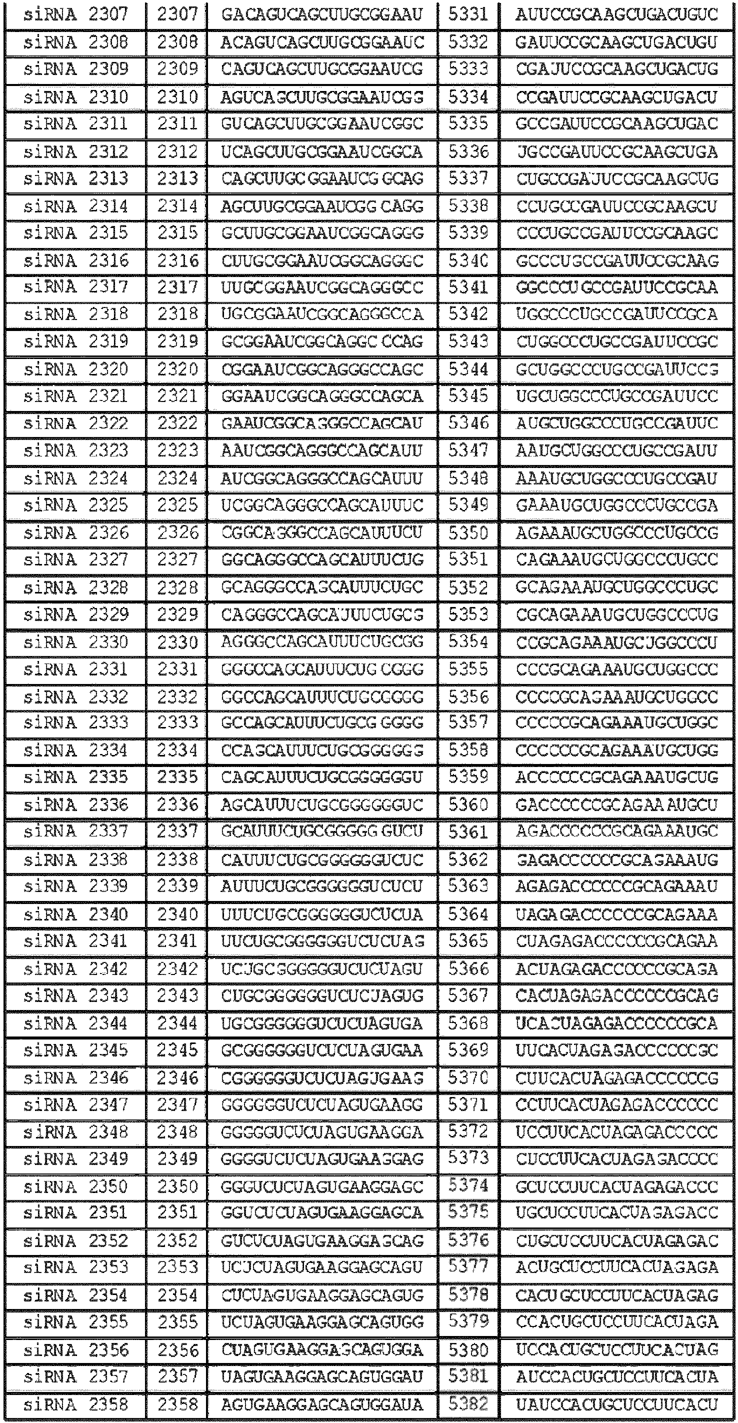

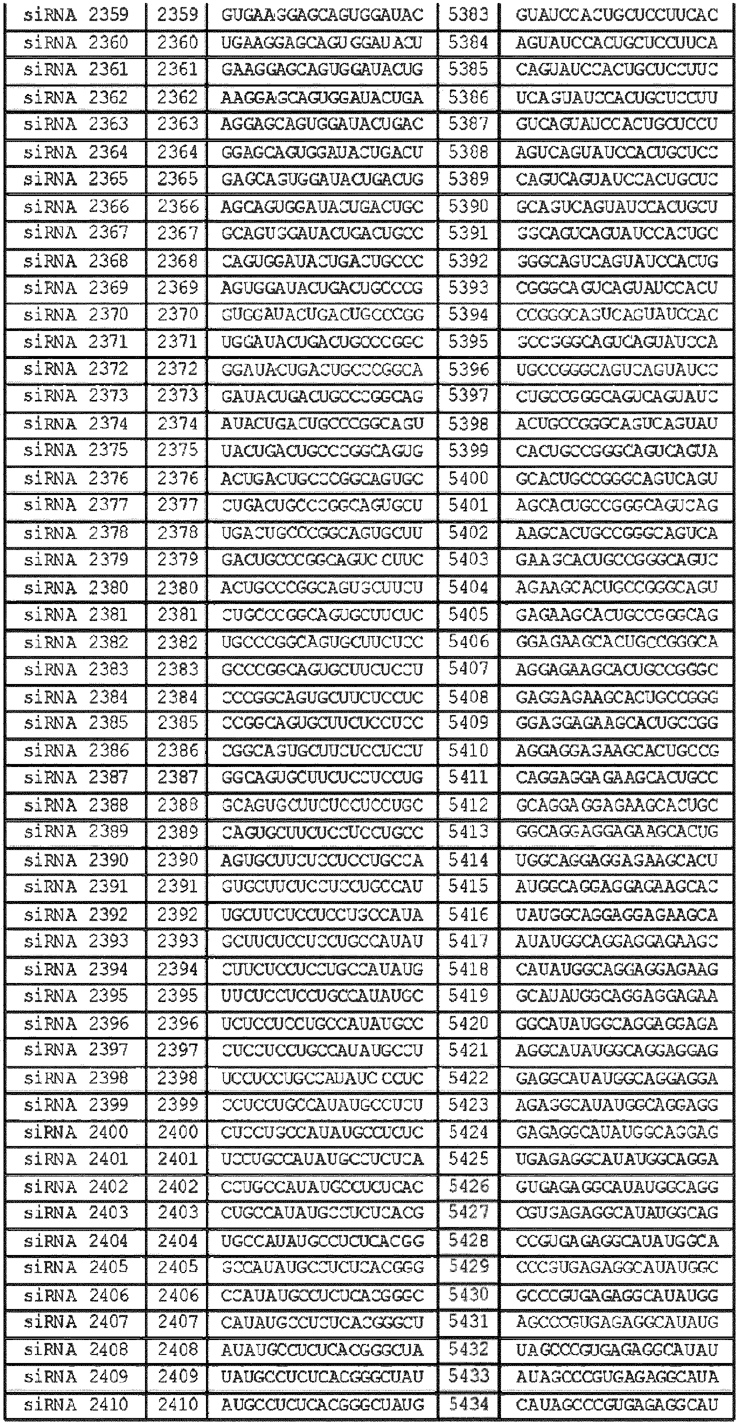

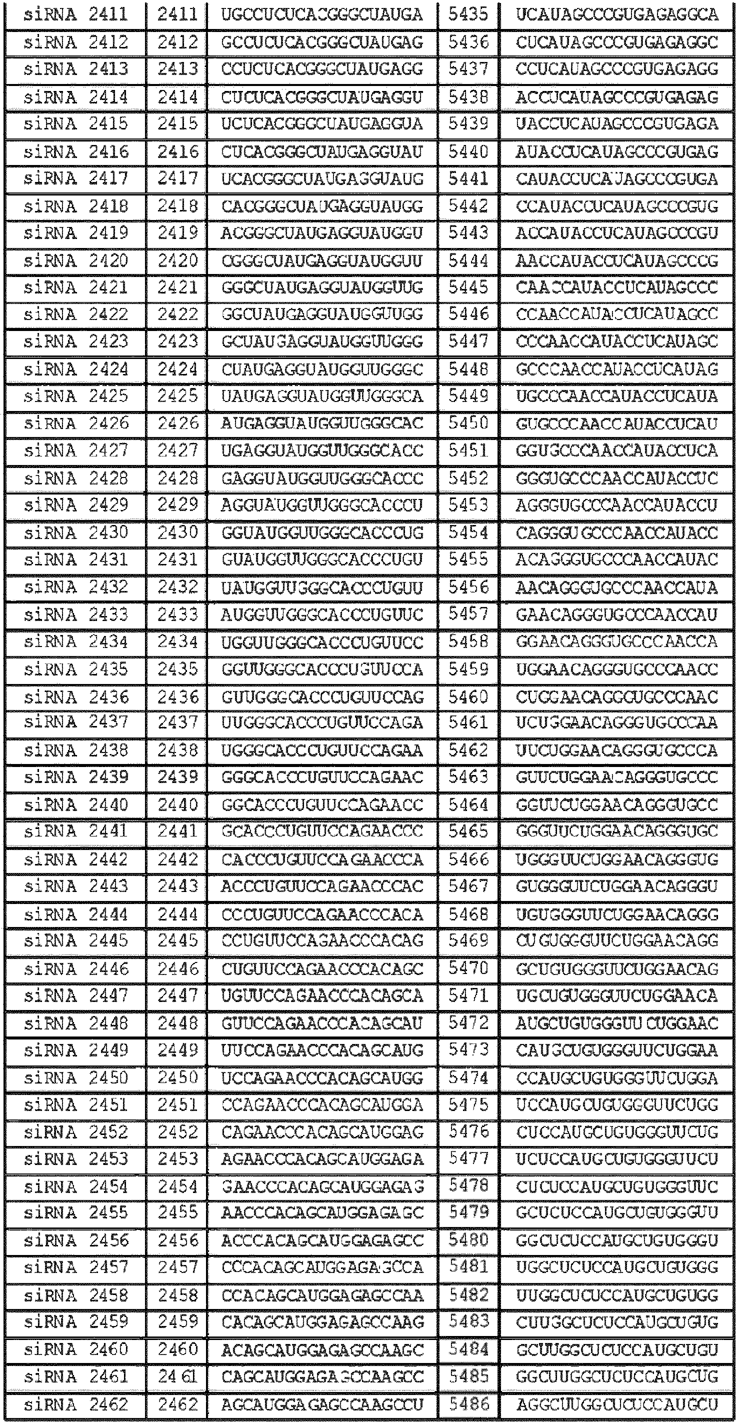

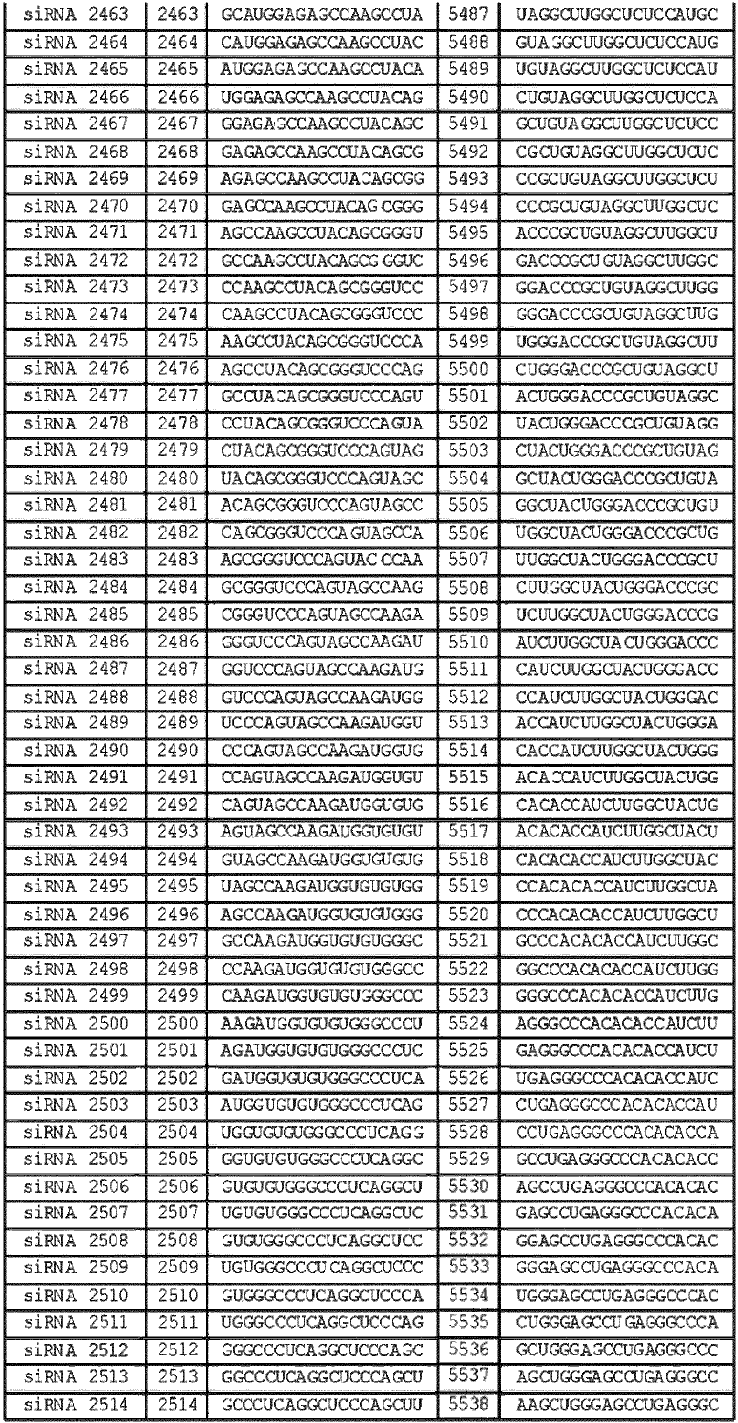

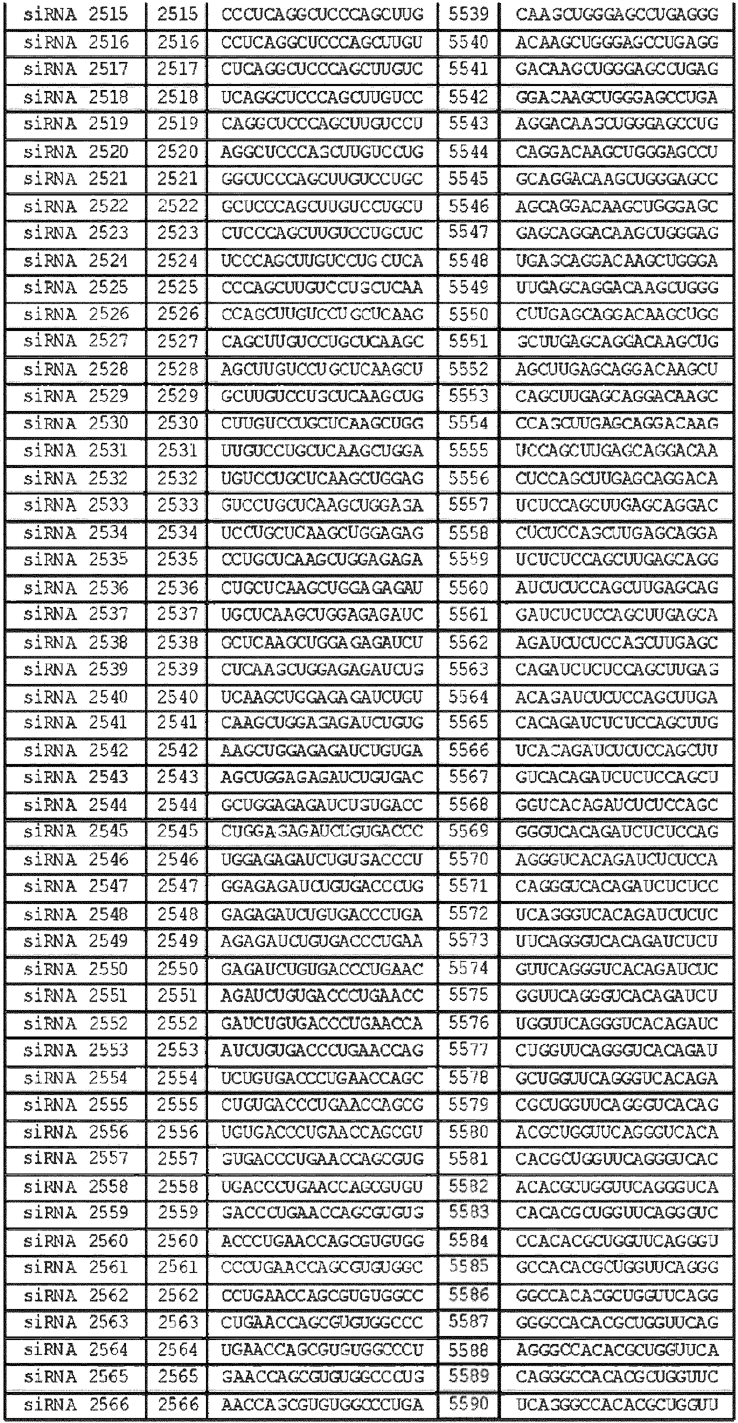

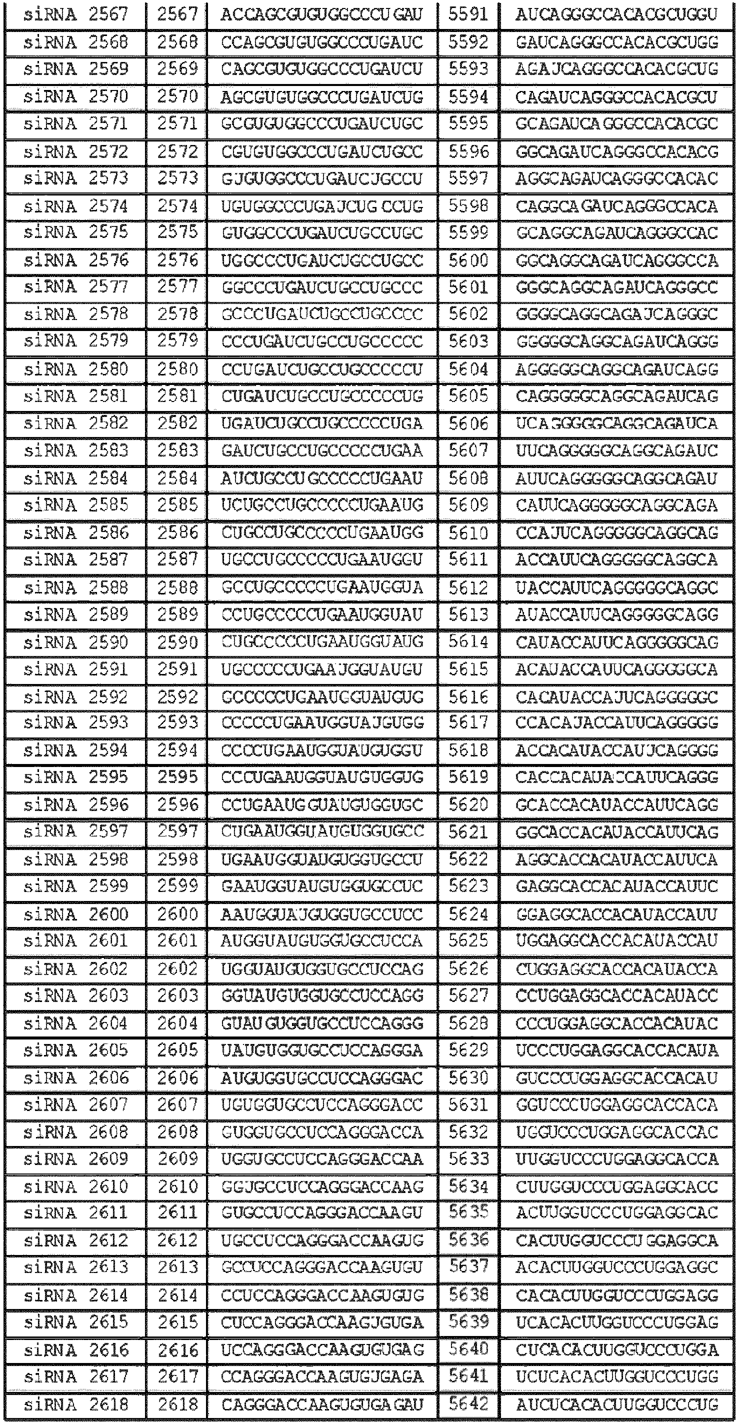

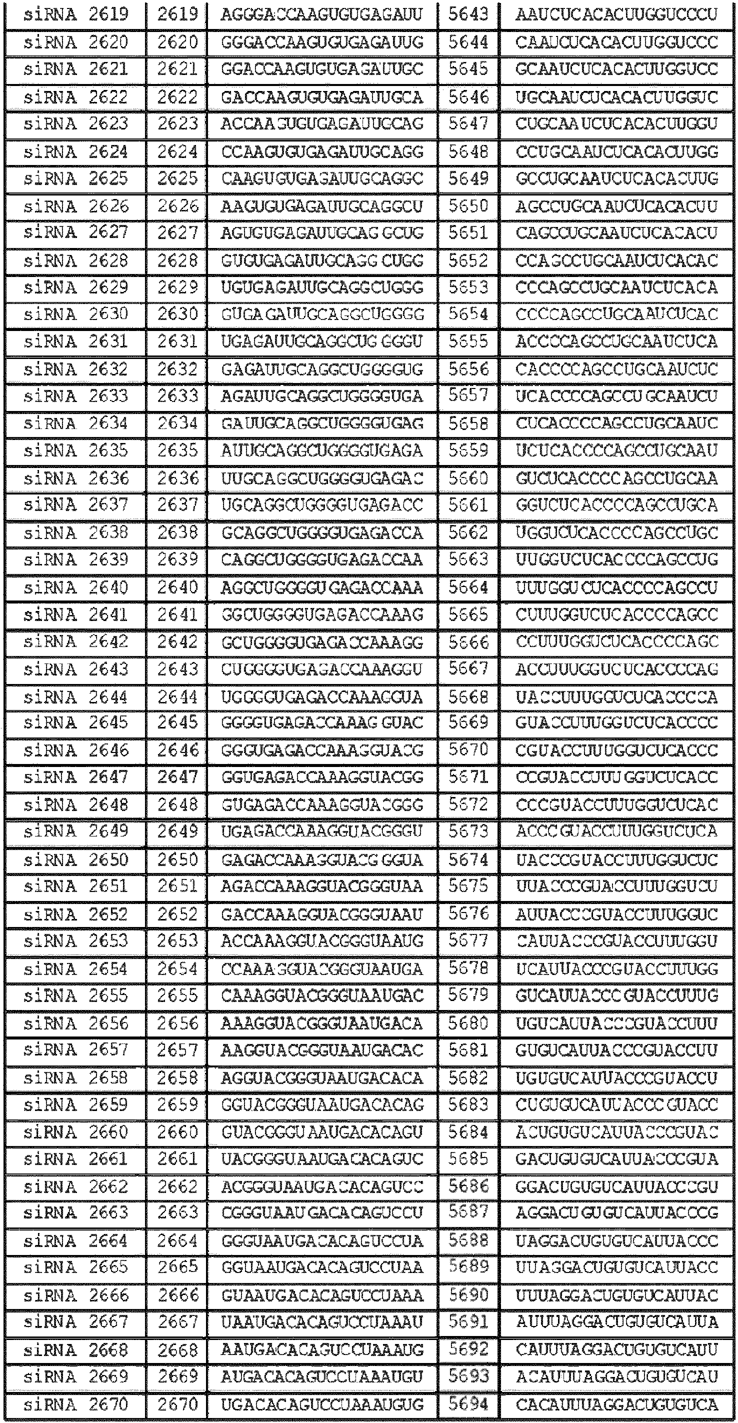

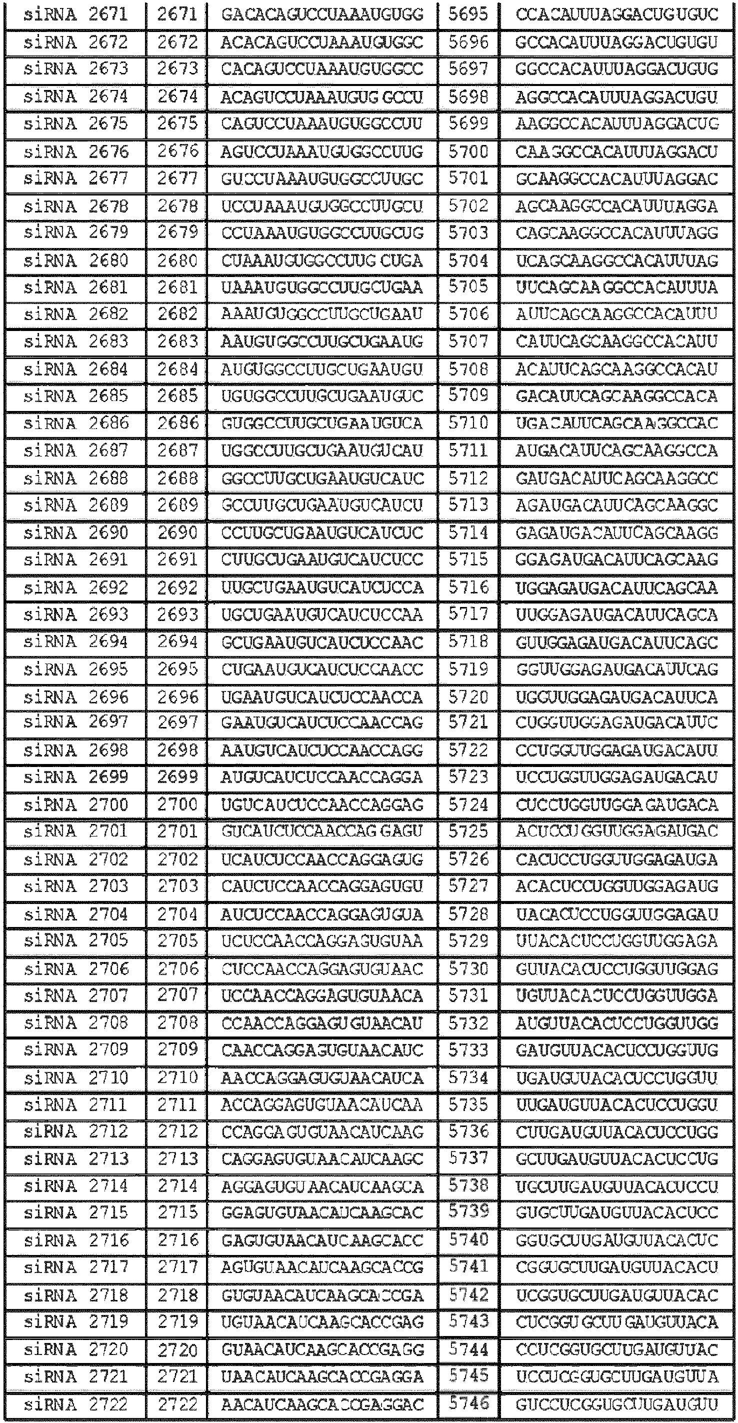

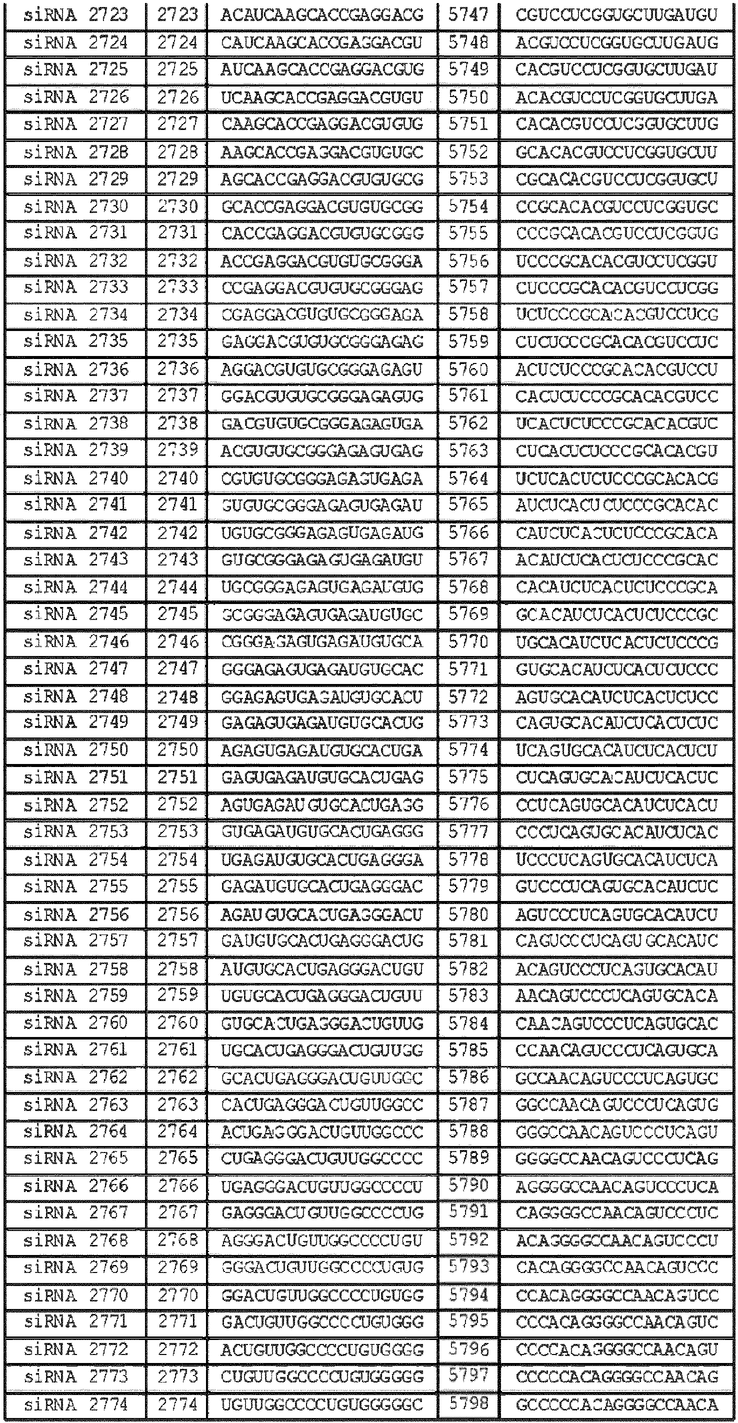

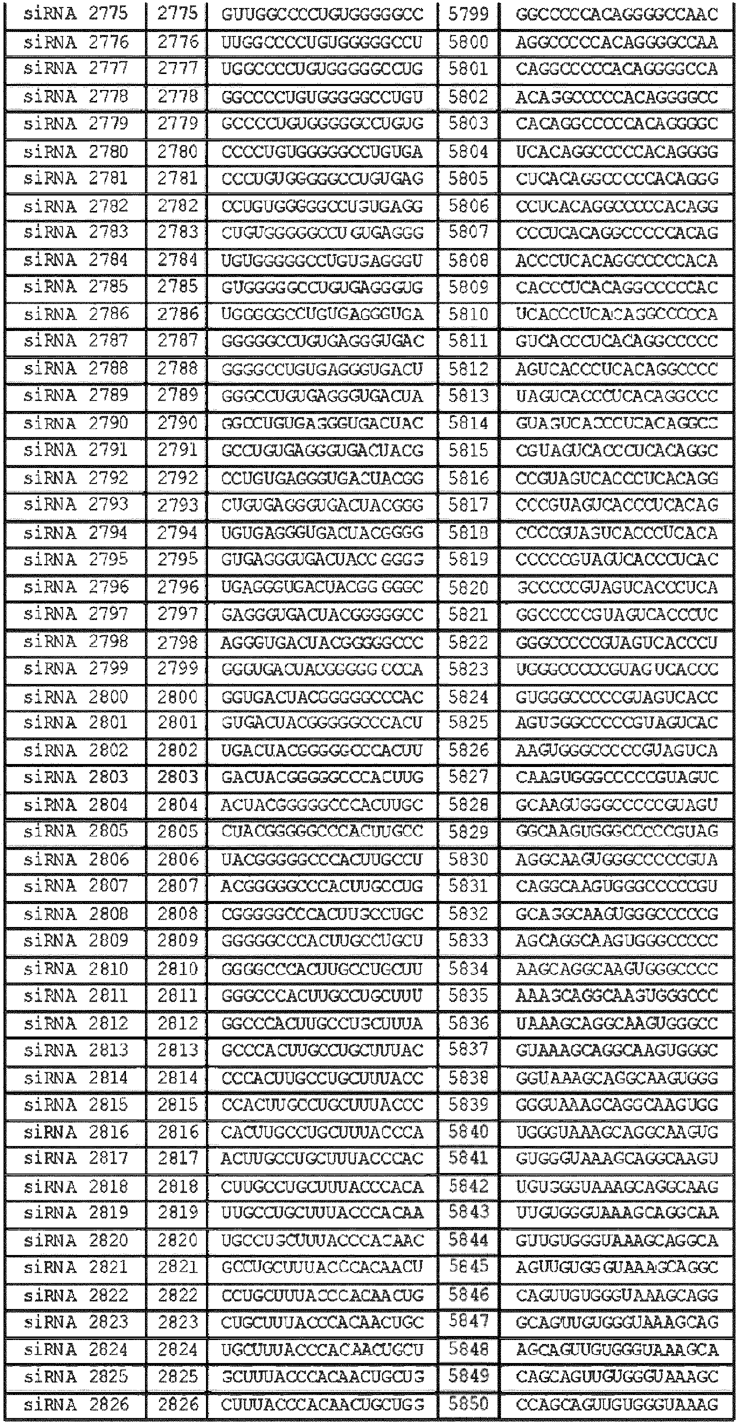

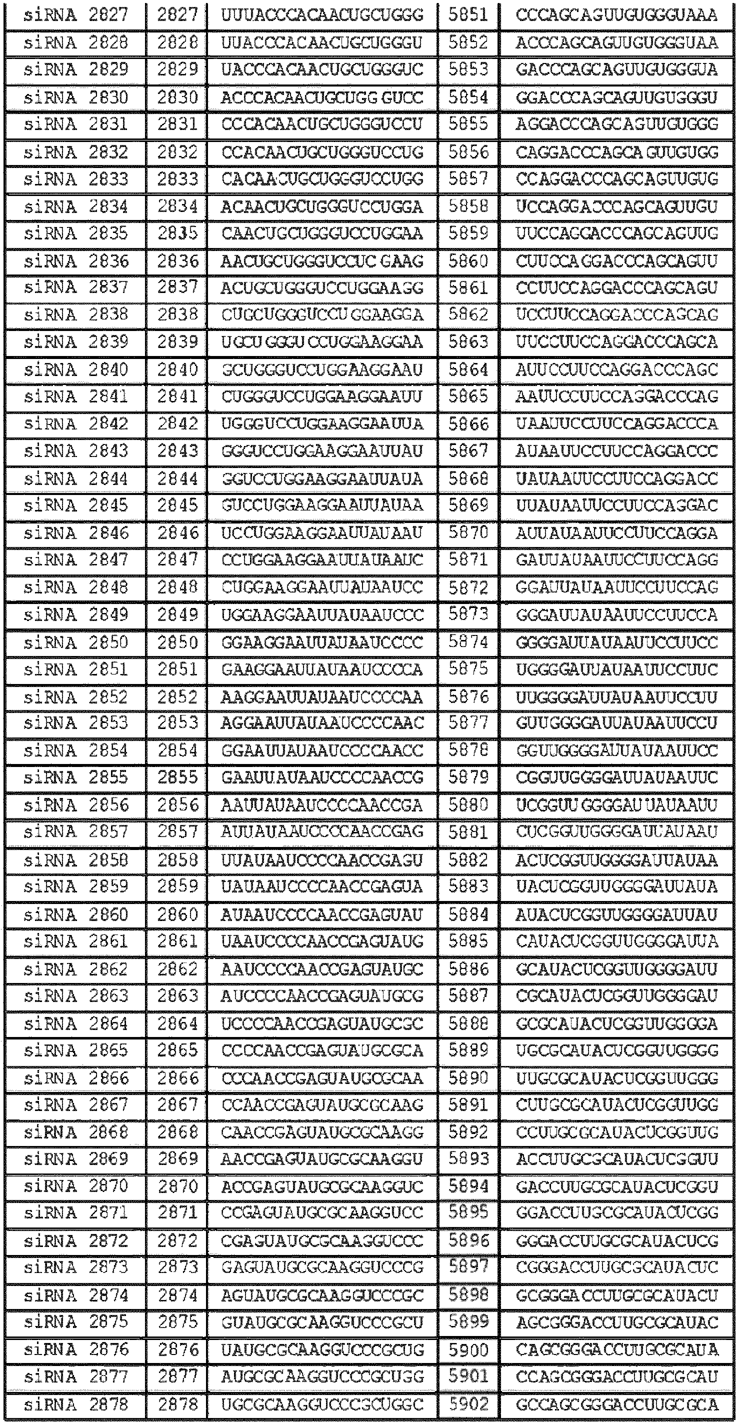

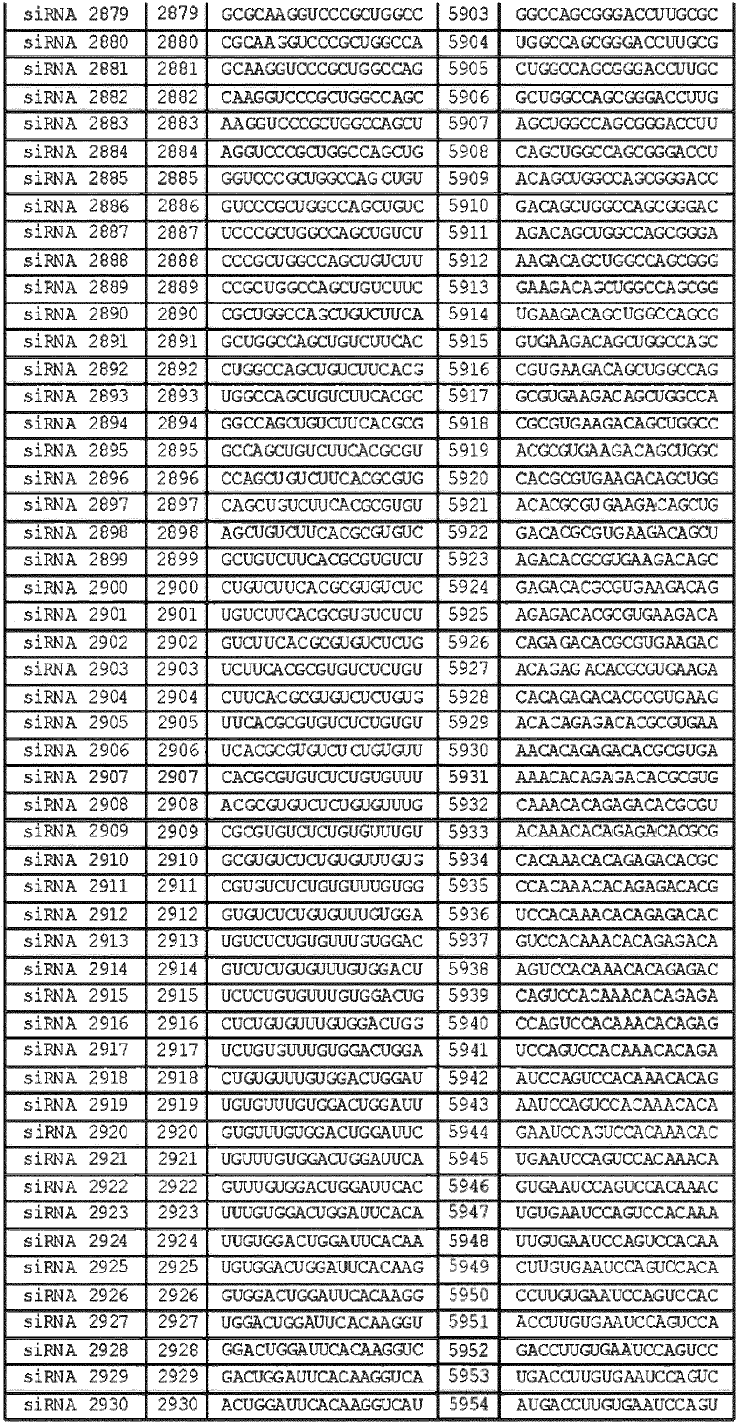

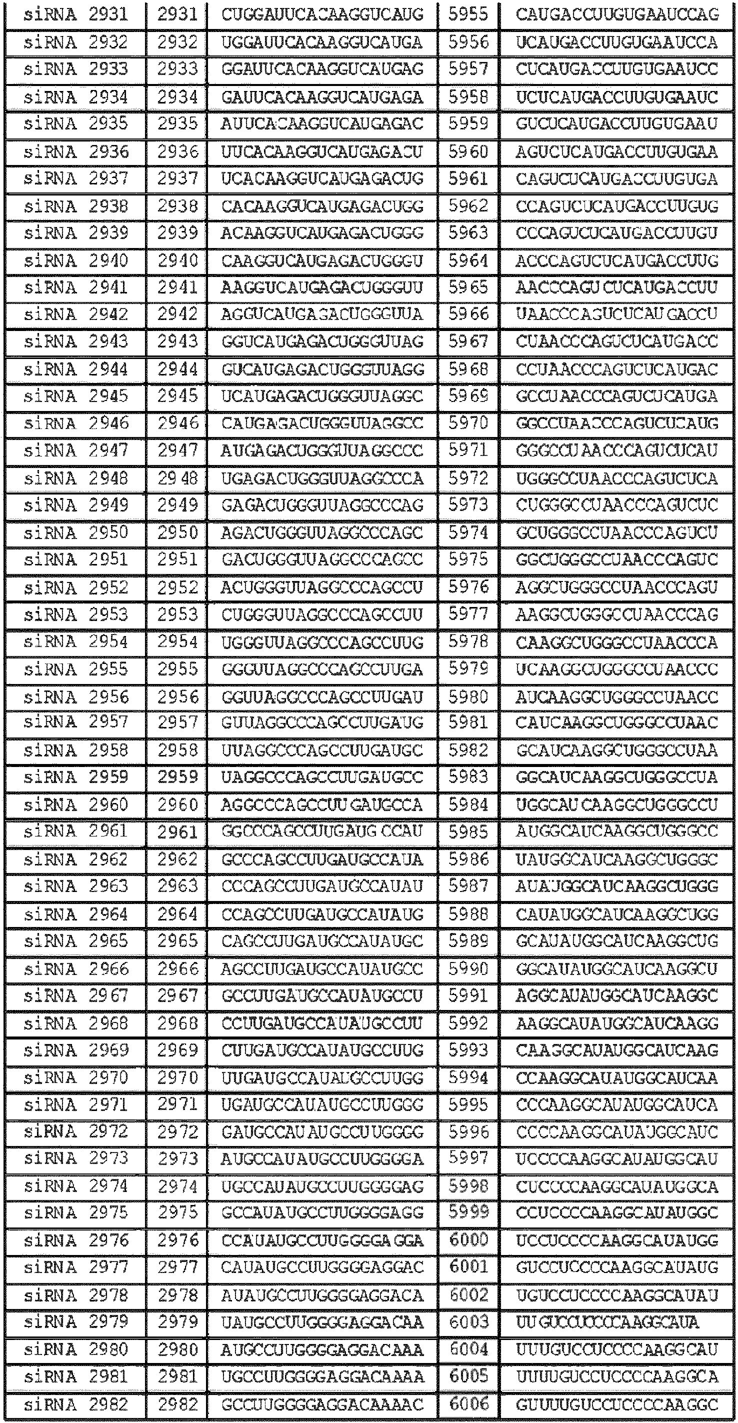

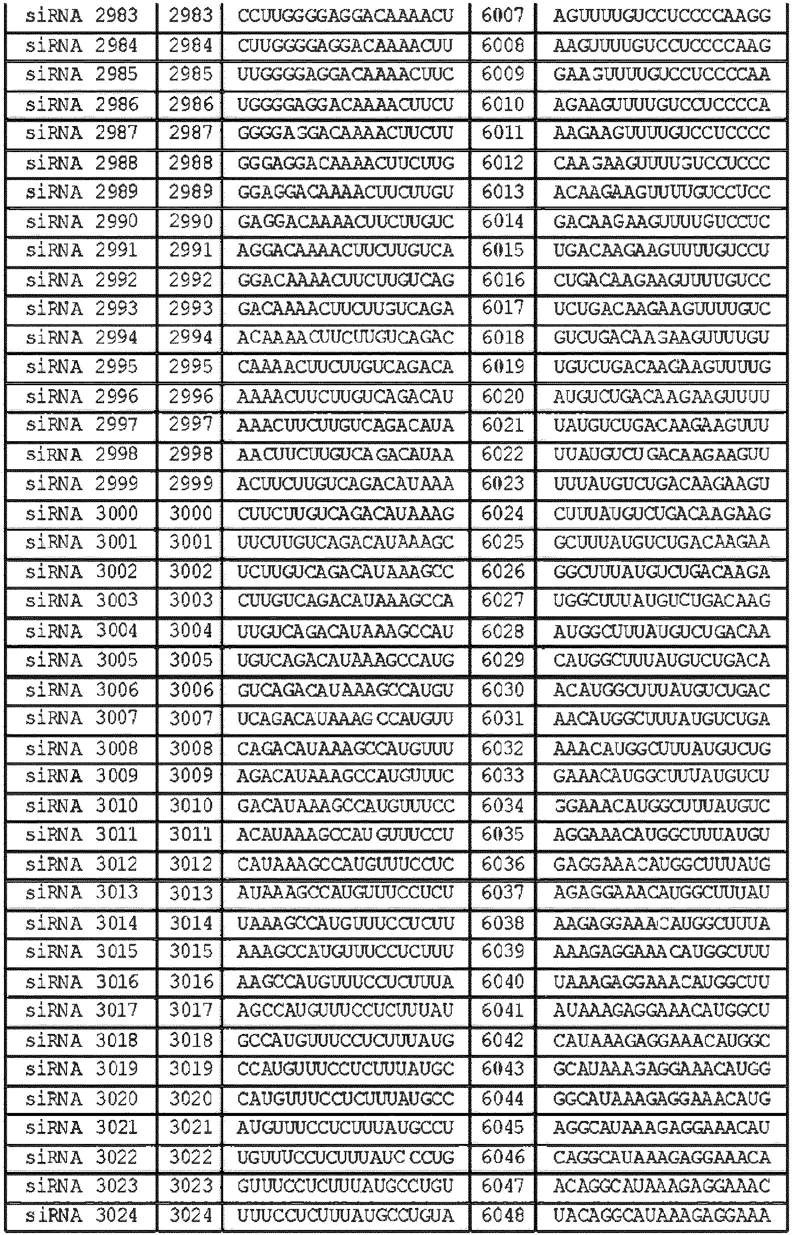

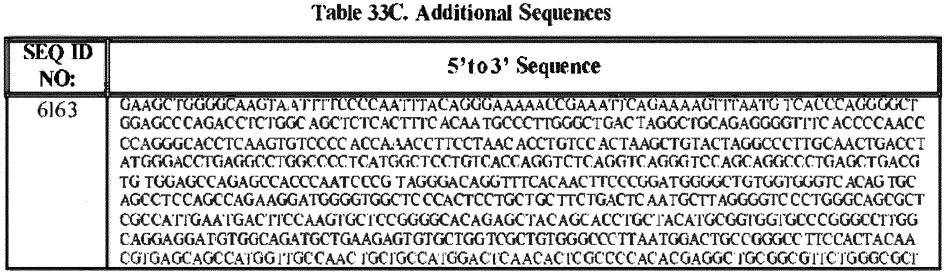

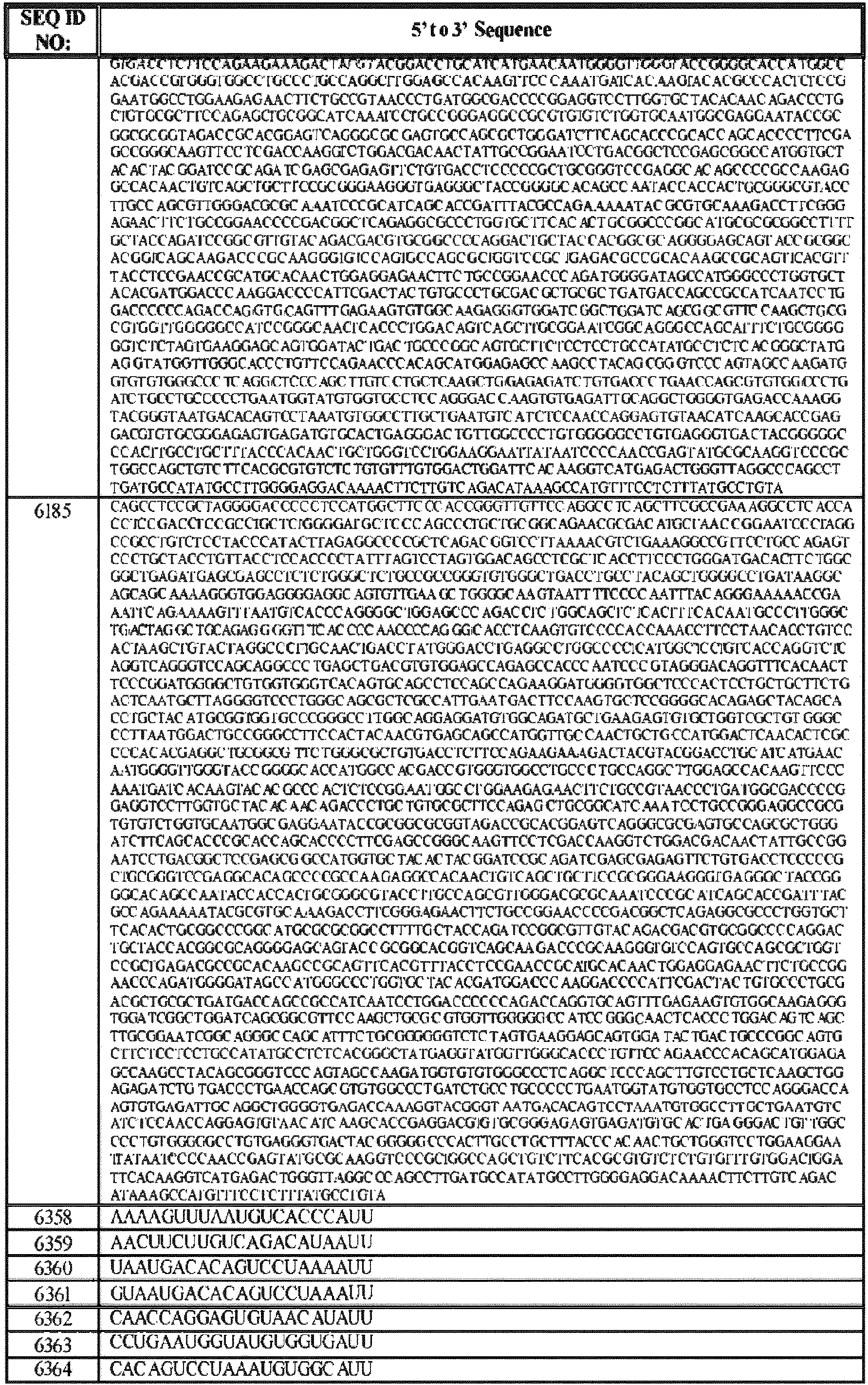

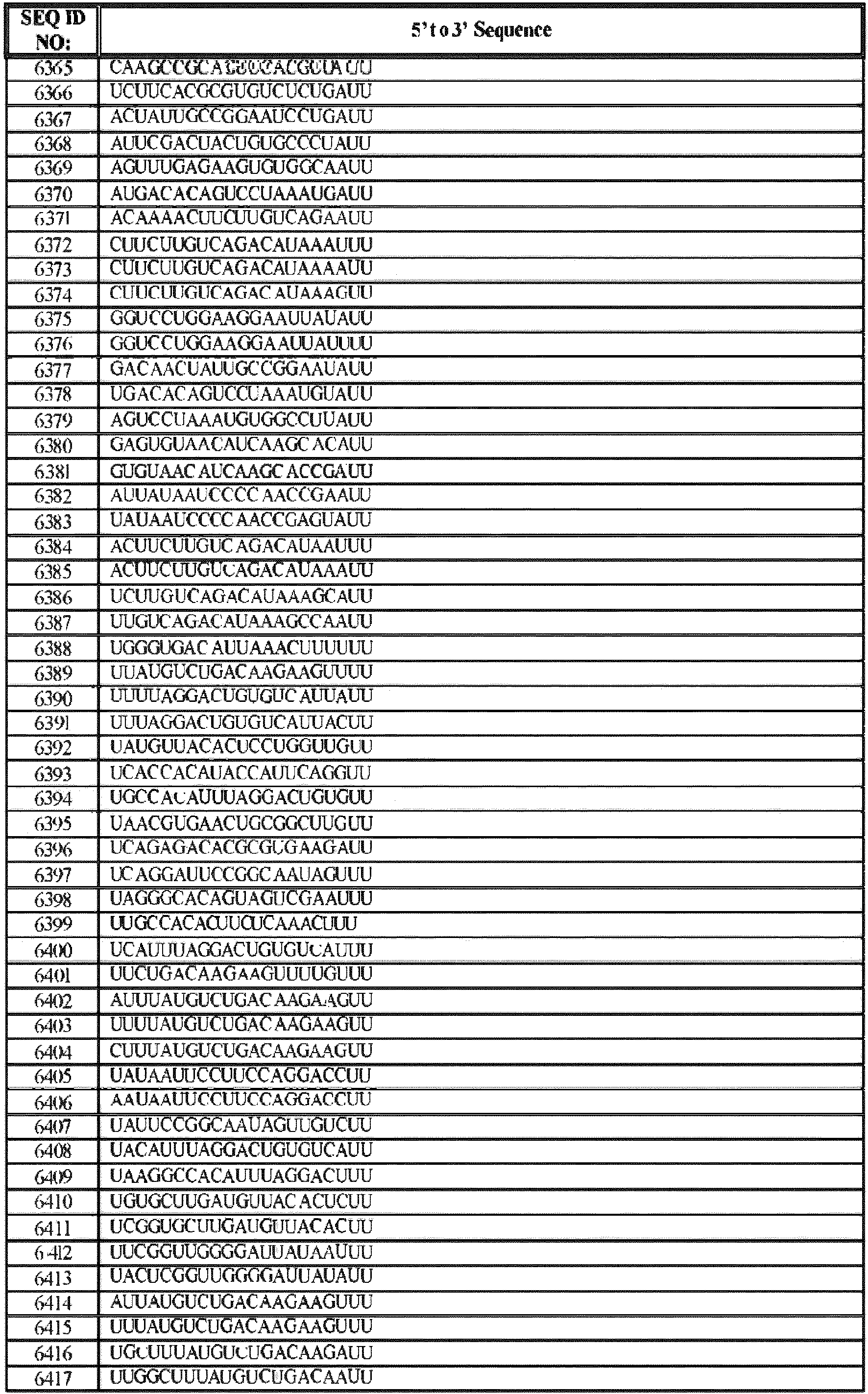

The disclosure may be further understood with reference to the following numbered clauses.
Clause 1. A composition comprising an oligonucleotide that targets *MST1* and when administered to a subject in an effective amount increases a lung function measurement.
Clause 2. The composition of clause 1, wherein the lung function measurement comprises a forced expiratory volume in 1 second (FEV 1) measurement, a forced expiratory volume in 1 second percent predicted (FEV I pp) measurement, a forced vital capacity (FVC) measurement, a FEV1/FVC ratio measurement, a forced expiratory volume, or a peak expiratory flow measurement.
Clause 3. The composition of clause 1, wherein the lung function measurement is increased by about 10% or more, as compared to prior to administration.
Clause 4. Acomposition comprising an oligonucleotide that targets *MST1* and when administered to a subject in an effective amount decreases a leukocyte measurement.
Clause 5. The composition of **clause** 4, wherei clauseukocyte measurement comprises a lung leukocyte measurement.
Clause 6. The composition of **clause** 4, wherein the leukocyte measurement comprises a circulating leukocyte measurement.
Clause 7. The composition of clause 4, wherein the leukocyte measurement comprises a neutrophil measurement, eosinophil measurement, basophil measurement, monocyte measurement, or lymphocyte measurement, or a combination thereof.
Clause 8. The composition of **clause** 4, wherein the leukocyte measurement is decreased by about 10%or more, as compared to prior to administration.
Clause 9 . A composition comprising an oligonucleotide that targets *MST1* and when administered to a subject in an effective amount decreases a chronic obstructive pulmonary disease (COPD) or asthma exacerbation measurement,
Clause 10. The composition of **clause 9** wherein the COPD or asthma exacerbation measurement is decreased by about 10% or more, as compared to prior to administration.
Clause 11. The composition of any one of claims 1, 4 or 9, wherein the oligonucleotide comprises a modified internucleoside linkage.
Clause12. The composition of clause 11, wherein the modified internucleoside linkage comprises alk ylphosphonate, phosphorothioate, methylphosphonate, phosphorodithioate, alkylphosphonothioate, phosphoramidate, carbamate, carbonate, phosphate triester, acetamidate, or carbox ymethyl ester, or a combination thereof.
Clause 13. The composition of clause 11, wherein the modified internucleoside linkage comprises one or more phosphorothioate linkages.
Clause 14. The composition of any one of clauses 1, 4 or 9, wherein the oligonucleotide comprises 1, 2 3,4, 5,6, 7, 8,9, 10, 11, 12, 13, 14, 15, 16, 17, 18, 19, or 20 modified internucleoside linkages.
Clause 15. The composition of any one of clauses 1, 4 or 9, wherein the oligonucleotide comprises a modified nucleoside.
Clause 16. The composition of clause 15, wherein the modified nucleoside comprises a locked nucleic acid (LNA), hexitol nucleic acid (HLA), cyclohexene nucleic acid (CeNA), 2'-methoxyethyl, 2'-O-alkyl, 2'-O-allyl, 2'-O-allyl, 2'-fluoro, or 2'-deoxy, or a combination thereof.
Clause 17. The composition of **clause** 15, wherein the modified nucleoside comprises a LNA.
Clause 18. The composition of clause 15, wherein the modified nucleoside comprises a2',4' constrained ethyl nucleic acid.
Clause 19. The composition of clause 15, wherein the modified nucleoside comprises a 2'-O-methyl nucleoside, 2'-deoxyfluoro nucleoside, 2'-O-N-methylacetamido (2'-O-NMA) nucleoside, a 2'-O-dimethylaminoethoxyethyl (2'-O-DMAEOE) nucleoside, 2'-O-aminopropyl (2'-O-AP) nucleoside, or 2'-ara-F, or a combination thereof.
Clause 20. The composition of **clause** 15, wherein the modified nucleoside comprises one or more 2' fluoro modified nucleosides.
Clause 21. The composition of clause 15, wherein the modified nucleoside comprises a 2' O-alkyl modified nucleoside.
Clause 22. The composition of any one of clauses 1, 4 or 9, wherein the oligonucleotide comprises 1, 2, 3, 4, 5, 6, 7, 8, 9, 10, 11, 12, 13, 14, 15, 16, 17, 18, 19, 20, or 21 modified nucleosides.
Clause 23. The composition of any one of **clauses** 1, 4 or 9, wherein the oligonucleotide comprises a lipid attached at a3' or 5' terminus of the oligonucleotide.
Clause 24. The composition of clause 23, wherein the lipid comprises cholesterol, myristoyl, palmitoyl, stearoyl, lithocholoyl, docosanoyl, docosahexaenoyl, myristyl, palmityl stearyl, or α-tocopherol, or a combination thereof.
Clause 25. The composition of any one of clauses 1, 4 or 9, wherein the oligonucleotide comprises a sugar moiety attached a a 3' or 5' terminus of the oligonucleotide.
Clause 26. The composition of clause 25, wherein the sugar comprises N-acety lgalactosamine (GalNAc), N-acetylglucosamine (GlcNAc), or mannose.
Clause 27. The composition of any one of clauses 1, 4 or 9, wherein the oligonucleotide comprises an integrin targeting ligand attached at a 3' or 5' terminus of the oligonucleotide.
Clause 28. The composition of clause 27, wherein the integrin comprises integrin alpha-v-beta-6.
Clause 29. The composition of clause 27, wherein the integrin targeting ligand comprises an arginineglycine-aspartic acid (RGD) peptide.
Clause 30. The composition of any one of **clauses 1, 4** or9, wherein the oligonucleotide comprises a small interfering RNA (si RNA) comprising a sense strand and an antisense strand.
Clause 31. The composition of clause 30, wherein the sense strand is 12-30 nucleosides in length.
Clause 32. The composition of clause 30, wherein the antisense strand is 12-30 nucleosides in length.
Clause 33. A composition comprising an oligonucleotide that inhibits the expression of *MST1,* wherein theoligonucleotide comprises an siRNA comprising a sense strand and an antisense strand, each strand is independently about 12-30 nucleosides in length, and at least one of the sense strand and the antisense strand comprises a nucleoside sequence comprising about 12-30 contiguous nucleosides of SEQ ID NO: 6185.
Clause 34. The composition of any one of clauses 1, 4, 9 or 33, wherein any one of the following is true with regard to the sense strand.
   all purines comprise 2' fluoro modified purines, and all pyrimidines comprise a mixture of 2' fluoro and 2' methyl modified pyrimidines;
   all purines comprise 2' methyl modified purines, and all pyrimidines comprise a mixture of 2' fluoro and 2' methyl modified pyrimidines;
   all purines comprise 2' fluoro modified purines, and all pyrimidines comprise2' methyl modified pyrimidines;
   all pyrimidines comprise 2' fluoro modified pyrimidines, and all purines comprise a mixture of 2' fluoro and 2' methyl modified purines;
   all pyrimidines comprise 2' methyl modified pyrimidines, and all purines comprise a mixture of 2' fluoro and 2' methyl modified purines; or
   all pyrimidines comprise 2' fluoro modified pyrimidines, and all purines comprise 2' methyl modified purines.
Clause 35. The composition of clause 30, wherein any one of the following is true with regard to the antisense strand:
   all purines comprise 2' fluoro modified purines, and all pyrimidines comprise a mixture of 2' fluoro and 2' methyl modified pyrimidines:
   all purines comprise 2' methyl modified purines, and all pyrimidines comprise a mixture of 2' fluoro and 2' methyl modified pyrimidines;
   all purines comprise 2' methyl modified purines, and all pyrimidines comprise 2' fluoro modified pyrimidines;
   all pyrimidines comprise 2' fluoro modified pyrimidines, and all purines comprise a mixture of 2' fluoro and 2' methyl modified purines;
   all pyrimidines comprise 2' methyl modified pyrimidines, and all purines comprise a mixture of 2' fluoro and 2' methyl modified purines; or
   all pyrimidines comprise 2' methyl modified pyrimidines, and all purines comprise2' fluoro modified purines.
Clause 36. The composition of any one of clauses 1, 4, or 9, wherein the oligonucleotide comprises an antisense oligonucleotide (ASO).
Clause 37. The composition of clause 36, wherein theASO is 12-30 nucleosides in length.
Clause 38. A composition comprising an oligonucleotide that inhibits the expression of *MST1*, wherein the oligonucleotide comprises an ASO about 12-30 nucleosides in length and a nucleoside sequence complementary to about 12-30 contiguous nucleosides of SEQ ID NO: 6185.
Clause 39. The composition of any one of clauses 1, 4, 9, 33 or 38, further comprising a pharmaceutically acceptable carrier.
Clause 40. A method of treating a subject having a lung disorder, comprising administering an effective amount of the composition of clause 39 to the subject.
Clause 41. The method of clause 40, wherein the lung disorder comprises COPD. acute exacerbation of COPD, emphysema, chronic bronchitis, asthma, status asthmaticus, asthma-COPD overlap syndrome (ACOS), cough, lung cancer, interstitial lung disease, or pulmonary fibrosis.

## Claims

1. A composition comprising an oligonucleotide that inhibits the expression of MST1 (macrophage-stimulating 1), wherein the oligonucleotide comprises an siRNA comprising a sense strand and an antisense strand,
wherein each strand is independently 12-30 nucleosides in length, at least one of the sense strand and the antisense strand comprises a nucleoside sequence comprising 12-30 contiguous nucleosides of SEQ ID NO: 6185; and
(a) the sense strand comprises modification pattern 30S (5'-snnnnnnNfnNfNfnnnnnnnnnsnsn-3'; SEQ ID NO:6340);
(b) the antisense strand comprises modification pattern 15AS (5'-nsNfsnnnnNfnnNfnNfnNfnNfnNfnsnsn-3'); or
(c) both (a) and (b),
wherein
n is 2'-O-methyl (2'-OMe) A, G, C, and U, respectively;
Nf is 2'-fluoro (2'-F) A, G, C, and U, respectively; and
s is a phosphorothioate linkage.

2. The composition of claim 1, wherein the sense strand comprises an oligonucleotide sequence of SEQ ID NO: 2999 and the antisense strand comprises an oligonucleotide sequence of SEQ ID NO: 6023.

3. The composition of claim 1 or 2, wherein the sense strand comprises an oligonucleotide sequence of SEQ ID NO: 6385.

4. The composition of claim 1 or 3, wherein the antisense strand comprises an oligonucleotide sequence of SEQ ID NO: 6415.

5. The composition of any one of claims 1-4, wherein the modified oligonucleotide is conjugated to a sugar moiety.

6. The composition of claim 5, wherein the sugar moiety comprises N-acetylgalactosamine (GalNAc), N-acetylglucosamine (GlcNAc), or mannose.

7. The composition of claim 5 or 6, wherein the sugar moiety is conjugated to the 5' or 3' terminus of the sense strand or antisense strand.

8. The composition of claim 7, wherein the sugar moiety is conjugated to the 5' terminus of the sense strand.

9. The composition of any one of claims 5-8, wherein the sugar moiety comprises the structure: 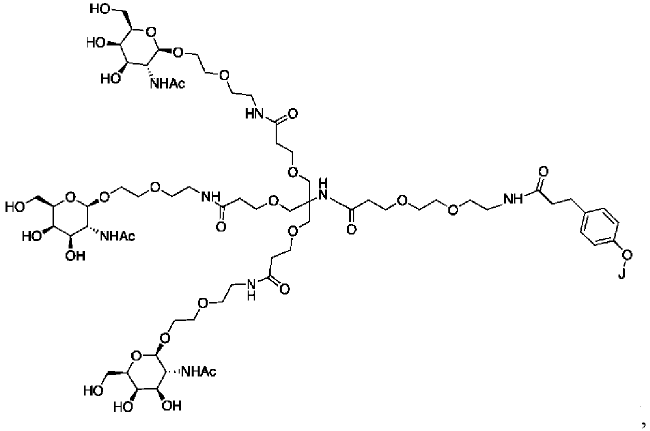 wherein J is the attachment point to the 5' phosphorothioate linkage of the sense strand.

10. The composition of any one of claims 1-9, wherein the sense strand comprises an oligonucleotide sequence of SEQ ID NO: 6229 and the antisense strand comprises an oligonucleotide sequence of SEQ ID NO: 6288.

11. A pharmaceutical composition comprising the composition of any one of claims 1-10 and a pharmaceutically acceptable carrier.

12. A composition of any one of claims 1-11, for use in a method of treating a subject having a lung disorder.

13. The composition for use of claim 12, wherein the lung disorder comprises COPD, acute exacerbation of COPD, emphysema, chronic bronchitis, asthma, status asthmaticus, asthma-COPD overlap syndrome (ACOS), cough, dyspnea, lung cancer, interstitial lung disease, or pulmonary fibrosis.

14. The composition for use of claim 13, wherein the lung disorder comprises COPD, acute exacerbation of COPD, or asthma-COPD overlap syndrome (ACOS).

15. The composition for use of any one of claims 12-14, wherein treating comprises administration to the subject via injection.
